(19)

Europäisches
Patentamt
European
Patent Office
Office européen
des brevets

(11) **EP 3 502 140 A1**

(12) **EUROPEAN PATENT APPLICATION**

(43) Date of publication:
26.06.2019 Bulletin 2019/26

(21) Application number: 17209444.3

(22) Date of filing: 21.12.2017

(51) Int Cl.:
*C07K 16/30* (2006.01)          *C07K 16/28* (2006.01)
*C07K 16/40* (2006.01)          *A61K 39/00* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(71) Applicant: **F. Hoffmann-La Roche AG
4070 Basel (CH)**

(72) Inventor: **The designation of the inventor has not
yet been filed**

(74) Representative: **Klostermeyer-Rauber, Dörte
F. Hoffmann-La Roche AG
Corporate Law Patents (CLP)
Grenzacherstrasse 124
4070 Basel (CH)**

(54) **COMBINATION THERAPY OF TUMOR TARGETED ICOS AGONISTS WITH T-CELL BISPECIFIC
MOLECULES**

(57)     The present invention relates to agonistic
ICOS-binding molecules comprising at least one antigen
binding domain that binds to a tumor-associated antigen
and their use in combination with T-cell bispecific molecules in the treatment of cancer, the agonistic ICOS-bind-
ing molecules as such, pharmaceutical compositions
containing these molecules, and methods of using the
same.

(1C)

**Description**

**FIELD OF THE INVENTION**

The present invention relates to tumor-targeted agonistic ICOS-binding molecules and their use as immunomodulators in combination with T-cell bispecific molecules in the treatment of cancer.

**BACKGROUND**

Modulating immune inhibitory pathways has been a major recent breakthrough in cancer treatment. Checkpoint blockade antibodies targeting cytotoxic T-lymphocyte antigen 4 (CTLA-4, YERVOY/ipilimumab) and programmed cell-death protein 1 (PD-1, OPDIVO/nivolumab or KEYTRUDA/pembrolizumab), respective PD-L1 (atezolizumab) have demonstrated acceptable toxicity, promising clinical responses, durable disease control, and improved survival in patients of various tumor indications. However, only a minority of patients experience durable responses to immune checkpoint blockade (ICB) therapy, the remainder of patients show primary or secondary resistance, demonstrating a clear need for regulating additional pathways to provide survival benefit for greater numbers of patients. Thus, combination strategies are needed to improve therapeutic benefit.

ICOS (CD278) is an inducible T-cell co-stimulator and belongs to the B7/CD28/CTLA-4 immunoglobulin superfamily (Hutloff, et al., Nature 1999, 397). Its expression seems to be restricted mainly to T cells with only weak expression on NK cells (Ogasawara et al., J Immunol. 2002, 169 and unpublished own data using human NK cells). Unlike CD28, which is constitutively expressed on T cells, ICOS is hardly expressed on naive $T_H1$ and $T_H2$ effector T cell populations (Paulos CM et al., Sci Transl Med 2010, 2), but on resting $T_H17$, T follicular helper ($T_{FH}$) and regulatory T (Treg) cells. However, ICOS is strongly induced on all T cell subsets upon previous antigen priming, respective TCR/CD3-engagement (Wakamatsu et al., Proc Natal Acad Sci USA, 2013, 110).

Signaling through the ICOS pathway occurs upon binding of its ligand, the so-called ICOS-L (B7h, B7RP-1, CD275), which is expressed on B cells, macrophages, dendritic cells, and on non-immune cells treated with TNF-$\alpha$ (Simpson et al., Current Opinion in Immunology 2010, 22). Neither B7-1 nor B7-2, the ligands for CD28 and CTLA4, are able to bind or activate ICOS. Nonetheless, ICOS-L has been shown to bind weakly to both CD28 and CTLA-4 (Yao et al., Immunity 2011, 34). Upon activation, ICOS, a disulfide-linked homodimer, induces a signal through the PI3K and AKT pathways. In contrast to CD28, ICOS has a unique YMFM SH2 binding motif, which recruits a PI3K variant with elevated lipid kinase activity compared to the isoform recruited by CD28. As a consequence, greater production of Phosphatidylinositol (3, 4, 5)-triphosphate and concomitant increase in AKT signaling can be observed, suggesting an important role of ICOS in T cell survival (Simpson et al., Current Opinion in Immunology 2010, 22).

As reviewed by Sharpe (Immunol Rev., 2009, 229), the ICOS/ICOS ligand pathway has critical roles in stimulating effector T-cell responses, T-dependent B-cell responses, and regulating T-cell tolerance by controlling IL-10 producing Tregs. Moreover, ICOS is important for generation of chemokine (C-X-C motif) receptor 5 (CXCR5)[+] follicular helper T cells ($T_{FH}$), a unique T-cell subset that regulates germinal center reactions and humoral immunity. Recent studies in ICOS-deficient mice indicate that ICOS can regulate interleukin-21 (IL-21) production, which in turn regulates the expansion of T helper (Th) type 17 ($T_H17$) cells and $T_{FH}$. In this context, ICOS is described to bipolarize CD4 T cells towards a $T_H1$-like $T_H17$ phenotype, which has been shown to correlate with improved survival of patients in several cancer indications, including melanoma, early stage ovarian cancer and more (Rita Young, J Clin Cell Immunol. 2016, 7).

ICOS-deficient mice show impaired germinal center formation and have decreased production of IL-10 and IL-17, which become manifest in an impaired development of autoimmunity phenotypes in various disease models, such as diabetes ($T_H1$), airway inflammation ($T_H2$) and EAE neuro-inflammatory models ($T_H17$) (Warnatz et al, Blood 2006). In line with this, human common variable immunodeficiency patients with mutated ICOS show profound hypogammaglobulinemia and a disturbed B-cell homeostasis (Sharpe, Immunol Rev., 2009, 229). Important to note, that efficient co-stimulatory signaling via ICOS receptor only occurs in T cells receiving a concurrent TCR activation signal (Wakamatsu et al., Proc Natal Acad Sci USA, 2013, 110).

T-cell bispecific (TCB) molecules are appealing immune cell engagers, since they bypass the need for recognition of MHCI-peptide by corresponding T-cell receptors, but enable a polyclonal T-cell response to cell-surface tumor-associated antigens (Yuraszeck et al., Clinical Pharmacology & Therapeutics 2017, 101). CEA CD3 TCB, an anti-CEA/anti-CD3 bispecific antibody, is an investigational, immunoglobulin G1 (IgG1) T-cell bispecific antibody to engage the immune system against cancer. It is designed to redirect T cells to tumor cells by simultaneous binding to human CD3$\epsilon$ on T cells and carcinoembryonic antigen (CEA), expressed by various cancer cells, including CRC (colorectal cancer), GC (gastric cancer), NSCLC (non-small-cell lung cancer) and BC (breast cancer). The cross-linking of T- and tumor cells, leads to CD3/TCR downstream signaling and to the formation of immunologic synapses, T-cell activation, secretion of cytotoxic granules and other cytokines and ultimately to a dose- and time-dependent lysis of tumor cells. Furthermore, CEA CD3 TCB is proposed to increase T-cell infiltration and generate a highly inflamed tumor microenvironment, making it an ideal combination partner for immune checkpoint blockade therapy (ICB), especially for tumors showing primary resistance to ICB because of the lack of sufficient endogenous adaptive and functional immune infiltrate. However, turning-off the brakes by blocking single or multiple inhibitory pathways on T cells might not be sufficient, given the paradoxical expression of several co-stimulatory receptors, such as 4-1BB (CD137), ICOS and OX40 on dysfunctional T cells in the tumor microenvironment (TME).

For ICOS, a growing body of literature actually supports the idea that engaging CD278 on CD4[+] and CD8[+] effector T cells has anti-tumor potential. Activating the ICOS-ICOS-L signaling has induced effective anti-tumor responses in several syngeneic mouse models both as monotherapy, as well in the context of anti-CLTA4 treatment, where activation of ICOS downstream signaling increased the efficacy of anti-CTLA4 therapy significantly (Fu T et al., Cancer Res, 2011, 71 and Allison et al., WO2011/041613 A2, 2009). Emerging data from patients treated with anti-CTLA4 antibodies also point to a correlation of sustained elevated levels of ICOS expression on CD4 and CD8 T cells and improved overall survival of tumor patients, e.g. with metastatic melanoma, urothelial, breast or prostate cancer (Giacomo et al., Cancer Immunol Immunother. 2013, 62; Carthon et al., Clin Cancer Res. 2010, 16; Vonderheide et al., Clin Cancer Res. 2010, 16; Liakou et al, Proc Natl Acad Sci USA 2008, 105 and Vonderheide et al., Clin Cancer Res. 2010, 16). Therefore, ICOS positive T effector cells are seen as a positive predictive biomarker of ipilimumab response.

It has been found that a better anti-tumor effect is achieved when an anti-CEA/anti-CD3 bispecific antibody, i.e. a CEA TCB, is combined with a tumor-targeted agonistic ICOS-binding molecule. Given, that ICOS is expressed already at baseline in various tumor indications (Allison et al., 2009, WO2011/041613 A2) and activation of ICOS downstream signaling depends on a simultaneous CD3 trigger for full activity, the combination of a TCB molecule with a tumor-targeted ICOS molecule acts synergistically to induce strong and long-lasting anti-tumor responses. The T-cell bispecific antibody provides the initial TCR activating signalling to T cells, and then the combination with the tumor-targeted agonistic ICOS-binding molecule leads to a further boost of anti-tumor T cell immunity. Thus, we herein describe a novel combination therapy for tumors expressing CEA (CEA-positive cancer).

## SUMMARY OF THE INVENTION

The present invention relates to agonistic ICOS-binding molecules comprising at least one antigen binding domain that binds to a tumor-associated antigen and their use in combination with anti-CD3 bispecific antibodies, in particular to their use in a method for treating or delaying progression of cancer. It has been found that the combination therapy described herein is more effective in inducing early T-cell activation, T-cell proliferation, induction of T memory cell and ultimately inhibiting tumor growth and eliminating tumor cells than treatment with the anti-CD3 bispecific antibodies alone.

In one aspect, the invention provides an agonistic ICOS-binding molecule comprising at least one antigen binding domain that binds to a tumor-associated antigen for use in a method for treating or delaying progression of cancer, wherein the agonistic ICOS-binding molecule comprising at least one antigen binding domain that binds to a tumor-associated antigen is used in combination with a T-cell activating anti-CD3 bispecific antibody specific for a tumor-associated antigen. In one aspect, the T-cell activating anti-CD3 bispecific antibody specific for a tumor-associated antigen is an anti-CEA/anti-CD3 bispecific antibody.

In a further aspect, provided is an agonistic ICOS-binding molecule comprising at least one antigen binding domain that binds to a tumor-associated antigen for use in a method for treating or delaying progression of cancer, wherein the agonistic ICOS-binding molecule comprising at least one antigen binding domain that binds to a tumor-associated antigen and the T-cell activating anti-CD3 bispecific antibody are administered together in a single composition or administered separately in two or more different compositions. In one aspect, they are administered separately in two different compositions. In a particular aspect, the agonistic ICOS-binding molecule comprising at least one antigen binding domain that binds to a tumor-associated antigen acts synergistically with the T-cell activating anti-CD3 bispecific antibody.

In one aspect, there is provided an agonistic ICOS-binding molecule comprising at least one antigen binding domain that binds to a tumor-associated antigen for use in combination with a T-cell activating anti-CD3 bispecific antibody specific for a tumor-associated antigen in a method for treating or delaying progression of cancer, wherein the agonistic ICOS-binding molecule comprising at least one antigen binding domain that binds to a tumor-associated antigen comprises at least one ICOS-L or fragments thereof. In one aspect, the agonistic ICOS-binding molecule comprising at least one antigen binding domain that binds to a tumor-associated antigen comprises two ICOS-L or fragments thereof. In a particular aspect, the agonistic ICOS-binding molecule comprising at least one antigen binding domain that binds to a tumor-associated antigen comprises at least one ICOS-L comprising the amino acid sequence of SEQ ID NO:1 or SEQ ID NO:2.

In a further aspect, there is provided an agonistic ICOS-binding molecule comprising at least one antigen binding domain that binds to a tumor-associated antigen for use in combination with a T-cell activating anti-CD3 bispecific antibody specific for a tumor-associated antigen in a method for treating or delaying progression of cancer, wherein the agonistic ICOS-binding molecule comprises at least one antigen binding domain that is capable of agonistic binding to ICOS. In particular, the agonistic ICOS-binding molecule comprising at least one antigen binding domain that binds to a tumor-associated antigen comprises at least one antigen binding domain that is capable of agonistic binding to human ICOS comprising the amino acid sequence of SEQ ID NO:3.

In another aspect, the invention provides an agonistic ICOS-binding molecule comprising at least one antigen binding domain that binds to a tumor-associated antigen for use in combination with a T-cell activating anti-CD3 bispecific antibody specific for a tumor-associated antigen in a method for treating or delaying progression of cancer, wherein the agonistic ICOS-binding molecule comprising at least one antigen binding domain that binds to a tumor-associated antigen is an agonistic ICOS-binding molecule comprising at least one antigen binding domain that binds to anti-Fibroblast activation protein (FAP). In one aspect, the agonistic ICOS-binding molecule comprises at least one antigen binding domain capable of specific binding to FAP comprising (a) a heavy chain variable region ($V_H$FAP) comprising (i) CDR-H1 comprising the amino acid sequence of SEQ ID NO:4, (ii) CDR-H2 comprising the amino acid sequence of SEQ ID NO:5, and (iii) CDR-H3 comprising the amino acid sequence of SEQ ID NO:6, and a light chain variable region ($V_L$FAP) comprising (iv) CDR-L1 comprising the amino acid sequence of SEQ ID NO:7, (v) CDR-L2 comprising the amino acid sequence of SEQ ID NO:8, and (vi) CDR-L3 comprising the amino acid sequence of SEQ ID NO:9, or (b) a heavy chain variable region ($V_H$FAP) comprising (i) CDR-H1 comprising the amino acid sequence of SEQ ID NO:12, (ii) CDR-H2 comprising the amino acid sequence of SEQ ID NO:13, and (iii) CDR-H3 comprising the amino acid sequence of SEQ ID NO:14, and a light chain variable region ($V_L$FAP) comprising (iv) CDR-L1 comprising the amino acid sequence of SEQ ID NO:15, (v) CDR-L2 comprising the amino acid sequence of SEQ ID NO:16, and (vi) CDR-L3 comprising the amino acid sequence of SEQ ID NO:17. In a particular aspect, the agonistic ICOS-binding molecule comprises at least one antigen binding domain capable of specific binding to FAP comprising (a) a heavy chain variable region ($V_H$FAP) comprising (i) CDR-H1 comprising the amino acid sequence of SEQ ID NO:4, (ii) CDR-H2 comprising the amino acid sequence of SEQ ID NO:5, and (iii) CDR-H3 comprising the amino acid sequence of SEQ ID NO:6, and a light chain variable region ($V_L$FAP) comprising (iv) CDR-L1 comprising the amino acid sequence of SEQ ID NO:7, (v) CDR-L2 comprising the amino acid sequence of SEQ ID NO:8, and (vi) CDR-L3 comprising the amino acid sequence of SEQ ID NO:9.

In one aspect, there is provided an agonistic ICOS-binding molecule comprising at least one antigen binding domain that binds to a tumor-associated antigen for use in combination with a T-cell activating anti-CD3 bispecific antibody specific for a tumor-associated antigen in a method for treating or delaying progression of cancer, wherein the agonistic ICOS-binding molecule comprises at least one antigen binding domain capable of specific binding to FAP comprising a heavy chain variable region ($V_H$FAP) comprising an amino acid sequence of SEQ ID NO:10 and a light chain variable region ($V_L$FAP) comprising an amino acid sequence of SEQ ID NO:11 or wherein the antigen binding domain capable of specific binding to FAP comprises a heavy chain variable region ($V_H$FAP) comprising an amino acid sequence of SEQ ID NO:18 and a light chain variable region ($V_L$FAP) comprising an amino acid sequence of SEQ ID NO:19. Particularly, the agonistic ICOS-binding molecule comprises at least one antigen binding domain capable of specific binding to FAP comprising a heavy chain variable region ($V_H$FAP) comprising an amino acid sequence of SEQ ID NO:10 and a light chain variable region ($V_L$FAP) comprising an amino acid sequence of SEQ ID NO:11.

In another aspect, provided is an agonistic ICOS-binding molecule comprising at least one antigen binding domain that binds to a tumor-associated antigen for use in combination with a T-cell activating anti-CD3 bispecific antibody specific for a tumor-associated antigen in a method for treating or delaying progression of cancer, wherein the agonistic ICOS-binding molecule comprising at least one antigen binding domain that binds to a tumor-associated antigen comprises at least one antigen binding domain capable of specific binding to ICOS comprising a heavy chain variable region ($V_H$ICOS) comprising (i) CDR-H1 comprising the amino acid sequence of SEQ ID NO:20, (ii) CDR-H2 comprising the amino acid sequence of SEQ ID NO:21, and (iii) CDR-H3 comprising the amino acid sequence of SEQ ID NO:22, and a light chain variable region ($V_L$ICOS) comprising (iv) CDR-L1 comprising the amino acid sequence of SEQ ID NO:23, (v) CDR-L2 comprising the amino acid sequence of SEQ ID NO:24, and (vi) CDR-L3 comprising the amino acid sequence of SEQ ID NO:25. In a particular aspect, the agonistic ICOS-binding molecule comprising at least one antigen binding domain that binds to a tumor-associated antigen comprises at least one antigen binding domain comprising a heavy chain variable region ($V_H$ICOS) comprising an amino acid sequence of SEQ ID NO:26 and a light chain variable region ($V_L$ICOS) comprising an amino acid sequence of SEQ ID NO:27.

In a further aspect, there is provided an agonistic ICOS-binding molecule comprising at least one antigen binding domain that binds to a tumor-associated antigen for use in combination with a T-cell activating anti-CD3 bispecific antibody specific for a tumor-associated antigen in a method for treating or delaying progression of cancer, wherein the agonistic ICOS-binding molecule comprising at least one antigen binding domain that binds to a tumor-associated antigen comprises an IgG Fc domain, specifically an IgG1 Fc domain or an IgG4 Fc domain. In a particular aspect, the IgG Fc domain is an IgG1 Fc domain. In yet another particular aspect, the the agonistic ICOS-binding molecule comprising at least one antigen binding domain that binds to a tumor-associated antigen comprises a Fc domain that comprises one or more amino acid substitution that reduces binding to an Fc receptor and/or effector function. More particularly, the agonistic ICOS-binding molecule comprising at least one antigen binding domain that binds to a tumor-associated antigen comprises an IgG1 Fc domain comprising the amino acid substitutions L234A, L235A and P329G (Kabat EU numbering).

In one aspect, the invention provides an agonistic ICOS-binding molecule comprising at least one antigen binding domain that binds to a tumor-associated antigen for use in combination with a T-cell activating anti-CD3 bispecific antibody specific for a tumor-associated antigen in a method for treating or delaying progression of cancer, wherein the agonistic ICOS-binding molecule comprises a first heavy chain comprising an amino acid sequence of SEQ ID NO:28, a first light chain comprising an amino acid sequence of SEQ ID NO:29, a second heavy chain comprising an amino acid sequence of SEQ ID NO:30, and a second light chain comprising an amino acid sequence of SEQ ID NO:31. In another aspect, the agonistic ICOS-binding molecule comprises a first heavy chain comprising an amino acid sequence of SEQ ID NO:32, a second heavy chain comprising an amino acid sequence of SEQ ID NO:66, and one light chain comprising the amino acid sequence of SEQ ID NO:29. In another aspect, there is provided an agonistic ICOS-binding molecule comprising at least one antigen binding domain that binds to a tumor-associated antigen for use in a method for treating or delaying progression of cancer, wherein the agonistic ICOS-binding molecule comprises monovalent binding to a tumor associated target and bivalent binding to ICOS. In one aspect, the agonistic ICOS-binding molecule comprises a first heavy chain comprising an amino acid sequence of SEQ ID NO:32, a second heavy chain comprising an amino acid sequence of SEQ ID NO:33, and a two light chains comprising an amino acid sequence of SEQ ID NO:29.

In another aspect, the invention provides an agonistic ICOS-binding molecule comprising at least one antigen binding domain that binds to a tumor-associated antigen for use in combination with a T-cell activating anti-CD3 bispecific antibody specific for a tumor-associated antigen in a method for treating or delaying progression of cancer, wherein the T-cell activating anti-CD3 bispecific antibody is an anti-CEA/anti-CD3 bispecific antibody. In one aspect, the T-cell activating anti-CD3 bispecific antibody comprises a first antigen binding domain comprising a heavy chain variable region ($V_H$CD3) and a light chain variable region ($V_L$CD3), and a second antigen binding domain comprising a heavy chain variable region ($V_H$CEA) and a light chain variable region ($V_L$CEA). In particular, the T-cell activating anti-CD3 bispecific antibody comprises a first antigen binding domain comprising a heavy chain variable region ($V_H$CD3) comprising (i) CDR-H1 comprising the amino acid sequence of SEQ ID NO:34, (ii) CDR-H2 comprising the amino acid sequence of SEQ ID NO:35, and (iii) CDR-H3 comprising the amino acid sequence of SEQ ID NO:36, and a light chain variable region ($V_L$CD3) comprising (iv) CDR-L1 comprising the amino acid sequence of SEQ ID NO:37, (v) CDR-L2 comprising the amino acid sequence of SEQ ID NO:38, and (vi) CDR-L3 comprising the amino acid sequence of SEQ ID NO:39. More particularly, the T-cell activating anti-CD3 bispecific antibody comprises a first antigen binding domain comprising a heavy chain variable region ($V_H$CD3) comprising the amino acid sequence of SEQ ID NO:40 and/or a light chain variable region ($V_L$CD3) comprising the amino acid sequence of SEQ ID NO:41.

In a further aspect, the T-cell activating anti-CD3 bispecific antibody as defined herein before comprises a second antigen binding domain comprising (a) a heavy chain variable region ($V_H$CEA) comprising (i) CDR-H1 comprising the amino acid sequence of SEQ ID NO:42, (ii) CDR-H2 comprising the amino acid sequence of SEQ ID NO:43, and (iii) CDR-H3 comprising the amino acid sequence of SEQ ID NO:44, and a light chain variable region ($V_L$CEA) comprising (iv) CDR-L1 comprising the amino acid sequence of SEQ ID NO:45, (v) CDR-L2 comprising the amino acid sequence of SEQ ID NO:46, and (vi) CDR-L3 comprising the amino acid sequence of SEQ ID NO:47, or (b) a heavy chain variable region ($V_H$CEA) comprising (i) CDR-H1 comprising the amino acid sequence of SEQ ID NO:50, (ii) CDR-H2 comprising the amino acid sequence of SEQ ID NO:51, and (iii) CDR-H3 comprising the amino acid sequence of SEQ ID NO:52, and a light chain variable region ($V_L$CEA) comprising (iv) CDR-L1 comprising the amino acid sequence of SEQ ID NO:53, (v) CDR-L2 comprising the amino acid sequence of SEQ ID NO:54, and (vi) CDR-L3 comprising the amino acid sequence of SEQ ID NO:55. In one aspect, the T-cell activating anti-CD3 bispecific antibody comprises a second antigen binding domain comprising a heavy chain variable region ($V_H$CEA) comprising the amino acid sequence of SEQ ID NO:48 and/or a light chain variable region ($V_L$CEA) comprising the amino acid sequence of SEQ ID NO:49 or a second antigen binding domain comprising a heavy chain variable region ($V_H$CEA) comprising the amino acid sequence of SEQ ID NO:56 and/or a light chain variable region ($V_L$CEA) comprising the amino acid sequence of SEQ ID NO:57.

In another aspect, the invention provides an agonistic ICOS-binding molecule comprising at least one antigen binding domain that binds to a tumor-associated antigen for use in combination with a T-cell activating anti-CD3 bispecific antibody specific for a tumor-associated antigen in a method for treating or delaying progression of cancer, wherein the T-cell activating anti-CD3 bispecific antibody is an anti-CEA/anti-CD3 bispecific antibody and wherein the anti-CEA/anti-CD3 bispecific antibody comprises a third antigen binding domain that binds to CEA. In particular, the third antigen binding domain comprises (a) a heavy chain variable region ($V_H$CEA) comprising (i) CDR-H1 comprising the amino acid sequence of SEQ ID NO:42, (ii) CDR-H2 comprising the amino acid sequence of SEQ ID NO:43, and (iii) CDR-H3 comprising the amino acid sequence of SEQ ID NO:44, and a light chain variable region ($V_L$CEA) comprising (iv) CDR-L1 comprising the amino acid sequence of SEQ ID NO:45, (v) CDR-L2 comprising the amino acid sequence of SEQ ID NO:46, and (vi) CDR-L3 comprising the amino acid sequence of SEQ ID NO:47, or (b) a heavy chain variable region ($V_H$CEA) comprising (i) CDR-H1 comprising the amino acid sequence of SEQ ID NO:50, (ii) CDR-H2 comprising the amino acid sequence of SEQ ID NO:51, and (iii) CDR-H3 comprising the amino acid sequence of SEQ ID NO:52, and a light chain variable region (V$_L$CEA) comprising (iv) CDR-L1 comprising the amino acid sequence of SEQ ID NO:53, (v) CDR-L2 comprising the amino acid sequence of SEQ ID NO:54, and (vi) CDR-L3 comprising the amino acid sequence of SEQ ID NO:55. More particularly, the third antigen binding domain comprises a heavy chain variable region (V$_H$CEA) comprising the amino acid sequence of SEQ ID NO:48 and/or a light chain variable region (V$_L$CEA) comprising the amino acid sequence of SEQ ID NO:49 or a heavy chain variable region (V$_H$CEA) comprising the amino acid sequence of SEQ ID NO:56 and/or a light chain variable region (V$_L$CEA) comprising the amino acid sequence of SEQ ID NO:57.

In a further aspect, there is provided an agonistic ICOS-binding molecule comprising at least one antigen binding domain that binds to a tumor-associated antigen for use in combination with a T-cell activating anti-CD3 bispecific antibody specific for a tumor-associated antigen in a method for treating or delaying progression of cancer, wherein the T-cell activating anti-CD3 bispecific antibody specific for a tumor-associated antigen comprises an IgG Fc domain, specifically an IgG1 Fc domain or an IgG4 Fc domain. In a particular aspect, the IgG Fc domain is an IgG1 Fc domain. In yet another particular aspect, the T-cell activating anti-CD3 bispecific antibody specific for a tumor-associated antigen comprises a Fc domain that comprises one or more amino acid substitution that reduces binding to an Fc receptor and/or effector function. More particularly, the T-cell activating anti-CD3 bispecific antibody specific for a tumor-associated antigen comprises an IgG1 Fc domain comprising the amino acid substitutions L234A, L235A and P329G (Kabat EU numbering).

In a particular aspect, there is provided an agonistic ICOS-binding molecule comprising at least one antigen binding domain that binds to a tumor-associated antigen for use in combination with a T-cell activating anti-CD3 bispecific antibody specific for a tumor-associated antigen in a method for treating or delaying progression of cancer, wherein the T-cell activating anti-CD3 bispecific antibody comprises (a) the amino acid sequences of SEQ ID NO:58, SEQ ID NO:59, SEQ ID NO:60 and SEQ ID NO:61, or (b) the amino acid sequences of SEQ ID NO:62, SEQ ID NO:63, SEQ ID NO:64 and SEQ ID NO:65. More particularly, the T-cell activating anti-CD3 bispecific antibody comprises the amino acid sequences of SEQ ID NO:58, SEQ ID NO:59, SEQ ID NO:60 and SEQ ID NO:61.

In another aspect, the invention provides an agonistic ICOS-binding molecule comprising at least one antigen binding domain that binds to a tumor-associated antigen for use in combination with a T-cell activating anti-CD3 bispecific antibody specific for a tumor-associated antigen in a method for treating or delaying progression of cancer, wherein the agonistic ICOS-binding molecule comprising at least one antigen binding domain that binds to a tumor-associated antigen is used in combination with a T-cell activating anti-CD3 bispecific antibody specific for a tumor-associated antigen and in combination with an agent blocking PD-L1/PD-1 interaction. In one aspect, the agent blocking PD-L1/PD-1 interaction is an anti-PD-L1 antibody or an anti-PDI antibody. More particularly, the agent blocking PD-L1/PD-1 interaction is selected from the group consisting of atezolizumab, durvalumab, pembrolizumab and nivolumab. In a specific aspect, the agent blocking PD-L1/PD-1 interaction is atezolizumab.

In a further aspect, the invention provides a pharmaceutical product comprising (A) a first composition comprising as active ingredient an agonistic ICOS-binding molecule comprising at least one antigen binding domain that binds to a tumor-associated antigen and a pharmaceutically acceptable excipient; and (B) a second composition comprising a T-cell activating anti-CD3 bispecific antibody specific for a tumor-associated antigen and a pharmaceutically acceptable excipient, for use in the combined, sequential or simultaneous, treatment of a disease, in particular cancer. In particular, the agonistic ICOS-binding molecule comprising at least one antigen binding domain that binds to a tumor-associated antigen is a molecule as defined herein before. In another aspect, the T-cell activating anti-CD3 bispecific antibody specific for a tumor-associated antigen is an anti-CD3 bispecific antibody as defined herein before.

In yet another aspect, the invention relates to a pharmaceutical composition comprising an agonistic ICOS-binding molecule comprising at least one antigen binding domain that binds to a tumor-associated antigen and a T-cell activating anti-CD3 bispecific antibody specific for a tumor-associated antigen and pharmaceutically acceptable excipients. In a particular aspect, there is provided a pharmaceutical composition comprising an agonistic ICOS-binding molecule comprising at least one antigen binding domain that binds to a tumor-associated antigen and a T-cell activating anti-CD3 bispecific antibody specific for a tumor-associated antigen and pharmaceutically acceptable excipients for use in the treatment of cancer, more particularly for the treatment of solid tumors.

In another aspect, the invention provides an agonistic ICOS-binding molecule comprising at least one antigen binding domain that binds to a tumor-associated antigen, wherein the tumor-associated antigen is selected from the group consisting of Fibroblast Activation Protein (FAP), Carcinoembryonic Antigen (CEA), Folate receptor alpha (FolR1), Melanoma-associated Chondroitin Sulfate Proteoglycan (MCSP), Epidermal Growth Factor Receptor (EGFR), human epidermal growth factor receptor 2 (HER2) and p95HER2.

In one aspect, there is provided an agonistic ICOS-binding molecule comprising at least one antigen binding domain that binds to a tumor-associated antigen, wherein the tumor-associated antigen is FAP. In particular, the agonistic ICOS-binding molecule comprises at least one antigen binding domain capable of specific binding to FAP comprising (a) a heavy chain variable region (V$_H$FAP) comprising (i) CDR-H1 comprising the amino acid sequence of SEQ ID NO:4, (ii) CDR-H2 comprising the amino acid sequence of SEQ ID NO:5, and (iii) CDR-H3 comprising the amino acid sequence of SEQ ID NO:6, and a light chain variable region (V$_L$FAP) comprising (iv) CDR-L1 comprising the amino acid sequence of SEQ ID NO:7, (v) CDR-L2 comprising the amino acid sequence of SEQ ID NO:8, and (vi) CDR-L3 comprising the amino acid sequence of SEQ ID NO:9, or (b) a heavy chain variable region ($V_H$FAP) comprising (i) CDR-H1 comprising the amino acid sequence of SEQ ID NO:12, (ii) CDR-H2 comprising the amino acid sequence of SEQ ID NO:13, and (iii) CDR-H3 comprising the amino acid sequence of SEQ ID NO:14, and a light chain variable region ($V_L$FAP) comprising (iv) CDR-L1 comprising the amino acid sequence of SEQ ID NO:15, (v) CDR-L2 comprising the amino acid sequence of SEQ ID NO:16, and (vi) CDR-L3 comprising the amino acid sequence of SEQ ID NO:17. More particularly, the agonistic ICOS-binding molecule comprises at least one antigen binding domain capable of specific binding to FAP comprising a heavy chain variable region ($V_H$FAP) comprising (i) CDR-H1 comprising the amino acid sequence of SEQ ID NO:4, (ii) CDR-H2 comprising the amino acid sequence of SEQ ID NO:5, and (iii) CDR-H3 comprising the amino acid sequence of SEQ ID NO:6, and a light chain variable region ($V_L$FAP) comprising (iv) CDR-L1 comprising the amino acid sequence of SEQ ID NO:7, (v) CDR-L2 comprising the amino acid sequence of SEQ ID NO:8, and (vi) CDR-L3 comprising the amino acid sequence of SEQ ID NO:9. In one aspect, the agonistic ICOS-binding molecule comprises at least one antigen binding domain capable of specific binding to FAP comprising a heavy chain variable region ($V_H$FAP) comprising an amino acid sequence of SEQ ID NO:10 and a light chain variable region ($V_L$FAP) comprising an amino acid sequence of SEQ ID NO:11 or at least one antigen binding domain capable of specific binding to FAP comprising a heavy chain variable region ($V_H$FAP) comprising an amino acid sequence of SEQ ID NO:18 and a light chain variable region ($V_L$FAP) comprising an amino acid sequence of SEQ ID NO:19. In a further aspect, the agonistic ICOS-binding molecule comprises at least one antigen binding domain capable of specific binding to ICOS comprising a heavy chain variable region ($V_H$ICOS) comprising (i) CDR-H1 comprising the amino acid sequence of SEQ ID NO:20, (ii) CDR-H2 comprising the amino acid sequence of SEQ ID NO:21, and (iii) CDR-H3 comprising the amino acid sequence of SEQ ID NO:22, and a light chain variable region ($V_L$ICOS) comprising (iv) CDR-L1 comprising the amino acid sequence of SEQ ID NO:23, (v) CDR-L2 comprising the amino acid sequence of SEQ ID NO:24, and (vi) CDR-L3 comprising the amino acid sequence of SEQ ID NO:25. More particularly, the agonistic ICOS-binding molecule comprising at least one antigen binding domain that binds to a tumor-associated antigen comprises at least one antigen binding domain comprising a heavy chain variable region ($V_H$ICOS) comprising an amino acid sequence of SEQ ID NO:26 and a light chain variable region ($V_L$ICOS) comprising an amino acid sequence of SEQ ID NO:27. In a further aspect, the agonistic ICOS-binding molecule comprising at least one antigen binding domain that binds to a tumor-associated antigen comprises an IgG Fc domain, specifically an IgG1 Fc domain or an IgG4 Fc domain. More particularly, the agonistic ICOS-binding molecule comprising at least one antigen binding domain that binds to a tumor-associated antigen comprises an IgG1 Fc domain. In a particular aspect, the agonistic ICOS-binding molecule comprising at least one antigen binding domain that binds to a tumor-associated antigen comprises a Fc domain that comprises one or more amino acid substitution that reduces binding to an Fc receptor and/or effector function. More particularly, the agonistic ICOS-binding molecule comprising at least one antigen binding domain that binds to a tumor-associated antigen comprises an IgG1 Fc domain comprising the amino acid substitutions L234A, L235A and P329G (Kabat EU numbering).

In one aspect, the invention provides an agonistic ICOS-binding molecule comprising at least one antigen binding domain that binds to a tumor-associated antigen, wherein the agonistic ICOS-binding molecule comprises a first heavy chain comprising an amino acid sequence of SEQ ID NO:28, a first light chain comprising an amino acid sequence of SEQ ID NO:29, a second heavy chain comprising an amino acid sequence of SEQ ID NO:30, and a second light chain comprising an amino acid sequence of SEQ ID NO:31. In another aspect, there is provided an agonistic ICOS-binding molecule comprising at least one antigen binding domain that binds to a tumor-associated antigen, wherein the agonistic ICOS-binding molecule comprises monovalent binding to a tumor associated target and bivalent binding to ICOS. In one aspect, the agonistic ICOS-binding molecule comprises a first heavy chain comprising an amino acid sequence of SEQ ID NO:32, a second heavy chain comprising an amino acid sequence of SEQ ID NO:33, and a two light chains comprising an amino acid sequence of SEQ ID NO:29.

According to another aspect of the invention, there is provided an isolated polynucleotide encoding an agonistic ICOS-binding molecule comprising at least one antigen binding domain that binds to a tumor-associated antigen as described herein before. The invention further provides a vector, particularly an expression vector, comprising the isolated polynucleotide of the invention and a host cell comprising the isolated polynucleotide or the vector of the invention. In some aspects the host cell is a eukaryotic cell, particularly a mammalian cell.

In another aspect, provided is a method for producing an agonistic ICOS-binding molecule comprising at least one antigen binding domain that binds to a tumor-associated antigen as described herein before, comprising the steps of (i) culturing the host cell of the invention under conditions suitable for expression of the antigen binding molecule, and (ii) recovering the antigen binding molecule. The invention also encompasses the agonistic ICOS-binding molecule comprising at least one antigen binding domain that binds to a tumor-associated antigen as described herein produced by the method of the invention.

The invention further provides a pharmaceutical composition comprising an agonistic ICOS-binding molecule comprising at least one antigen binding domain that binds to a tumor-associated antigen as described herein before and at least one pharmaceutically acceptable excipient.

Also encompassed by the invention is the agonistic ICOS-binding molecule comprising at least one antigen binding domain that binds to a tumor-associated antigen as described herein before, or the pharmaceutical composition comprising the agonistic ICOS-binding molecule comprising at least one antigen binding domain that binds to a tumor-associated antigen, for use as a medicament.

In one aspect, provided is the agonistic ICOS-binding molecule comprising at least one antigen binding domain that binds to a tumor-associated antigen as described herein before or the pharmaceutical composition comprising the agonistic ICOS-binding molecule comprising at least one antigen binding domain that binds to a tumor-associated antigen, for use

> (i) in stimulating T cell response,
> (ii) in supporting survival of activated T cells,
> (iii) in the treatment of infections,
> (iv) in the treatment of cancer,
> (v) in delaying progression of cancer, or
> (vi) in prolonging the survival of a patient suffering from cancer.

In a specific aspect, there is provided the agonistic ICOS-binding molecule comprising at least one antigen binding domain that binds to a tumor-associated antigen as described herein before, or the pharmaceutical composition comprising the agonistic ICOS-binding molecule comprising at least one antigen binding domain that binds to a tumor-associated antigen as described herein before, for use in the treatment of cancer.

In another specific aspect, the invention provides the agonistic ICOS-binding molecule comprising at least one antigen binding domain that binds to a tumor-associated antigen as described herein before as described herein for use in the treatment of cancer, wherein the agonistic ICOS-binding molecule comprising at least one antigen binding domain that binds to a tumor-associated antigen is administered in combination with a chemotherapeutic agent, radiation and/or other agents for use in cancer immunotherapy.

In a further aspect, provided is the agonistic ICOS-binding molecule comprising at least one antigen binding domain that binds to a tumor-associated antigen as described herein before as described herein for use in the treatment of cancer, wherein the agonistic ICOS-binding molecule comprising at least one antigen binding domain that binds to a tumor-associated antigen is administered in combination with an agent blocking PD-L1/PD-1 interaction. In one aspect, the agent blocking PD-L1/PD-1 interaction is an anti-PD-L1 antibody or an anti-PD1 antibody. More particularly, the agent blocking PD-L1/PD-1 interaction is selected from the group consisting of atezolizumab, durvalumab, pembrolizumab and nivolumab. In a specific aspect, the agent blocking PD-L1/PD-1 interaction is atezolizumab.

In a further aspect, the invention provides a method of inhibiting the growth of tumor cells in an individual comprising administering to the individual an effective amount of the agonistic ICOS-binding molecule comprising at least one antigen binding domain that binds to a tumor-associated antigen as described herein before, or the pharmaceutical composition comprising the agonistic ICOS-binding molecule comprising at least one antigen binding domain that binds to a tumor-associated antigen as described herein before, to inhibit the growth of the tumor cells.

Also provided is the use of the agonistic ICOS-binding molecule comprising at least one antigen binding domain that binds to a tumor-associated antigen as described herein before for the manufacture of a medicament for the treatment of a disease in an individual in need thereof, in particular for the manufacture of a medicament for the treatment of cancer, as well as a method of treating a disease in an individual, comprising administering to said individual a therapeutically effective amount of a composition comprising the agonistic ICOS-binding molecule comprising at least one antigen binding domain that binds to a tumor-associated antigen as described herein before in a pharmaceutically acceptable form. In a specific aspect, the disease is cancer. In any of the above aspects the individual is a mammal, particularly a human.

## BRIEF DESCRIPTION OF THE DRAWINGS

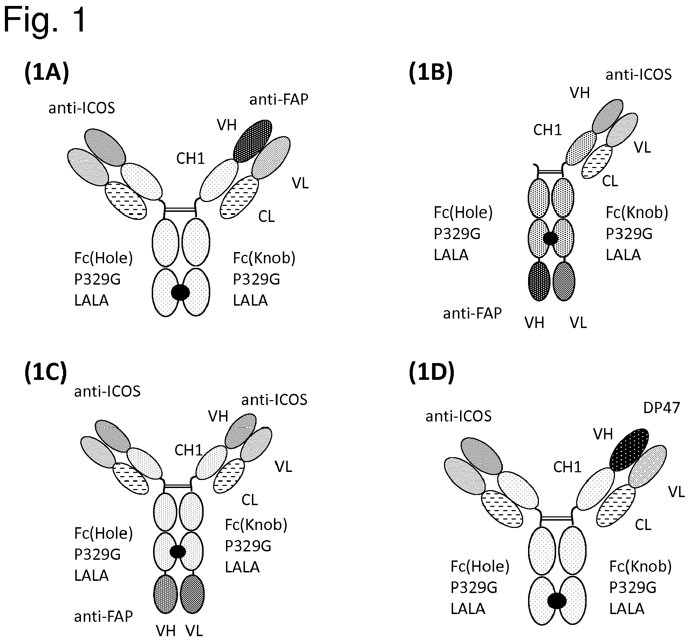

Figure 1 A - E: Schematic Figures of all FAP-targeted ICOS molecules & their untargeted (DP47) reference molecules.

Figure IF: Schematic Figure of a human T-cell activating anti-CD3 bispecific antibody specific for a tumor-associated antigen (CEA TCB).

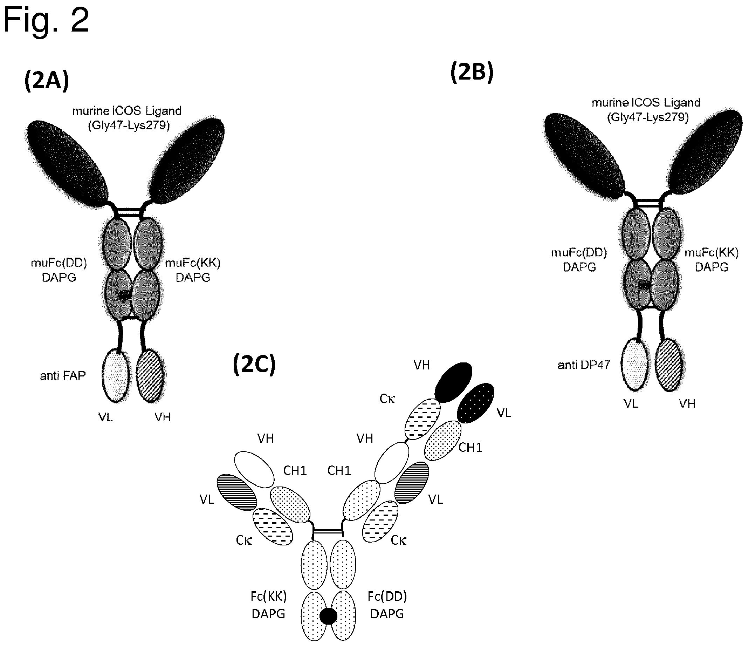

Figures 2A and 2B: Schematic Figure of a FAP-targeted mICOS-L molecule and an untargeted (DP47)-mICOS-L reference molecule.

Figure 2C: Schematic Figure of a murine T-cell activating anti-CD3 bispecific antibody specific for a tumor-associated antigen (CEA TCB).

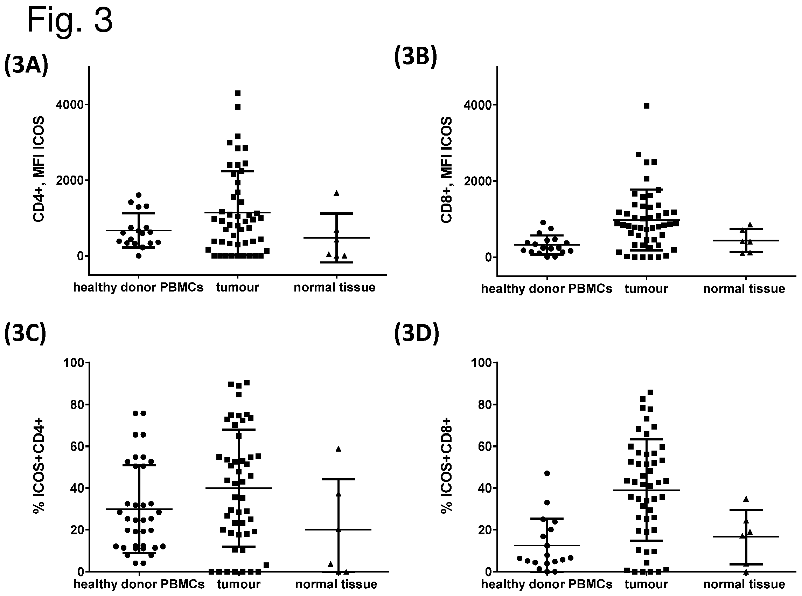

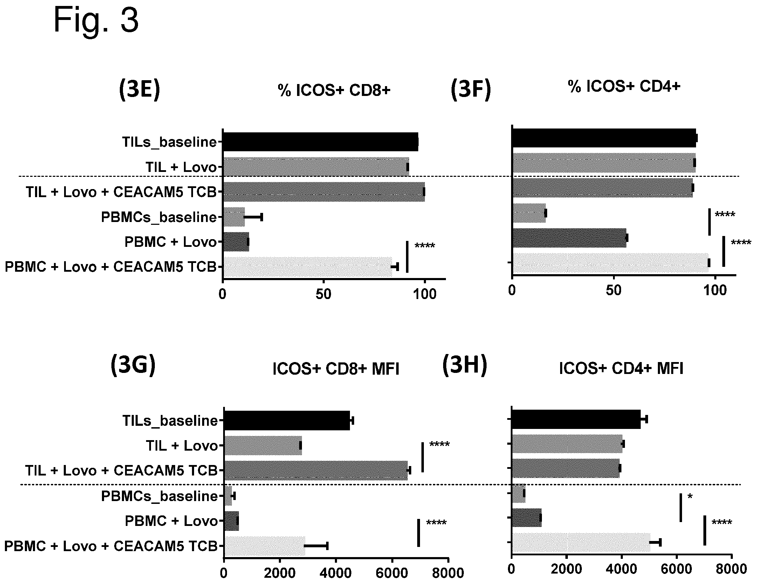

Figures 3A - D: ICOS expression on T-cells from healthy donors, TILs or on T-cells isolated from normal tissue of the same patient. Figures 3E- 3H: ICOS expression on TILs versus human PBMCs at baseline versus upon TCB treatment. Median fluorescence intensities and percent of ICOS-positive T-cells, as determined by flow cytometry. Depicted are technical triplicates with SD. Isotypes were substracted.

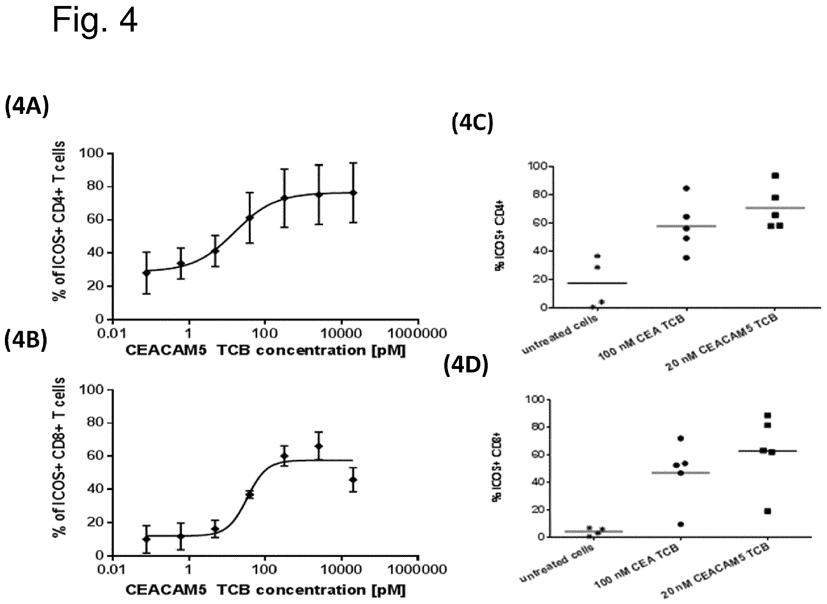

Figures 4A - 4D: Increase of human ICOS expression upon CEACAM5-TCB-mediated activation of human T cells, as determined by flow cytometry. Figures 4A and 4B show the dose-dependent increase in ICOS$^+$ CD4 (Figure 4A) and CD8$^+$ (Figure 4B) T-Cells upon incubation with CEACAM5 TCB for 48 hours. The graphs show technical triplicates, error bars indicate SD. Figures 4C and 4D show the increase in percentage of ICOS$^+$ CD4$^+$ (Figure 4C) and CD8 $^+$ (Figure 4D) T Cells upon CEA TCB (100 nM) or CEACAM5 TCB (20 nM) treatment for 48 h for 5 different healthy human PBMC donors.

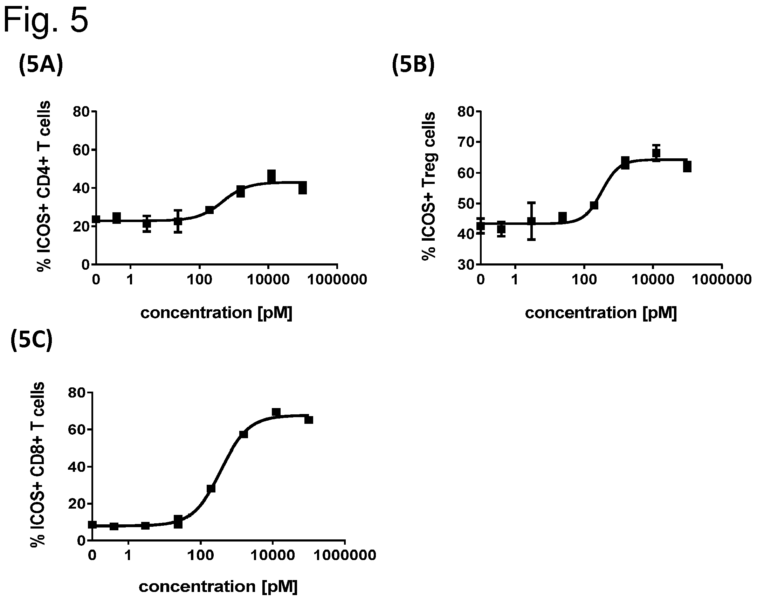

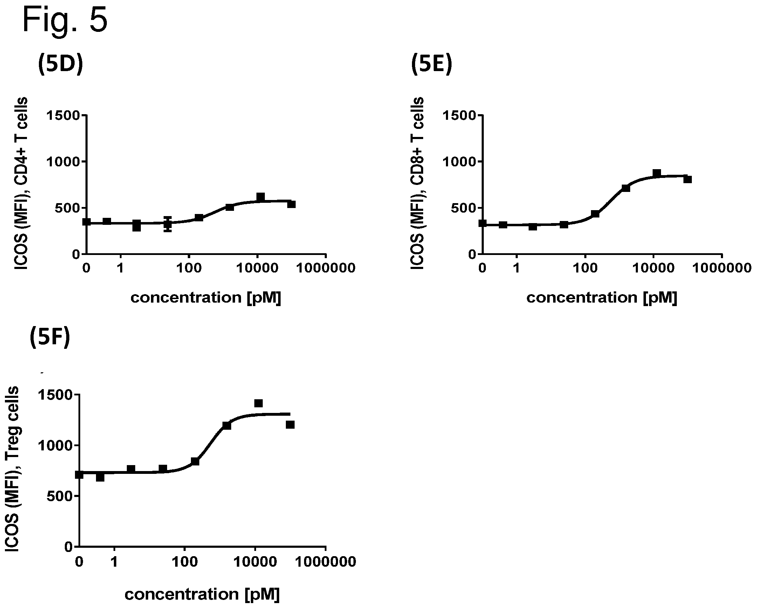

Figures 5A-5F: Increase of murine ICOS expression on murine CD4$^+$, Treg or CD8$^+$ T-cells upon mTCB-mediated activation of murine T cells. Percentage (Figures 5A-5C) and Median Fluorescence Intensities (MFI, Figures 5D-5F) of ICOS-positive cells of either murine CD4 (Figures 5A and 5D), Treg (Figures 5B and 5E) or CD8 T cells (Figures 5C and 5F) after 48h of co-incubation of MC38-hCEA tumor target cells, murine splenocytes from hCEA(HO) Tg mice and increasing concentrations of mCEA-TCB. The graphs show technical triplicates, error bars indicate SD, isotype values have been substracted.

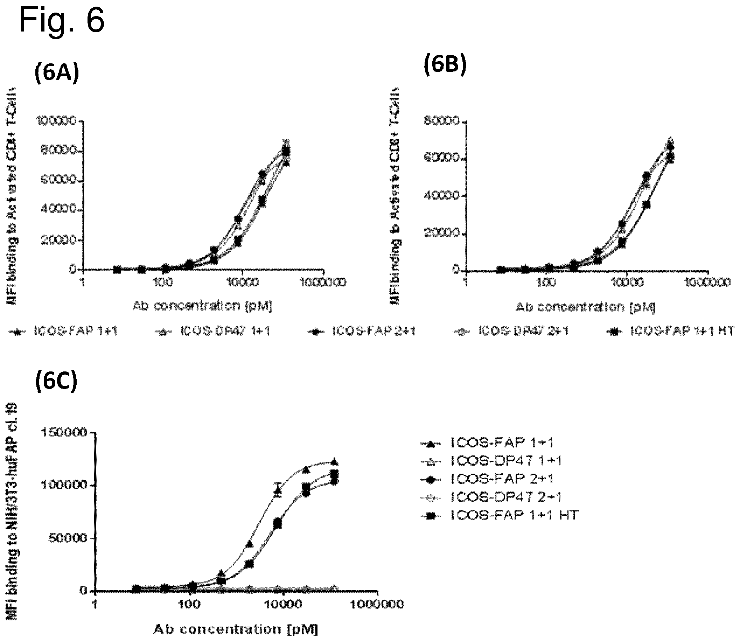

Figures 6A-6C: Binding of different FAP-ICOS molecules to human ICOS (Figures 6A and 6B) and human FAP (Figure 6C) on cells, as determined by flow cytometry. Median fluorescence intensities for binding of different FAP- or DP47-ICOS molecules to activated PBMCs (Using Gibco #11161D, 48 hours, 1:2 Bead to Cell Ratio), respective to human FAP-expressing NIH/3T3-hFAP cells, as measured by flow cytometry. Depicted are technical triplicates with SD.

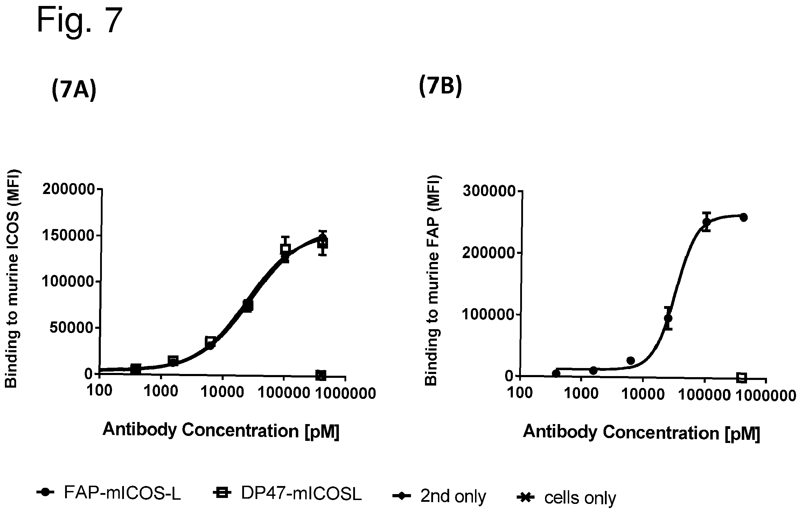

Figures 7A and 7B: Binding of FAP-mICOS-L to murine ICOS (Figure 7A)- and murine FAP (Figure 7B)-expressing cells, as determined by flow cytometry. Median fluorescence intensities for binding of FAP-mICOS-L to stable CHO-K1 transfectants, over-expressing murine ICOS (A), respective to 3T3-mFAP cells (parental cell line ATCC #CCL-92, modified to stably overexpress murine FAP (B), as measured by flow cytometry. Depicted are technical triplicates with SD.

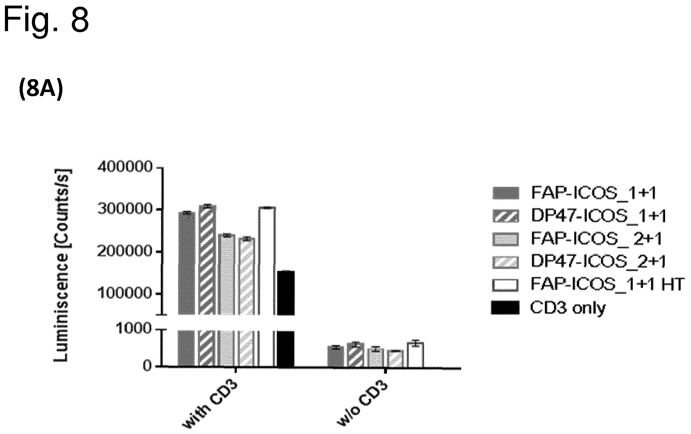

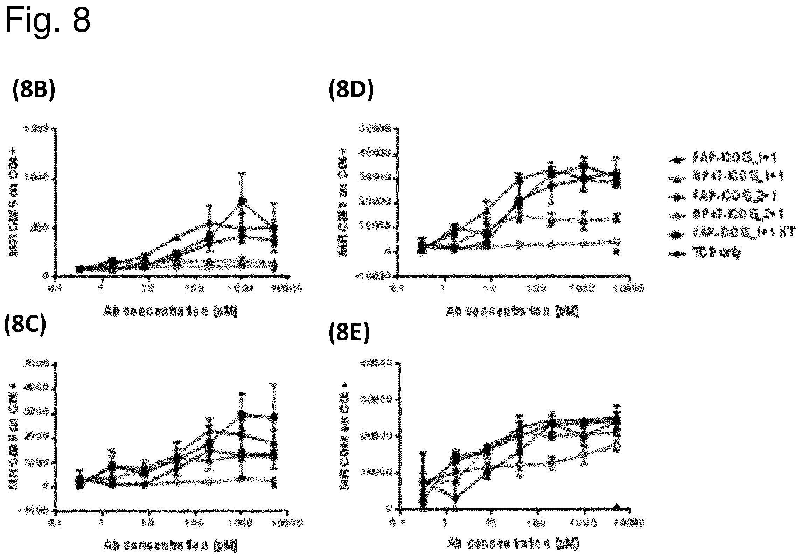

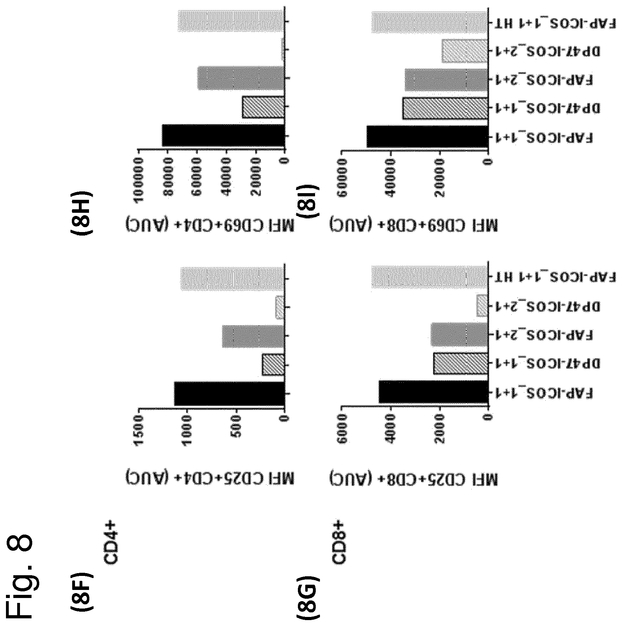

Figures 8A-8I: Evaluation of different FAP- versus untargeted ICOS molecules. In Figure 8A, potential superagonism was addressed, using a Jurkat-NFAT reporter assay. The graphs show luminescence signal intensities, i.e. luminescence intensities of preactivated Jurkat-NFAT-Luc2P cells (Promega #CS176501) with different plate-bound aICOS constructs in presence or absence of simultaneously coated aCD3. Depicted are technical triplicates with SEM. In Figures 8B to 8E, the impact of crosslinking of ICOS molecules via FAP, as assessed in a T-cell activation assay by flow cytometry is shown. Depicted are surface expression levels of early T-cell activation CD69 and late activation marker CD25 on CD4$^+$ T cells (Figures 8B and 8C) or CD8$^+$ T-cells (Figures 8D and 8E) as Median Fluorescence Intensities (MFI) or as Area under the curve AUC (Figures 8F to 8I). Median Fluorescence Intensitites (MFI) were measured after 48h of co-incubation of human PBMC effector, MKN45 tumor cells and 3T3-hFAP fibroblasts at an E:T of 5:1:1 in presence of 80 pM CEACAM5 TCB in presence of increasing concentration of FAP targeted FAP-ICOS constructs or untargeted DP47-ICOS constructs. The graphs show technical triplicates, error bars indicate SD.

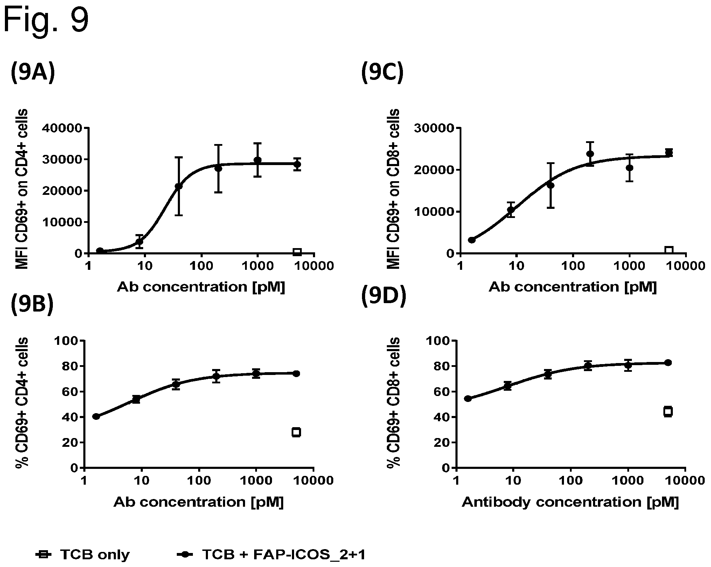

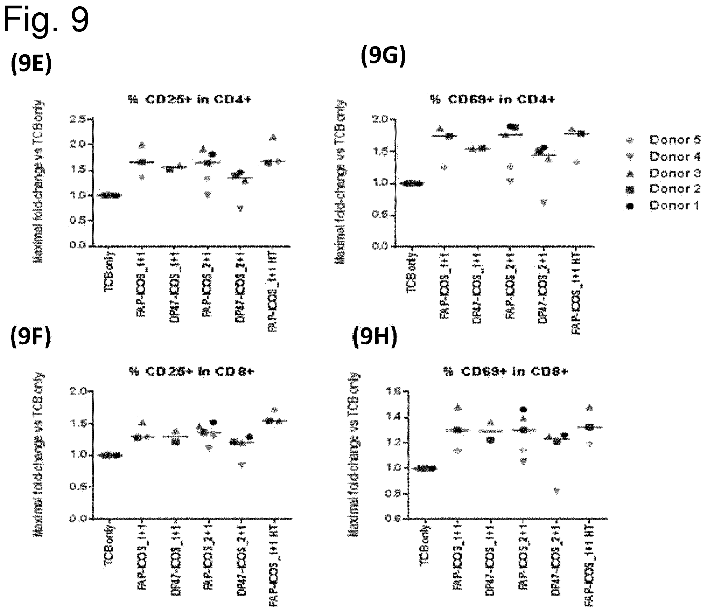

Figures 9A -9H: Increase of CEACAM5-TCB-mediated T-cell activation by FAP-ICOS bispecific molecules, crosslinked by binding to 3T3-hFAP cells, as determined by flow cytometry. Depicted are expression levels of the CD69 as MFI or percent of positive CD4$^+$ and CD8$^+$ T-cells in the presence of increasing concentrations of the FAP-ICOS_2+1 molecule (Figures 9A-9D) as well as the maximal fold increase of T-cell activation, induced by co-incubation of FAP-ICOS molecules and CEACAM5-TCB for up to 5 different healthy human PBMC donors (Figures 9E-9H). Median Fluorescence Intensitites (MFI, Figures 9A and 9C) and percentage (Figures 9B and 9D) of CD69-positive CD4$^+$ T cells (Figures 9A and 9B) or CD8$^+$ T cells (Figures 9C and 9D) are shown after 48h of co-incubation of human PBMC effector, MKN45 tumor cells and 3T3-hFAP fibroblasts at an E:T of 5:1:1 in presence of 80 pM CEACAM5 TCB in presence of increasing concentration of FAP-ICOS_2+1. The graphs show technical triplicates, error bars indicate SD. Figures 9E-9H show the maximal fold increase of T-cell activation, induced by co-incubation of the indicated FAP-ICOS molecules and 80 pM TCB for up to 5 different healthy human PBMC donors.

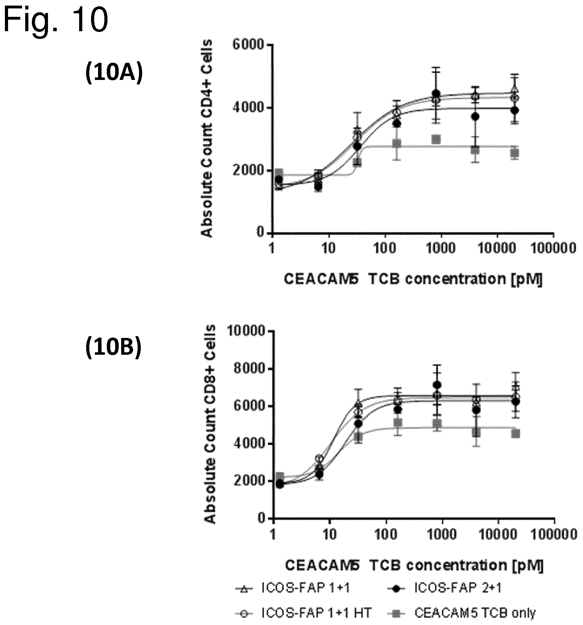

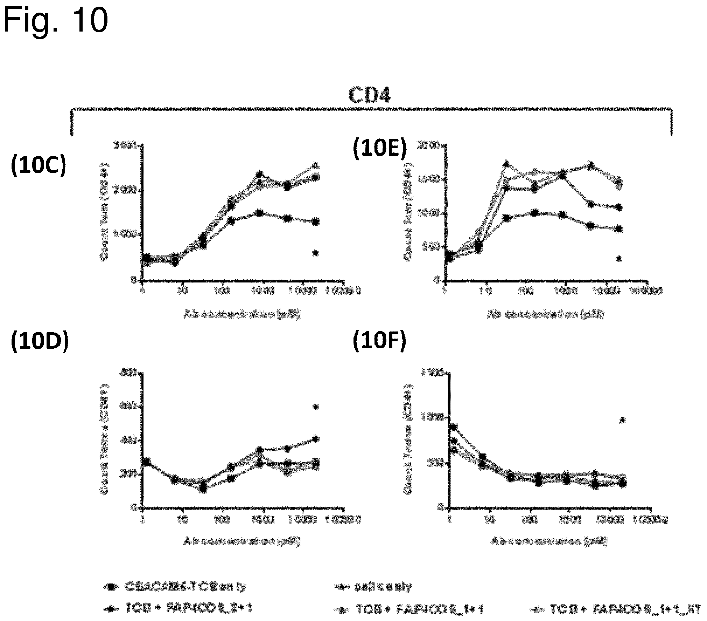

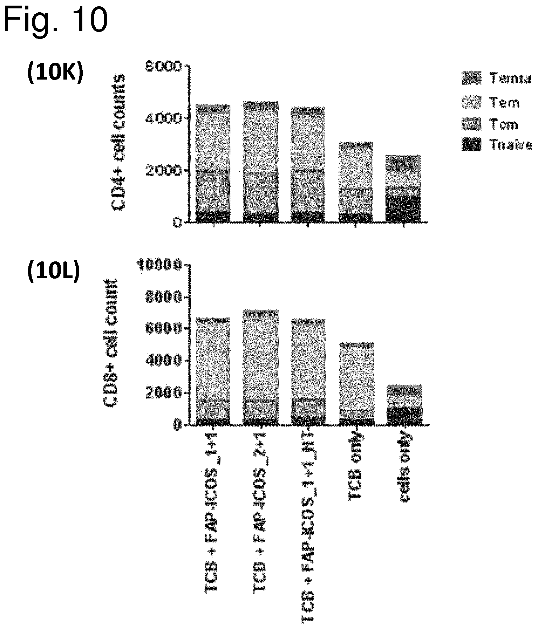

Figures 10 A-L: Increased CEACAM5-TCB-mediated T-cell Proliferation and T-Cell Differentiation in absence versus presence of several FAP-ICOS Formats. The graphs show the number of absolute CD4$^+$ and CD8$^+$ T-cells (Figures 10A and 10B), respective numbers of naive, central memory (Tcm), effector memory (Tem) and CD45RA-positive effector memory (Temra) cells with increasing TCB concentration (Figures 10C to 10J) or at a fixed concentration of 2.9 pM (Figures 10K and 10L), as determined by flow cytometry. Absolute Cell Counts of CD4$^+$ and CD8+ (Figures 10A and 10B) or CD4 or CD8$^+$ Tem, Tcm, Tnaive and Temra Memory T Cell Subsets (Figures 10C to 10J) after 96h of co-incubation of human PBMC effector, MKN45 tumor cells and 3T3-hFAP fibroblasts at an E:T of 5:1:1 in presence of CEACAM5-TCB (increasing concentrations) in presence of InM FAP-ICOS_1+1, FAP-ICOS_2+1., FAP-ICOS_1+1 HT. The graphs show means of technical triplicates, error bars indicate SD. Figures 10K and 10L refer to the data set depicted in Figures 10C to 10J, highlighting the absolute numbers of naive, central memory, effector memory or Temra cells at a fixed concentration of 2.9 pM CEACAM5-TCB in absence versus presence of InM of the indicated ICOS molecules. Gating was done as follows: $T_{em}$ = CD45RO+CCR7-, $T_{cm}$ = CD45RO+CCR7+, $T_{emra}$ = CD45RO-CCR7-, $T_{naive}$ = CD45RO-CCR7+.

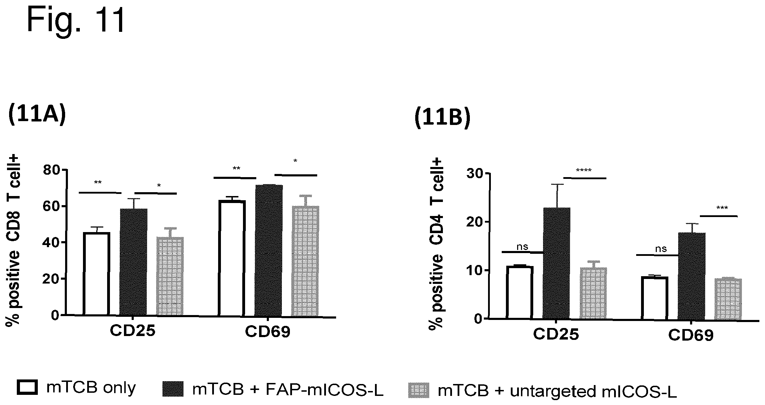

Figures 11A and 11B. Increased mCEA-TCB-mediated T-cell activation in presence of FAP-mICOS-L. The graphs show percent of CD25 and CD69-positive CD8$^+$ and CD4$^+$ T-cells, as assessed by flow cytometry. Percentage of CD25- and CD69-positive CD8$^+$ T cells (Figure 11A) or CD4$^+$ T cells (Figure 11B) after 48h of co-incubation of MC38-hCEA tumor cells, 3T3-mFAP fibroblasts and murine splenocyte effector cells (C57 Bl/6 mice) at an E:T of 50:1 in presence of 1.5 nM mCEA-TCB in presence or absence of 50 nM FAP-targeted or untargeted reference mICOS-L molecules, as indicated. The graphs show triplicates, error bars indicate SD, isotype values have been substracted.

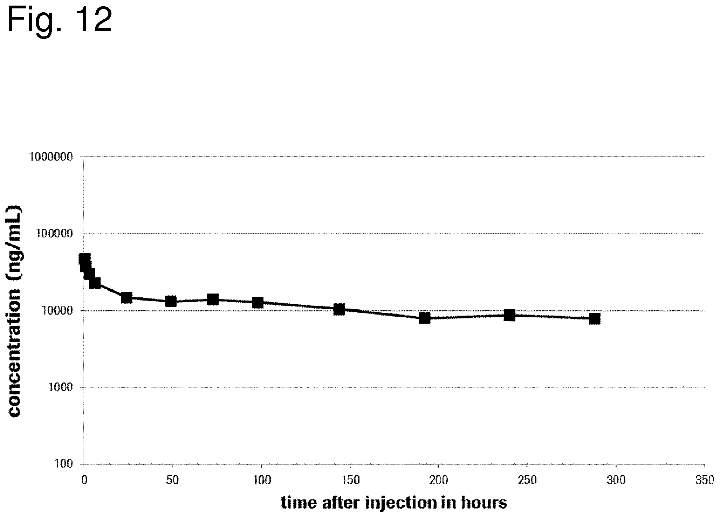

Figure 12: Pharmacokinetic profile of FAP-ICOS_1+1 after single injection in NSG mice.

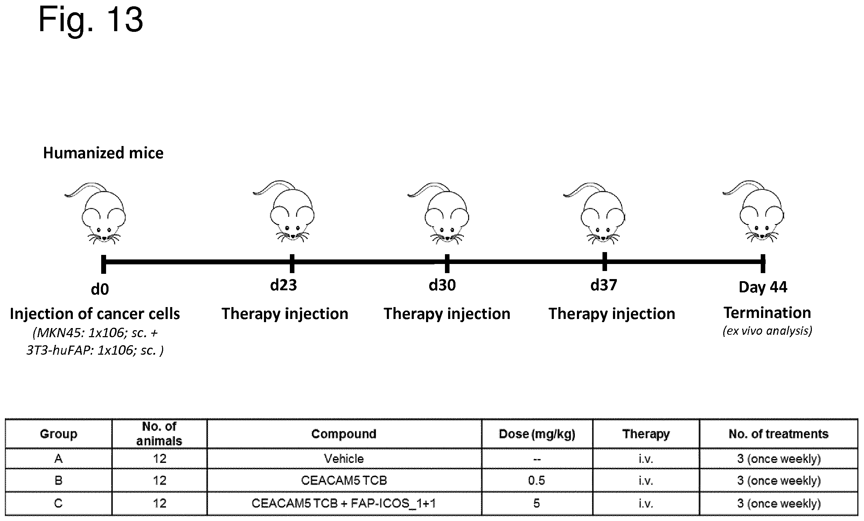

Figure 13: Efficacy study with FAP-ICOS_1+1 and CEACAM5-TCB combination in MKN45 Xenograft in humanized mice. Depicted is the study design and the treatment groups.

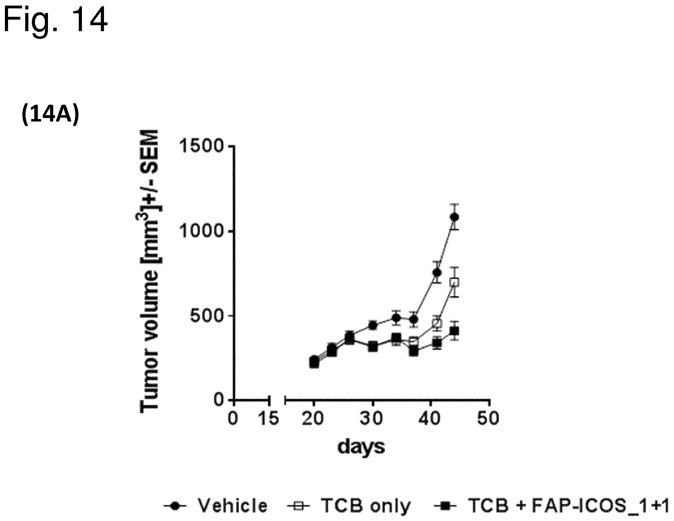

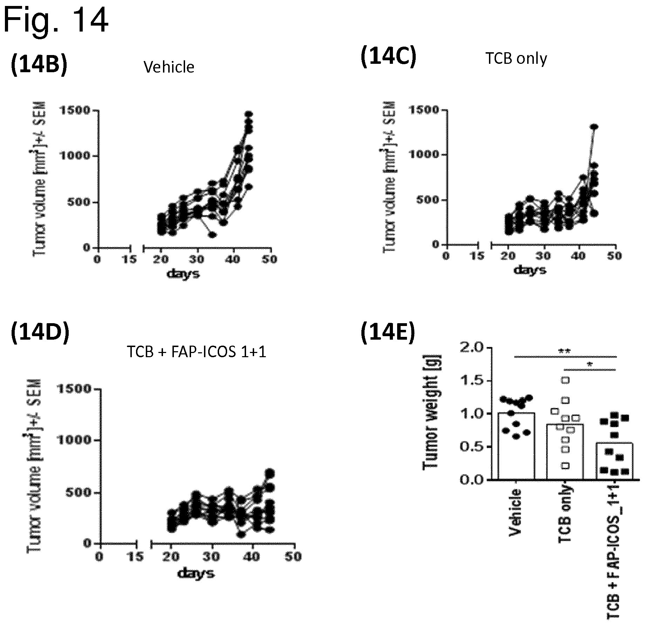

Figures 14A- 14E: Efficacy study with FAP-ICOS_1+1 and CEACAM5 TCB combination in MKN45 Xenograft in humanized mice. Shown is the average tumor volume (Figure 14A) or the growth of tumors in individual mice as plotted on the y-axis (Figures 14B to 14D). Tumor weight at day 44 as plotted for individual mice is summarized in Figure 14E. It can be seen that there is increased TCB-mediated Tumor Regression in the presence of FAP-ICOS_1+1.

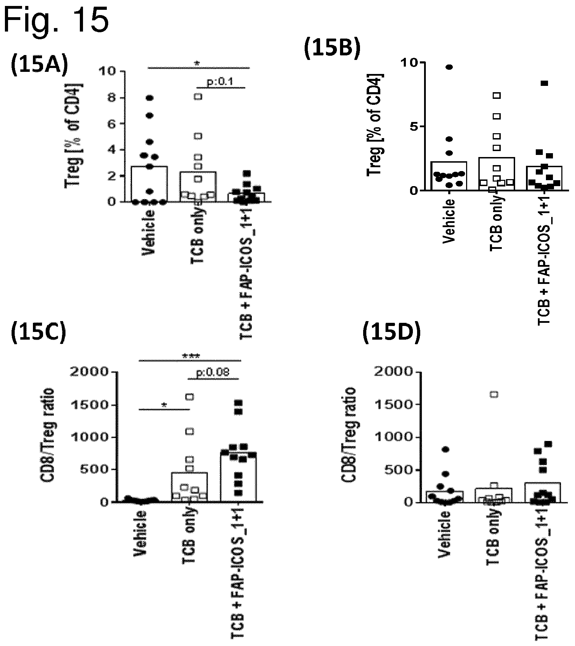

Figures 15A-15D. Tumor-specific depletion of FoxP3+ Tregs upon combination therapy of FAP-ICOS_1+1 antibody and CEACAM5-TCB in a co-grafting model of MKN45 and 3T3-hFAP cells in humanized NSG mice. Frequency of Treg among CD4$^+$ T-cells and ratio of CD8 and Treg cells in spleen (Figures 15C and 15D) and tumor (Figures 15A and 15B) is shown. Each shape indicates an individual mouse.

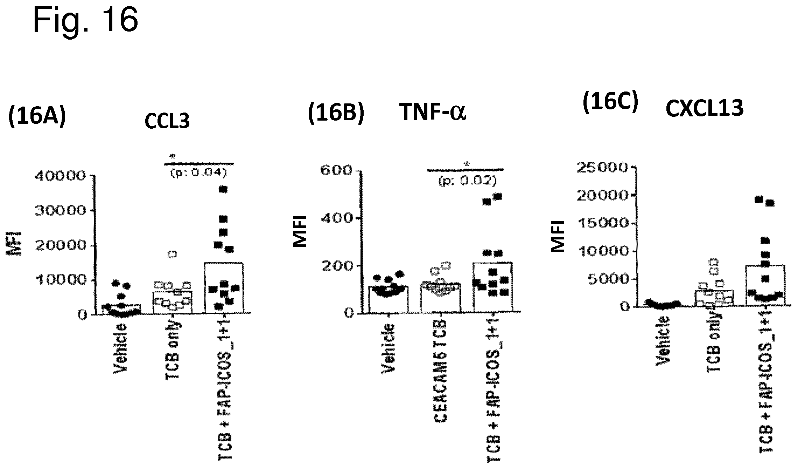

Figures 16A-16C: Cytokine analysis. Intra-tumoral changes in selected chemokine and cytokine expression upon combination therapy of FAP-ICOS_1+1 antibody and CEACAM5-TCB in a co-grafting model of MKN45 and 3T3-hFAP cells in humanized NSG mice. Each shape indicates an individual mouse. Shown are the data for CCL3 (Figure 16A), TNF-$\alpha$, (Figuer 16B) and CXCL13 (Figure 16C).

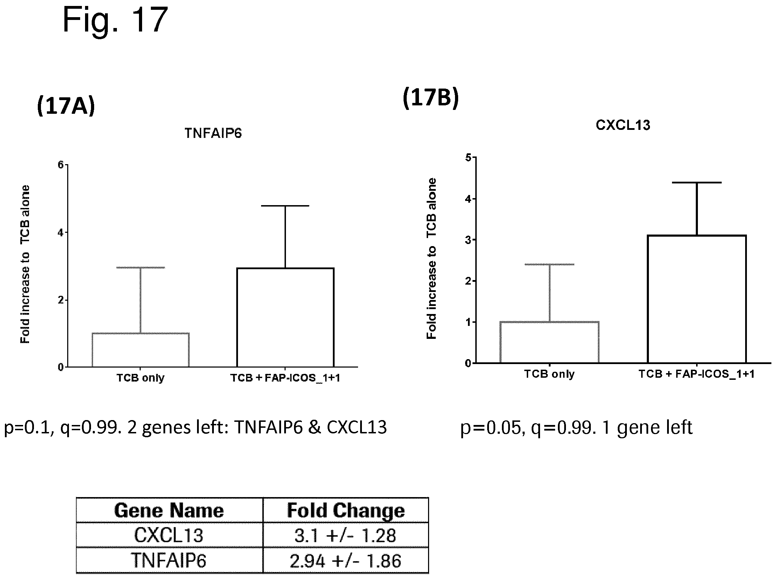

Figures 17A and 17B: Gene expression analysis of remaining tumours of an FAP-ICOS_1+1 combination study with CEACAM5-TCB in a co-grafting model of MKN45 and 3T3-hFAP in humanized NSG mice to identify ICOS-regulated genes. Depicted is the fold increase of significantly upregulated genes TNFAIP6 (p-value is 0.1) and CXCL13 (p value of 0.05), threshold was set to have at least a fold change of 2 as compared to the TCB monotherapy effect. The data for TNFAIP6 are provided in Figure 17A and the data for CXCL13 are depicted in Figure 17B.

## DETAILED DESCRIPTION OF THE INVENTION

### Definitions

Unless defined otherwise, technical and scientific terms used herein have the same meaning as generally used in the art to which this invention belongs. For purposes of interpreting this specification, the following definitions will apply and whenever appropriate, terms used in the singular will also include the plural and vice versa.

As used herein, the term "**antigen binding molecule**" refers in its broadest sense to a molecule that specifically binds an antigenic determinant. Examples of antigen binding molecules are antibodies, antibody fragments and scaffold antigen binding proteins.

As used herein, the term "**antigen binding domain that binds to a tumor-associated antigen**" or "moiety capable of specific binding to a tumor-associated antigen" refers to a polypeptide molecule that specifically binds to an antigenic determinant. In one aspect, the antigen binding domain is able to activate signaling through its target cell antigen. In a particular aspect, the antigen binding domain is able to direct the entity to which it is attached (e.g. the ICOS agonist) to a target site, for example to a specific type of tumor cell or tumor stroma bearing the antigenic determinant. Antigen binding domains capable of specific binding to a target cell antigen include antibodies and fragments thereof as further defined herein. In addition, antigen binding domains capable of specific binding to a target cell antigen include scaffold antigen binding proteins as further defined herein, e.g. binding domains which are based on designed repeat proteins or designed repeat domains (see e.g. WO 2002/020565).

In relation to an antibody or fragment thereof, the term "antigen binding domain that binds to a target cell antigen" refers to the part of the molecule that comprises the area which specifically binds to and is complementary to part or all of an antigen. An antigen binding domain capable of specific antigen binding may be provided, for example, by one or more antibody variable domains (also called antibody variable regions). Particularly, an antigen binding domain capable of specific antigen binding comprises an antibody light chain variable region (VL) and an antibody heavy chain variable region (VH). In another aspect, the "antigen binding domain capable of specific binding to a target cell antigen" can also be a Fab fragment or a cross-Fab fragment.

The term "**antibody**" herein is used in the broadest sense and encompasses various antibody structures, including but not limited to monoclonal antibodies, polyclonal antibodies, monospecific and multispecific antibodies (e.g., bispecific antibodies), and antibody fragments so long as they exhibit the desired antigen-binding activity.

The term "**monoclonal antibody**" as used herein refers to an antibody obtained from a population of substantially homogeneous antibodies, i.e., the individual antibodies comprising the population are identical and/or bind the same epitope, except for possible variant antibodies, e.g. containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen.

The term "**monospecific**" antibody as used herein denotes an antibody that has one or more binding sites each of which bind to the same epitope of the same antigen. The term "**bispecific**" means that the antigen binding molecule is able to specifically bind to at least two distinct antigenic determinants. Typically, a bispecific antigen binding molecule comprises two antigen binding sites, each of which is specific for a different antigenic determinant. In certain embodiments the bispecific antigen binding molecule is capable of simultaneously binding two antigenic determinants, particularly two antigenic determinants expressed on two distinct cells.

The term "**valent**" as used within the current application denotes the presence of a specified number of binding sites specific for one distinct antigenic determinant in an antigen binding molecule that are specific for one distinct antigenic determinant. As such, the terms "bivalent", "tetravalent", and "hexavalent" denote the presence of two binding sites, four binding sites, and six binding sites specific for a certain antigenic determinant, respectively, in an antigen binding molecule. In particular aspects of the invention, the bispecific antigen binding molecules according to the invention can be monovalent for a certain antigenic determinant, meaning that they have only one binding site for said antigenic determinant or they can be bivalent or tetravalent for a certain antigenic determinant, meaning that they have two binding sites or four binding sites, respectively, for said antigenic determinant.

The terms "full length antibody", "intact antibody", and "whole antibody" are used herein interchangeably to refer to an antibody having a structure substantially similar to a native antibody structure. "**Native antibodies**" refer to naturally occurring immunoglobulin molecules with varying structures. For example, native IgG-class antibodies are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by three constant domains (CH1, CH2, and CH3), also called a heavy chain constant region. Similarly, from N- to C-terminus, each light chain has a variable region (VL), also called a variable light domain or a light chain variable domain, followed by a light chain constant domain (CL), also called a light chain constant region. The heavy chain of an antibody may be assigned to one of five types, called $\alpha$ (IgA), $\delta$ (IgD), $\varepsilon$ (IgE), $\gamma$ (IgG), or $\mu$ (IgM), some of which may be further divided into subtypes, e.g. $\gamma$1 (IgG1), $\gamma$2 (IgG2), y3 (IgG3), y4 (IgG4), $\alpha$1 (IgA1) and $\alpha$2 (IgA2). The light chain of an antibody may be assigned to one of two types, called kappa ($\kappa$) and lambda ($\lambda$), based on the amino acid sequence of its constant domain.

An "**antibody fragment**" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')$_2$; diabodies, triabodies, tetrabodies, cross-Fab fragments; linear antibodies; single-chain antibody molecules (e.g. scFv); and single domain antibodies. For a review of certain antibody fragments, see Hudson et al., Nat Med 9, 129-134 (2003). For a review of scFv fragments, see e.g. Plückthun, in The Pharmacology of Monoclonal Antibodies, vol. 113, Rosenburg and Moore eds., Springer-Verlag, New York, pp. 269-315 (1994); see also WO 93/16185; and U.S. Patent Nos. 5,571,894 and 5,587,458. For discussion of Fab and F(ab')2 fragments comprising salvage receptor binding epitope residues and having increased in vivo half-life, see U.S. Patent No. 5,869,046. Diabodies are antibody fragments with two antigen-binding sites that may be bivalent or bispecific, see, for example, EP 404,097; WO 1993/01161; Hudson et al., Nat Med 9, 129-134 (2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see e.g. U.S. Patent No. 6,248,516 B1). Antibody fragments can be made by various techniques, including but not limited to proteolytic digestion of an intact antibody as well as production by recombinant host cells (e.g. E. coli or phage), as described herein.

Papain digestion of intact antibodies produces two identical antigen-binding fragments, called "Fab" fragments containing each the heavy- and light-chain variable domains and also the constant domain of the light chain and the first constant domain (CH1) of the heavy chain. As used herein, Thus, the term "**Fab fragment**" refers to an antibody fragment comprising a light chain fragment comprising a VL domain and a constant domain of a light chain (CL), and a VH domain and a first constant domain (CH1) of a heavy chain. Fab' fragments differ from Fab fragments by the addition of a few residues at the carboxy terminus of the heavy chain CH1 domain including one or more cysteins from the antibody hinge region. Fab'-SH are Fab' fragments in which the cysteine residue(s) of the constant domains bear a free thiol group. Pepsin treatment yields an $F(ab')_2$ fragment that has two antigen-combining sites (two Fab fragments) and a part of the Fc region.

The term "**cross-Fab fragment**" or "**xFab fragment**" or "**crossover Fab fragment**" refers to a Fab fragment, wherein either the variable regions or the constant regions of the heavy and light chain are exchanged. Two different chain compositions of a crossover Fab molecule are possible and comprised in the bispecific antibodies of the invention: On the one hand, the variable regions of the Fab heavy and light chain are exchanged, i.e. the crossover Fab molecule comprises a peptide chain composed of the light chain variable region (VL) and the heavy chain constant region (CH1), and a peptide chain composed of the heavy chain variable region (VH) and the light chain constant region (CL). This crossover Fab molecule is also referred to as CrossFab $_{(VLVH)}$. On the other hand, when the constant regions of the Fab heavy and light chain are exchanged, the crossover Fab molecule comprises a peptide chain composed of the heavy chain variable region (VH) and the light chain constant region (CL), and a peptide chain composed of the light chain variable region (VL) and the heavy chain constant region (CH1). This crossover Fab molecule is also referred to as CrossFab $_{(CLCH1)}$.

A "single chain Fab fragment" or "**scFab**" is a polypeptide consisting of an antibody heavy chain variable domain (VH), an antibody constant domain 1 (CH1), an antibody light chain variable domain (VL), an antibody light chain constant domain (CL) and a linker, wherein said antibody domains and said linker have one of the following orders in N-terminal to C-terminal direction: a) VH-CH1-linker-VL-CL, b) VL-CL-linker-VH-CH1, c) VH-CL-linker-VL-CH1 or d) VL-CH1-linker-VH-CL; and wherein said linker is a polypeptide of at least 30 amino acids, preferably between 32 and 50 amino acids. Said single chain Fab fragments are stabilized via the natural disulfide bond between the CL domain and the CH1 domain. In addition, these single chain Fab molecules might be further stabilized by generation of interchain disulfide bonds via insertion of cysteine residues (e.g. position 44 in the variable heavy chain and position 100 in the variable light chain according to Kabat numbering).

A "crossover single chain Fab fragment" or "**x-scFab**" is a is a polypeptide consisting of an antibody heavy chain variable domain (VH), an antibody constant domain 1 (CH1), an antibody light chain variable domain (VL), an antibody light chain constant domain (CL) and a linker, wherein said antibody domains and said linker have one of the following orders in N-terminal to C-terminal direction: a) VH-CL-linker-VL-CH1 and b) VL-CH1-linker-VH-CL; wherein VH and VL form together an antigen-binding site which binds specifically to an antigen and wherein said linker is a polypeptide of at least 30 amino acids. In addition, these x-scFab molecules might be further stabilized by generation of interchain disulfide bonds via insertion of cysteine residues (e.g. position 44 in the variable heavy chain and position 100 in the variable light chain according to Kabat numbering).

A "**single-chain variable fragment (scFv)**" is a fusion protein of the variable regions of the heavy ($V_H$) and light chains ($V_L$) of an antibody, connected with a short linker peptide of ten to about 25 amino acids. The linker is usually rich in glycine for flexibility, as well as serine or threonine for solubility, and can either connect the N-terminus of the $V_H$ with the C-terminus of the $V_L$, or *vice versa*. This protein retains the specificity of the original antibody, despite removal of the constant regions and the introduction of the linker. scFv antibodies are, e.g. described in Houston, J.S., Methods in Enzymol. 203 (1991) 46-96). In addition, antibody fragments comprise single chain polypeptides having the characteristics of a VH domain, namely being able to assemble together with a VL domain, or of a VL domain, namely being able to assemble together with a VH domain to a functional antigen binding site and thereby providing the antigen binding property of full length antibodies.

"**Scaffold antigen binding proteins**" are known in the art, for example, fibronectin and designed ankyrin repeat proteins (DARPins) have been used as alternative scaffolds for antigen-binding domains, see, e.g., Gebauer and Skerra, Engineered protein scaffolds as next-generation antibody therapeutics. Curr Opin Chem Biol 13:245-255 (2009) and Stumpp et al., Darpins: A new generation of protein therapeutics. Drug Discovery Today 13: 695-701 (2008). In one aspect of the invention, a scaffold antigen binding protein is selected from the group consisting of CTLA-4 (Evibody), Lipocalins (Anticalin), a Protein A-derived molecule such as Z-domain of Protein A (Affibody), an A-domain (Avimer/Maxibody), a serum transferrin (*trans*-body); a designed ankyrin repeat protein (DARPin), a variable domain of antibody light chain or heavy chain (single-domain antibody, sdAb), a variable domain of antibody heavy chain (nanobody, aVH), $V_{NAR}$ fragments, a fibronectin (AdNectin), a C-type lectin domain (Tetranectin); a variable domain of a new antigen receptor beta-lactamase ($V_{NAR}$ fragments), a human gamma-crystallin or ubiquitin (Affilin molecules); a kunitz type domain of human protease inhibitors, microbodies such as the proteins from the knottin family, peptide aptamers and fibronectin (adnectin). CTLA-4 (Cytotoxic T Lymphocyte-associated Antigen 4) is a CD28-family receptor expressed on mainly CD4$^+$ T-cells. Its extracellular domain has a variable domain- like Ig fold. Loops corresponding to CDRs of antibodies can be substituted with heterologous sequence to confer different binding properties. CTLA-4 molecules engineered to have different binding specificities are also known as Evibodies (e.g. US7166697B1). Evibodies are around the same size as the isolated variable region of an antibody (e.g. a domain antibody). For further details see Journal of Immunological Methods 248 (1-2), 31-45 (2001). Lipocalins are a family of extracellular proteins which transport small hydrophobic molecules such as steroids, bilins, retinoids and lipids. They have a rigid beta-sheet secondary structure with a number of loops at the open end of the conical structure which can be engineered to bind to different target antigens. Anticalins are between 160-180 amino acids in size, and are derived from lipocalins. For further details see Biochim Biophys Acta 1482: 337-350 (2000), US7250297B1 and US20070224633. An affibody is a scaffold derived from Protein A of Staphylococcus aureus which can be engineered to bind to antigen. The domain consists of a three-helical bundle of approximately 58 amino acids. Libraries have been generated by randomization of surface residues. For further details see Protein Eng. Des. Sel. 2004, 17, 455-462 and EP 1641818A1. Avimers are multidomain proteins derived from the A-domain scaffold family. The native domains of approximately 35 amino acids adopt a defined disulfide bonded structure. Diversity is generated by shuffling of the natural variation exhibited by the family of A-domains. For further details see Nature Biotechnology 23(12), 1556 - 1561 (2005) and Expert Opinion on Investigational Drugs 16(6), 909-917 (June 2007). A transferrin is a monomeric serum transport glycoprotein. Transferrins can be engineered to bind different target antigens by insertion of peptide sequences in a permissive surface loop. Examples of engineered transferrin scaffolds include the Trans-body. For further details see J. Biol. Chem 274, 24066-24073 (1999). Designed Ankyrin Repeat Proteins (DARPins) are derived from Ankyrin which is a family of proteins that mediate attachment of integral membrane proteins to the cytoskeleton. A single ankyrin repeat is a 33 residue motif consisting of two alpha-helices and a beta-turn. They can be engineered to bind different target antigens by randomizing residues in the first alpha-helix and a beta-turn of each repeat. Their binding interface can be increased by increasing the number of modules (a method of affinity maturation). For further details see J. Mol. Biol. 332, 489-503 (2003), PNAS 100(4), 1700-1705 (2003) and J. Mol. Biol. 369, 1015-1028 (2007) and US20040132028A1. A single-domain antibody is an antibody fragment consisting of a single monomeric variable antibody domain. The first single domains were derived from the variable domain of the antibody heavy chain from camelids (nanobodies or $V_HH$ fragments). Furthermore, the term single-domain antibody includes an autonomous human heavy chain variable domain (aVH) or $V_{NAR}$ fragments derived from sharks. Fibronectin is a scaffold which can be engineered to bind to antigen. Adnectins consists of a backbone of the natural amino acid sequence of the 10th domain of the 15 repeating units of human fibronectin type III (FN3). Three loops at one end of the .beta.-sandwich can be engineered to enable an Adnectin to specifically recognize a therapeutic target of interest. For further details see Protein Eng. Des. Sel. 18, 435- 444 (2005), US20080139791, WO2005056764 and US6818418B1. Peptide aptamers are combinatorial recognition molecules that consist of a constant scaffold protein, typically thioredoxin (TrxA) which contains a constrained variable peptide loop inserted at the active site. For further details see Expert Opin. Biol. Ther. 5, 783-797 (2005). Microbodies are derived from naturally occurring microproteins of 25-50 amino acids in length which contain 3-4 cysteine bridges - examples of microproteins include KalataBI and conotoxin and knottins. The microproteins have a loop which can beengineered to include upto 25 amino acids without affecting the overall fold of the microprotein. For further details of engineered knottin domains, see WO2008098796.

An "**antigen binding molecule that binds to the same epitope**" as a reference molecule refers to an antigen binding molecule that blocks binding of the reference molecule to its antigen in a competition assay by 50% or more, and conversely, the reference molecule blocks binding of the antigen binding molecule to its antigen in a competition assay by 50% or more.

The term "**antigen binding domain**" refers to the part of an antigen binding molecule that comprises the area which specifically binds to and is complementary to part or all of an antigen. Where an antigen is large, an antigen binding molecule may only bind to a particular part of the antigen, which part is termed an epitope. An antigen binding domain may be provided by, for example, one or more variable domains (also called variable regions). Preferably, an antigen binding domain comprises an antibody light chain variable region (VL) and an antibody heavy chain variable region (VH).

As used herein, the term "**antigenic determinant**" is synonymous with "antigen" and "epitope," and refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM). The proteins useful as antigens herein can be any native form the proteins from any vertebrate source, including mammals such as primates (e.g. humans) and rodents (e.g. mice and rats), unless otherwise indicated. In a particular embodiment the antigen is a human protein. Where reference is made to a specific protein herein, the term encompasses the "full-length", unprocessed protein as well as any form of the protein that results from processing in the cell. The term also encompasses naturally occurring variants of the protein, e.g. splice variants or allelic variants.

By "**specific binding**" is meant that the binding is selective for the antigen and can be discriminated from unwanted or non-specific interactions. The ability of an antigen binding molecule to bind to a specific antigen can be measured either through an enzyme-linked immunosorbent assay (ELISA) or other techniques familiar to one of skill in the art, e.g. Surface Plasmon Resonance (SPR) technique (analyzed on a BIAcore instrument) (Liljeblad et al., Glyco J 17, 323-329 (2000)), and traditional binding assays (Heeley, Endocr Res 28, 217-229 (2002)). In one embodiment, the extent of binding of an antigen binding molecule to an unrelated protein is less than about 10% of the binding of the antigen binding molecule to the antigen as measured, e.g. by SPR. In certain embodiments, an molecule that binds to the antigen has a dissociation constant (Kd) of $\leq 1 \mu M$, $\leq 100$ nM, $\leq 10$ nM, $\leq 1$ nM, $\leq 0.1$ nM, $\leq 0.01$ nM, or $\leq 0.001$ nM (e.g. $10^{-8}$ M or less, e.g. from $10^{-8}$ M to $10^{-13}$ M, e.g. from $10^{-9}$ M to $10^{-13}$ M).

"**Affinity**" or "binding affinity" refers to the strength of the sum total of non-covalent interactions between a single binding site of a molecule (e.g. an antibody) and its binding partner (e.g. an antigen). Unless indicated otherwise, as used herein, "binding affinity" refers to intrinsic binding affinity which reflects a 1:1 interaction between members of a binding pair (e.g. antibody and antigen). The affinity of a molecule X for its partner Y can generally be represented by the dissociation constant (Kd), which is the ratio of dissociation and association rate constants (koff and kon, respectively). Thus, equivalent affinities may comprise different rate constants, as long as the ratio of the rate constants remains the same. Affinity can be measured by common methods known in the art, including those described herein. A particular method for measuring affinity is Surface Plasmon Resonance (SPR).

A "**tumor-associated antigen**" or TAA as used herein refers to an antigenic determinant presented on the surface of a target cell, for example a cell in a tumor such as a cancer cell or a cell of the tumor stroma. In certain embodiments, the target cell antigen is an antigen on the surface of a tumor cell. In one embodiment, TAA is selected from the group consisting of Fibroblast Activation Protein (FAP), Carcinoembryonic Antigen (CEA), Folate receptor alpha (FolR1), Melanoma-associated Chondroitin Sulfate Proteoglycan (MCSP), Epidermal Growth Factor Receptor (EGFR), human epidermal growth factor receptor 2 (HER2) and p95HER2. In particular, the tumor-associated antigen is Fibroblast Activation Protein (FAP).

The term "**Fibroblast activation protein (FAP)**", also known as Prolyl endopeptidase FAP or Seprase (EC 3.4.21), refers to any native FAP from any vertebrate source, including mammals such as primates (e.g. humans) non-human primates (e.g. cynomolgus monkeys) and rodents (e.g. mice and rats), unless otherwise indicated. The term encompasses "full-length," unprocessed FAP as well as any form of FAP that results from processing in the cell. The term also encompasses naturally occurring variants of FAP, e.g., splice variants or allelic variants. In one embodiment, the antigen binding molecule of the invention is capable of specific binding to human, mouse and/or cynomolgus FAP. The amino acid sequence of human FAP is shown in UniProt (www.uniprot.org) accession no. Q12884 (version 149, SEQ ID NO:79), or NCBI (www.ncbi.nlm.nih.gov/) RefSeq NP_004451.2. The extracellular domain (ECD) of human FAP extends from amino acid position 26 to 760. The amino acid sequence of a His-tagged human FAP ECD is shown in SEQ ID NO 80. The amino acid sequence of mouse FAP is shown in UniProt accession no. P97321 (version 126, SEQ ID NO:81), or NCBI RefSeq NP_032012.1. The extracellular domain (ECD) of mouse FAP extends from amino acid position 26 to 761. SEQ ID NO 82 shows the amino acid sequence of a His-tagged mouse FAP ECD. SEQ ID NO 83 the amino acid sequence, of a His-tagged cynomolgus FAP ECD. Preferably, an anti-FAP binding molecule of the invention binds to the extracellular domain of FAP.

The term "**Carcinoembroynic antigen (CEA)**", also known as Carcinoembryonic antigen-related cell adhesion molecule 5 (CEACAM5), refers to any native CEA from any vertebrate source, including mammals such as primates (e.g. humans) non-human primates (e.g. cynomolgus monkeys) and rodents (e.g. mice and rats), unless otherwise indicated. The amino acid sequence of human CEA is shown in UniProt accession no. P06731 (version 151, SEQ ID NO:84). CEA has long been identified as a tumor-associated antigen (Gold and Freedman, J Exp Med., 121:439-462, 1965; Berinstein N. L., J Clin Oncol., 20:2197-2207, 2002). Originally classified as a protein expressed only in fetal tissue, CEA has now been identified in several normal adult tissues. These tissues are primarily epithelial in origin, including cells of the gastrointestinal, respiratory, and urogential tracts, and cells of colon, cervix, sweat glands, and prostate (Nap et al., Tumour Biol., 9(2-3):145-53, 1988; Nap et al., Cancer Res., 52(8):2329-23339, 1992). Tumors of epithelial origin, as well as their metastases, contain CEA as a tumor associated antigen. While the presence of CEA itself does not indicate transformation to a cancerous cell, the distribution of CEA is indicative. In normal tissue, CEA is generally expressed on the apical surface of the cell (Hammarström S., Semin Cancer Biol. 9(2):67-81 (1999)), making it inaccessible to antibody in the blood stream. In contrast to normal tissue, CEA tends to be expressed over the entire surface of cancerous cells (Hammarström S., Semin Cancer Biol. 9(2):67-81 (1999)). This change of expression pattern makes CEA accessible to antibody binding in cancerous cells. In addition, CEA expression increases in cancerous cells. Furthermore, increased CEA expression promotes increased intercellular adhesions, which may lead to metastasis (Marshall J., Semin Oncol., 30(a Suppl. 8):30-6, 2003). The prevalence of CEA expression in various tumor entities is generally very high. In concordance with published data, own analyses performed in tissue samples confirmed its high prevalence, with approximately 95% in colorectal carcinoma (CRC), 90% in pancreatic cancer, 80% in gastric cancer, 60% in non-small cell lung cancer (NSCLC, where it is co-expressed with HER3), and 40% in breast cancer; low expression was found in small cell lung cancer and glioblastoma.

CEA is readily cleaved from the cell surface and shed into the blood stream from tumors, either directly or via the lymphatics. Because of this property, the level of serum CEA has been used as a clinical marker for diagnosis of cancers and screening for recurrence of cancers, particularly colorectal cancer (Goldenberg D M., The International Journal of Biological Markers, 7:183-188, 1992; Chau I., et al., J Clin Oncol., 22:1420-1429, 2004; Flamini et al., Clin Cancer Res; 12(23):6985-6988, 2006).

The term "**FolR1**" refers to Folate receptor alpha and has been identified as a potential prognostic and therapeutic target in a number of cancers. It refers to any native FolR1 from any vertebrate source, including mammals such as primates (e.g. humans) non-human primates (e.g. cynomolgus monkeys) and rodents (e.g. mice and rats), unless otherwise indicated. The amino acid sequence of human FolR1 is shown in UniProt accession no. P15328 (SEQ ID NO: 85), murine FolR1 has the amino acid sequence of UniProt accession no. P35846 (SEQ ID NO:86) and cynomolgus FolR1 has the amino acid sequence as shown in UniProt accession no. G7PR14 (SEQ ID NO:87). FolR1 is an N-glycosylated protein expressed on plasma membrane of cells. FolR1 has a high affinity for folic acid and for several reduced folic acid derivatives and mediates delivery of the physiological folate, 5-methyltetrahydrofolate, to the interior of cells. FOLR1 is a desirable target for FOLR1-directed cancer therapy as it is overexpressed in vast majority of ovarian cancers, as well as in many uterine, endometrial, pancreatic, renal, lung, and breast cancers, while the expression of FOLR1 on normal tissues is restricted to the apical membrane of epithelial cells in the kidney proximal tubules, alveolar pneumocytes of the lung, bladder, testes, choroid plexus, and thyroid. Recent studies have identified that FolR1 expression is particularly high in triple negative breast cancers (Necela et al. PloS One 2015, 10(3), e0127133).

The term "**Melanoma-associated Chondroitin Sulfate Proteoglycan (MCSP)**", also known as Chondroitin Sulfate Proteoglycan 4 (CSPG4) refers to any native MCSP from any vertebrate source, including mammals such as primates (e.g. humans) non-human primates (e.g. cynomolgus monkeys) and rodents (e.g. mice and rats), unless otherwise indicated. The amino acid sequence of human MCSP is shown in UniProt accession no. Q6UVK1 (version 103, SEQ ID NO:88). MCSP is a highly glycosylated integral membrane chondroitin sulfate proteoglycan consisting of an N-linked 280 kDa glycoprotein component and a 450-kDa chondroitin sulfate proteoglycan component expressed on the cell membrane (Ross et al., Arch. Biochem. Biophys. 1983, 225:370-38). MCSP is more broadly distributed in a number of normal and transformed cells. In particular, MCSP is found in almost all basal cells of the epidermis. MCSP is differentially expressed in melanoma cells, and was found to be expressed in more than 90% of benign nevi and melanoma lesions analyzed. MCSP has also been found to be expressed in tumors of nonmelanocytic origin, including basal cell carcinoma, various tumors of neural crest origin, and in breast carcinomas.

The term "**Epidermal Growth Factor Receptor (EGFR)**", also named Proto-oncogene c-ErbB-1 or Receptor tyrosine-protein kinase erbB-1, refers to any native EGFR from any vertebrate source, including mammals such as primates (e.g. humans) non-human primates (e.g. cynomolgus monkeys) and rodents (e.g. mice and rats), unless otherwise indicated. The amino acid sequence of human EGFR is shown in UniProt accession no. P00533 (version 211, SEQ ID NO:89). The proto-oncogene "**HER2**", (human epidermal growth factor receptor 2) encodes a protein tyrosine kinase (p185HER2) that is related to and somewhat homologous to the human epidermal growth factor receptor. HER2 is also known in the field as c-erbB-2, and sometimes by the name of the rat homolog, neu. Amplification and/or overexpression of HER2 is associated with multiple human malignancies and appears to be integrally involved in progression of 25-30% of human breast and ovarian cancers. Furthermore, the extent of amplification is inversely correlated with the observed median patient survival time (Slamon, D. J. et al., Science 244:707-712 (1989)). The amino acid sequence of human HER2 is shown in UniProt accession no. P04626 (version 230, SEQ ID NO:90). The term "**p95HER2**" as used herein refers to a carboxy terminal fragment (CTF) of the HER2 receptor protein, which is also known as "611-CTF" or "100-115 kDa p95HER2". The p95HER2 fragment is generated in the cell through initiation of translation of the HER2 mRNA at codon position 611 of the full-length HER2 molecule (Anido et al, EMBO J 25; 3234-44 (2006)). It has a molecular weight of 100 to 115 kDa and is expressed at the cell membrane, where it can form homodimers maintained by intermolecular disulfide bonds (Pedersen et al., Mol Cell Biol 29, 3319-31 (2009)). An exemplary sequence of human p95HER2 is given in SEQ ID NO: 91.

The term "**ICOS**" (Inducible T cell COStimulator) refers to any Inducible T cell costimulatory protein from any vertebrate source, including mammals such as primates (e.g. humans) non-human primates (e.g. cynomolgus monkeys) and rodents (e.g. mice and rats), unless otherwise indicated. ICOS, also named AILIM or CD278, is a member of the CD28 superfamily (CD28/CTLA-4 cell-surface receptor family) and is specifically expressed on T cells after initial T cell activation. ICOS also plays a role in the development and function of other T cell subsets, including Th1, Th2, and Th17. Notably, ICOS co-stimulates T cell proliferation and cytokine secretion associated with both Th1 and Th2 cells. Accordingly, ICOS KO mice demonstrate impaired development of autoimmune phenotypes in a variety of disease models, including diabetes (Th1), airway inflammation (Th2) and EAE neuro-inflammatory models (Th17). In addition to its role in modulating T effector (Teff) cell function, ICOS also modulates T regulatory cells (Tregs). ICOS is expressed at high levels on Tregs, and has been implicated in Treg homeostasis and function. Upon activation, ICOS, a disulfide-linked homodimer, induces a signal through the PI3K and AKT pathways. Subsequent signaling events result in expression of lineage specific transcription factors (e.g., T-bet, GATA-3) and, in turn, effects on T cell proliferation and survival. The term also encompasses naturally occurring variants of ICOS, e.g., splice variants or allelic variants. The amino acid sequence of human ICOS is shown in UniProt (www.uniprot.org) accession no. Q9Y6W8 (SEQ ID NO:3)

As described herein before, **ICOS ligand** (ICOS-L; B7-H2; B7RP-1; CD275; GL50), also a member of the B7 superfamily, is the membrane bound natural ligand for ICOS and is expressed on the cell surface of B cells, macrophages and dendritic cells. ICOS-L functions as a non-covalently linked homodimer on the cell surface in its interaction with ICOS. Human ICOS-L has also been reported to bind to human CD28 and CTLA-4 (Yao et al., 2011, Immunity, 34: 729-740). An exemplary amino acid sequence of the ectodomain of huICOS-L is given in SEQ ID NO: 1, an exemplary amino acid sequence of murine ICOS-L (muICOS-L) is provided in SEQ ID NO:2.

The term "**variable region**" or "variable domain" refers to the domain of an antibody heavy or light chain that is involved in binding the antigen binding molecule to antigen. The variable domains of the heavy chain and light chain (VH and VL, respectively) of a native antibody generally have similar structures, with each domain comprising four conserved framework regions (FRs) and three hypervariable regions (HVRs). See, e.g., Kindt et al., Kuby Immunology, 6th ed., W.H. Freeman and Co., page 91 (2007). A single VH or VL domain may be sufficient to confer antigen-binding specificity.

The term "**hypervariable region**" or "HVR," as used herein refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (H1), 53-55 (H2), and 96-101 (H3). (Chothia and Lesk, J. Mol. Biol. 196:901-917 (1987).) Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat et al., Sequences of Proteins of Immunological Interest, 5th Ed. Public Health Service, National Institutes of Health, Bethesda, MD (1991).) Hypervariable regions (HVRs) are also referred to as complementarity determining regions (CDRs), and these terms are used herein interchangeably in reference to portions of the variable region that form the antigen binding regions. This particular region has been described by Kabat et al., U.S. Dept. of Health and Human Services, "Sequences of Proteins of Immunological Interest" (1983) and by Chothia et al., J. Mol. Biol. 196:901-917 (1987), where the definitions include overlapping or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth below in Table A as a comparison. The exact residue numbers which encompass a particular CDR will vary depending on the sequence and size of the CDR. Those skilled in the art can routinely determine which residues comprise a particular CDR given the variable region amino acid sequence of the antibody.

**TABLE A. CDR Definitions[1]**

| CDR | Kabat | Chothia | AbM[2] |
|---|---|---|---|
| $V_H$CDR1 | 31-35 | 26-32 | 26-35 |
| $V_H$ CDR2 | 50-65 | 52-58 | 50-58 |
| $V_H$ CDR3 | 95-102 | 95-102 | 95-102 |
| $V_L$CDR1 | 24-34 | 26-32 | 24-34 |
| $V_L$ CDR2 | 50-56 | 50-52 | 50-56 |

(continued)

| CDR | Kabat | Chothia | AbM[2] |
|---|---|---|---|
| $V_L$ CDR3 | 89-97 | 91-96 | 89-97 |

Numbering of all CDR definitions in Table A is according to the numbering conventions set forth by Kabat et al. (see below).
[2] "AbM" with a lowercase "b" as used in Table A refers to the CDRs as defined by Oxford Molecular's "AbM" antibody modeling software.

Kabat *et al.* also defined a numbering system for variable region sequences that is applicable to any antibody. One of ordinary skill in the art can unambiguously assign this system of "Kabat numbering" to any variable region sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983). Unless otherwise specified, references to the numbering of specific amino acid residue positions in an antibody variable region are according to the Kabat numbering system.

With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-LI, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3. (See Almagro and Fransson, Front. Biosci. 13:1619-1633 (2008).) Unless otherwise indicated, HVR residues and other residues in the variable domain (e.g., FR residues) are numbered herein according to Kabat et al., *supra.*

As used herein, the term "**affinity matured**" in the context of antigen binding molecules (e.g., antibodies) refers to an antigen binding molecule that is derived from a reference antigen binding molecule, e.g., by mutation, binds to the same antigen, preferably binds to the same epitope, as the reference antibody; and has a higher affinity for the antigen than that of the reference antigen binding molecule. Affinity maturation generally involves modification of one or more amino acid residues in one or more CDRs of the antigen binding molecule. Typically, the affinity matured antigen binding molecule binds to the same epitope as the initial reference antigen binding molecule.

"**Framework**" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

An "**acceptor human framework**" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

The term "**chimeric**" antibody refers to an antibody in which a portion of the heavy and/or light chain is derived from a particular source or species, while the remainder of the heavy and/or light chain is derived from a different source or species.

The "**class**" of an antibody refers to the type of constant domain or constant region possessed by its heavy chain. There are five major classes of antibodies: IgA, IgD, IgE, IgG, and IgM, and several of these may be further divided into subclasses (isotypes), e.g. $IgG_1$, $IgG_2$, $IgG_3$, $IgG_4$, $IgA_1$, and $IgA_2$. The heavy chain constant domains that correspond to the different classes of immunoglobulins are called $\alpha$, $\delta$, $\varepsilon$, $\gamma$, and $\mu$ respectively..

A "**humanized**" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "**humanized form**" of an antibody, e.g., a non-human antibody, refers to an antibody that has undergone humanization. Other forms of "humanized antibodies" encompassed by the present invention are those in which the constant region has been additionally modified or changed from that of the original antibody to generate the properties according to the invention, especially in regard to C1q binding and/or Fc receptor (FcR) binding.

A "**human**" antibody is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

The term "**Fc domain**" or "**Fc region**" herein is used to define a C-terminal region of an antibody heavy chain that contains at least a portion of the constant region. The term includes native sequence Fc regions and variant Fc regions. An IgG Fc region comprises an IgG CH2 and an IgG CH3 domain. The "CH2 domain" of a human IgG Fc region usually extends from an amino acid residue at about position 231 to an amino acid residue at about position 340. In one embodiment, a carbohydrate chain is attached to the CH2 domain. The CH2 domain herein may be a native sequence CH2 domain or variant CH2 domain. The "CH3 domain" comprises the stretch of residues C-terminal to a CH2 domain in an Fc region (i.e. from an amino acid residue at about position 341 to an amino acid residue at about position 447 of an IgG). The CH3 region herein may be a native sequence CH3 domain or a variant CH3 domain (e.g. a CH3 domain with an introduced "protuberance" ("knob") in one chain thereof and a corresponding introduced "cavity" ("hole") in the other chain thereof; see US Patent No. 5,821,333, expressly incorporated herein by reference). Such variant CH3 domains may be used to promote heterodimerization of two non-identical antibody heavy chains as herein described. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

The "**knob-into-hole**" technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine). The protuberance and cavity can be made by altering the nucleic acid encoding the polypeptides, e.g. by site-specific mutagenesis, or by peptide synthesis. In a specific embodiment a knob modification comprises the amino acid substitution T366W in one of the two subunits of the Fc domain, and the hole modification comprises the amino acid substitutions T366S, L368A and Y407V in the other one of the two subunits of the Fc domain. In a further specific embodiment, the subunit of the Fc domain comprising the knob modification additionally comprises the amino acid substitution S354C, and the subunit of the Fc domain comprising the hole modification additionally comprises the amino acid substitution Y349C. Introduction of these two cysteine residues results in the formation of a disulfide bridge between the two subunits of the Fc region, thus further stabilizing the dimer (Carter, J Immunol Methods 248, 7-15 (2001)).

A "region equivalent to the Fc region of an immunoglobulin" is intended to include naturally occurring allelic variants of the Fc region of an immunoglobulin as well as variants having alterations which produce substitutions, additions, or deletions but which do not decrease substantially the ability of the immunoglobulin to mediate effector functions (such as antibody-dependent cellular cytotoxicity). For example, one or more amino acids can be deleted from the N-terminus or C-terminus of the Fc region of an immunoglobulin without substantial loss of biological function. Such variants can be selected according to general rules known in the art so as to have minimal effect on activity (see, e.g., Bowie, J. U. et al., Science 247:1306-10 (1990)).

The term "**effector functions**" refers to those biological activities attributable to the Fc region of an antibody, which vary with the antibody isotype. Examples of antibody effector functions include: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen presenting cells, down regulation of cell surface receptors (e.g. B cell receptor), and B cell activation.

Fc receptor binding dependent effector functions can be mediated by the interaction of the Fc-region of an antibody with Fc receptors (FcRs), which are specialized cell surface receptors on hematopoietic cells. Fc receptors belong to the immunoglobulin superfamily, and have been shown to mediate both the removal of antibody-coated pathogens by phagocytosis of immune complexes, and the lysis of erythrocytes and various other cellular targets (e.g. tumor cells) coated with the corresponding antibody, via antibody dependent cell mediated cytotoxicity (ADCC) (see e.g. Van de Winkel, J.G. and Anderson, C.L., J. Leukoc. Biol. 49 (1991) 511-524). FcRs are defined by their specificity for immunoglobulin isotypes: Fc receptors for IgG antibodies are referred to as FcγR. Fc receptor binding is described e.g. in Ravetch, J.V. and Kinet, J.P., Annu. Rev. Immunol. 9 (1991) 457-492; Capel, P.J., et al., Immunomethods 4 (1994) 25-34; de Haas, M., et al., J. Lab. Clin. Med. 126 (1995) 330-341; and Gessner, J.E., et al., Ann. Hematol. 76 (1998) 231-248.

Cross-linking of receptors for the Fc-region of IgG antibodies (FcγR) triggers a wide variety of effector functions including phagocytosis, antibody-dependent cellular cytotoxicity, and release of inflammatory mediators, as well as immune complex clearance and regulation of antibody production. In humans, three classes of FcγR have been characterized, which are:

- FcγRI (CD64) binds monomeric IgG with high affinity and is expressed on macrophages, monocytes, neutrophils and eosinophils. Modification in the Fc-region IgG at least at one of the amino acid residues E233-G236, P238, D265, N297, A327 and P329 (numbering according to EU index of Kabat) reduce binding to FcγRI. IgG2 residues at positions 233-236, substituted into IgG1 and IgG4, reduced binding to FcγRI by 103-fold and eliminated the human monocyte response to antibody-sensitized red blood cells (Armour, K.L., et al., Eur. J. Immunol. 29 (1999) 2613-2624).

- FcγRII (CD32) binds complexed IgG with medium to low affinity and is widely expressed. This receptor can be divided into two sub-types, FcγRIIA and FcγRIIB. FcγRIIA is found on many cells involved in killing (e.g. macrophages, monocytes, neutrophils) and seems able to activate the killing process. FcγRIIB seems to play a role in inhibitory processes and is found on B cells, macrophages and on mast cells and eosinophils. On B-cells it seems to function to suppress further immunoglobulin production and isotype switching to, for example, the IgE class. On macrophages, FcγRIIB acts to inhibit phagocytosis as mediated through FcγRIIA. On eosinophils and mast cells the B-form may help to suppress activation of these cells through IgE binding to its separate receptor. Reduced binding for FcγRIIA is found e.g. for antibodies comprising an IgG Fc-region with mutations at least at one of the amino acid residues E233-G236, P238, D265, N297, A327, P329, D270, Q295, A327, R292, and K414 (numbering according to EU index of Kabat).

- FcγRIII (CD16) binds IgG with medium to low affinity and exists as two types. FcγRIIIA is found on NK cells, macrophages, eosinophils and some monocytes and T cells and mediates ADCC. FcγRIIIB is highly expressed on neutrophils. Reduced binding to FcγRIIIA is found e.g. for antibodies comprising an IgG Fc-region with mutation at least at one of the amino acid residues E233-G236, P238, D265, N297, A327, P329, D270, Q295, A327, S239, E269, E293, Y296, V303, A327, K338 and D376 (numbering according to EU index of Kabat).

Mapping of the binding sites on human IgG1 for Fc receptors, the above mentioned mutation sites and methods for measuring binding to FcγRI and FcγRIIA are described in Shields, R.L., et al. J. Biol. Chem. 276 (2001) 6591-6604.

The term "**ADCC**" or "antibody-dependent cellular cytotoxicity" is a function mediated by Fc receptor binding and refers to lysis of target cells by an antibody as reported herein in the presence of effector cells. The capacity of the antibody to induce the initial steps mediating ADCC is investigated by measuring their binding to Fcγ receptors expressing cells, such as cells, recombinantly expressing FcγRI and/or FcγRIIA or NK cells (expressing essentially FcγRIIIA). In particular, binding to FcγR on NK cells is measured.

An "**activating Fc receptor**" is an Fc receptor that following engagement by an Fc region of an antibody elicits signaling events that stimulate the receptor-bearing cell to perform effector functions. Activating Fc receptors include FcγRIIIa (CD16a), FcγRI (CD64), FcγRIIa (CD32), and FcaRI (CD89). A particular activating Fc receptor is human FcγRIIIa (see UniProt accession no. P08637, version 141).

An "**ectodomain**" is the domain of a membrane protein that extends into the extracellular space (i.e. the space outside the target cell). Ectodomains are usually the parts of proteins that initiate contact with surfaces, which leads to signal transduction.

The term "**peptide linker**" refers to a peptide comprising one or more amino acids, typically about 2 to 20 amino acids. Peptide linkers are known in the art or are described herein. Suitable, non-immunogenic linker peptides are, for example, $(G_4S)_n$, $(SG_4)_n$ or $G_4(SG_4)_n$ peptide linkers, wherein "n" is generally a number between 1 and 10, typically between 2 and 4, in particular 2, i.e. the peptides selected from the group consisting of GGGGS (SEQ ID NO: 92) GGGGSGGGGS (SEQ ID NO:93), SGGGGSGGGG (SEQ ID NO:94) and GGGGSGGGGSGGGG (SEQ ID NO:95), but also include the sequences GSPGSSSSGS (SEQ ID NO:96), $(G4S)_3$ (SEQ ID NO:97), $(G4S)_4$ (SEQ ID NO:98), GSGSGSGS (SEQ ID NO:99), GSGSGNGS (SEQ ID NO:100), GGSGSGSG (SEQ ID NO:101), GGSGSG (SEQ ID NO:102), GGSG (SEQ ID NO:103), GGSGNGSG (SEQ ID NO:104), GGNGSGSG (SEQ ID NO:105) and GGNGSG (SEQ ID NO: 106). Peptide linkers of particular interest are (G4S) (SEQ ID NO:92), $(G_4S)_2$ or GGGGSGGGGS (SEQ ID NO:93), $(G4S)_3$ (SEQ ID NO:97) and $(G4S)_4$ (SEQ ID NO:98).

The term "**amino acid**" as used within this application denotes the group of naturally occurring carboxy $\alpha$-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

By "fused" or "connected" is meant that the components (e.g. a polypeptide and an ectodomain of said TNF ligand family member) are linked by peptide bonds, either directly or via one or more peptide linkers.

"**Percent (%) amino acid sequence identity**" with respect to a reference polypeptide (protein) sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN. SAWI or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

In certain embodiments, **amino acid sequence variants** of the agonistic ICOS-binding molecules provided herein are contemplated. For example, it may be desirable to improve the binding affinity and/or other biological properties of the agonistic ICOS-binding molecules. Amino acid sequence variants of the agonistic ICOS-binding molecules may be prepared by introducing appropriate modifications into the nucleotide sequence encoding the molecules, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding. Sites of interest for substitutional mutagenesis include the HVRs and Framework (FRs). Conservative substitutions are provided in Table B under the heading "Preferred Substitutions" and further described below in reference to amino acid side chain classes (1) to (6). Amino acid substitutions may be introduced into the molecule of interest and the products screened for a desired activity, e.g., retained/improved antigen binding, decreased immunogenicity, or improved ADCC or CDC.

**TABLE B**

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gin |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |

(continued)

| Original Residue | Exemplary Substitutions | Preferred Substitutions |
|---|---|---|
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;

(2) neutral hydrophilic: Cys, Ser, Thr, Asn, Gln;

(3) acidic: Asp, Glu;

(4) basic: His, Lys, Arg;

(5) residues that influence chain orientation: Gly, Pro;

(6) aromatic: Trp, Tyr, Phe.

Non-conservative substitutions will entail exchanging a member of one of these classes for another class.

The term "**amino acid sequence variants**" includes substantial variants wherein there are amino acid substitutions in one or more hypervariable region residues of a parent antigen binding molecule (*e.g.* a humanized or human antibody). Generally, the resulting variant(s) selected for further study will have modifications (e.g., improvements) in certain biological properties (e.g., increased affinity, reduced immunogenicity) relative to the parent antigen binding molecule and/or will have substantially retained certain biological properties of the parent antigen binding molecule. An exemplary substitutional variant is an affinity matured antibody, which may be conveniently generated, e.g., using phage display-based affinity maturation techniques such as those described herein. Briefly, one or more HVR residues are mutated and the variant antigen binding molecules displayed on phage and screened for a particular biological activity (e.g. binding affinity). In certain embodiments, substitutions, insertions, or deletions may occur within one or more HVRs so long as such alterations do not substantially reduce the ability of the antigen binding molecule to bind antigen. For example, conservative alterations (e.g., conservative substitutions as provided herein) that do not substantially reduce binding affinity may be made in HVRs. A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as Arg, Asp, His, Lys, and Glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antigen binding molecule complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of insertions include agonistic ICOS-binding molecules with a fusion to the N- or C-terminus to a polypeptide which increases the serum half-life of the agonistic ICOS-binding molecules.

In certain embodiments, the agonistic ICOS-binding molecules provided herein are altered to increase or decrease the extent to which the antibody is glycosylated. Glycosylation variants of the molecules may be conveniently obtained by altering the amino acid sequence such that one or more glycosylation sites is created or removed. Where the agonistic ICOS-binding molecule comprises an Fc domain, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in agonistic ICOS-binding molecules may be made in order to create variants with certain improved properties. In one aspect, variants of agonistic ICOS-binding molecules are provided having a carbohydrate structure that lacks fucose attached (directly or indirectly) to an Fc region. Such fucosylation variants may have improved ADCC function, see e.g. US Patent Publication Nos. US 2003/0157108 (Presta, L.) or US 2004/0093621 (Kyowa Hakko Kogyo Co., Ltd). Further variants of the agonistic ICOS-binding molecules of the invention include those with bisected oligosaccharides, e.g., in which a biantennary oligosaccharide attached to the Fc region is bisected by GlcNAc. Such variants may have reduced fucosylation and/or improved ADCC function., see for example WO 2003/011878 (Jean-Mairet et al.); US Patent No. 6,602,684 (Umana et al.); and US 2005/0123546 (Umana et al.). Variants with at least one galactose residue in the oligosaccharide attached to the Fc region are also provided. Such antibody variants may have improved CDC function and are described, e.g., in WO 1997/30087 (Patel et al.); WO 1998/58964 (Raju, S.); and WO 1999/22764 (Raju, S.).

In certain embodiments, it may be desirable to create **cysteine engineered variants** of the agonistic ICOS-binding molecules of the invention, e.g., "thioMAbs," in which one or more residues of the molecule are substituted with cysteine residues. In particular embodiments, the substituted residues occur at accessible sites of the molecule. By substituting those residues with cysteine, reactive thiol groups are thereby positioned at accessible sites of the antibody and may be used to conjugate the antibody to other moieties, such as drug moieties or linker-drug moieties, to create an immunoconjugate. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antigen binding molecules may be generated as described, e.g., in U.S. Patent No. 7,521,541.

In certain aspects, the agonistic ICOS-binding molecules provided herein may be further modified to contain additional non-proteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the bispecific antibody derivative will be used in a therapy under defined conditions, etc. In another aspect, conjugates of an antibody and non-proteinaceous moiety that may be selectively heated by exposure to radiation are provided. In one embodiment, the non-proteinaceous moiety is a carbon nanotube (Kam, N.W. et al., Proc. Natl. Acad. Sci. USA 102 (2005) 11600-11605). The radiation may be of any wavelength, and includes, but is not limited to, wavelengths that do not harm ordinary cells, but which heat the non-proteinaceous moiety to a temperature at which cells proximal to the antibody-non-proteinaceous moiety are killed. In another aspect, immunoconjugates of the agonistic ICOS-binding molecules provided herein maybe obtained. An "**immunoconjugate**" is an antibody conjugated to one or more heterologous molecule(s), including but not limited to a cytotoxic agent.

The term "**polynucleotide**" refers to an isolated nucleic acid molecule or construct, e.g. messenger RNA (mRNA), virally-derived RNA, or plasmid DNA (pDNA). A polynucleotide may comprise a conventional phosphodiester bond or a non-conventional bond (e.g. an amide bond, such as found in peptide nucleic acids (PNA). The term "nucleic acid molecule" refers to any one or more nucleic acid segments, e.g. DNA or RNA fragments, present in a polynucleotide.

By "**isolated**" nucleic acid molecule or polynucleotide is intended a nucleic acid molecule, DNA or RNA, which has been removed from its native environment. For example, a recombinant polynucleotide encoding a polypeptide contained in a vector is considered isolated for the purposes of the present invention. Further examples of an isolated polynucleotide include recombinant polynucleotides maintained in heterologous host cells or purified (partially or substantially) polynucleotides in solution. An isolated polynucleotide includes a polynucleotide molecule contained in cells that ordinarily contain the polynucleotide molecule, but the polynucleotide molecule is present extrachromosomally or at a chromosomal location that is different from its natural chromosomal location. Isolated RNA molecules include in vivo or in vitro RNA transcripts of the present invention, as well as positive and negative strand forms, and double-stranded forms. Isolated polynucleotides or nucleic acids according to the present invention further include such molecules produced synthetically. In addition, a polynucleotide or a nucleic acid may be or may include a regulatory element such as a promoter, ribosome binding site, or a transcription terminator.

By a nucleic acid or polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These alterations of the reference sequence may occur at the 5' or 3' terminal positions of the reference nucleotide sequence or anywhere between those terminal positions, interspersed either individually among residues in the reference sequence or in one or more contiguous groups within the reference sequence. As a practical matter, whether any particular polynucleotide sequence is at least 80%, 85%, 90%, 95%, 96%, 97%, 98% or 99% identical to a nucleotide sequence of the present invention can be determined conventionally using known computer programs, such as the ones discussed above for polypeptides (e.g. ALIGN-2).

The term "**expression cassette**" refers to a polynucleotide generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter. In certain embodiments, the expression cassette of the invention comprises polynucleotide sequences that encode bispecific antigen binding molecules of the invention or fragments thereof.

The term "**vector**" or "expression vector" is synonymous with "expression construct" and refers to a DNA molecule that is used to introduce and direct the expression of a specific gene to which it is operably associated in a target cell. The term includes the vector as a self-replicating nucleic acid structure as well as the vector incorporated into the genome of a host cell into which it has been introduced. The expression vector of the present invention comprises an expression cassette. Expression vectors allow transcription of large amounts of stable mRNA. Once the expression vector is inside the target cell, the ribonucleic acid molecule or protein that is encoded by the gene is produced by the cellular transcription and/or translation machinery. In one embodiment, the expression vector of the invention comprises an expression cassette that comprises polynucleotide sequences that encode bispecific antigen binding molecules of the invention or fragments thereof.

The terms "**host cell**", "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. A host cell is any type of cellular system that can be used to generate the bispecific antigen binding molecules of the present invention. Host cells include cultured cells, e.g. mammalian cultured cells, such as CHO cells, BHK cells, NS0 cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue.

An "**effective amount**" of an agent refers to the amount that is necessary to result in a physiological change in the cell or tissue to which it is administered.

A "**therapeutically effective amount**" of an agent, e.g. a pharmaceutical composition, refers to an amount effective, at dosages and for periods of time necessary, to achieve the desired therapeutic or prophylactic result. A therapeutically effective amount of an agent for example eliminates, decreases, delays, minimizes or prevents adverse effects of a disease.

An "**individual**" or "subject" is a mammal. Mammals include, but are not limited to, domesticated animals (e.g. cows, sheep, cats, dogs, and horses), primates (e.g. humans and non-human primates such as monkeys), rabbits, and rodents (e.g. mice and rats). Particularly, the individual or subject is a human.

The term "**pharmaceutical composition**" refers to a preparation which is in such form as to permit the biological activity of an active ingredient contained therein to be effective, and which contains no additional components which are unacceptably toxic to a subject to which the formulation would be administered.

A "**pharmaceutically acceptable excipient**" refers to an ingredient in a pharmaceutical composition, other than an active ingredient, which is nontoxic to a subject. A pharmaceutically acceptable excipient includes, but is not limited to, a buffer, a stabilizer, or a preservative.

The term "**package insert**" is used to refer to instructions customarily included in commercial packages of therapeutic products, that contain information about the indications, usage, dosage, administration, combination therapy, contraindications and/or warnings concerning the use of such therapeutic products.

As used herein, "**treatment**" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, the molecules of the invention are used to delay development of a disease or to slow the progression of a disease.

The term "**cancer**" as used herein refers to proliferative diseases, such as lymphomas, lymphocytic leukemias, lung cancer, non-small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma and Ewings sarcoma, including refractory versions of any of the above cancers, or a combination of one or more of the above cancers.

## Agonistic ICOS-binding molecules of the invention

The invention provides novel bispecific antigen binding molecules with particularly advantageous properties such as producibility, stability, binding affinity, biological activity, targeting efficiency, reduced toxicity, an extended dosage range that can be given to a patient and thereby a possibly enhanced efficacy.

## Exemplary agonistic ICOS-binding molecules comprising at least one antigen binding domain that binds to a tumor-associated antigen

In one aspect, the invention provides bispecific agonistic ICOS-binding molecules, comprising

(a) at least one antigen binding domain capable of specific binding to ICOS, and
(b) at least one antigen binding domain capable of specific binding to a tumor-associated antigen, and
(c) a Fc domain.

In a particular aspect, the agonistic ICOS-binding molecules comprise a Fc domain comprising mutations that reduce or abolish effector function. The use of a Fc domain comprising mutations that reduce or abolish effector function will prevent unspecific agonism by crosslinking via Fc receptors and will prevent ADCC of ICOS$^+$ cells.

The agonistic ICOS-binding molecules as described herein possess the advantage over conventional antibodies capable of specific binding to ICOS in that they selectively induce immune response at the target cells, which are typically cancer cells or tumor stroma. In one aspect, the tumor-associated antigen is selected from the group consisting of Fibroblast Activation Protein (FAP), Carcinoembryonic Antigen (CEA), Folate receptor alpha (FolR1), Melanoma-associated Chondroitin Sulfate Proteoglycan (MCSP), Epidermal Growth Factor Receptor (EGFR), human epidermal growth factor receptor 2 (HER2) and p95HER2. In particular, the tumor-associated antigen is FAP.

In particular, the agonistic ICOS-binding molecule comprises at least one antigen binding domain capable of specific binding to FAP comprising

(a) a heavy chain variable region ($V_H$FAP) comprising (i) CDR-H1 comprising the amino acid sequence of SEQ ID NO:4, (ii) CDR-H2 comprising the amino acid sequence of SEQ ID NO:5, and (iii) CDR-H3 comprising the amino acid sequence of SEQ ID NO:6, and a light chain variable region ($V_L$FAP) comprising (iv) CDR-L1 comprising the amino acid sequence of SEQ ID NO:7, (v) CDR-L2 comprising the amino acid sequence of SEQ ID NO:8, and (vi) CDR-L3 comprising the amino acid sequence of SEQ ID NO:9, or
(b) a heavy chain variable region ($V_H$FAP) comprising (i) CDR-H1 comprising the amino acid sequence of SEQ ID NO:12, (ii) CDR-H2 comprising the amino acid sequence of SEQ ID NO:13, and (iii) CDR-H3 comprising the amino acid sequence of SEQ ID NO:14, and a light chain variable region ($V_L$FAP) comprising (iv) CDR-L1 comprising the amino acid sequence of SEQ ID NO:15, (v) CDR-L2 comprising the amino acid sequence of SEQ ID NO:16, and (vi) CDR-L3 comprising the amino acid sequence of SEQ ID NO:17.

More particularly, the agonistic ICOS-binding molecule comprises at least one antigen binding domain capable of specific binding to FAP comprising a heavy chain variable region ($V_H$FAP) comprising (i) CDR-H1 comprising the amino acid sequence of SEQ ID NO:4, (ii) CDR-H2 comprising the amino acid sequence of SEQ ID NO:5, and (iii) CDR-H3 comprising the amino acid sequence of SEQ ID NO:6, and a light chain variable region ($V_L$FAP) comprising (iv) CDR-L1 comprising the amino acid sequence of SEQ ID NO:7, (v) CDR-L2 comprising the amino acid sequence of SEQ ID NO:8, and (vi) CDR-L3 comprising the amino acid sequence of SEQ ID NO:9.

In a specific aspect, the agonistic ICOS-binding molecule comprises at least one antigen binding domain capable of specific binding to FAP comprising a heavy chain variable region ($V_H$FAP) comprising an amino acid sequence that is at least about 95%, 96%, 97%, 98%, 99% or 100% identical to the amino acid sequence of SEQ ID NO:10 and a light chain variable region ($V_L$FAP) comprising an amino acid sequence that is at least about 95%, 96%, 97%, 98%, 99% or 100% identical to the amino acid sequence of SEQ ID NO:11, or at least one antigen binding domain capable of specific binding to FAP comprising a heavy chain variable region ($V_H$FAP) comprising an amino acid sequence that is at least about 95%, 96%, 97%, 98%, 99% or 100% identical to the amino acid sequence of SEQ ID NO:18 and a light chain variable region ($V_L$FAP) comprising an amino acid sequence that is at least about 95%, 96%, 97%, 98%, 99% or 100% identical to the amino acid sequence of SEQ ID NO:19. In a more specific aspect, the agonistic ICOS-binding molecule comprises at least one antigen binding domain capable of specific binding to FAP comprising a heavy chain variable region ($V_H$FAP) comprising an amino acid sequence of SEQ ID NO:10 and a light chain variable region ($V_L$FAP) comprising an amino acid sequence of SEQ ID NO:11 or at least one antigen binding domain capable of specific binding to FAP comprising a heavy chain variable region ($V_H$FAP) comprising an amino acid sequence of SEQ ID NO:18 and a light chain variable region ($V_L$FAP) comprising an amino acid sequence of SEQ ID NO:19. More particularly, the agonistic ICOS-binding molecule comprises at least one antigen binding domain capable of specific binding to FAP comprising a heavy chain variable region ($V_H$FAP) comprising an amino acid sequence of SEQ ID NO:10 and a light chain variable region ($V_L$FAP) comprising an amino acid sequence of SEQ ID NO:11.

In a further aspect, the agonistic ICOS-binding molecule comprises at least one antigen binding domain capable of specific binding to ICOS comprising a heavy chain variable region ($V_H$ICOS) comprising (i) CDR-H1 comprising the amino acid sequence of SEQ ID NO:20, (ii) CDR-H2 comprising the amino acid sequence of SEQ ID NO:21, and (iii) CDR-H3 comprising the amino acid sequence of SEQ ID NO:22, and a light chain variable region ($V_L$ICOS) comprising (iv) CDR-L1 comprising the amino acid sequence of SEQ ID NO:23, (v) CDR-L2 comprising the amino acid sequence of SEQ ID NO:24, and (vi) CDR-L3 comprising the amino acid sequence of SEQ ID NO:25.

More particularly, the agonistic ICOS-binding molecule comprises at least one antigen binding domain that binds to a tumor-associated antigen comprises at least one antigen binding domain comprising a heavy chain variable region ($V_H$ICOS) comprising an amino acid sequence that is at least about 95%, 96%, 97%, 98%, 99% or 100% identical to the amino acid sequence of SEQ ID NO:26 and a light chain variable region ($V_L$ICOS) comprising an amino acid sequence that is at least about 95%, 96%, 97%, 98%, 99% or 100% identical to the amino acid sequence of SEQ ID NO:27. More specifically, the agonistic ICOS-binding molecule comprises at least one antigen binding domain that binds to a tumor-associated antigen comprises at least one antigen binding domain comprising a heavy chain variable region ($V_H$ICOS) comprising an amino acid sequence of SEQ ID NO:26 and a light chain variable region ($V_L$ICOS) comprising an amino acid sequence of SEQ ID NO:27.

In one aspect, provided is an agonistic ICOS-binding molecule, comprising

(a) at least one antigen binding domain capable of specific binding to ICOS comprising a heavy chain variable region ($V_H$ICOS) comprising an amino acid sequence of SEQ ID NO:26 and a light chain variable region ($V_L$ICOS) comprising an amino acid sequence of SEQ ID NO:27, and
(b) at least one antigen binding domain capable of specific binding to a tumor-associated antigen comprising a heavy chain variable region ($V_H$FAP) comprising an amino acid sequence of SEQ ID NO:10 and a light chain variable region ($V_L$FAP) comprising an amino acid sequence of SEQ ID NO:11 or comprising a heavy chain variable region ($V_H$FAP) comprising an amino acid sequence of SEQ ID NO:18 and a light chain variable region ($V_L$FAP) comprising an amino acid sequence of SEQ ID NO:19.

In one aspect, the invention provides bispecific agonistic ICOS-binding molecules, comprising (a) one antigen binding domain capable of specific binding to ICOS, and (b) one antigen binding domain capable of specific binding to a tumor-associated antigen, and (c) a Fc domain. Thus, in this case the agonistic ICOS-binding molecule is monovalent for the binding to ICOS and monovalent for the binding to the tumor-associated antigen (1+1 format).

In a particular aspect, provided is an agonistic ICOS-binding molecule, wherein said molecule comprises (a) a first Fab fragment capable of specific binding to ICOS, (b) a second Fab fragment capable of specific binding to a tumor-associated antigen, and (c) a Fc domain composed of a first and a second subunit capable of stable association with each other.

More particularly, provided is a bispecific antigen binding molecule, wherein said molecule comprises

(i) a first Fab fragment capable of specific binding to ICOS, comprising a heavy chain variable region (V$_H$ICOS) comprising an amino acid sequence of SEQ ID NO:26 and a light chain variable region (V$_L$ICOS) comprising an amino acid sequence of SEQ ID NO:27, and

(ii) a second Fab fragment capable of specific binding to FAP, comprising a heavy chain variable region (V$_H$FAP) comprising an amino acid sequence of SEQ ID NO:10 and a light chain variable region (V$_L$FAP) comprising an amino acid sequence of SEQ ID NO:11 or comprising a heavy chain variable region (V$_H$FAP) comprising an amino acid sequence of SEQ ID NO:18 and a light chain variable region (V$_L$FAP) comprising an amino acid sequence of SEQ ID NO:19.

In a particular aspect, provided is a bispecific antigen binding molecule comprising a first heavy chain (HC1) comprising the amino acid sequence of SEQ ID NO:28, a second heavy chain (HC2) comprising the amino acid sequence of SEQ ID NO:30, a first light chain comprising the amino acid sequence of SEQ ID NO:29 and a second light chain comprising the amino acid sequence of SEQ ID NO:31.

In one aspect, provided is an agonistic ICOS-binding molecule, wherein said molecule comprises (a) a first Fab fragment capable of specific binding to ICOS, (b) a second antigen binding domain capable of specific binding to a tumor-associated antigen comprising a VH and VL domain, and (c) a Fc domain composed of a first and a second subunit capable of stable association with each other, and wherein one of the VH and VL domain of the antigen binding domain capable of specific binding to a tumor-associated antigen is fused to the C-terminus of the first subunit of the Fc domain and the other one of VH and VL is fused to the C-terminus of the second subunit of the Fc domain. Such a molecule is termed 1+1 head-to-tail.

In a particular aspect, provided is a bispecific agonistic ICOS-binding molecule comprising a first heavy chain (HC1) comprising the amino acid sequence of SEQ ID NO:32, a second heavy chain (HC2) comprising the amino acid sequence of SEQ ID NO:66, and a light chain comprising the amino acid sequence of SEQ ID NO:29.

In another aspect, the invention provides bispecific agonistic ICOS-binding molecules, comprising (a) two antigen binding domain capable of specific binding to ICOS, and (b) one antigen binding domain capable of specific binding to a tumor-associated antigen, and (c) a Fc domain. Thus, in this case the agonistic ICOS-binding molecule is bivalent for the binding to ICOS and monovalent for the binding to the tumor-associated antigen (2+1 format).

In one aspect, provided is an agonistic ICOS-binding molecule, wherein said molecule comprises (a) two Fab fragments capable of specific binding to ICOS, (b) a second antigen binding domain capable of specific binding to a tumor-associated antigen comprising a VH and VL domain, and (c) a Fc domain composed of a first and a second subunit capable of stable association with each other, and wherein one of the VH and VL domain of the antigen binding domain capable of specific binding to a tumor-associated antigen is fused to the C-terminus of the first subunit of the Fc domain and the other one of VH and VL is fused to the C-terminus of the second subunit of the Fc domain. Such a molecule is termed 2+1.

In a particular aspect, provided is a bispecific agonistic ICOS-binding molecule comprising a first heavy chain (HC1) comprising the amino acid sequence of SEQ ID NO:32, a second heavy chain (HC2) comprising the amino acid sequence of SEQ ID NO:33, and a light chain comprising the amino acid sequence of SEQ ID NO:29.

The invention also provides agonistic ICOS-binding molecules comprising (a) at least one ectodomain of the murine ICOS ligand, (b) one antigen binding domain capable of specific binding to the target cell antigen, and (c) a Fc domain composed of a first and a second subunit capable of stable association. In particular, the agonistic ICOS-binding molecules comprise two ectodomains of the murine ICOS ligand.

More particularly, provided is a bispecific agonistic murine ICOS-binding molecule comprising a first heavy chain (HC1) comprising the amino acid sequence of SEQ ID NO:71 and a second heavy chain (HC2) comprising the amino acid sequence of SEQ ID NO:72.

**Fc domain modifications reducing Fc receptor binding and/or effector function**

The Fc domain of the agonistic ICOS-binding molecules of the invention consists of a pair of polypeptide chains comprising heavy chain domains of an immunoglobulin molecule. For example, the Fc domain of an immunoglobulin G (IgG) molecule is a dimer, each subunit of which comprises the CH2 and CH3 IgG heavy chain constant domains. The two subunits of the Fc domain are capable of stable association with each other.

Thus, the agonistic ICOS-binding molecule comprising at least one antigen binding domain that binds to a tumor-associated antigen comprises an IgG Fc domain, specifically an IgG1 Fc domain or an IgG4 Fc domain. More particularly, the agonistic ICOS-binding molecule comprising at least one antigen binding domain that binds to a tumor-associated antigen comprises an IgG1 Fc domain.

The Fc domain confers favorable pharmacokinetic properties to the antigen binding molecules of the invention, including a long serum half-life which contributes to good accumulation in the target tissue and a favorable tissue-blood distribution ratio. At the same time it may, however, lead to undesirable targeting of the bispecific antibodies of the invention to cells expressing Fc receptors rather than to the preferred antigen-bearing cells. Accordingly, in particular aspects, the Fc domain of the agonistic ICOS-binding molecules of the invention exhibits reduced binding affinity to an Fc receptor and/or reduced effector function, as compared to a native IgG1 Fc domain. In one aspect, the Fc domain does not substantially bind to an Fc receptor and/or does not induce effector function. In a particular aspect, the Fc receptor is an Fcγ receptor. In one aspect, the Fc receptor is a human Fc receptor. In a specific aspect, the Fc receptor is an activating human Fcγ receptor, more specifically human FcγRIIIa, FcγRI or FcγRIIa, most specifically human FcγRIIIa. In one aspect, the Fc domain does not induce effector function. The reduced effector function can include, but is not limited to, one or more of the following: reduced complement dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen-presenting cells, reduced binding to NK cells, reduced binding to macrophages, reduced binding to monocytes, reduced binding to polymorphonuclear cells, reduced direct signaling inducing apoptosis, reduced dendritic cell maturation, or reduced T cell priming.

In certain aspects, one or more amino acid modifications may be introduced into the Fc domain of an antibody provided herein, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions.

In a particular aspect, the invention provides an antibody, wherein the Fc domain comprises one or more amino acid substitution that reduces binding to an Fc receptor, in particular towards Fcγ receptor.

In one aspect, the Fc domain of the antibody of the invention comprises one or more amino acid mutation that reduces the binding affinity of the Fc domain to an Fc receptor and/or effector function. Typically, the same one or more amino acid mutation is present in each of the two subunits of the Fc domain. In particular, the Fc domain comprises an amino acid substitution at a position of E233, L234, L235, N297, P331 and P329 (EU numbering). In particular, the Fc domain comprises amino acid substitutions at positions 234 and 235 (EU numbering) and/or 329 (EU numbering) of the IgG heavy chains. More particularly, provided is an antibody according to the invention which comprises an Fc domain with the amino acid substitutions L234A, L235A and P329G ("P329G LALA", Kabat EU numbering) in the IgG heavy chains. The amino acid substitutions L234A and L235A refer to the so-called LALA mutation. The "P329G LALA" combination of amino acid substitutions almost completely abolishes Fcγ receptor binding of a human IgG1 Fc domain and is described in International Patent Appl. Publ. No. WO 2012/130831 A1 which also describes methods of preparing such mutant Fc domains and methods for determining its properties such as Fc receptor binding or effector functions.

Fc domains with reduced Fc receptor binding and/or effector function also include those with substitution of one or more of Fc domain residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

In another aspect, the Fc domain is an IgG4 Fc domain. IgG4 antibodies exhibit reduced binding affinity to Fc receptors and reduced effector functions as compared to IgG1 antibodies. In a more specific aspect, the Fc domain is an IgG4 Fc domain comprising an amino acid substitution at position S228 (Kabat numbering), particularly the amino acid substitution S228P. In a more specific aspect, the Fc domain is an IgG4 Fc domain comprising amino acid substitutions L235E and S228P and P329G (EU numbering). Such IgG4 Fc domain mutants and their Fcγ receptor binding properties are also described in WO 2012/130831.

Antibodies with increased half lives and improved binding to the neonatal Fc receptor (FcRn), which is responsible for the transfer of maternal IgGs to the fetus (Guyer, R.L. et al., J. Immunol. 117 (1976) 587-593, and Kim, J.K. et al., J. Immunol. 24 (1994) 2429-2434), are described in US 2005/0014934. Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826). See also Duncan, A.R. and Winter, G., Nature 322 (1988) 738-740; US 5,648,260; US 5,624,821; and WO 94/29351 concerning other examples of Fc region variants.

Binding to Fc receptors can be easily determined e.g. by ELISA, or by Surface Plasmon Resonance (SPR) using standard instrumentation such as a BIAcore instrument (GE Healthcare), and Fc receptors such as may be obtained by recombinant expression. A suitable such binding assay is described herein. Alternatively, binding affinity of Fc domains or cell activating bispecific antigen binding molecules comprising an Fc domain for Fc receptors may be evaluated using cell lines known to express particular Fc receptors, such as human NK cells expressing FcγIIIa receptor. Effector function of an Fc domain, or bispecific antigen binding molecules of the invention comprising an Fc domain, can be measured by methods known in the art. A suitable assay for measuring ADCC is described herein. Other examples of *in vitro* assays to assess ADCC activity of a molecule of interest are described in U.S. Patent No. 5,500,362; Hellstrom et al. Proc Natl Acad Sci USA 83, 7059-7063 (1986) and Hellstrom et al., Proc Natl Acad Sci USA 82, 1499-1502 (1985); U.S. Patent No. 5,821,337; Bruggemann et al., J Exp Med 166, 1351-1361 (1987). Alternatively, non-radioactive assays methods may be employed (see, for example, ACTI™ non-radioactive cytotoxicity assay for flow cytometry (CellTechnology, Inc. Mountain View, CA); and CytoTox 96® non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed *in vivo,* e.g. in a animal model such as that disclosed in Clynes et al., Proc Natl Acad Sci USA 95, 652-656 (1998).

The following section describes preferred aspects of the agonistic ICOS-binding molecules of the invention comprising Fc domain modifications that reduce Fc receptor binding and/or effector function. In one aspect, the invention relates to the bispecific antigen binding molecule (a) at least one antigen binding domain capable of specific binding to ICOS, (b) at least one antigen binding domain capable of specific binding to a tumor-associated antigen, and (c) a Fc domain composed of a first and a second subunit capable of stable association, wherein the Fc domain comprises one or more amino acid substitution that reduces the binding affinity of the antibody to an Fc receptor, in particular towards Fcγ receptor. In another aspect, the invention relates to the agonistic ICOS-binding molecule comprising (a) at least one antigen binding domain capable of specific binding to ICOS, (b) at least one antigen binding domain capable of specific binding to a target cell antigen, and (c) a Fc domain composed of a first and a second subunit capable of stable association, wherein the Fc domain comprises one or more amino acid substitution that reduces effector function. In particular aspect, the Fc domain is of human IgG1 subclass with the amino acid mutations L234A, L235A and P329G (numbering according to Kabat EU index).

In one aspect of the invention, the Fc region comprises an amino acid substitution at positions D265, and P329. In some aspects, the Fc region comprises the amino acid substitutions D265A and P329G ("DAPG") in the CH2 domain. In one such embodiment, the Fc region is an IgG1 Fc region, particularly a mouse IgG1 Fc region. DAPG mutations are described e.g. in WO 2016/030350 A1, and can be introduced in CH2 regions of heavy chains to abrogate binding of antigen binding molecules to murine Fc gamma receptors.

**Fc domain modifications promoting heterodimerization**

The agonistic ICOS-binding molecules of the invention comprise different antigen-binding sites, fused to one or the other of the two subunits of the Fc domain, thus the two subunits of the Fc domain may be comprised in two non-identical polypeptide chains. Recombinant co-expression of these polypeptides and subsequent dimerization leads to several possible combinations of the two polypeptides. To improve the yield and purity of the agonistic ICOS-binding molecules of the invention in recombinant production, it will thus be advantageous to introduce in the Fc domain of the bispecific antigen binding molecules of the invention a modification promoting the association of the desired polypeptides.

Accordingly, in particular aspects the invention relates to agonistic ICOS-binding molecules comprising (a) at least one antigen binding domain capable of specific binding to ICOS, (b) at least one antigen binding domain capable of specific binding to a tumor-associated antigen, and (c) a Fc domain composed of a first and a second subunit capable of stable association with each other, wherein the Fc domain comprises a modification promoting the association of the first and second subunit of the Fc domain. The site of most extensive protein-protein interaction between the two subunits of a human IgG Fc domain is in the CH3 domain of the Fc domain. Thus, in one aspect said modification is in the CH3 domain of the Fc domain.

In a specific aspect, said modification is a so-called "knob-into-hole" modification, comprising a "knob" modification in one of the two subunits of the Fc domain and a "hole" modification in the other one of the two subunits of the Fc domain. Thus, the invention relates to the agonistic ICOS-binding molecule comprising (a) at least one antigen binding domain capable of specific binding to ICOS, (b) at least one antigen binding domain capable of specific binding to a tumor-associated antigen, and (c) a Fc domain composed of a first and a second subunit capable of stable association with each other, wherein the first subunit of the Fc domain comprises knobs and the second subunit of the Fc domain comprises holes according to the knobs into holes method. In a particular aspect, the first subunit of the Fc domain comprises the amino acid substitutions S354C and T366W (EU numbering) and the second subunit of the Fc domain comprises the amino acid substitutions Y349C, T366S and Y407V (numbering according to Kabat EU index).

The knob-into-hole technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine).

Accordingly, in one aspect, in the CH3 domain of the first subunit of the Fc domain of the agonistic ICOS-binding molecules of the invention an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable. The protuberance and cavity can be made by altering the nucleic acid encoding the polypeptides, e.g. by site-specific mutagenesis, or by peptide synthesis. In a specific aspect, in the CH3 domain of the first subunit of the Fc domain the threonine residue at position 366 is replaced with a tryptophan residue (T366W), and in the CH3 domain of the second subunit of the Fc domain the tyrosine residue at position 407 is replaced with a valine residue (Y407V). In one aspect, in the second subunit of the Fc domain additionally the threonine residue at position 366 is replaced with a serine residue (T366S) and the leucine residue at position 368 is replaced with an alanine residue (L368A).

In yet a further aspect, in the first subunit of the Fc domain additionally the serine residue at position 354 is replaced with a cysteine residue (S354C), and in the second subunit of the Fc domain additionally the tyrosine residue at position 349 is replaced by a cysteine residue (Y349C). Introduction of these two cysteine residues results in the formation of a disulfide bridge between the two subunits of the Fc domain, further stabilizing the dimer (Carter (2001), J Immunol Methods 248, 7-15). In a particular aspect, the first subunit of the Fc domain comprises the amino acid substitutions S354C and T366W (EU numbering) and the second subunit of the Fc domain comprises the amino acid substitutions Y349C, T366S and Y407V (numbering according to Kabat EU index).

In one aspect, the first subunit of the Fc region comprises aspartic acid residues (D) at positions 392 and 409, and the second subunit of the Fc region comprises lysine residues (K) at positions 356 and 399. In some embodiments, in the first subunit of the Fc region the lysine residues at positions 392 and 409 are replaced with aspartic acid residues (K392D, K409D), and in the second subunit of the Fc region the glutamate residue at position 356 and the aspartic acid residue at position 399 are replaced with lysine residues (E356K, D399K). "DDKK" knob-into-hole technology is described e.g. in WO 2014/131694 A1, and favours the assembly of the heavy chains bearing subunits providing the complementary amino acid residues.

In an alternative aspect, a modification promoting association of the first and the second subunit of the Fc domain comprises a modification mediating electrostatic steering effects, e.g. as described in PCT publication WO 2009/089004. Generally, this method involves replacement of one or more amino acid residues at the interface of the two Fc domain subunits by charged amino acid residues so that homodimer formation becomes electrostatically unfavorable but heterodimerization electrostatically favorable.

The C-terminus of the heavy chain of the bispecific antibody as reported herein can be a complete C-terminus ending with the amino acid residues PGK. The C-terminus of the heavy chain can be a shortened C-terminus in which one or two of the C terminal amino acid residues have been removed. In one preferred aspect, the C-terminus of the heavy chain is a shortened C-terminus ending PG. In one aspect of all aspects as reported herein, a bispecific antibody comprising a heavy chain including a C-terminal CH3 domain as specified herein, comprises the C-terminal glycine-lysine dipeptide (G446 and K447, numbering according to Kabat EU index). In one embodiment of all aspects as reported herein, a bispecific antibody comprising a heavy chain including a C-terminal CH3 domain, as specified herein, comprises a C-terminal glycine residue (G446, numbering according to Kabat EU index).

**Exemplary anti-CEA/anti-CD3 bispecific antibodies for use in the invention**

The present invention relates to anti-CEA/anti-CD3 bispecific antibodies and their use in combination with agonistic ICOS-binding molecules, in particular to their use in a method for treating or delaying progression of cancer, more particularly for treating or delaying progression of solid tumors. The anti-CEA/anti-CD3 bispecific antibodies as used herein are bispecific antibodies comprising a first antigen binding domain that binds to CD3, and a second antigen binding domain that binds to CEA.

Thus, the anti-CEA/anti-CD3 bispecific antibody as used herein comprises a first antigen binding domain comprising a heavy chain variable region ($V_H$CD3) and a light chain variable region ($V_L$CD3), and a second antigen binding domain comprising a heavy chain variable region ($V_H$CEA) and a light chain variable region ($V_L$CEA).

In a particular aspect, the anti-CEA/anti-CD3 bispecific antibody for use in the combination comprises a first antigen binding domain comprising a heavy chain variable region ($V_H$CD3) comprising CDR-H1 sequence of SEQ ID NO:33, CDR-H2 sequence of SEQ ID NO:34, and CDR-H3 sequence of SEQ ID NO:35; and/or a light chain variable region ($V_L$CD3) comprising CDR-L1 sequence of SEQ ID NO:36, CDR-L2 sequence of SEQ ID NO:37, and CDR-L3 sequence of SEQ ID NO:38. More particularly, the anti-CEA/anti-CD3 bispecific antibody comprises a first antigen binding domain comprising a heavy chain variable region ($V_H$CD3) that is at least 90%, 95%, 96%, 97%, 98%, or 99% identical to the amino acid sequence of SEQ ID NO:39 and/or a light chain variable region ($V_L$CD3) that is at least 90%, 95%, 96%, 97%, 98%, or 99% identical to the amino acid sequence of SEQ ID NO:40. In a further aspect, the anti-CEA/anti-CD3 bispecific antibody comprises a heavy chain variable region (V$_H$CD3) comprising the amino acid sequence of SEQ ID NO:39 and/or a light chain variable region (V$_L$CD3) comprising the amino acid sequence of SEQ ID NO:40.

In one aspect, the antibody that specifically binds to CD3 is a full-length antibody. In one aspect, the antibody that specifically binds to CD3 is an antibody of the human IgG class, particularly an antibody of the human IgG1 class. In one aspect, the antibody that specifically binds to CD3 is an antibody fragment, particularly a Fab molecule or a scFv molecule, more particularly a Fab molecule. In a particular aspect, the antibody that specifically binds to CD3 is a crossover Fab molecule wherein the variable domains or the constant domains of the Fab heavy and light chain are exchanged (i.e. replaced by each other). In one aspect, the antibody that specifically binds to CD3 is a humanized antibody.

In another aspect, the anti-CEA/anti-CD3 bispecific antibody comprises a second antigen binding domain comprising

(a) a heavy chain variable region (V$_H$CEA) comprising CDR-H1 sequence of SEQ ID NO:41, CDR-H2 sequence of SEQ ID NO:42, and CDR-H3 sequence of SEQ ID NO:43, and/or a light chain variable region (V$_L$CEA) comprising CDR-L1 sequence of SEQ ID NO:44, CDR-L2 sequence of SEQ ID NO:45, and CDR-L3 sequence of SEQ ID NO:46, or (b) a heavy chain variable region (V$_H$CEA) comprising CDR-H1 sequence of SEQ ID NO:49, CDR-H2 sequence of SEQ ID NO:50, and CDR-H3 sequence of SEQ ID NO:51, and/or a light chain variable region (V$_L$CEA) comprising CDR-L1 sequence of SEQ ID NO:52, CDR-L2 sequence of SEQ ID NO:53, and CDR-L3 sequence of SEQ ID NO:54.

More particularly, the anti-CEA/anti-CD3 bispecific comprises a second antigen binding domain comprising a heavy chain variable region (V$_H$CEA) that is at least 90%, 95%, 96%, 97%, 98%, or 99% identical to the amino acid sequence of SEQ ID NO:47 and/or a light chain variable region (V$_L$CEA) that is at least 90%, 95%, 96%, 97%, 98%, or 99% identical to the amino acid sequence of SEQ ID NO:48. In a further aspect, the anti-CEA/anti-CD3 bispecific comprises a second antigen binding domain comprising a heavy chain variable region (V$_H$CEA) comprising the amino acid sequence of SEQ ID NO:47 and/or a light chain variable region (V$_L$CEA) comprising the amino acid sequence of SEQ ID NO:48. In another aspect, the anti-CEA/anti-CD3 bispecific comprises a second antigen binding domain comprising a heavy chain variable region (V$_H$CEA) that is at least 90%, 95%, 96%, 97%, 98%, or 99% identical to the amino acid sequence of SEQ ID NO:55 and/or a light chain variable region (V$_L$CEA) that is at least 90%, 95%, 96%, 97%, 98%, or 99% identical to the amino acid sequence of SEQ ID NO:56. In a further aspect, the anti-CEA/anti-CD3 bispecific comprises a second antigen binding domain comprising a heavy chain variable region (V$_H$CEA) comprising the amino acid sequence of SEQ ID NO:55 and/or a light chain variable region (V$_L$CEA) comprising the amino acid sequence of SEQ ID NO:56.

In another particular aspect, the anti-CEA/anti-CD3 bispecific antibody comprises a third antigen binding domain that binds to CEA. In particular, the anti-CEA/anti-CD3 bispecific antibody comprises a third antigen binding domain comprising (a) a heavy chain variable region (V$_H$CEA) comprising CDR-H1 sequence of SEQ ID NO:41, CDR-H2 sequence of SEQ ID NO:42, and CDR-H3 sequence of SEQ ID NO:43, and/or a light chain variable region (V$_L$CEA) comprising CDR-L1 sequence of SEQ ID NO:44, CDR-L2 sequence of SEQ ID NO:45, and CDR-L3 sequence of SEQ ID NO:46, or (b) a heavy chain variable region (V$_H$CEA) comprising CDR-H1 sequence of SEQ ID NO:49, CDR-H2 sequence of SEQ ID NO:50, and CDR-H3 sequence of SEQ ID NO:51, and/or a light chain variable region (V$_L$CEA) comprising CDR-L1 sequence of SEQ ID NO:52, CDR-L2 sequence of SEQ ID NO:53, and CDR-L3 sequence of SEQ ID NO:54.

More particularly, the anti-CEA/anti-CD3 bispecific comprises a third antigen binding domain comprising a heavy chain variable region (V$_H$CEA) that is at least 90%, 95%, 96%, 97%, 98%, or 99% identical to the amino acid sequence of SEQ ID NO:47 and/or a light chain variable region (V$_L$CEA) that is at least 90%, 95%, 96%, 97%, 98%, or 99% identical to the amino acid sequence of SEQ ID NO:48. In a further aspect, the anti-CEA/anti-CD3 bispecific comprises a third antigen binding domain comprising a heavy chain variable region (V$_H$CEA) comprising the amino acid sequence of SEQ ID NO:47 and/or a light chain variable region (V$_L$CEA) comprising the amino acid sequence of SEQ ID NO:48. In another particular aspect, the anti-CEA/anti-CD3 bispecific comprises a third antigen binding domain comprising a heavy chain variable region (V$_H$CEA) that is at least 90%, 95%, 96%, 97%, 98%, or 99% identical to the amino acid sequence of SEQ ID NO:55 and/or a light chain variable region (V$_L$CEA) that is at least 90%, 95%, 96%, 97%, 98%, or 99% identical to the amino acid sequence of SEQ ID NO:56. In a further aspect, the anti-CEA/anti-CD3 bispecific comprises a third antigen binding domain comprising a heavy chain variable region (V$_H$CEA) comprising the amino acid sequence of SEQ ID NO:55 and/or a light chain variable region (V$_L$CEA) comprising the amino acid sequence of SEQ ID NO:56.

In a further aspect, the anti-CEA/anti-CD3 bispecific antibody is a bispecific antibody, wherein the first antigen binding domain is a cross-Fab molecule wherein the variable domains or the constant domains of the Fab heavy and light chain are exchanged, and the second and third, if present, antigen binding domain is a conventional Fab molecule.

In another aspect, the anti-CEA/anti-CD3 bispecific antibody is bispecific antibody, wherein (i) the second antigen binding domain is fused at the C-terminus of the Fab heavy chain to the N-terminus of the Fab heavy chain of the first antigen binding domain, the first antigen binding domain is fused at the C-terminus of the Fab heavy chain to the N-terminus of the first subunit of the Fc domain, and the third antigen binding domain is fused at the C-terminus of the Fab heavy chain to the N-terminus of the second subunit of the Fc domain, or (ii) the first antigen binding domain is fused at the C-terminus of the Fab heavy chain to the N-terminus of the Fab heavy chain of the second antigen binding domain, the second antigen binding domain is fused at the C-terminus of the Fab heavy chain to the N-terminus of the first subunit of the Fc domain, and the third antigen binding domain is fused at the C-terminus of the Fab heavy chain to the N-terminus of the second subunit of the Fc domain.

The Fab molecules may be fused to the Fc domain or to each other directly or through a peptide linker, comprising one or more amino acids, typically about 2-20 amino acids. Peptide linkers are known in the art and are described herein. Suitable, non-immunogenic peptide linkers include, for example, $(G_4S)_n$, $(SG_4)_n$, $(G_4S)_n$ or $G_4(SG_4)_n$ peptide linkers. "n" is generally an integer from 1 to 10, typically from 2 to 4. In one embodiment said peptide linker has a length of at least 5 amino acids, in one embodiment a length of 5 to 100, in a further embodiment of 10 to 50 amino acids. In one embodiment said peptide linker is $(GxS)_n$ or $(GxS)_nG_m$ with G=glycine, S=serine, and (x=3, n= 3, 4, 5 or 6, and m=0, 1, 2 or 3) or (x=4, n=2, 3, 4 or 5 and m= 0, 1, 2 or 3), in one embodiment x=4 and n=2 or 3, in a further embodiment x=4 and n=2. In one embodiment said peptide linker is $(G_4S)_2$. A particularly suitable peptide linker for fusing the Fab light chains of the first and the second Fab molecule to each other is $(G_4S)_2$. An exemplary peptide linker suitable for connecting the Fab heavy chains of the first and the second Fab fragments comprises the sequence $(D)-(G_4S)_2$. Another suitable such linker comprises the sequence $(G_4S)_4$. Additionally, linkers may comprise (a portion of) an immunoglobulin hinge region. Particularly where a Fab molecule is fused to the N-terminus of an Fc domain subunit, it may be fused via an immunoglobulin hinge region or a portion thereof, with or without an additional peptide linker.

In a further aspect, the anti-CEA/anti-CD3 bispecific antibody comprises an Fc domain comprising one or more amino acid substitutions that reduce binding to an Fc receptor and/or effector function. In particular, the anti-CEA/anti-CD3 bispecific antibody comprises an IgG1 Fc domain comprising the amino aciod substitutions L234A, L235A and P329G.

In a particular aspect, the anti-CEA/anti-CD3 bispecific antibody comprises a polypeptide that is at least 95%, 96%, 97%, 98%, or 99% identical to the sequence of SEQ ID NO: 61, a polypeptide that is at least 95%, 96%, 97%, 98%, or 99% identical to the sequence of SEQ ID NO: 62, a polypeptide that is at least 95%, 96%, 97%, 98%, or 99% identical to the sequence of SEQ ID NO: 63, and a polypeptide that is at least 95%, 96%, 97%, 98%, or 99% identical to the sequence of SEQ ID NO: 64. In a further particular embodiment, the bispecific antibody comprises a polypeptide sequence of SEQ ID NO: 61, a polypeptide sequence of SEQ ID NO: 62, a polypeptide sequence of SEQ ID NO: 63 and a polypeptide sequence of SEQ ID NO: 64 (CEA CD3 TCB).

In a further particular aspect, the anti-CEA/anti-CD3 bispecific antibody comprises a polypeptide that is at least 95%, 96%, 97%, 98%, or 99% identical to the sequence of SEQ ID NO:57, a polypeptide that is at least 95%, 96%, 97%, 98%, or 99% identical to the sequence of SEQ ID NO:58, a polypeptide that is at least 95%, 96%, 97%, 98%, or 99% identical to the sequence of SEQ ID NO:59, and a polypeptide that is at least 95%, 96%, 97%, 98%, or 99% identical to the sequence of SEQ ID NO:60. In a further particular embodiment, the bispecific antibody comprises a polypeptide sequence of SEQ ID NO:57, a polypeptide sequence of SEQ ID NO:58, a polypeptide sequence of SEQ ID NO:59 and a polypeptide sequence of SEQ ID NO:60 (CEACAM5 CD3 TCB).

Particular bispecific antibodies are described in PCT publication no. WO 2014/131712 A1.

In a further aspect, the anti-CEA/anti-CD3 bispecific antibody may also comprise a bispecific T cell engager (BiTE®). In a further aspect, the anti-CEA/anti-CD3 bispecific antibody is a bispecific antibody as described in WO 2007/071426 or WO 2014/131712. In another aspect, the bispecific antibody is MEDI565.

In another aspect, the invention relates to a murine anti-CEA/anti-CD3 bispecific antibody comprising a first antigen binding domain comprising a heavy chain variable region ($V_H$muCD3) and a light chain variable region ($V_L$muCD3), a second antigen binding domain comprising a heavy chain variable region ($V_H$muCEA) and a light chain variable region ($V_L$muCEA) and a third antigen binding domain comprising a heavy chain variable region ($V_H$muCEA) and a light chain variable region ($V_L$muCEA).

In a particular aspect, the murine anti-CEA/anti-CD3 bispecific antibody comprises a polypeptide that is at least 95%, 96%, 97%, 98%, or 99% identical to the sequence of SEQ ID NO:75, a polypeptide that is at least 95%, 96%, 97%, 98%, or 99% identical to the sequence of SEQ ID NO: 62, a polypeptide that is at least 95%, 96%, 97%, 98%, or 99% identical to the sequence of SEQ ID NO:76, a polypeptide that is at least 95%, 96%, 97%, 98%, or 99% identical to the sequence of SEQ ID NO:77 and a polypeptide that is at least 95%, 96%, 97%, 98%, or 99% identical to the sequence of SEQ ID NO:78. In a further particular aspect, the murine anti-CEA/anti-CD3 bispecific antibody comprises a polypeptide sequence of SEQ ID NO:75, a polypeptide sequence of SEQ ID NO:76, a polypeptide sequence of SEQ ID NO:77 and a polypeptide sequence of SEQ ID NO:78 (mu CEA CD3 TCB).

**Agents blocking PD-L1/PD-1 interaction for use in the invention**

In one aspect of the invention, the T-cell activating anti-CD3 bispecific antibodies specific for a tumor-associated antigen, in particular the anti-CEA/anti-CD3 antibodies are for use in a method for treating or delaying progression of cancer, wherein the T-cell activating anti-CD3 bispecific antibodies specific for a tumor-associated antigen are used in combination with a 4-1BB (CD137) agonist and additionally they are combined with an agent blocking PD-L1/PD-1 interaction. In another aspect, the agent blocking PD-L1/PD-1 interaction is only combined with a targeted 4-1BB agonist. In all these aspects, an agent blocking PD-L1/PD-1 interaction is a PD-L1 binding antagonist or a PD-1 binding antagonist. In particular, the agent blocking PD-L1/PD-1 interaction is an anti-PD-Ll antibody or an anti-PD-1 antibody.

The term "**PD-L1**", also known as CD274 or B7-H1, refers to any native PD-L1 from any vertebrate source, including mammals such as primates (e.g. humans) non-human primates (e.g. cynomolgus monkeys) and rodents (e.g. mice and rats), in particular to "human PD-L1". The amino acid sequence of complete human PD-L1 is shown in UniProt (www.uniprot.org) accession no. Q9NZQ7 (SEQ ID NO:107). The term "**PD-L1 binding antagonist**" refers to a molecule that decreases, blocks, inhibits, abrogates or interferes with signal transduction resulting from the interaction of PD-L1 with either one or more of its binding partners, such as PD-1, B7-1. In some embodiments, a PD-L1 binding antagonist is a molecule that inhibits the binding of PD-L1 to its binding partners. In a specific aspect, the PD-L1 binding antagonist inhibits binding of PD-L1 to PD-1 and/or B7-1. In some embodiments, the PD-L1 binding antagonists include anti-PD-Ll antibodies, antigen binding fragments thereof, immunoadhesins, fusion proteins, oligopeptides and other molecules that decrease, block, inhibit, abrogate or interfere with signal transduction resulting from the interaction of PD-L1 with one or more of its binding partners, such as PD-1, B7-1. In one embodiment, a PD-L1 binding antagonist reduces the negative co-stimulatory signal mediated by or through cell surface proteins expressed on T lymphocytes mediated signaling through PD-L1 so as to render a dysfunctional T-cell less dysfunctional (e.g., enhancing effector responses to antigen recognition). In particular, a PD-L1 binding antagonist is an anti-PD-Ll antibody. The term "**anti-PD-L1 antibody**" or "antibody binding to human PD-L1" or "antibody that specifically binds to human PD-L1" or "antagonistic anti-PD-Ll" refers to an antibody specifically binding to the human PD-L1 antigen with a binding affinity of KD-value of $1.0 \times 10^{-8}$ mol/l or lower, in one aspect of a KD-value of $1.0 \times 10^{-9}$ mol/l or lower. The binding affinity is determined with a standard binding assay, such as surface plasmon resonance technique (BIAcore®, GE-Healthcare Uppsala, Sweden).

In a particular aspect, the agent blocking PD-L1/PD-1 interaction is an anti-PD-L1 antibody. In a specific aspect, the anti-PD-Ll antibody is selected from the group consisting of atezolizumab (MPDL3280A, RG7446), durvalumab (MEDI4736), avelumab (MSB0010718C) and MDX-1105. In a specific aspect, an anti-PD-Ll antibody is YW243.55.S70 described herein. In another specific aspect, an anti-PD-Ll antibody is MDX-1105 described herein. In still another specific aspect, an anti-PD-Ll antibody is MEDI4736 (durvalumab). In yet a further aspect, an anti-PD-Ll antibody is MSB0010718C (avelumab). More particularly, the agent blocking PD-L1/PD-1 interaction is atezolizumab (MPDL3280A). In another aspect, the agent blocking PD-L1/PD-1 interaction is an anti-PD-Ll antibody comprising a heavy chain variable domain VH(PDL-1) of SEQ ID NO:109 and a light chain variable domain VL(PDL-1) of SEQ ID NO:110. In another aspect, the agent blocking PD-L1/PD-1 interaction is an anti-PD-Ll antibody comprising a heavy chain variable domain VH(PDL-1) of SEQ ID NO:111 and a light chain variable domain VL(PDL-1) of SEQ ID NO:112.

The term "**PD-1**", also known as CD279, PD1 or programmed cell death protein 1, refers to any native PD-L1 from any vertebrate source, including mammals such as primates (e.g. humans) non-human primates (e.g. cynomolgus monkeys) and rodents (e.g. mice and rats), in particular to the human protein PD-1 with the amino acid sequence as shown in UniProt (www.uniprot.org) accession no. Q15116 (SEQ ID NO:108). The term "**PD-1 binding antagonist**" refers to a molecule that inhibits the binding of PD-1 to its ligand binding partners. In some embodiments, the PD-1 binding antagonist inhibits the binding of PD-1 to PD-L1. In some embodiments, the PD-1 binding antagonist inhibits the binding of PD-1 to PD-L2. In some embodiments, the PD-1 binding antagonist inhibits the binding of PD-1 to both PD-L1 and PD-L2. In particular, a PD-L1 binding antagonist is an anti-PD-Ll antibody. The term "**anti-PD-1 antibody**" or "antibody binding to human PD-1" or "antibody that specifically binds to human PD-1" or "antagonistic anti-PD-1" refers to an antibody specifically binding to the human PD1 antigen with a binding affinity of KD-value of $1.0 \times 10^{-8}$ mol/l or lower, in one aspect of a KD-value of $1.0 \times 10^{-9}$ mol/l or lower. The binding affinity is determined with a standard binding assay, such as surface plasmon resonance technique (BIAcore®, GE-Healthcare Uppsala, Sweden).

In one aspect, the agent blocking PD-L1/PD-1 interaction is an anti-PD-1 antibody. In a specific aspect, the anti-PD-1 antibody is selected from the group consisting of MDX 1106 (nivolumab), MK-3475 (pembrolizumab), CT-011 (pidilizumab), MEDI-0680 (AMP-514), PDR001, REGN2810, and BGB-108, in particular from pembrolizumab and nivolumab. In another aspect, the agent blocking PD-L1/PD-1 interaction is an anti-PD-1 antibody comprising a heavy chain variable domain VH(PD-1) of SEQ ID NO:113 and a light chain variable domain VL(PD-1) of SEQ ID NO:114. In another aspect, the agent blocking PD-L1/PD-1 interaction is an anti-PD-1 antibody comprising a heavy chain variable domain VH(PD-1) of SEQ ID NO:115 and a light chain variable domain VL(PD-1) of SEQ ID NO:116.

**Polynucleotides**

The invention further provides isolated polynucleotides encoding agonistic ICOS-binding molecule or a T-cell bispecific antibody as described herein or a fragment thereof.

The isolated polynucleotides encoding the bispecific antibodies of the invention may be expressed as a single polynucleotide that encodes the entire antigen binding molecule or as multiple (e.g., two or more) polynucleotides that are co-expressed. Polypeptides encoded by polynucleotides that are co-expressed may associate through, e.g., disulfide bonds or other means to form a functional antigen binding molecule. For example, the light chain portion of an immunoglobulin may be encoded by a separate polynucleotide from the heavy chain portion of the immunoglobulin. When co-expressed, the heavy chain polypeptides will associate with the light chain polypeptides to form the immunoglobulin.

In some aspects, the isolated polynucleotide encodes the entire antigen-binding molecule according to the invention as described herein. In other embodiments, the isolated polynucleotide encodes a polypeptide comprised in the antibody according to the invention as described herein.

In certain embodiments the polynucleotide or nucleic acid is DNA. In other embodiments, a polynucleotide of the present invention is RNA, for example, in the form of messenger RNA (mRNA). RNA of the present invention may be single stranded or double stranded.

**Recombinant Methods**

Bispecific antibodies of the invention may be obtained, for example, by solid-state peptide synthesis (e.g. Merrifield solid phase synthesis) or recombinant production. For recombinant production one or more polynucleotide encoding the antibody or polypeptide fragments thereof, e.g., as described above, is isolated and inserted into one or more vectors for further cloning and/or expression in a host cell. Such polynucleotide may be readily isolated and sequenced using conventional procedures. In one aspect of the invention, a vector, preferably an expression vector, comprising one or more of the polynucleotides of the invention is provided. Methods which are well known to those skilled in the art can be used to construct expression vectors containing the coding sequence of the antibody (fragment) along with appropriate transcriptional/translational control signals. These methods include in vitro recombinant DNA techniques, synthetic techniques and in vivo recombination/genetic recombination. See, for example, the techniques described in Maniatis et al., MOLECULAR CLONING: A LABORATORY MANUAL, Cold Spring Harbor Laboratory, N.Y. (1989); and Ausubel et al., CURRENT PROTOCOLS IN MOLECULAR BIOLOGY, Greene Publishing Associates and Wiley Interscience, N.Y. (1989). The expression vector can be part of a plasmid, virus, or may be a nucleic acid fragment. The expression vector includes an expression cassette into which the polynucleotide encoding the antibody or polypeptide fragments thereof (i.e. the coding region) is cloned in operable association with a promoter and/or other transcription or translation control elements. As used herein, a "coding region" is a portion of nucleic acid which consists of codons translated into amino acids. Although a "stop codon" (TAG, TGA, or TAA) is not translated into an amino acid, it may be considered to be part of a coding region, if present, but any flanking sequences, for example promoters, ribosome binding sites, transcriptional terminators, introns, 5' and 3' untranslated regions, and the like, are not part of a coding region. Two or more coding regions can be present in a single polynucleotide construct, e.g. on a single vector, or in separate polynucleotide constructs, e.g. on separate (different) vectors. Furthermore, any vector may contain a single coding region, or may comprise two or more coding regions, e.g. a vector of the present invention may encode one or more polypeptides, which are post- or co-translationally separated into the final proteins via proteolytic cleavage. In addition, a vector, polynucleotide, or nucleic acid of the invention may encode heterologous coding regions, either fused or unfused to a polynucleotide encoding the antibody of the invention or polypeptide fragments thereof, or variants or derivatives thereof. Heterologous coding regions include without limitation specialized elements or motifs, such as a secretory signal peptide or a heterologous functional domain. An operable association is when a coding region for a gene product, e.g. a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription.

Suitable promoters and other transcription control regions are disclosed herein. A variety of transcription control regions are known to those skilled in the art. These include, without limitation, transcription control regions, which function in vertebrate cells, such as, but not limited to, promoter and enhancer segments from cytomegaloviruses (e.g. the immediate early promoter, in conjunction with intron-A), simian virus 40 (e.g. the early promoter), and retroviruses (such as, e.g. Rous sarcoma virus). Other transcription control regions include those derived from vertebrate genes such as actin, heat shock protein, bovine growth hormone and rabbit â-globin, as well as other sequences capable of controlling gene expression in eukaryotic cells. Additional suitable transcription control regions include tissue-specific promoters and enhancers as well as inducible promoters (e.g. promoters inducible tetracyclins). Similarly, a variety of translation control elements are known to those of ordinary skill in the art. These include, but are not limited to ribosome binding sites, translation initiation and termination codons, and elements derived from viral systems (particularly an internal ribosome entry site, or IRES, also referred to as a CITE sequence). The expression cassette may also include other features such as an origin of replication, and/or chromosome integration elements such as retroviral long terminal repeats (LTRs), or adeno-associated viral (AAV) inverted terminal repeats (ITRs).

Polynucleotide and nucleic acid coding regions of the present invention may be associated with additional coding regions which encode secretory or signal peptides, which direct the secretion of a polypeptide encoded by a polynucleotide of the present invention. For example, if secretion of the antibody or polypeptide fragments thereof is desired, DNA encoding a signal sequence may be placed upstream of the nucleic acid an antibody of the invention or polypeptide fragments thereof. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the translated polypeptide to produce a secreted or "mature" form of the polypeptide. In certain embodiments, the native signal peptide, e.g. an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence may be substituted with the leader sequence of human tissue plasminogen activator (TPA) or mouse β-glucuronidase.

DNA encoding a short protein sequence that could be used to facilitate later purification (e.g. a histidine tag) or assist in labeling the fusion protein may be included within or at the ends of the polynucleotide encoding a bispecific antibody of the invention or polypeptide fragments thereof.

In a further aspect of the invention, a host cell comprising one or more polynucleotides of the invention is provided. In certain embodiments a host cell comprising one or more vectors of the invention is provided. The polynucleotides and vectors may incorporate any of the features, singly or in combination, described herein in relation to polynucleotides and vectors, respectively. In one aspect, a host cell comprises (e.g. has been transformed or transfected with) a vector comprising a polynucleotide that encodes (part of) an antibody of the invention of the invention. As used herein, the term "host cell" refers to any kind of cellular system which can be engineered to generate the fusion proteins of the invention or fragments thereof. Host cells suitable for replicating and for supporting expression of antigen binding molecules are well known in the art. Such cells may be transfected or transduced as appropriate with the particular expression vector and large quantities of vector containing cells can be grown for seeding large scale fermenters to obtain sufficient quantities of the antigen binding molecule for clinical applications. Suitable host cells include prokaryotic microorganisms, such as E. coli, or various eukaryotic cells, such as Chinese hamster ovary cells (CHO), insect cells, or the like. For example, polypeptides may be produced in bacteria in particular when glycosylation is not needed. After expression, the polypeptide may be isolated from the bacterial cell paste in a soluble fraction and can be further purified. In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of a polypeptide with a partially or fully human glycosylation pattern. See Gerngross, Nat Biotech 22, 1409-1414 (2004), and Li et al., Nat Biotech 24, 210-215 (2006).

Suitable host cells for the expression of (glycosylated) polypeptides are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells. Plant cell cultures can also be utilized as hosts. See e.g. US Patent Nos. 5,959,177, 6,040,498, 6,420,548, 7,125,978, and 6,417,429 (describing PLANTIBODIES™ technology for producing antibodies in transgenic plants). Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293T cells as described, e.g., in Graham et al., J Gen Virol 36, 59 (1977)), baby hamster kidney cells (BHK), mouse sertoli cells (TM4 cells as described, e.g., in Mather, Biol Reprod 23, 243-251 (1980)), monkey kidney cells (CV1), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor cells (MMT 060562), TRI cells (as described, e.g., in Mather et al., Annals N.Y. Acad Sci 383, 44-68 (1982)), MRC 5 cells, and FS4 cells. Other useful mammalian host cell lines include Chinese hamster ovary (CHO) cells, including dhfr- CHO cells (Urlaub et al., Proc Natl Acad Sci USA 77, 4216 (1980)); and myeloma cell lines such as YO, NS0, P3X63 and Sp2/0. For a review of certain mammalian host cell lines suitable for protein production, see, e.g., Yazaki and Wu, Methods in Molecular Biology, Vol. 248 (B.K.C. Lo, ed., Humana Press, Totowa, NJ), pp. 255-268 (2003). Host cells include cultured cells, e.g., mammalian cultured cells, yeast cells, insect cells, bacterial cells and plant cells, to name only a few, but also cells comprised within a transgenic animal, transgenic plant or cultured plant or animal tissue. In one embodiment, the host cell is a eukaryotic cell, preferably a mammalian cell, such as a Chinese Hamster Ovary (CHO) cell, a human embryonic kidney (HEK) cell or a lymphoid cell (e.g., Y0, NS0, Sp20 cell). Standard technologies are known in the art to express foreign genes in these systems. Cells expressing a polypeptide comprising either the heavy or the light chain of an immunoglobulin, may be engineered so as to also express the other of the immunoglobulin chains such that the expressed product is an immunoglobulin that has both a heavy and a light chain.

In one aspect, a method of producing an agonistic ICOS-binding molecule of the invention or polypeptide fragments thereof is provided, wherein the method comprises culturing a host cell comprising polynucleotides encoding the agonistic ICOS-binding molecule or polypeptide fragments thereof, as provided herein, under conditions suitable for expression of the antibody of the invention or polypeptide fragments thereof, and recovering the antibody of the invention or polypeptide fragments thereof from the host cell (or host cell culture medium).

In certain embodiments the antigen binding domains capable of specific binding to a tumor-associated antigen or antigen binding domains capable of specific binding to ICOS (e.g. Fab fragments or VH and VL) forming part of the antigen binding molecule comprise at least an immunoglobulin variable region capable of binding to an antigen. Variable regions can form part of and be derived from naturally or non-naturally occurring antibodies and fragments thereof. Methods to produce polyclonal antibodies and monoclonal antibodies are well known in the art (see e.g. Harlow and Lane, "Antibodies, a laboratory manual", Cold Spring Harbor Laboratory, 1988). Non-naturally occurring antibodies can be constructed using solid phase-peptide synthesis, can be produced recombinantly (e.g. as described in U.S. patent No. 4,186,567) or can be obtained, for example, by screening combinatorial libraries comprising variable heavy chains and variable light chains (see e.g. U.S. Patent. No. 5,969,108 to McCafferty).

Any animal species of immunoglobulin can be used in the invention. Non-limiting immunoglobulins useful in the present invention can be of murine, primate, or human origin. If the fusion protein is intended for human use, a chimeric form of immunoglobulin may be used wherein the constant regions of the immunoglobulin are from a human. A humanized or fully human form of the immunoglobulin can also be prepared in accordance with methods well known in the art (see e. g. U.S. Patent No. 5,565,332 to Winter). Humanization may be achieved by various methods including, but not limited to (a) grafting the non-human (e.g., donor antibody) CDRs onto human (e.g. recipient antibody) framework and constant regions with or without retention of critical framework residues (e.g. those that are important for retaining good antigen binding affinity or antibody functions), (b) grafting only the non-human specificity-determining regions (SDRs or a-CDRs; the residues critical for the antibody-antigen interaction) onto human framework and constant regions, or (c) transplanting the entire non-human variable domains, but "cloaking" them with a human-like section by replacement of surface residues. Humanized antibodies and methods of making them are reviewed, e.g., in Almagro and Fransson, Front Biosci 13, 1619-1633 (2008), and are further described, e.g., in Riechmann et al., Nature 332, 323-329 (1988); Queen et al., Proc Natl Acad Sci USA 86, 10029-10033 (1989); US Patent Nos. 5,821,337, 7,527,791, 6,982,321, and 7,087,409; Jones et al., Nature 321, 522-525 (1986); Morrison et al., Proc Natl Acad Sci 81, 6851-6855 (1984); Morrison and Oi, Adv Immunol 44, 65-92 (1988); Verhoeyen et al., Science 239, 1534-1536 (1988); Padlan, Molec Immun 31(3), 169-217 (1994); Kashmiri et al., Methods 36, 25-34 (2005) (describing SDR (a-CDR) grafting); Padlan, Mol Immunol 28, 489-498 (1991) (describing "resurfacing"); Dall'Acqua et al., Methods 36, 43-60 (2005) (describing "FR shuffling"); and Osbourn et al., Methods 36, 61-68 (2005) and Klimka et al., Br J Cancer 83, 252-260 (2000) (describing the "guided selection" approach to FR shuffling). Particular immunoglobulins according to the invention are human immunoglobulins. Human antibodies and human variable regions can be produced using various techniques known in the art. Human antibodies are described generally in van Dijk and van de Winkel, Curr Opin Pharmacol 5, 368-74 (2001) and Lonberg, Curr Opin Immunol 20, 450-459 (2008). Human variable regions can form part of and be derived from human monoclonal antibodies made by the hybridoma method (see e.g. Monoclonal Antibody Production Techniques and Applications, pp. 51-63 (Marcel Dekker, Inc., New York, 1987)). Human antibodies and human variable regions may also be prepared by administering an immunogen to a transgenic animal that has been modified to produce intact human antibodies or intact antibodies with human variable regions in response to antigenic challenge (see e.g. Lonberg, Nat Biotech 23, 1117-1125 (2005). Human antibodies and human variable regions may also be generated by isolating Fv clone variable region sequences selected from human-derived phage display libraries (see e.g., Hoogenboom et al. in Methods in Molecular Biology 178, 1-37 (O'Brien et al., ed., Human Press, Totowa, NJ, 2001); and McCafferty et al., Nature 348, 552-554; Clackson et al., Nature 352, 624-628 (1991)). Phage typically display antibody fragments, either as single-chain Fv (scFv) fragments or as Fab fragments.

In certain aspects, the antikgne binding domains are engineered to have enhanced binding affinity according to, for example, the methods disclosed in PCT publication WO 2012/020006 (see Examples relating to affinity maturation) or U.S. Pat. Appl. Publ. No. 2004/0132066. The ability of the antigen binding molecules of the invention to bind to a specific antigenic determinant can be measured either through an enzyme-linked immunosorbent assay (ELISA) or other techniques familiar to one of skill in the art, e.g. surface plasmon resonance technique (Liljeblad, et al., Glyco J 17, 323-329 (2000)), and traditional binding assays (Heeley, Endocr Res 28, 217-229 (2002)). Competition assays may be used to identify an antigen binding molecule that competes with a reference antibody for binding to a particular antigen. In certain embodiments, such a competing antigen binding molecule binds to the same epitope (e.g. a linear or a conformational epitope) that is bound by the reference antigen binding molecule. Detailed exemplary methods for mapping an epitope to which an antigen binding molecule binds are provided in Morris (1996) "Epitope Mapping Protocols", in Methods in Molecular Biology vol. 66 (Humana Press, Totowa, NJ). In an exemplary competition assay, immobilized antigen is incubated in a solution comprising a first labeled antigen binding molecule that binds to the antigen and a second unlabeled antigen binding molecule that is being tested for its ability to compete with the first antigen binding molecule for binding to the antigen. The second antigen binding molecule may be present in a hybridoma supernatant. As a control, immobilized antigen is incubated in a solution comprising the first labeled antigen binding molecule but not the second unlabeled antigen binding molecule. After incubation under conditions permissive for binding of the first antibody to the antigen, excess unbound antibody is removed, and the amount of label associated with immobilized antigen is measured. If the amount of label associated with immobilized antigen is substantially reduced in the test sample relative to the control sample, then that indicates that the second antigen binding molecule is competing with the first antigen binding molecule for binding to the antigen. See Harlow and Lane (1988) Antibodies: A Laboratory Manual ch.14 (Cold Spring Harbor Laboratory, Cold Spring Harbor, NY).

Agonistic ICOS-binding molecules of the invention prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the bispecific antigen binding molecule binds. For example, for affinity chromatography purification of fusion proteins of the invention, a matrix with protein A or protein G may be used. Sequential Protein A or G affinity chromatography and size exclusion chromatography can be used to isolate an antigen binding molecule essentially as described in the Examples. The purity of the bispecific antigen binding molecule or fragments thereof can be determined by any of a variety of well-known analytical methods including gel electrophoresis, high pressure liquid chromatography, and the like. For example, the bispecific antigen binding molecules expressed as described in the Examples were shown to be intact and properly assembled as demonstrated by reducing and non-reducing SDS-PAGE.

### Assays

The antigen binding molecules provided herein may be identified, screened for, or characterized for their physical/chemical properties and/or biological activities by various assays known in the art.

### 1. Affinity assays

The affinity of the antibody provided herein for ICOS or the tumor-associated antigen can be determined in accordance with the methods set forth in the Examples by surface plasmon resonance (SPR), using standard instrumentation such as a BIAcore instrument (GE Healthcare), and receptors or target proteins such as may be obtained by recombinant expression. The affinity of the bispecific antigen binding molecule for the target cell antigen can also be determined by surface plasmon resonance (SPR), using standard instrumentation such as a BIAcore instrument (GE Healthcare), and receptors or target proteins such as may be obtained by recombinant expression. A specific illustrative and exemplary embodiment for measuring binding affinity is described in Example 9. According to one aspect, $K_D$ is measured by surface plasmon resonance using a BIACORE® T100 machine (GE Healthcare) at 25 °C.

### 2. Binding assays and other assays

In one aspect, an antibody as reported herein is tested for its antigen binding activity, e.g., by known methods such as ELISA, Western blot, flow cytometry, etc.

### 3. Activity assays

Several cell-based in vitro assays were performed to evaluate the activity of the agonistic ICOS-binding molecules comprising at least one antigen binding domain that binds to a tumor-associated antigen. The assays were designed to show additional agonistic/co-stimulatory activity of the anti-ICOS bispecific molecules in presence of T-cell bispecific-(TCB) mediated activation of T-cells. For example, a Jurkat assay with a reporter cell line with NFAT-regulated expression of luciferase, induced upon engagement of the CD3/TCR and ICOS), wherein ICOS IgG molecules, plate-bound vs. in solution and in absence versus presence of a coated CD3 IgG stimulus were measured, is described in more detail in Example 11,

Furthermore, primary human PBMC co-culture assays, wherein FAP-targeted ICOS molecules, cross-linked by simultaneous binding to human ICOS on T-cells and human FAP, expressed on 3T3-hFAP cells (parental cell line ATCC #CCL-92, modified to stably overexpress human FAP), in the presence of a TCB molecule being crosslinked by simultaneous binding to CD3 on T-cells and human CEA on tumor cells were tested and described in Example 12. A primary murine splenocyte co-culture assay is described in Example 13.

In certain aspects, an antibody as reported herein is tested for such biological activity.

**Pharmaceutical Compositions, Formulations and Routes of Administation**

In a further aspect, the invention provides pharmaceutical compositions comprising an agonistic ICOS-binding molecule comprising at least one antigen binding domain that binds to a tumor-associated antigen and a T-cell activating anti-CD3 bispecific antibody specific for a tumor-associated antigen and pharmaceutically acceptable excipients. In a particular aspect, there is provided a pharmaceutical composition comprising an agonistic ICOS-binding molecule comprising at least one antigen binding domain that binds to a tumor-associated antigen and a T-cell activating anti-CD3 bispecific antibody specific for a tumor-associated antigen and pharmaceutically acceptable excipients for use in the treatment of cancer, more particularly for the treatment of solid tumors. In another aspect, a pharmaceutical composition comprises an an agonistic ICOS-binding molecule provided herein and at least one pharmaceutically acceptable excipient. In another aspect, a pharmaceutical composition comprises an agonistic ICOS-binding molecule provided herein and at least one additional therapeutic agent, e.g., as described below.

In yet another aspect, the invention provides a pharmaceutical compositions comprising an agonistic ICOS-binding molecule comprising at least one antigen binding domain that binds to a tumor-associated antigen for use in a method for treating or delaying progression of cancer, wherein the agonistic ICOS-binding molecule comprising at least one antigen binding domain that binds to a tumor-associated antigen is used in combination with a a T-cell activating anti-CD3 bispecific antibody specific for a tumor-associated antigen and in combination with an agent blocking PD-L1/PD-1 interaction. In particular, the agent blocking PD-L1/PD-1 interaction is an anti-PD-LI antibody or an anti-PDI antibody. More particularly, the agent blocking PD-L1/PD-1 interaction is selected from the group consisting of atezolizumab, durvalumab, pembrolizumab and nivolumab. In a specific aspect, the agent blocking PD-L1/PD-1 interaction is atezolizumab.

Pharmaceutical compositions of the present invention comprise a therapeutically effective amount of one or more antibodies dissolved or dispersed in a pharmaceutically acceptable excipient. The phrases "pharmaceutical or pharmacologically acceptable" refers to molecular entities and compositions that are generally non-toxic to recipients at the dosages and concentrations employed, i.e. do not produce an adverse, allergic or other untoward reaction when administered to an animal, such as, for example, a human, as appropriate. The preparation of a pharmaceutical composition that contains at least one antibody and optionally an additional active ingredient will be known to those of skill in the art in light of the present disclosure, as exemplified by Remington's Pharmaceutical Sciences, 18th Ed. Mack Printing Company, 1990, incorporated herein by reference. In particular, the compositions are lyophilized formulations or aqueous solutions. As used herein, "pharmaceutically acceptable excipient" includes any and all solvents, buffers, dispersion media, coatings, surfactants, antioxidants, preservatives (e.g. antibacterial agents, antifungal agents), isotonic agents, salts, stabilizers and combinations thereof, as would be known to one of ordinary skill in the art.

Parenteral compositions include those designed for administration by injection, e.g. subcutaneous, intradermal, intralesional, intravenous, intraarterial intramuscular, intrathecal or intraperitoneal injection. For injection, the TNF family ligand trimer-containing antigen binding molecules of the invention may be formulated in aqueous solutions, preferably in physiologically compatible buffers such as Hanks' solution, Ringer's solution, or physiological saline buffer. The solution may contain formulatory agents such as suspending, stabilizing and/or dispersing agents. Alternatively, the fusion proteins may be in powder form for constitution with a suitable vehicle, e.g., sterile pyrogen-free water, before use. Sterile injectable solutions are prepared by incorporating the fusion proteins of the invention in the required amount in the appropriate solvent with various of the other ingredients enumerated below, as required. Sterility may be readily accomplished, e.g., by filtration through sterile filtration membranes. Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein. Suitable pharmaceutically acceptable excipients include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Aqueous injection suspensions may contain compounds which increase the viscosity of the suspension, such as sodium carboxymethyl cellulose, sorbitol, dextran, or the like. Optionally, the suspension may also contain suitable stabilizers or agents which increase the solubility of the compounds to allow for the preparation of highly concentrated solutions. Additionally, suspensions of the active compounds may be prepared as appropriate oily injection suspensions. Suitable lipophilic solvents or vehicles include fatty oils such as sesame oil, or synthetic fatty acid esters, such as ethyl cleats or triglycerides, or liposomes.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences (18th Ed. Mack Printing Company, 1990). Sustained-release preparations may be prepared. Suitable examples of sustained-release preparations include semipermeable matrices of solid hydrophobic polymers containing the polypeptide, which matrices are in the form of shaped articles, e.g. films, or microcapsules. In particular embodiments, prolonged absorption of an injectable composition can be brought about by the use in the compositions of agents delaying absorption, such as, for example, aluminum monostearate, gelatin or combinations thereof.

Exemplary pharmaceutically acceptable excipients herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

In addition to the compositions described previously, the fusion proteins may also be formulated as a depot preparation. Such long acting formulations may be administered by implantation (for example subcutaneously or intramuscularly) or by intramuscular injection. Thus, for example, the fusion proteins may be formulated with suitable polymeric or hydrophobic materials (for example as an emulsion in an acceptable oil) or ion exchange resins, or as sparingly soluble derivatives, for example, as a sparingly soluble salt.

Pharmaceutical compositions comprising the fusion proteins of the invention may be manufactured by means of conventional mixing, dissolving, emulsifying, encapsulating, entrapping or lyophilizing processes. Pharmaceutical compositions may be formulated in conventional manner using one or more physiologically acceptable carriers, diluents, excipients or auxiliaries which facilitate processing of the proteins into preparations that can be used pharmaceutically. Proper formulation is dependent upon the route of administration chosen.

The agonistic ICOS-binding molecule of the invention may be formulated into a composition in a free acid or base, neutral or salt form. Pharmaceutically acceptable salts are salts that substantially retain the biological activity of the free acid or base. These include the acid addition salts, e.g. those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine. Pharmaceutical salts tend to be more soluble in aqueous and other protic solvents than are the corresponding free base forms.

The composition herein may also contain more than one active ingredients as necessary for the particular indication being treated, preferably those with complementary activities that do not adversely affect each other. Such active ingredients are suitably present in combination in amounts that are effective for the purpose intended.

The formulations to be used for in vivo administration are generally sterile. Sterility may be readily accomplished, e.g., by filtration through sterile filtration membranes.

**Therapeutic methods and compositions**

In one aspect, provided is a method for treating or delaying progression of cancer in a subject comprising administering to the subject an effective amount of an agonistic ICOS-binding molecule comprising at least one antigen binding domain that binds to a tumor-associated antigen and a T-cell activating anti-CD3 bispecific antibody, in particular a anti-CEA/anti-CD3 bispecific antibody.

In one such aspect, the method further comprises administering to the subject an effective amount of at least one additional therapeutic agent. In further embodiments, herein is provided a method for tumor shrinkage comprising administering to the subject an effective amount of an agonistic ICOS-binding molecule comprising at least one antigen binding domain that binds to a tumor-associated antigen and a T-cell activating anti-CD3 bispecific antibody, in particular an anti-CEA/anti-CD3 bispecific antibody. An "individual" or a "subject" according to any of the above aspects is preferably a human.

In further aspects, a composition for use in cancer immunotherapy is provided comprising an agonistic ICOS-binding molecule comprising at least one antigen binding domain that binds to a tumor-associated antigen and a T-cell activating anti-CD3 bispecific antibody, in particular a anti-CEA/anti-CD3 bispecific antibody. In certain embodiments, a composition comprising an agonistic ICOS-binding molecule comprising at least one antigen binding domain that binds to a tumor-associated antigen and a T-cell activating anti-CD3 bispecific antibody, in particular an anti-CEA/anti-CD3 bispecific antibody, for use in a method of cancer immunotherapy is provided.

In a further aspect, herein is provided for the use of a composition comprising an agonistic ICOS-binding molecule comprising at least one antigen binding domain that binds to a tumor-associated antigen and a T-cell activating anti-CD3 bispecific antibody, in particular a anti-CEA/anti-CD3 bispecific antibody, in the manufacture or preparation of a medicament. In one aspect, the medicament is for treatment of cancer. In a further aspect, the medicament is for use in a method of tumor shrinkage comprising administering to an individual having a solid tumor an effective amount of the medicament. In one such aspect, the method further comprises administering to the individual an effective amount of at least one additional therapeutic agent. In a further embodiment, the medicament is for treating solid tumors. In some aspects, the individual has CEA positive cancer. In some aspects, CEA positive cancer is colon cancer, lung cancer, ovarian cancer, gastric cancer, bladder cancer, pancreatic cancer, endometrial cancer, breast cancer, kidney cancer, esophageal cancer, or prostate cancer. In some aspects, the breast cancer is a breast carcinoma or a breast adenocarcinoma. In some aspects, the breast carcinoma is an invasive ductal carcinoma. In some aspects, the lung cancer is a lung adenocarcinoma. In some embodiments, the colon cancer is a colorectal adenocarcinoma. A "subject" or an "individual" according to any of the above embodiments may be a human.

In another aspect, provided is a method for treating or delaying progression of cancer in a subject comprising administering to the subject an effective amount of an agonistic ICOS-binding molecule comprising at least one antigen binding domain that binds to a tumor-associated antigen and a T-cell activating anti-CD3 bispecific antibody, in particular a anti-CEA/anti-CD3 bispecific antibody, wherein the subject comprises a low ICOS baseline expression on T cells before treatment with the agonistic ICOS-binding molecule.

The combination therapies noted above encompass combined administration (where two or more therapeutic agents are included in the same or separate formulations), and separate administration, in which case, administration of the antibody as reported herein can occur prior to, simultaneously, and/or following, administration of the additional therapeutic agent or agents. In one aspect, administration of a T-cell activating anti-CD3 bispecific antibody, in particular a anti-CEA/anti-CD3 bispecific antibody, and of an agonistic ICOS-binding molecule comprising at least one antigen binding domain that binds to a tumor-associated antigen and optionally the administration of an additional therapeutic agent occur within about one month, or within about one, two or three weeks, or within about one, two, three, four, five, or six days, of each other.

Both the T-cell activating anti-CD3 bispecific antibody, in particular a anti-CEA/anti-CD3 bispecific antibody, and the agonistic ICOS-binding molecule comprising at least one antigen binding domain that binds to a tumor-associated antigen as reported herein (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Both the T-cell activating anti-CD3 bispecific antibody, in particular a anti-CEA/anti-CD3 bispecific antibody, and the agonistic ICOS-binding molecule comprising at least one antigen binding domain that binds to a tumor-associated antigen as reported herein would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibodies need not be, but are optionally formulated with one or more agents currently used to prevent or treat the disorder in question. The effective amount of such other agents depends on the amount of antibodies present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

**Other agents and treatments**

The agonistic ICOS-binding molecules comprising at least one antigen binding domain that binds to a tumor-associated antigen of the invention may be administered in combination with one or more other agents in therapy. For instance, an agonistic ICOS-binding molecules of the invention may be co-administered with at least one additional therapeutic agent. The term "therapeutic agent" encompasses any agent that can be administered for treating a symptom or disease in an individual in need of such treatment. Such additional therapeutic agent may comprise any active ingredients suitable for the particular indication being treated, preferably those with complementary activities that do not adversely affect each other. In certain embodiments, an additional therapeutic agent is another anti-cancer agent. In one aspect, the additional therapeutic agent is selected from the group consisting of a chemotherapeutic agent, radiation and other agents for use in cancer immunotherapy. In a further aspect, provided is the agonistic ICOS-binding molecule comprising at least one antigen binding domain that binds to a tumor-associated antigen as described herein before as described herein for use in the treatment of cancer, wherein the agonistic ICOS-binding molecule comprising at least one antigen binding domain that binds to a tumor-associated antigen is administered in combination with another immunomodulator.

The term "immunomodulator" refers to any substance including a monoclonal antibody that effects the immune system. The molecules of the inventions can be considered immunomodulators. Immunomodulators can be used as anti-neoplastic agents for the treatment of cancer. In one aspect, immunomodulators include, but are not limited to anti-CTLA4 antibodies (e.g. ipilimumab), anti-PDI antibodies (e.g. nivolumab or pembrolizumab), PD-L1 antibodies (e.g. atezolizumab, avelumab or durvalumab), OX-40 antibodies, LAG3 antibodies, TIM-3 antibodies, 4-1BB antibodies and GITR antibodies.

In a further aspect, provided is the agonistic ICOS-binding molecule comprising at least one antigen binding domain that binds to a tumor-associated antigen as described herein before as described herein for use in the treatment of cancer, wherein the agonistic ICOS-binding molecule comprising at least one antigen binding domain that binds to a tumor-associated antigen is administered in combination with an agent blocking PD-L1/PD-1 interaction. In one aspect, the agent blocking PD-L1/PD-1 interaction is an anti-PD-L1 antibody or an anti-PD1 antibody. More particularly, the agent blocking PD-L1/PD-1 interaction is selected from the group consisting of atezolizumab, durvalumab, pembrolizumab and nivolumab. In a specific aspect, the agent blocking PD-L1/PD-1 interaction is atezolizumab.Such other agents are suitably present in combination in amounts that are effective for the purpose intended. The effective amount of such other agents depends on the amount of fusion protein used, the type of disorder or treatment, and other factors discussed above. The agonistic ICOS-binding molecules comprising at least one antigen binding domain that binds to a tumor-associated antigen are generally used in the same dosages and with administration routes as described herein, or about from 1 to 99% of the dosages described herein, or in any dosage and by any route that is empirically/clinically determined to be appropriate.

Such combination therapies noted above encompass combined administration (where two or more therapeutic agents are included in the same or separate compositions), and separate administration, in which case, administration of the agonistic ICOS-binding molecules comprising at least one antigen binding domain that binds to a tumor-associated antigen of the invention can occur prior to, simultaneously, and/or following, administration of the additional therapeutic agent and/or adjuvant.

**Articles of Manufacture**

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper that is pierceable by a hypodermic injection needle). At least one active agent in the composition is an agonistic ICOS-binding molecule comprising at least one antigen binding domain that binds to a tumor-associated antigen of the invention.

The label or package insert indicates that the composition is used for treating the condition of choice. Moreover, the article of manufacture may comprise (a) a first container with a composition contained therein, wherein the composition comprises an agonistic ICOS-binding molecule comprising at least one antigen binding domain that binds to a tumor-associated antigen of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition.

Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

**Table C (Sequences):**

| SEQ ID NO: | Name | Sequence |
|---|---|---|
| 1 | huICOS-L (ECD) | DTQEKEVRAM VGSDVELSCA CPEGSRFDLN DVYVYWQTSE SKTVVTYHIP QNSSLENVDS RYRNRALMSP AGMLRGDFSL RLFNVTPQDE QKFHCLVLSQ SLGFQEVLSV EVTLHVAANF SVPVVSAPHS PSQDELTFTC TSINGYPRPN VYWINKTDNS LLDQALQNDT VFLNMRGLYD VVSVLRIART PSVNIGCCIE NVLLQQNLTV GSQTGNDIGE RDKITENPVS TGEKNAAT |
| 2 | muICOS-L | ETEVGAMVGS NVVLSCIDPH RRHFNLSGLY VYWQIENPEV SVTYYLPYKS PGINVDSSYK NRGHLSLDSM KQGNFSLYLK NVTPQDTQEF TCRVFMNTAT ELVKILEEVV RLRVAANFST PVISTSDSSN PGQERTYTCM SKNGYPEPNL YWINTTDNSL IDTALQNNTV YLNKLGLYDV ISTLRLPWTS RGDVLCCVEN VALHQNITSI SQAESFTGNN TKNPQETHNN ELK |
| 3 | human ICOS | UniProt Q9Y6W8: <br><br> MKSGLWYFFL FCLRIKVLTG EINGSANYEM FIFHNGGVQI LCKYPDIVQQ FKMQLLKGGQ ILCDLTKTKG SGNTVSIKSL KFCHSQLSNN SVSFFLYNLD HSHANYYFCN LSIFDPPPFK VTLTGGYLHI YESQLCCQLK FWLPIGCAAF VVVCILGCIL ICWLTKKKYS SSVHDPNGEY MFMRAVNTAK KSRLTDVTL |
| 4 | FAP(4B9) CDR-H1 | SYAMS |
| 5 | FAP(4B9) CDR-H2 | AIIGSGASTYYADSVKG |
| 6 | FAP(4B9) CDR-H3 | GWFGGFNY |
| 7 | FAP(4B9) CDR-L1 | RASQSVTSSYLA |
| 8 | FAP(4B9) CDR-L2 | VGSRRAT |
| 9 | FAP(4B9) CDR-L3 | QQGIMLPPT |
| 10 | FAP(4B9) VH | EVQLLESGGGLVQPGGSLRLSCAASGFTFSSYAMSW VRQAPGKGLEWVSAIIGSGASTYYADSVKGRFTISRD NSKNTLYLQMNSLRAEDTAVYYCAKGWFGGFNYW GQGTLVTVSS |
| 11 | FAP(4B9) VL | EIVLTQSPGTLSLSPGERATLSCRASQSVTSSYLAWY QQKPGQAPRLLINVGSRRATGIPDRFSGSGSGTDFTL TISRLEPEDFAVYYCQQGIMLPPTFGQGTKVEIK |
| 12 | FAP (28H1) CDR-H1 | SHAMS |
| 13 | FAP (28H1) CDR-H2 | AIWASGEQYYADSVKG |
| 14 | FAP (28H1) CDR-H3 | GWLGNFDY |

(continued)

| SEQ ID NO: | Name | Sequence |
|---|---|---|
| 15 | FAP (28H1) CDR-L1 | RASQSVSRSYLA |
| 16 | FAP (28H1) CDR-L2 | GASTRAT |
| 17 | FAP (28H1) CDR-L3 | QQGQVIPPT |
| 18 | FAP(28H1) VH | EVQLLESGGGLVQPGGSLRLSCAASGFTFSSHAMSW VRQAPGKGLEWVSAIWASGEQYYADSVKGRFTISRD NSKNTLYLQMNSLRAEDTAVYYCAKGWLGNFDYW GQGTLVTVSS |
| 19 | FAP(28H1) VL | EIVLTQSPGTLSLSPGERATLSCRASQSVSRSYLAWY QQKPGQAPRLLIIGASTRATGIPDRFSGSGSGTDFTLTI SRLEPEDFAVYYCQQGQVIPPTFGQGTKVEIK |
| 20 | ICOS CDR-H1 | GYTFTGYYMH |
| 21 | ICOS CDR-H2 | WINPHSGGTNYAQKFQG |
| 22 | ICOS CDR-H3 | TYYYDSSGYYHDAFDI |
| 23 | ICOS CDR-L1 | RASQGISRLLA |
| 24 | ICOS CDR-L2 | VASSLQS |
| 25 | ICOS CDR-L3 | QQANSFPWT |
| 26 | ICOS VH | QVQLVQSGAEVKKPGASVKVSCKAS<u>GYTFTGYYMH</u> WVRQAPGQGLEWMG<u>WINPHSGGTNYAQKFQG</u>RVT MTRDTSISTAYMELSRLRSDDTAVYYCAR<u>TYYYDSS GYYHDAFDI</u>WGQGTMVTVSS |
| 27 | ICOS VL | DIQMTQSPSSVSASVGDRVTITC<u>RASQGISRLLA</u>WYQ QKPGKAPKLLIY<u>VASSLQS</u>GVPSRFSGSGSGTDFTLTI SSLQPEDFATYYC<u>QQANSFPWT</u>FGQGTKVEIK |
| 28 | VHCH1(JMAb136)- Fc knob chain | see Table 1 |
| 29 | VLCL(JMAb 136) Light chain | see Table 1 |
| 30 | VHCH1 (4B9)-Fc hole chain | see Table 1 |
| 31 | VLCL(4B9) Light chain | see Table 1 |
| 32 | VHCH1 (JMAb136)-Fc knob chain-VH (4B9) | see Table 2 |
| 33 | VHCH1 (JMAb136)-Fc hole chain-VL (4B9) | see Table 3 |
| 34 | CD3 CDR-H1 | TYAMN |
| 35 | CD3 CDR-H2 | RIRSKYNNYATYYADSVKG |
| 36 | CD3 CDR-H3 | HGNFGNSYVSWFAY |
| 37 | CD3 CDR-L1 | GSSTGAVTTSNYAN |
| 38 | CD3 CDR-L2 | GTNKRAP |
| 39 | CD3 CDR-L3 | ALWYSNLWV |

(continued)

| SEQ ID NO: | Name | Sequence |
|---|---|---|
| 40 | CD3 VH | EVQLLESGGGLVQPGGSLRLSCAASGFTFSTYAMNW VRQAPGKGLEWVSRIRSKYNNYATYYADSVKGRFTI SRDDSKNTLYLQMNSLRAEDTAVYYCVRHGNFGNS YVSWFAYWGQGTLVTVSS |
| 41 | CD3 VL | QAVVTQEPSLTVSPGGTVTLTCGSSTGAVTTSNYAN WVQEKPGQAFRGLIGGTNKRAPGTPARFSGSLLGGK AALTLSGAQPEDEAEYYCALWYSNLWVFGGGTKLT VL |
| 42 | CEA CDR-H1 | EFGMN |
| 43 | CEA CDR-H2 | WINTKTGEATYVEEFKG |
| 44 | CEA CDR-H3 | WDFAYYVEAMDY |
| 45 | CEA CDR-L1 | KASAAVGTYVA |
| 46 | CEA CDR-L2 | SASYRKR |
| 47 | CEA CDR-L3 | HQYYTYPLFT |
| 48 | CEA VH | QVQLVQSGAEVKKPGASVKVSCKASGYTFTEFGMN WVRQAPGQGLEWMGWINTKTGEATYVEEFKGRVTF TTDTSTSTAYMELRSLRSDDTAVYYCARWDFAYYV EAMDYWGQGTTVTVSS |
| 49 | CEA VL | DIQMTQSPSSLSASVGDRVTITCKASAAVGTYVAWY QQKPGKAPKLLIYSASYRKRGVPSRFSGSGSGTDFTL TISSLQPEDFATYYCHQYYTYPLFTFGQGTKLEIK |
| 50 | CEA CDR-H1 (CEACAM5) | DTYMH |
| 51 | CEA CDR-H2 (CEACAM5) | RIDPANGNSKYVPKFQG |
| 52 | CEA CDR-H3 (CEACAM5) | FGYYVSDYAMAY |
| 53 | CEA CDR-L1 (CEACAM5) | RAGESVDIFGVGFLH |
| 54 | CEA CDR-L2 (CEACAM5) | RASNRAT |
| 55 | CEA-CDR-L3 (CEACAM5) | QQTNEDPYT |
| 56 | CEA VH (CEACAM5) | QVQLVQSGAEVKKPGSSVKVSCKASGFNIKDTYMH WVRQAPGQGLEWMGRIDPANGNSKYVPKFQGRVTI TADTSTSTAYMELSSLRSEDTAVYYCAPFGYYVSDY AMAYWGQGTLVTVSS |
| 57 | CEA VL (CEACAM5) | EIVLTQSPATLSLSPGERATLSCRAGESVDIFGVGFLH WYQQKPGQAPRLLIYRASNRATGIPARFSGSGSGTDF TLTISSLEPEDFAVYYCQQTNEDPYTFGQGTKLEIK |

(continued)

| SEQ ID NO: | Name | Sequence |
|---|---|---|
| 58 | Light chain "CEA 2F1" (CEA TCB) | DIQMTQSPSSLSASVGDRVTITCKASAAVGTYVAWY QQKPGKAPKLLIYSASYRKRGVPSRFSGSGSGTDFTL TISSLQPEDFATYYCHQYYTYPLFTFGQGTKLEIKRT VAAPSVFIFPPSDEQLKSGTASVVCLLNNFYPREAKV QWKVDNALQSGNSQESVTEQDSKDSTYSLSSTLTLS KADYEKHKVYACEVTHQGLSSPVTKSFNRGEC |
| 59 | Light Chain humanized CD3 CH2527 (Crossfab, VL-CH1) (CEA TCB) | QAVVTQEPSLTVSPGGTVTLTCGSSTGAVTTSNYAN WVQEKPGQAFRGLIGGTNKRAPGTPARFSGSLLGGK AALTLSGAQPEDEAEYYCALWYSNLWVFGGGTKLT VLSSASTKGPSVFPLAPSSKSTSGGTAALGCLVKDYF PEPVTVSWNSGALTSGVHTFPAVLQSSGLYSLSSVVT VPSSSLGTQTYICNVNHKPSNTKVDKKVEPKSC |
| 60 | CEA CH1A1A 98/99 - humanized CD3 CH2527 (Crossfab VH-Ck)-Fc(knob) P329GLALA (CEA TCB) | QVQLVQSGAEVKKPGASVKVSCKASGYTFTEFGMN WVRQAPGQGLEWMGWINTKTGEATYVEEFKGRVTF TTDTSTSTAYMELRSLRSDDTAVYYCARWDFAYYV EAMDYWGQGTTVTVSSASTKGPSVFPLAPSSKSTSG GTAALGCLVKDYFPEPVTVSWNSGALTSGVHTFPAV LQSSGLYSLSSVVTVPSSSLGTQTYICNVNHKPSNTK VDKKVEPKSCDGGGGSGGGGSEVQLLESGGGLVQP GGSLRLSCAASGFTFSTYAMNWVRQAPGKGLEWVS RIRSKYNNYATYYADSVKGRFTISRDDSKNTLYLQM NSLRAEDTAVYYCVRHGNFGNSYVSWFAYWGQGT LVTVSSASVAAPSVFIFPPSDEQLKSGTASVVCLLNNF YPREAKVQWKVDNALQSGNSQESVTEQDSKDSTYS LSSTLTLSKADYEKHKVYACEVTHQGLSSPVTKSFN RGECDKTHTCPPCPAPEAAGGPSVFLFPPKPKDTLMI SRTPEVTCVVVDVSHEDPEVKFNWYVDGVEVHNAK TKPREEQYNSTYRVVSVLTVLHQDWLNGKEYKCKV SNKALGAPIEKTISKAKGQPREPQVYTLPPCRDELTK NQVSLWCLVKGFYPSDIAVEWESNGQPENNYKTTPP VLDSDGSFFLYSKLTVDKSRWQQGNVFSCSVMHEAL HNHYTQKSLSLSPGK |
| 61 | CEA CH1A1A 98/99 (VH-CH1)-Fc (hole) P329GLALA (CEA TCB) | QVQLVQSGAEVKKPGASVKVSCKASGYTFTEFGMN WVRQAPGQGLEWMGWINTKTGEATYVEEFKGRVTF TTDTSTSTAYMELRSLRSDDTAVYYCARWDFAYYV EAMDYWGQGTTVTVSSASTKGPSVFPLAPSSKSTSG GTAALGCLVKDYFPEPVTVSWNSGALTSGVHTFPAV LQSSGLYSLSSVVTVPSSSLGTQTYICNVNHKPSNTK VDKKVEPKSCDKTHTCPPCPAPEAAGGPSVFLFPPKP KDTLMISRTPEVTCVVVDVSHEDPEVKFNWYVDGV EVHNAKTKPREEQYNSTYRVVSVLTVLHQDWLNGK EYKCKVSNKALGAPIEKTISKAKGQPREPQVCTLPPS RDELTKNQVSLSCAVKGFYPSDIAVEWESNGQPENN YKTTPPVLDSDGSFFLVSKLTVDKSRWQQGNVFSCS VMHEALHNHYTQKSLSLSPGK |

(continued)

| SEQ ID NO: | Name | Sequence |
|---|---|---|
| 62 | CD3 VH-CL (CEACAM5 TCB) | EVQLLESGGGLVQPGGSLRLSCAASGFTFSTYAMNW VRQAPGKGLEWVSRIRSKYNNYATYYADSVKGRFTI SRDDSKNTLYLQMNSLRAEDTAVYYCVRHGNFGNS YVSWFAYWGQGTLVTVSSASVAAPSVFIFPPSDEQL KSGTASVVCLLNNFYPREAKVQWKVDNALQSGNSQ ESVTEQDSKDSTYSLSSTLTLSKADYEKHKVYACEV THQGLSSPVTKSFNRGEC |
| 63 | humanized CEA VH-CH1 (EE)-Fc (hole, P329G LALA) (CEACAM5 TCB) | QVQLVQSGAEVKKPGSSVKVSCKASGFNIKDTYMH WVRQAPGQGLEWMGRIDPANGNSKYVPKFQGRVTI TADTSTSTAYMELSSLRSEDTAVYYCAPFGYYVSDY AMAYWGQGTLVTVSSASTKGPSVFPLAPSSKSTSGG TAALGCLVEDYFPEPVTVSWNSGALTSGVHTFPAVL QSSGLYSLSSVVTVPSSSLGTQTYICNVNHKPSNTKV DEKVEPKSCDKTHTCPPCPAPEAAGGPSVFLFPPKPK DTLMISRTPEVTCVVVDVSHEDPEVKFNWYVDGVE VHNAKTKPREEQYNSTYRVVSVLTVLHQDWLNGKE YKCKVSNKALGAPIEKTISKAKGQPREPQVCTLPPSR DELTKNQVSLSCAVKGFYPSDIAVEWESNGQPENNY |
| | | KTTPPVLDSDGSFFLVSKLTVDKSRWQQGNVFSCSV MHEALHNHYTQKSLSLSP |
| 64 | humanized CEA VH-CH1 (EE)-CD3 VL-CH1-Fc (knob, P329G LALA) (CEACAM5 TCB) | QVQLVQSGAEVKKPGSSVKVSCKASGFNIKDTYMH WVRQAPGQGLEWMGRIDPANGNSKYVPKFQGRVTI TADTSTSTAYMELSSLRSEDTAVYYCAPFGYYVSDY AMAYWGQGTLVTVSSASTKGPSVFPLAPSSKSTSGG TAALGCLVEDYFPEPVTVSWNSGALTSGVHTFPAVL QSSGLYSLSSVVTVPSSSLGTQTYICNVNHKPSNTKV DEKVEPKSCDGGGGSGGGGSQAVVTQEPSLTVSPGG TVTLTCGSSTGAVTTSNYANWVQEKPGQAFRGLIGG TNKRAPGTPARFSGSLLGGKAALTLSGAQPEDEAEY YCALWYSNLWVFGGGTKLTVLSSASTKGPSVFPLAP SSKSTSGGTAALGCLVKDYFPEPVTVSWNSGALTSG VHTFPAVLQSSGLYSLSSVVTVPSSSLGTQTYICNVN HKPSNTKVDKKVEPKSCDKTHTCPPCPAPEAAGGPS VFLFPPKPKDTLMISRTPEVTCVVVDVSHEDPEVKFN WYVDGVEVHNAKTKPREEQYNSTYRVVSVLTVLHQ DWLNGKEYKCKVSNKALGAPIEKTISKAKGQPREPQ VYTLPPCRDELTKNQVSLWCLVKGFYPSDIAVEWES NGQPENNYKTTPPVLDSDGSFFLYSKLTVDKSRWQQ GNVFSCSVMHEALHNHYTQKSLSLSP |
| 65 | humanized CEA VL-CL(RK) (CEACAM5 TCB) | EIVLTQSPATLSLSPGERATLSCRAGESVDIFGVGFLH WYQQKPGQAPRLLIYRASNRATGIPARFSGSGSGTDF TLTISSLEPEDFAVYYCQQTNEDPYTFGQGTKLEIKRT VAAPSVFIFPPSDRKLKSGTASVVCLLNNFYPREAKV QWKVDNALQSGNSQESVTEQDSKDSTYSLSSTLTLS KADYEKHKVYACEVTHQGLSSPVTKSFNRGEC |

(continued)

| SEQ ID NO: | Name | Sequence |
|---|---|---|
| 66 | Fc hole chain-VL (4B9) | see Table 2 |
| 67 | VHCH1 (DP47) Fc hole chain | see Table 4 |
| 68 | VLCL(DP47) Light chain | see Table 4 |
| 69 | VHCH1 (JMAb136)-Fc knob chain-VH (DP47) | see Table 5 |
| 70 | VHCH1 (JMAb136)-Fc hole chain-VL (DP47) | see Table 5 |
| 71 | Murine ICOSL Linker muIgG1 Fc (DAPG KK) 4GS linker FAP (28H1) VH | see Table 7 |
| 72 | Murine ICOSL linker muIgG1 Fc (DAPG DD) 4GS linker FAP (28H1) VL | see Table 7 |
| 73 | Murine ICOSL Linker muIgG1 Fc (DAPG KK) G4S linker DP47 VH | see Table 8 |
| 74 | Murine ICOSL linker muIgG1 Fc (DAPG DD) 4GS linker DP47 VL | see Table 8 |
| 75 | VHCH1(CH1A1A 98/99 2F1)-Fc (KK) DAPG chain | see Table 10 |
| 76 | VLCL (CH1A1A 98/99 2F1) Light chain | see Table 10 |
| 77 | VHCL VHCH1 (2C11-CH1A1A 98/99 2F1)-Fc(DD) DAPG chain | see Table 10 |
| 78 | VLCH1 (2C11) Light chain | see Table 10 |
| 79 | human FAP | UniProt accession no. Q12884 |

(continued)

| SEQ ID NO: | Name | Sequence |
|---|---|---|
| 80 | His-tagged human FAP ECD | RPSRVHNSEENTMRALTLKDILNGTFSYKTFFPNWIS GQEYLHQSADNNIVLYNIETGQSYTILSNRTMKSVNA SNYGLSPDRQFVYLESDYSKLWRYSYTATYYIYDLS NGEFVRGNELPRPIQYLCWSPVGSKLAYVYQNNIYL KQRPGDPPFQITFNGRENKIFNGIPDWVYEEEMLATK YALWWSPNGKFLAYAEFNDTDIPVIAYSYYGDEQYP RTINIPYPKAGAKNPVVRIFIIDTTYPAYVGPQEVPVP AMIASSDYYFSWLTWVTDERVCLQWLKRVQNVSVL SICDFREDWQTWDCPKTQEHIEESRTGWAGGFFVST PVFSYDAISYYKIFSDKDGYKHIHYIKDTVENAIQITS GKWEAINIFRVTQDSLFYSSNEFEEYPGRRNIYRISIGS YPPSKKCVTCHLRKERCQYYTASFSDYAKYYALVCY GPGIPISTLHDGRTDQEIKILEENKELENALKNIQLPKE EIKKLEVDEITLWYKMILPPQFDRSKKYPLLIQVYGG PCSQSVRSVFAVNWISYLASKEGMVIALVDGRGTAF QGDKLLYAVYRKLGVYEVEDQITAVRKFIEMGFIDE KRIAIWGWSYGGYVSSLALASGTGLFKCGIAVAPVSS WEYYASVYTERFMGLPTKDDNLEHYKNSTVMARAE YFRNVDYLLIHGTADDNVHFQNSAQIAKALVNAQV DFQAMWYSDQNHGLSGLSTNHLYTHMTHFLKQCFS LSDGKKKKKKGHHHHHH |
| 81 | mouse FAP | UniProt accession no. P97321 |
| 82 | His-tagged mouse FAP ECD | RPSRVYKPEGNTKRALTLKDILNGTFSYKTYFPNWIS EQEYLHQSEDDNIVFYNIETRESYIILSNSTMKSVNAT DYGLSPDRQFVYLESDYSKLWRYSYTATYYIYDLQN GEFVRGYELPRPIQYLCWSPVGSKLAYVYQNNIYLK QRPGDPPFQITYTGRENRIFNGIPDWVYEEEMLATKY ALWWSPDGKFLAYVEFNDSDIPIIAYSYYGDGQYPR TINIPYPKAGAKNPVVRVFIVDTTYPHHVGPMEVPVP EMIASSDYYFSWLTWVSSERVCLQWLKRVQNVSVL SICDFREDWHAWECPKNQEHVEESRTGWAGGFFVST PAFSQDATSYYKIFSDKDGYKHIHYIKDTVENAIQITS GKWEAIYIFRVTQDSLFYSSNEFEGYPGRRNIYRISIG NSPPSKKCVTCHLRKERCQYYTASFSYKAKYYALVC YGPGLPISTLHDGRTDQEIQVLEENKELENSLRNIQLP KVEIKKLKDGGLTFWYKMILPPQFDRSKKYPLLIQVY GGPCSQSVKSVFAVNWITYLASKEGIVIALVDGRGTA FQGDKFLHAVYRKLGVYEVEDQLTAVRKFIEMGFID EERIAIWGWSYGGYVSSLALASGTGLFKCGIAVAPVS SWEYYASIYSERFMGLPTKDDNLEHYKNSTVMARA EYFRNVDYLLIHGTADDNVHFQNSAQIAKALVNAQV DFQAMWYSDQNHGILSGRSQNHLYTHMTHFLKQCF |
| | | SLSDGKKKKKKGHHHHHH |

(continued)

| SEQ ID NO: | Name | Sequence |
|---|---|---|
| 83 | His-tagged cynomolgus FAP ECD | RPPRVHNSEENTMRALTLKDILNGTFSYKTFFPNWIS GQEYLHQSADNNIVLYNIETGQSYTILSNRTMKSVNA SNYGLSPDRQFVYLESDYSKLWRYSYTATYYIYDLS NGEFVRGNELPRPIQYLCWSPVGSKLAYVYQNNIYL KQRPGDPPFQITFNGRENKIFNGIPDWVYEEEMLATK YALWWSPNGKFLAYAEFNDTDIPVIAYSYYGDEQYP RTINIPYPKAGAKNPFVRIFIIDTTYPAYVGPQEVPVP AMIASSDYYFSWLTWVTDERVCLQWLKRVQNVSVL SICDFREDWQTWDCPKTQEHIEESRTGWAGGFFVST PVFSYDAISYYKIFSDKDGYKHIHYIKDTVENAIQITS GKWEAINIFRVTQDSLFYSSNEFEDYPGRRNIYRISIG SYPPSKKCVTCHLRKERCQYYTASFSDYAKYYALVC YGPGIPISTLHDGRTDQEIKILEENKELENALKNIQLP KEEIKKLEVDEITLWYKMILPPQFDRSKKYPLLIQVY GGPCSQSVRSVFAVNWISYLASKEGMVIALVDGRGT AFQGDKLLYAVYRKLGVYEVEDQITAVRKFIEMGFI DEKRIAIWGWSYGGYVSSLALASGTGLFKCGIAVAP VSSWEYYASVYTERFMGLPTKDDNLEHYKNSTVMA RAEYFRNVDYLLIHGTADDNVHFQNSAQIAKALVNA QVDFQAMWYSDQNHGLSGLSTNHLYTHMTHFLKQ CFSLSDGKKKKKKGHHHHHH |
| 84 | human CEA | UniProt accession no. P06731 |
| 85 | human FolR1 | UniProt accession no. P15328 |
| 86 | murine FolR1 | UniProt accession no. P35846 |
| 87 | cynomolgus FolR1 | UniProt accession no. G7PR14 |
| 88 | human MCSP | UniProt accession no. Q6UVK1 |
| 89 | human EGFR | UniProt accession no. P00533 |
| 90 | human HER2 | Uniprot accession no. P04626 |

(continued)

| SEQ ID NO: | Name | Sequence |
|---|---|---|
| 91 | p95 HER2 | MPIWKFPDEEGACQPCPINCTHSCVDLDDKGCPAEQ RASPLTSIISAVVGILLVVVLGVVFGILIKRRQQKIRKY TMRRLLQETELVEPLTPSGAMPNQAQMRILKETELR KVKVLGSGAFGTVYKGIWIPDGENVKIPVAIKVLREN TSPKANKEILDEAYVMAGVGSPYVSRLLGICLTSTVQ LVTQLMPYGCLLDHVRENRGRLGSQDLLNWCMQIA KGMSYLEDVRLVHRDLAARNVLVKSPNHVKITDFG LARLLDIDETEYHADGGKVPIKWMALESILRRRFTHQ SDVWSYGVTVWELMTFGAKPYDGIPAREIPDLLEKG ERLPQPPICTIDVYMIMVKCWMIDSECRPRFRELVSEF SRMARDPQRFVVIQNEDLGPASPLDSTFYRSLLEDDD MGDLVDAEEYLVPQQGFFCPDPAPGAGGMVHHRHR SSSTRSGGGDLTLGLEPSEEEAPRSPLAPSEGAGSDVF DGDLGMGAAKGLQSLPTHDPSPLQRYSEDPTVPLPS ETDGYVAPLTCSPQPEYVNQPDVRPQPPSPREGPLPA ARPAGATLERPKTLSPGKNGVVKDVFAFGGAVENPE YLTPQGGAAPQPHPPPAFSPAFDNLYYWDQDPPERG APPSTFKGTPTAENPEYLGLDVPV |
| 92 | Peptide linker (G4S) | GGGGS |
| 93 | Peptide linker (G4S)$_2$ | GGGGSGGGGS |
| 94 | Peptide linker (SG4)$_2$ | SGGGGSGGGG |
| 95 | Peptide linker G4(SG4)$_2$ | GGGGSGGGGSGGGG |
| 96 | peptide linker | GSPGSSSSGS |
| 97 | (G4S)$_3$ peptide linker | GGGGSGGGGSGGGGS$_3$ |
| 98 | (G4S)$_4$ peptide linker | GGGGSGGGGSGGGGSGGGGS |
| 99 | peptide linker | GSGSGSGS |
| 100 | peptide linker | GSGSGNGS |
| 101 | peptide linker | GGSGSGSG |
| 102 | peptide linker | GGSGSG |
| 103 | peptide linker | GGSG |
| 104 | peptide linker | GGSGNGSG |
| 105 | peptide linker | GGNGSGSG |
| 106 | peptide linker | GGNGSG |
| 107 | human PD-L1 | UniProt accession no. Q9NZQ7 |
| 108 | human PD-1 | UniProt accession no. Q15116 |
| 109 | VH (PD-L1) | EVQLVESGGGLVQPGGSLRLSCAASGFTFSDSWIHW VRQAPGKGLEWVAWISPYGGSTYYADSVKGRFTISA DTSKNTAYLQMNSLRAEDTAVYYCARRHWPGGFDY WGQGTLVTVSS |

(continued)

| SEQ ID NO: | Name | Sequence |
|---|---|---|
| 110 | VL (PD-L1) | DIQMTQSPSSLSASVGDRVTITCRASQDVSTAVAWY QQKPGKAPKLLIYSASFLYSGVPSRFSGSGSGTDFTLT ISSLQPEDFATYYCQQYLYHPATFGQGTKVEIK |
| 111 | VH (PD-L1) 2 | EVQLVESGGGLVQPGGSLRLSCAASGFTFSRYWMS WVRQAPGKGLEWVANIKQDGSEKYYVDSVKGRFTI SRDNAKNSLYLQMNSLRAEDTAVYYCAREGGWFGE LAFDYWGQGTLVTVSS |
| 112 | VL (PD-L1) 2 | EIVLTQSPGTLSLSPGERATLSCRASQRVSSSYLAWY QQKPGQAPRLLIYDASSRATGIPDRFSGSGSGTDFTLT ISRLEPEDFAVYYCQQYGSLPWTFGQGTKVEIK |
| 113 | VH (PD-1) | QVQLVQSGVEVKKPGASVKVSCKASGYTFTNYYMY WVRQAPGQGLEWMGGINPSNGGTNFNEKFKNRVTL TTDSSTTTAYMELKSLQFDDTAVYYCARRDYRFDM GFDYWGQGTTVTVSS |
| 114 | VL (PD-1) | EIVLTQSPATLSLSPGERATLSCRASKGVSTSGYSYLH WYQQKPGQAPRLLIYLASYLESGVPARFSGSGSGTDF TLTISSLEPEDFAVYYCQHSRDLPLTFGGGTKVEIK |
| 115 | VH (PD-1) 2 | QVQLVESGGGVVQPGRSLRLDCKASGITFSNSGMHW VRQAPGKGLEWVAVIWYDGSKRYYADSVKGRFTIS RDNSKNTLFLQMNSLRAEDTAVYYCATNDDYWGQ GTLVTVSS |
| 116 | VL (PD-1) 2 | EIVLTQSPATLSLSPGERATLSCRASQSVSSYLAWYQ QKPGQAPRLLIYDASNRATGIPARFSGSGSGTDFTLTI SSLEPEDFAVYYCQQSSNWPRTFGQGTKVEIK |

General information regarding the nucleotide sequences of human immunoglobulins light and heavy chains is given in: Kabat, E.A., et al., Sequences of Proteins of Immunological Interest, 5th ed., Public Health Service, National Institutes of Health, Bethesda, MD (1991). Amino acids of antibody chains are numbered and referred to according to the numbering systems according to Kabat (Kabat, E.A., et al., Sequences of Proteins of Immunological Interest, 5th ed., Public Health Service, National Institutes of Health, Bethesda, MD (1991)) as defined above.

The following numbered paragraphs (paras) describe aspects of the present invention:

1. An agonistic ICOS-binding molecule comprising at least one antigen binding domain that binds to a tumor-associated antigen for use in a method for treating or delaying progression of cancer, wherein the agonistic ICOS-binding molecule comprising at least one antigen binding domain that binds to a tumor-associated antigen is used in combination with a T-cell activating anti-CD3 bispecific antibody specific for a tumor-associated antigen.

2. The agonistic ICOS-binding molecule comprising at least one antigen binding domain that binds to a tumor-associated antigen for use in a method of para 1, wherein the T-cell activating anti-CD3 bispecific antibody specific for a tumor-associated antigen is an anti-CEA/anti-CD3 bispecific antibody.

3. The agonistic ICOS-binding molecule comprising at least one antigen binding domain that binds to a tumor-associated antigen for use in a method of paras 1 or 2, wherein the agonistic ICOS-binding molecule comprising at least one antigen binding domain that binds to a tumor-associated antigen and the T-cell activating anti-CD3 bispecific antibody are administered together in a single composition or administered separately in two or more different compositions.

4. The agonistic ICOS-binding molecule comprising at least one antigen binding domain that binds to a tumor-associated antigen for use in a method of paras 1 to 3, wherein the agonistic ICOS-binding molecule comprising at least one antigen binding domain that binds to a tumor-associated antigen acts synergistically with the T-cell activating anti-CD3 bispecific antibody.

5. The agonistic ICOS-binding molecule comprising at least one antigen binding domain that binds to a tumor-associated antigen for use in a method of any one of paras 1 to 4, wherein the agonistic ICOS-binding molecule comprising at least one antigen binding domain that binds to a tumor-associated antigen comprises at least one ICOS-L or fragments thereof.

6. The agonistic ICOS-binding molecule comprising at least one antigen binding domain that binds to a tumor-associated antigen for use in a method of any one of paras 1 to 5, wherein the agonistic ICOS-binding molecule comprising at least one antigen binding domain that binds to a tumor-associated antigen comprises at least one ICOS-L comprising the amino acid sequence of SEQ ID NO:1 or SEQ ID NO:2.

7. The agonistic ICOS-binding molecule comprising at least one antigen binding domain that binds to a tumor-associated antigen for use in a method of any one of paras 1 to 4, wherein the agonistic ICOS-binding molecule comprising at least one antigen binding domain that binds to a tumor-associated antigen comprises at least one antigen binding domain that is capable of agonistic binding to ICOS.

8. The agonistic ICOS-binding molecule comprising at least one antigen binding domain that binds to a tumor-associated antigen for use in a method of any one of paras 1 to 4, wherein the agonistic ICOS-binding molecule comprising at least one antigen binding domain that binds to a tumor-associated antigen comprises at least one antigen binding domain that is capable of agonistic binding to human ICOS comprising the amino acid sequence of SEQ ID NO:3.

9. The agonistic ICOS-binding molecule comprising at least one antigen binding domain that binds to a tumor-associated antigen for use in a method of any one of paras 1 to 8, wherein the agonistic ICOS-binding molecule comprising at least one antigen binding domain that binds to a tumor-associated antigen is an agonistic ICOS-binding molecule comprising at least one antigen binding domain that binds to anti-Fibroblast activation protein (FAP).

10. The agonistic ICOS-binding molecule comprising at least one antigen binding domain that binds to a tumor-associated antigen for use in a method of any one of paras 1 to 9, wherein the agonistic ICOS-binding molecule comprises at least one antigen binding domain capable of specific binding to FAP comprising

(a) a heavy chain variable region (V$_H$FAP) comprising (i) CDR-H1 comprising the amino acid sequence of SEQ ID NO:4, (ii) CDR-H2 comprising the amino acid sequence of SEQ ID NO:5, and (iii) CDR-H3 comprising the amino acid sequence of SEQ ID NO:6, and a light chain variable region (V$_L$FAP) comprising (iv) CDR-L1 comprising the amino acid sequence of SEQ ID NO:7, (v) CDR-L2 comprising the amino acid sequence of SEQ ID NO:8, and (vi) CDR-L3 comprising the amino acid sequence of SEQ ID NO:9, or
(b) a heavy chain variable region (V$_H$FAP) comprising (i) CDR-H1 comprising the amino acid sequence of SEQ ID NO:12, (ii) CDR-H2 comprising the amino acid sequence of SEQ ID NO:13, and (iii) CDR-H3 comprising the amino acid sequence of SEQ ID NO:14, and a light chain variable region (V$_L$FAP) comprising (iv) CDR-L1 comprising the amino acid sequence of SEQ ID NO:15, (v) CDR-L2 comprising the amino acid sequence of SEQ ID NO:16, and (vi) CDR-L3 comprising the amino acid sequence of SEQ ID NO:17.

11. The agonistic ICOS-binding molecule comprising at least one antigen binding domain that binds to a tumor-associated antigen for use in a method of any one of paras 1 to 10, wherein the agonistic ICOS-binding molecule comprises at least one antigen binding domain capable of specific binding to FAP comprising a heavy chain variable region (V$_H$FAP) comprising an amino acid sequence of SEQ ID NO:10 and a light chain variable region (V$_L$FAP) comprising an amino acid sequence of SEQ ID NO:11 or wherein the antigen binding domain capable of specific binding to FAP comprises a heavy chain variable region (V$_H$FAP) comprising an amino acid sequence of SEQ ID NO:18 and a light chain variable region (V$_L$FAP) comprising an amino acid sequence of SEQ ID NO:19.

12. The agonistic ICOS-binding molecule comprising at least one antigen binding domain that binds to a tumor-associated antigen for use in a method of any one of paras 1 to 4 or 7 to 11, wherein the agonistic ICOS-binding molecule comprising at least one antigen binding domain that binds to a tumor-associated antigen comprises at least one antigen binding domain capable of specific binding to ICOS comprising a heavy chain variable region ($V_H$ICOS) comprising (i) CDR-H1 comprising the amino acid sequence of SEQ ID NO:20, (ii) CDR-H2 comprising the amino acid sequence of SEQ ID NO:21, and (iii) CDR-H3 comprising the amino acid sequence of SEQ ID NO:22, and a light chain variable region ($V_L$ICOS) comprising (iv) CDR-L1 comprising the amino acid sequence of SEQ ID NO:23, (v) CDR-L2 comprising the amino acid sequence of SEQ ID NO:24, and (vi) CDR-L3 comprising the amino acid sequence of SEQ ID NO:25.

13. The agonistic ICOS-binding molecule comprising at least one antigen binding domain that binds to a tumor-associated antigen for use in a method of any one of paras 1 to 4 or 7 to 12, wherein the agonistic ICOS-binding molecule comprising at least one antigen binding domain that binds to a tumor-associated antigen comprises at least one antigen binding domain comprising a heavy chain variable region ($V_H$ICOS) comprising an amino acid sequence of SEQ ID NO:26 and a light chain variable region ($V_L$ICOS) comprising an amino acid sequence of SEQ ID NO:27.

14. The agonistic ICOS-binding molecule comprising at least one antigen binding domain that binds to a tumor-associated antigen for use in a method of any one of paras 1 to 13, wherein the agonistic ICOS-binding molecule comprising at least one antigen binding domain that binds to a tumor-associated antigen comprises an IgG Fc domain, specifically an IgG1 Fc domain or an IgG4 Fc domain.

15. The agonistic ICOS-binding molecule comprising at least one antigen binding domain that binds to a tumor-associated antigen for use in a method of any one of paras 1 to 14, wherein the agonistic ICOS-binding molecule comprising at least one antigen binding domain that binds to a tumor-associated antigen comprises a Fc domain that comprises one or more amino acid substitution that reduces binding to an Fc receptor and/or effector function.

16. The agonistic ICOS-binding molecule comprising at least one antigen binding domain that binds to a tumor-associated antigen for use in a method of any one of paras 1 to 4 or 7 to 15, wherein the agonistic ICOS-binding molecule comprises a first heavy chain comprising an amino acid sequence of SEQ ID NO:28, a first light chain comprising an amino acid sequence of SEQ ID NO:29, a second heavy chain comprising an amino acid sequence of SEQ ID NO:30, and a second light chain comprising an amino acid sequence of SEQ ID NO:31 or wherein the agonistic ICOS-binding molecule comprises a first heavy chain comprising an amino acid sequence of SEQ ID NO:32, a second heavy chain comprising an amino acid sequence of SEQ ID NO:66, and one light chain comprising the amino acid sequence of SEQ ID NO:29.

17. The agonistic ICOS-binding molecule comprising at least one antigen binding domain that binds to a tumor-associated antigen for use in a method of any one of paras 1 to 4 or 7 to 15, wherein the agonistic ICOS-binding molecule comprises monovalent binding to a tumor associated target and bivalent binding to ICOS.

18. The agonistic ICOS-binding molecule comprising at least one antigen binding domain that binds to a tumor-associated antigen for use in a method of any one of paras 1 to 4 or 7 to 15 or 17, wherein the agonistic ICOS-binding molecule comprises a first heavy chain comprising an amino acid sequence of SEQ ID NO:32, a second heavy chain comprising an amino acid sequence of SEQ ID NO:33, and a two light chains comprising an amino acid sequence of SEQ ID NO:29.

19. The agonistic ICOS-binding molecule comprising at least one antigen binding domain that binds to a tumor-associated antigen for use in a method of any one of paras 1 to 18, wherein the T-cell activating anti-CD3 bispecific antibody is an anti-CEA/anti-CD3 bispecific antibody.

20. The agonistic ICOS-binding molecule comprising at least one antigen binding domain that binds to a tumor-associated antigen for use in a method of any one of paras 1 to 19, wherein the T-cell activating anti-CD3 bispecific antibody comprises a first antigen binding domain comprising a heavy chain variable region ($V_H$CD3) and a light chain variable region ($V_L$CD3), and a second antigen binding domain comprising a heavy chain variable region ($V_H$CEA) and a light chain variable region ($V_L$CEA).

21. The agonistic ICOS-binding molecule comprising at least one antigen binding domain that binds to a tumor-associated antigen for use in a method of any one of paras 1 to 20, wherein the T-cell activating anti-CD3 bispecific antibody comprises a first antigen binding domain comprising a heavy chain variable region ($V_H$CD3) comprising (i) CDR-H1 comprising the amino acid sequence of SEQ ID NO:34, (ii) CDR-H2 comprising the amino acid sequence of SEQ ID NO:35, and (iii) CDR-H3 comprising the amino acid sequence of SEQ ID NO:36, and a light chain variable region ($V_L$CD3) comprising (iv) CDR-L1 comprising the amino acid sequence of SEQ ID NO:37, (v) CDR-L2 comprising the amino acid sequence of SEQ ID NO:38, and (vi) CDR-L3 comprising the amino acid sequence of SEQ ID NO:39.

22. The agonistic ICOS-binding molecule comprising at least one antigen binding domain that binds to a tumor-associated antigen for use in a method of any one of paras 1 to 21, wherein the T-cell activating anti-CD3 bispecific antibody comprises a first antigen binding domain comprising a heavy chain variable region ($V_H$CD3) comprising the amino acid sequence of SEQ ID NO:40 and/or a light chain variable region ($V_L$CD3) comprising the amino acid sequence of SEQ ID NO:41.

23. The agonistic ICOS-binding molecule comprising at least one antigen binding domain that binds to a tumor-associated antigen for use in a method of any one of paras 1 to 22, wherein the T-cell activating anti-CD3 bispecific antibody comprises a second antigen binding domain comprising

(a) a heavy chain variable region ($V_H$CEA) comprising (i) CDR-H1 comprising the amino acid sequence of SEQ ID NO:42, (ii) CDR-H2 comprising the amino acid sequence of SEQ ID NO:43, and (iii) CDR-H3 comprising the amino acid sequence of SEQ ID NO:44, and a light chain variable region ($V_L$CEA) comprising (iv) CDR-L1 comprising the amino acid sequence of SEQ ID NO:45, (v) CDR-L2 comprising the amino acid sequence of SEQ ID NO:46, and (vi) CDR-L3 comprising the amino acid sequence of SEQ ID NO:47, or
(b) a heavy chain variable region ($V_H$CEA) comprising (i) CDR-H1 comprising the amino acid sequence of SEQ ID NO:50, (ii) CDR-H2 comprising the amino acid sequence of SEQ ID NO:51, and (iii) CDR-H3 comprising the amino acid sequence of SEQ ID NO:52, and a light chain variable region ($V_L$CEA) comprising (iv) CDR-L1 comprising the amino acid sequence of SEQ ID NO:53, (v) CDR-L2 comprising the amino acid sequence of SEQ ID NO:54, and (vi) CDR-L3 comprising the amino acid sequence of SEQ ID NO:55.

24. The agonistic ICOS-binding molecule comprising at least one antigen binding domain that binds to a tumor-associated antigen for use in a method of any one of paras 1 to 23, wherein the T-cell activating anti-CD3 bispecific antibody comprises a second antigen binding domain comprising a heavy chain variable region ($V_H$CEA) comprising the amino acid sequence of SEQ ID NO:48 and/or a light chain variable region ($V_L$CEA) comprising the amino acid sequence of SEQ ID NO:49 or a second antigen binding domain comprising a heavy chain variable region ($V_H$CEA) comprising the amino acid sequence of SEQ ID NO:56 and/or a light chain variable region ($V_L$CEA) comprising the amino acid sequence of SEQ ID NO:57.

25. The agonistic ICOS-binding molecule comprising at least one antigen binding domain that binds to a tumor-associated antigen for use in a method of any one of paras 1 to 24, wherein the anti-CEA/anti-CD3 bispecific antibody comprises a third antigen binding domain that binds to CEA.

26. The agonistic ICOS-binding molecule comprising at least one antigen binding domain that binds to a tumor-associated antigen for use in a method of any one of paras 1 to 25, wherein the T-cell activating anti-CD3 bispecific antibody comprises an Fc domain comprising one or more amino acid substitutions that reduce binding to an Fc receptor and/or effector function.

27. The agonistic ICOS-binding molecule comprising at least one antigen binding domain that binds to a tumor-associated antigen for use in a method of any one of paras 1 to 25, wherein the T-cell activating anti-CD3 bispecific antibody comprises (a) the amino acid sequences of SEQ ID NO:58, SEQ ID NO:59, SEQ ID NO:60 and SEQ ID NO:61, or (b) the amino acid sequences of SEQ ID NO:62, SEQ ID NO:63, SEQ ID NO:64 and SEQ ID NO:65.

28. The agonistic ICOS-binding molecule comprising at least one antigen binding domain that binds to a tumor-associated antigen for use in a method of any one of paras 1 to 27, wherein the agonistic ICOS-binding molecule comprising at least one antigen binding domain that binds to a tumor-associated antigen is used in combination with a T-cell activating anti-CD3 bispecific antibody specific for a tumor-associated antigen and in combination with an agent blocking PD-L1/PD-1 interaction.

29. The agonistic ICOS-binding molecule comprising at least one antigen binding domain that binds to a tumor-associated antigen for use in a method of para 28, wherein the agent blocking PD-L1/PD-1 interaction is a anti-PD-LI antibody or an anti-PDI antibody.

30. A pharmaceutical product comprising (A) a first composition comprising as active ingredient an agonistic ICOS-binding molecule comprising at least one antigen binding domain that binds to a tumor-associated antigen and a pharmaceutically acceptable excipient; and (B) a second composition comprising a T-cell activating anti-CD3 bispecific antibody specific for a tumor-associated antigen and a pharmaceutically acceptable excipient, for use in the combined, sequential or simultaneous, treatment of a disease, in particular cancer.

31. A pharmaceutical composition comprising an agonistic ICOS-binding molecule comprising at least one antigen binding domain that binds to a tumor-associated antigen and a T-cell activating anti-CD3 bispecific antibody specific for a tumor-associated antigen and pharmaceutically acceptable excipients.

32. The pharmaceutical composition of para 31 for use in the treatment of solid tumors.

33. An agonistic ICOS-binding molecule comprising at least one antigen binding domain that binds to a tumor-associated antigen, wherein the tumor-associated antigen is selected from the group consisting of Fibroblast Activation Protein (FAP), Carcinoembryonic Antigen (CEA), Folate receptor alpha (FolR1), Melanoma-associated Chondroitin Sulfate Proteoglycan (MCSP), Epidermal Growth Factor Receptor (EGFR), human epidermal growth factor receptor 2 (HER2) and p95HER2.

34. The agonistic ICOS-binding molecule comprising at least one antigen binding domain that binds to a tumor-associated antigen of para 33, wherein the tumor-associated antigen is FAP.

35. The agonistic ICOS-binding molecule comprising at least one antigen binding domain that binds to a tumor-associated antigen of paras 33 or 34, wherein the agonistic ICOS-binding molecule comprises at least one antigen binding domain capable of specific binding to FAP comprising

(a) a heavy chain variable region ($V_H$FAP) comprising (i) CDR-H1 comprising the amino acid sequence of SEQ ID NO:4, (ii) CDR-H2 comprising the amino acid sequence of SEQ ID NO:5, and (iii) CDR-H3 comprising the amino acid sequence of SEQ ID NO:6, and a light chain variable region ($V_L$FAP) comprising (iv) CDR-L1 comprising the amino acid sequence of SEQ ID NO:7, (v) CDR-L2 comprising the amino acid sequence of SEQ ID NO:8, and (vi) CDR-L3 comprising the amino acid sequence of SEQ ID NO:9, or
(b) a heavy chain variable region ($V_H$FAP) comprising (i) CDR-H1 comprising the amino acid sequence of SEQ ID NO:12, (ii) CDR-H2 comprising the amino acid sequence of SEQ ID NO:13, and (iii) CDR-H3 comprising the amino acid sequence of SEQ ID NO:14, and a light chain variable region ($V_L$FAP) comprising (iv) CDR-L1 comprising the amino acid sequence of SEQ ID NO:15, (v) CDR-L2 comprising the amino acid sequence of SEQ ID NO:16, and (vi) CDR-L3 comprising the amino acid sequence of SEQ ID NO:17.

36. The agonistic ICOS-binding molecule comprising at least one antigen binding domain that binds to a tumor-associated antigen of any one of paras 33 to 35, wherein the agonistic ICOS-binding molecule comprises at least one antigen binding domain capable of specific binding to FAP comprising a heavy chain variable region ($V_H$FAP) comprising an amino acid sequence of SEQ ID NO:10 and a light chain variable region ($V_L$FAP) comprising an amino acid sequence of SEQ ID NO:11 or wherein the antigen binding domain capable of specific binding to FAP comprises a heavy chain variable region ($V_H$FAP) comprising an amino acid sequence of SEQ ID NO:18 and a light chain variable region ($V_L$FAP) comprising an amino acid sequence of SEQ ID NO:19.

37. The agonistic ICOS-binding molecule comprising at least one antigen binding domain that binds to a tumor-associated antigen of any one of paras 33 to 36, wherein the agonistic ICOS-binding molecule comprises at least one antigen binding domain capable of specific binding to ICOS comprising a heavy chain variable region ($V_H$ICOS) comprising (i) CDR-H1 comprising the amino acid sequence of SEQ ID NO:20, (ii) CDR-H2 comprising the amino acid sequence of SEQ ID NO:21, and (iii) CDR-H3 comprising the amino acid sequence of SEQ ID NO:22, and a light chain variable region ($V_L$ICOS) comprising (iv) CDR-L1 comprising the amino acid sequence of SEQ ID NO:23, (v) CDR-L2 comprising the amino acid sequence of SEQ ID NO:24, and (vi) CDR-L3 comprising the amino acid sequence of SEQ ID NO:25.

38. The agonistic ICOS-binding molecule comprising at least one antigen binding domain that binds to a tumor-associated antigen of any one of paras 33 to 37, wherein the agonistic ICOS-binding molecule comprising at least one antigen binding domain that binds to a tumor-associated antigen comprises at least one antigen binding domain comprising a heavy chain variable region ($V_H$ICOS) comprising an amino acid sequence of SEQ ID NO:26 and a light chain variable region ($V_L$ICOS) comprising an amino acid sequence of SEQ ID NO:27.

39. The agonistic ICOS-binding molecule comprising at least one antigen binding domain that binds to a tumor-associated antigen of any one of paras 33 to 38, wherein the agonistic ICOS-binding molecule comprising at least one antigen binding domain that binds to a tumor-associated antigen comprises an IgG Fc domain, specifically an IgG1 Fc domain or an IgG4 Fc domain.

40. The agonistic ICOS-binding molecule comprising at least one antigen binding domain that binds to a tumor-associated antigen of any one of paras 33 to 39, wherein the agonistic ICOS-binding molecule comprising at least one antigen binding domain that binds to a tumor-associated antigen comprises a Fc domain that comprises one or more amino acid substitution that reduces binding to an Fc receptor and/or effector function.

41. The agonistic ICOS-binding molecule comprising at least one antigen binding domain that binds to a tumor-associated antigen of any one of paras 33 to 40, wherein the agonistic ICOS-binding molecule comprises a first heavy chain comprising an amino acid sequence of SEQ ID NO:28, a first light chain comprising an amino acid sequence of SEQ ID NO:29, a second heavy chain comprising an amino acid sequence of SEQ ID NO:30, and a second light chain comprising an amino acid sequence of SEQ ID NO:31.

42. The agonistic ICOS-binding molecule comprising at least one antigen binding domain that binds to a tumor-associated antigen of any one of paras 33 to 40, wherein the agonistic ICOS-binding molecule comprises monovalent binding to a tumor associated target and bivalent binding to ICOS.

43. The agonistic ICOS-binding molecule comprising at least one antigen binding domain that binds to a tumor-associated antigen of any one of paras 33 to 40 or 42, wherein the agonistic ICOS-binding molecule comprises a first heavy chain comprising an amino acid sequence of SEQ ID NO:32, a second heavy chain comprising an amino acid sequence of SEQ ID NO:33, and a two light chains comprising an amino acid sequence of SEQ ID NO:29.

## EXAMPLES

The following are examples of methods and compositions of the invention. It is understood that various other embodiments may be practiced, given the general description provided above.

### Recombinant DNA techniques

Standard methods were used to manipulate DNA as described in Sambrook et al., Molecular cloning: A laboratory manual; Cold Spring Harbor Laboratory Press, Cold Spring Harbor, New York, 1989. The molecular biological reagents were used according to the manufacturer's instructions. General information regarding the nucleotide sequences of human immunoglobulin light and heavy chains is given in: Kabat, E.A. et al., (1991) Sequences of Proteins of Immunological Interest, Fifth Ed., NIH Publication No 91-3242.

### DNA sequencing

DNA sequences were determined by double strand sequencing.

### Gene synthesis

Desired gene segments were either generated by PCR using appropriate templates or were synthesized by Geneart AG (Regensburg, Germany) from synthetic oligonucleotides and PCR products by automated gene synthesis. In cases where no exact gene sequence was available, oligonucleotide primers were designed based on sequences from closest homologues and the genes were isolated by RT-PCR from RNA originating from the appropriate tissue. The gene segments flanked by singular restriction endonuclease cleavage sites were cloned into standard cloning / sequencing vectors. The plasmid DNA was purified from transformed bacteria and concentration determined by UV spectroscopy. The DNA sequence of the subcloned gene fragments was confirmed by DNA sequencing. Gene segments were designed with suitable restriction sites to allow sub-cloning into the respective expression vectors. All constructs were designed with a 5'-end DNA sequence coding for a leader peptide which targets proteins for secretion in eukaryotic cells.

### Cell culture techniques

Standard cell culture techniques were used as described in Current Protocols in Cell Biology (2000), Bonifacino, J.S., Dasso, M., Harford, J.B., Lippincott-Schwartz, J. and Yamada, K.M. (eds.), John Wiley & Sons, Inc.

**Protein purification**

Proteins were purified from filtered cell culture supernatants referring to standard protocols. In brief, antibodies were applied to a Protein A Sepharose column (GE healthcare) and washed with PBS. Elution of antibodies was achieved at pH 2.8 followed by immediate neutralization of the sample. Aggregated protein was separated from monomeric antibodies by size exclusion chromatography (Superdex 200, GE Healthcare) in PBS or in 20 mM Histidine, 150 mM NaCl pH 6.0. Monomeric antibody fractions were pooled, concentrated (if required) using e.g., a MILLIPORE Amicon Ultra (30 MWCO) centrifugal concentrator, frozen and stored at -20°C or -80°C. Part of the samples were provided for subsequent protein analytics and analytical characterization e.g. by SDS-PAGE, size exclusion chromatography (SEC) or mass spectrometry.

**SDS-PAGE**

The NuPAGE® Pre-Cast gel system (Invitrogen) was used according to the manufacturer's instruction. In particular, 10% or 4-12% NuPAGE® Novex® Bis-TRIS Pre-Cast gels (pH 6.4) and a NuPAGE® MES (reduced gels, with NuPAGE® Antioxidant running buffer additive) or MOPS (non-reduced gels) running buffer was used.

**Analytical size exclusion chromatography**

Size exclusion chromatography (SEC) for the determination of the aggregation and oligomeric state of antibodies was performed by HPLC chromatography. Briefly, Protein A purified antibodies were applied to a Tosoh TSKgel G3000SW column in 300 mM NaCl, 50 mM $KH_2PO_4/K_2HPO_4$, pH 7.5 on an Agilent HPLC 1100 system or to a Superdex 200 column (GE Healthcare) in 2 x PBS on a Dionex HPLC-System. The eluted protein was quantified by UV absorbance and integration of peak areas. BioRad Gel Filtration Standard 151-1901 served as a standard.

**Mass spectrometry**

This section describes the characterization of the multispecific antibodies with VH/VL exchange (VH/VL Cross-Mabs) with emphasis on their correct assembly. The expected primary structures were analyzed by electrospray ionization mass spectrometry (ESI-MS) of the deglycosylated intact CrossMabs and deglycosylated/plasmin digested or alternatively deglycosylated/limited LysC digested CrossMabs.

The VH/VL CrossMabs were deglycosylated with N-Glycosidase F in a phosphate or Tris buffer at 37°C for up to 17 h at a protein concentration of 1 mg/ml. The plasmin or limited LysC (Roche) digestions were performed with 100 μg deglycosylated VH/VL CrossMabs in a Tris buffer pH 8 at room temperature for 120 hours and at 37°C for 40 min, respectively. Prior to mass spectrometry the samples were desalted via HPLC on a Sephadex G25 column (GE Healthcare). The total mass was determined via ESI-MS on a maXis 4G UHR-QTOF MS system (Bruker Daltonik) equipped with a TriVersa NanoMate source (Advion).

**Determination of binding and binding affinity of multispecific antibodies to the respective antigens using surface plasmon resonance (SPR) (BIACORE)**

Binding of the generated antibodies to the respective antigens is investigated by surface plasmon resonance using a BIACORE instrument (GE Healthcare Biosciences AB, Uppsala, Sweden). Briefly, for affinity measurements Goat-Anti-Human IgG, JIR 109-005-098 antibodies are immobilized on a CM5 chip via amine coupling for presentation of the antibodies against the respective antigen. Binding is measured in HBS buffer (HBS-P (10 mM HEPES, 150 mM NaCl, 0.005% Tween 20, ph 7.4), 25°C (or alternatively at 37°C). Antigen (R&D Systems or in house purified) was added in various concentrations in solution. Association was measured by an antigen injection of 80 seconds to 3 minutes; dissociation was measured by washing the chip surface with HBS buffer for 3 - 10 minutes and a KD value was estimated using a 1:1 Langmuir binding model. Negative control data (e.g. buffer curves) are subtracted from sample curves for correction of system intrinsic baseline drift and for noise signal reduction. The respective Biacore Evaluation Software is used for analysis of sensorgrams and for calculation of affinity data.

**Example 1**

**Generation of bispecific antibodies with a monovalent or bivalent binding to ICOS and a monovalent binding to FAP**

The following bispecific agonistic ICOS antibodies with monovalent or bivalent binding for ICOS and monovalent binding for FAP have been prepared as depicted in **Figures 1A-1C,** respectively.

A) FAP-ICOS_1+1, huIgG1 P329G LALA, monovalent ICOS (JMAb136), monovalent FAP (4B9) (Figure 1A, SEQ ID Nos: 28-31)

B) FAP-ICOS_1+1_HT, huIgG1 P329G LALA, monovalent ICOS (JMAb136), monovalent FAP (4B9) c-terminal fused of the heavy chain C region (Figure 1B, SEQ ID Nos: 29, 32, 66)

C) FAP-ICOS_2+1, huIgG1 P329G LALA, bivalent ICOS (JMAb136), monovalent FAP (4B9) (Figure 1C, SEQ ID Nos: 29, 32, 33)

In example 1A, the HC1 of the FAP-ICOS_1+1 construct was comprised of the following components, VHCH1 of an anti-ICOS (JMAb136) followed by Fc hole. HC2 was comprised of VHCH1 of anti-FAP (4B9) followed by Fc knob.

The FAP-ICOS_1+1_HT construct (example 1B) was comprised of the following components, Fc hole, at which C-terminus a VH of anti-FAP binder (4B9) was fused. HC2 was comprised of VHCH1 of anti-ICOS (JMAb136) followed by Fc knob, at which C-terminus a VL of anti-FAP binder (4B9) was fused.

The FAP-ICOS_2+1 construct (example 1C) was comprised of the following components, VHCH1 of an anti-ICOS (JMAb136) followed by Fc hole, at which C-terminus a VH of anti-FAP binder (4B9) was fused. HC2 was comprised of VHCH1 of anti-ICOS (JMAb136) followed by Fc knob, at which C-terminus a VL of anti-FAP binder (4B9) was fused.

For the ICOS binder, the VH and VL sequences of clone JMAb136 were obtained from patent US 2008/0199466 A1.

Combination of the Fc knob with the Fc hole chain allows generation of a heterodimer. The Pro329Gly, Leu234Ala and Leu235Ala mutations have been introduced in the constant region of the knob and hole heavy chains to abrogate binding to Fc$\gamma$ receptor according to the method described in International Patent Appl. Publ. No.WO2012/130831A1.

The amino acid sequences for bispecific agonistic ICOS antibodies can be found respectively in **Tables 1, 2 and 3.**

The targeted bispecific agonistic ICOS molecule encoding sequences were cloned into a plasmid vector driving expression of the insert from a Cytomegalovirus (CMV) promoter and containing a synthetic polyA sequence located at the 3' end of the CDS. In addition, the vector contained an Epstein-Barr virus (EBV) oriP sequence for episomal maintenance of the plasmid.

The bispecific agonistic ICOS antibodies were produced by co-transfecting HEK293-EBNA cells with the mammalian expression vectors using polyethylenimine (PEI). The cells were transfected with the corresponding expression vectors at a 1:1:1:1 ratio (A: "VHCH1(JMAb136)- Fc knob chain" : "VLCL(JMAb136) Light chain" : "VHCH1 (4B9)- Fc hole chain" : "VLCL(4B9) Light chain") or in a 1:1:1 ratio (B: "VHCH1 (JMAb136)-Fc knob chain-VH (4B9)" : "Fc hole chain-VL (4B9)" : "VLCL(JMAb136) Light chain") or in a 1:1:2 ratio (C: "VHCH1 (JMAb136)-Fc knob chain-VH (4B9)": "VHCH1 (JMAb136)-Fc hole chain-VL (4B9)" : "VLCL(JMAb136) Light chain").

For production in 500 mL shake flasks, 400 million HEK293-EBNA cells were seeded 24 hours before transfection. For transfection cells were centrifuged for 5 minutes at 210 x g, and the supernatant was replaced by pre-warmed CD CHO medium. Expression vectors were mixed in 20 mL CD CHO medium to a final amount of 200 $\mu$g DNA. After addition of 540 $\mu$L PEI, the solution was vortexed for 15 seconds and incubated for 10 minutes at room temperature.

Afterwards, cells were mixed with the DNA/PEI solution, transferred to a 500 mL shake flask and incubated for 3 hours at 37°C in an incubator with a 5% $CO_2$ atmosphere. After the incubation, 160 mL of EX-CELL 293 (Sigma) medium was added and cells were cultured for 24 hours. One day after transfection 1 mM valproic acid and 7% Feed with supplements were added. After culturing for 7 days, the supernatant was collected by centrifugation for 15 minutes at 210 x g. The solution was sterile filtered (0.22 $\mu$m filter), supplemented with sodium azide to a final concentration of 0.01 % (w/v), and kept at 4°C.

Secreted proteins were purified from cell culture supernatants by affinity chromatography using Protein A, followed by size exclusion chromatography. For affinity chromatography, the supernatant was loaded on a Protein A MabSelectSure column (CV = 5 mL, GE Healthcare) equilibrated with 40 mL 20 mM sodium phosphate, 20 mM sodium citrate pH 7.5. Unbound protein was removed by washing with at least 10 column volumes of 20 mM sodium phosphate, 20 mM sodium citrate containing buffer (pH 7.5). The bound protein was eluted using a linear pH-gradient of sodium chloride (from 20 to 100 mM) created over 15 column volumes of 20 mM sodium citrate, 100 mM NaCl, 100 mM Glycine, 0.01% Tween20 pH 3.0. The column was then washed with 10 column volumes of 20 mM sodium citrate, 100 mM NaCl, 100 mM Glycine, 0.01% Tween20 pH 3.0. The pH of collected fractions was adjusted by adding 1/40 (v/v) of 2 M Tris, pH 8.0. The protein was concentrated and filtered prior to loading on a HiLoad Superdex 50/600 S200 column (GE Healthcare) equilibrated with 20 mM Histidine, 140 mM NaCl, 0.01% Tween20 pH6.0.

The protein concentration of purified bispecific constructs was determined by measuring the OD at 280 nm, using the molar extinction coefficient calculated on the basis of the amino acid sequence. Purity and molecular weight of the bispecific constructs were analyzed by CE-SDS in the presence and absence of a reducing agent (Invitrogen, USA) using a LabChipGXII (Caliper). The aggregate content of bispecific constructs was analyzed using a TSKgel G3000 SW XL analytical size-exclusion column (Tosoh) equilibrated in a 25 mM $K_2HPO_4$, 125 mM NaCl, 200 mM L-Arginine Monohydrocloride, 0.02 % (w/v) $NaN_3$, pH 6.7 running buffer at 25°C.

**Table 1-** amino acid sequences of bispecific 1+1 FAP(4B9)-targeted anti-ICOS(JMAb136) human IgG1 P329G LALA (Figure 1A).

| SEQ ID NO: | Description | Sequence |
|---|---|---|
| 28 | VHCH1 (JMAb136)-Fc knob chain | QVQLVQSGAEVKKPGASVKVSCKAS<u>GYTFTGYYMH</u>WVRQA PGQGLEWMG<u>WINPHSGGTNYAQKFQG</u>RVTMTRDTSISTAY MELSRLRSDDTAVYYCAR<u>TYYYDSSGYYHDAFDI</u>WGQGTM VTVSSASTKGPSVFPLAPSSKSTSGGTAALGCLVEDYFPEPVT VSWNSGALTSGVHTFPAVLQSSGLYSLSSVVTVPSSSLGTQT YICNVNHKPSNTKVDEKVEPKSCDKTHTCPPCPAPEAAGGPS VFLFPPKPKDTLMISRTPEVTCVVVDVSHEDPEVKFNWYVDG VEVHNAKTKPREEQYNSTYRVVSVLTVLHQDWLNGKEYKC KVSNKALGAPIEKTISKAKGQPREPQVYTLPPCRDELTKNQVS LWCLVKGFYPSDIAVEWESNGQPENNYKTTPPVLDSDGSFFL YSKLTVDKSRWQQGNVFSCSVMHEALHNHYTQKSLSLSPGK |
| 29 | VLCL(JMAb136) Light chain | DIQMTQSPSSVSASVGDRVTITC<u>RASQGISRLLA</u>WYQQKPGK APKLLIY<u>VASSLQS</u>GVPSRFSGSGSGTDFTLTISSLQPEDFATY YC<u>QQANSFPWT</u>FGQGTKVEIKRTVAAPSVFIFPPSDRKLKSGT ASVVCLLNNFYPREAKVQWKVDNALQSGNSQESVTEQDSKD STYSLSSTLTLSKADYEKHKVYACEVTHQGLSSPVTKSFNRG EC |
| 30 | VHCH1 (4B9)-Fc hole chain | EIVLTQSPGTLSLSPGERATLSCRASQSVTSSYLAWYQQKPGQ APRLLINVGSRRATGIPDRFSGSGSGTDFTLTISRLEPEDFAVY YCQQGIMLPPTFGQGTKVEIKSSASTKGPSVFPLAPSSKSTSG GTAALGCLVKDYFPEPVTVSWNSGALTSGVHTFPAVLQSSGL YSLSSVVTVPSSSLGTQTYICNVNHKPSNTKVDKKVEPKSCD KTHTCPPCPAPEAAGGPSVFLFPPKPKDTLMISRTPEVTCVVV DVSHEDPEVKFNWYVDGVEVHNAKTKPREEQYNSTYRVVS VLTVLHQDWLNGKEYKCKVSNKALGAPIEKTISKAKGQPRE PQVCTLPPSRDELTKNQVSLSCAVKGFYPSDIAVEWESNGQP ENNYKTTPPVLDSDGSFFLVSKLTVDKSRWQQGNVFSCSVM HEALHNHYTQKSLSLSPGK |

(continued)

| SEQ ID NO: | Description | Sequence |
|---|---|---|
| 31 | VLCL(4B9) Light chain | EVQLLESGGGLVQPGGSLRLSCAASGFTFSSYAMSWVRQAP GKGLEWVSAIIGSGASTYYADSVKGRFTISRDNSKNTLYLQM NSLRAEDTAVYYCAKGWFGGFNYWGQGTLVTVSSASVAAP SVFIFPPSDEQLKSGTASVVCLLNNFYPREAKVQWKVDNALQ SGNSQESVTEQDSKDSTYSLSSTLTLSKADYEKHKVYACEVT HQGLSSPVTKSFNRGEC |

**Table 2** - amino acid sequences of bispecific 1+1 head-to-tail FAP(4B9)-targeted anti-ICOS (JMAb136) human IgG1 P329G LALA (Figure 1B).

| SEQ ID NO: | Description | Sequence |
|---|---|---|
| 29 | VLCL(JMAb13 6) Light chain | DIQMTQSPSSVSASVGDRVTITC<u>RASQGISRLLA</u>WYQQKPGK APKLLIY<u>VASSLQS</u>GVPSRFSGSGSGTDFTLTISSLQPEDFATY YC<u>QQANSFPWT</u>FGQGTKVEIKRTVAAPSVFIFPPSDRKLKSGT ASVVCLLNNFYPREAKVQWKVDNALQSGNSQESVTEQDSKD STYSLSSTLTLSKADYEKHKVYACEVTHQGLSSPVTKSFNRG EC |
| 32 | VHCH1 (JMAb136)-Fc knob chain-VH (4B9) | QVQLVQSGAEVKKPGASVKVSCKAS<u>GYTFTGYYMH</u>WVRQA PGQGLEWMG<u>WINPHSGGTNYAQKFQG</u>RVTMTRDTSISTAY MELSRLRSDDTAVYYCAR<u>TYYYDSSGYYHDAFDI</u>WGQGTM VTVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPEAAGGPS VFLFPPKPKDTLMISRTPEVTCVVVDVSHEDPEVKFNWYVDG VEVHNAKTKPREEQYNSTYRVVSVLTVLHQDWLNGKEYKC KVSNKALGAPIEKTISKAKGQPREPQVYTLPPCRDELTKNQVS LWCLVKGFYPSDIAVEWESNGQPENNYKTTPPVLDSDGSFFL YSKLTVDKSRWQQGNVFSCSVMHEALHNHYTQKSLSLSPGG GGGSGGGGSGGGGSGGGGSEVQLLESGGGLVQPGGSLRLSC AASGFTFSSYAMSWVRQAPGKGLEWVSAIIGSGASTYYADS VKGRFTISRDNSKNTLYLQMNSLRAEDTAVYYCAKGWFGGF NYWGQGTLVTVSS |

(continued)

| SEQ ID NO: | Description | Sequence |
|---|---|---|
| 66 | Fc hole chain-VL (4B9) | DKTHTCPPCPAPEAAGGPSVFLFPPKPKDTLMISRTPEVTCVV VDVSHEDPEVKFNWYVDGVEVHNAKTKPREEQYNSTYRVV SVLTVLHQDWLNGKEYKCKVSNKALGAPIEKTISKAKGQPR EPQVCTLPPSRDELTKNQVSLSCAVKGFYPSDIAVEWESNGQ PENNYKTTPPVLDSDGSFFLVSKLTVDKSRWQQGNVFSCSV MHEALHNHYTQKSLSLSPGGGGGSGGGGSGGGGSGGGGSEI VLTQSPGTLSLSPGERATLSCRASQSVTSSYLAWYQQKPGQA PRLLINVGSRRATGIPDRFSGSGSGTDFTLTISRLEPEDFAVYY CQQGIMLPPTFGQGTKVEIK |

**Table 3**- amino acid sequences of bispecific 2+1 FAP(4B9)-targeted anti-ICOS(JMAb136) human IgG1 P329G LALA (Figure 1C)

| SEQ ID NO: | Description | Sequence |
|---|---|---|
| 29 | VLCL(JMAb136) Light chain | DIQMTQSPSSVSASVGDRVTITCRASQGISRLLAWYQQKPGK APKLLIYVASSLQSGVPSRFSGSGSGTDFTLTISSLQPEDFATY YCQQANSFPWTFGQGTKVEIKRTVAAPSVFIFPPSDEQLKSGT ASVVCLLNNFYPREAKVQWKVDNALQSGNSQESVTEQDSKD STYSLSSTLTLSKADYEKHKVYACEVTHQGLSSPVTKSFNRG |
| | | EC |
| 32 | VHCH1 (JMAb136)-Fc knob chain-VH (4B9) | QVQLVQSGAEVKKPGASVKVSCKASGYTFTGYYMHWVRQA PGQGLEWMGWINPHSGGTNYAQKFQGRVTMTRDTSISTAY MELSRLRSDDTAVYYCARTYYYDSSGYYHDAFDIWGQGTM VTVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPEAAGGPS VFLFPPKPKDTLMISRTPEVTCVVVDVSHEDPEVKFNWYVDG VEVHNAKTKPREEQYNSTYRVVSVLTVLHQDWLNGKEYKC KVSNKALGAPIEKTISKAKGQPREPQVYTLPPCRDELTKNQVS LWCLVKGFYPSDIAVEWESNGQPENNYKTTPPVLDSDGSFFL YSKLTVDKSRWQQGNVFSCSVMHEALHNHYTQKSLSLSPGG GGGSGGGGSGGGGSGGGGSEVQLLESGGGLVQPGGSLRLSC AASGFTFSSYAMSWVRQAPGKGLEWVSAIIGSGASTYYADS VKGRFTISRDNSKNTLYLQMNSLRAEDTAVYYCAKGWFGGF NYWGQGTLVTVSS |

(continued)

| SEQ ID NO: | Description | Sequence |
|---|---|---|
| 33 | VHCH1 (JMAb136)-Fc hole chain-VL (4B9) | QVQLVQSGAEVKKPGASVKVSCKAS<u>GYTFTGYYMH</u>WVRQA PGQGLEWMG<u>WINPHSGGTNYAQKFQG</u>RVTMTRDTSISTAY MELSRLRSDDTAVYYCAR<u>TYYYDSSGYYHDAFDI</u>WGQGTM VTVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPEAAGGPS VFLFPPKPKDTLMISRTPEVTCVVVDVSHEDPEVKFNWYVDG VEVHNAKTKPREEQYNSTYRVVSVLTVLHQDWLNGKEYKC KVSNKALGAPIEKTISKAKGQPREPQVCTLPPSRDELTKNQVS LSCAVKGFYPSDIAVEWESNGQPENNYKTTPPVLDSDGSFFL VSKLTVDKSRWQQGNVFSCSVMHEALHNHYTQKSLSLSPGG GGGSGGGGSGGGGSGGGGSEIVLTQSPGTLSLSPGERATLSC RASQSVTSSYLAWYQQKPGQAPRLLINVGSRRATGIPDRFSG SGSGTDFTLTISRLEPEDFAVYYCQQGIMLPPTFGQGTKVEIK |

**Example 2**

**Generation of bispecific antibodies with a monovalent or bivalent binding to ICOS and an untargeted moiety (control molecules)**

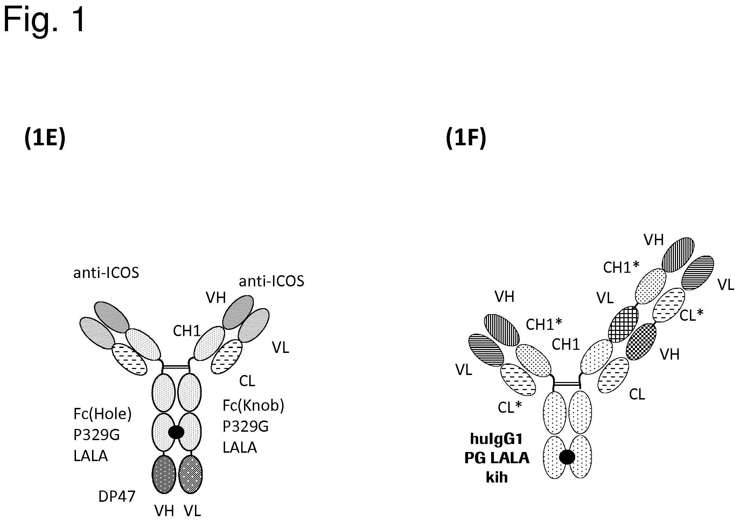

The following bispecific agonistic ICOS antibodies with monovalent or bivalent binding for ICOS and containing an untargeted moiety were prepared similarly to the targeted formats, and as depicted in Figures 1D and 1E, respectively.

A) DP47-ICOS_1+1, huIgG1 P329G LALA, monovalent ICOS (JMAb136), monovalent DP47 (Figure 1D, SEQ ID NO 1, 2, 8, 9)

B) DP47-ICOS_2+1, huIgG1 P329G LALA, bivalent ICOS (JMAb136), monovalent DP47 (Figure 1E, SEQ ID NO 2, 10, 11)

In this example the HC1 of the DP47-ICOS_1+1 construct was comprised of the following components, VHCH1 of JMAb136 as anti-ICOS antibody followed by Fc hole. HC2 was comprised of VHCH1 of DP47 as non-binding antibody followed by Fc knob.
The DP47-ICOS_2+1 construct was comprised of the following components, VHCH1 of an anti-ICOS (JMAb136) followed by Fc hole, at which C-terminus a VH of of a non-binding clone (DP47) was fused. HC2 was comprised of VHCH1 of anti-ICOS (JMAb136) followed by Fc knob, at which C-terminus a VL of a non-binding clone (DP47) was fused.
The untargeted bispecific agonistic ICOS molecules were prepared as described in Example 1 for the FAP(4B9)-targeted bispecific agonistic ICOS antibodies.

**Table 4**- amino acid sequences of bispecific 1+1 untargeted DP47 anti-ICOS(JMAb136) human IgG1 P329G LALA.

| SEQ ID NO: | Description | Sequence |
|---|---|---|
| 28 | VHCH1 (JMAb136)-Fc knob chain | QVQLVQSGAEVKKPGASVKVSCKAS<u>GYTFTGYYMH</u>WVRQA PGQGLEWMG<u>WINPHSGGTNYAQKFQG</u>RVTMTRDTSISTAY MELSRLRSDDTAVYYCAR<u>TYYYDSSGYYHDAFDI</u>WGQGTM VTVSSASTKGPSVFPLAPSSKSTSGGTAALGCLVEDYFPEPVT VSWNSGALTSGVHTFPAVLQSSGLYSLSSVVTVPSSSLGTQT YICNVNHKPSNTKVDEKVEPKSCDKTHTCPPCPAPEAAGGPS VFLFPPKPKDTLMISRTPEVTCVVVDVSHEDPEVKFNWYVDG VEVHNAKTKPREEQYNSTYRVVSVLTVLHQDWLNGKEYKC KVSNKALGAPIEKTISKAKGQPREPQVYTLPPCRDELTKNQVS LWCLVKGFYPSDIAVEWESNGQPENNYKTTPPVLDSDGSFFL YSKLTVDKSRWQQGNVFSCSVMHEALHNHYTQKSLSLSPGK |
| 29 | VLCL(JMAb136) Light chain | DIQMTQSPSSVSASVGDRVTITC<u>RASQGISRLLA</u>WYQQKPGK APKLLIY<u>VASSLQS</u>GVPSRFSGSGSGTDFTLTISSLQPEDFATY YC<u>QQANSFPWT</u>FGQGTKVEIKRTVAAPSVFIFPPSDRKLKSGT ASVVCLLNNFYPREAKVQWKVDNALQSGNSQESVTEQDSKD STYSLSSTLTLSKADYEKHKVYACEVTHQGLSSPVTKSFNRG EC |
| 67 | VHCH1 (DP47)-Fc hole chain | EIVLTQSPGTLSLSPGERATLSCRASQSVSSSYLAWYQQKPGQ APRLLIYGASSRATGIPDRFSGSGSGTDFTLTISRLEPEDFAVY YCQQYGSSPLTFGQGTKVEIKSSASTKGPSVFPLAPSSKSTSG GTAALGCLVKDYFPEPVTVSWNSGALTSGVHTFPAVLQSSGL YSLSSVVTVPSSSLGTQTYICNVNHKPSNTKVDKKVEPKSCD |
|  |  | KTHTCPPCPAPEAAGGPSVFLFPPKPKDTLMISRTPEVTCVVV DVSHEDPEVKFNWYVDGVEVHNAKTKPREEQYNSTYRVVS VLTVLHQDWLNGKEYKCKVSNKALGAPIEKTISKAKGQPRE PQVCTLPPSRDELTKNQVSLSCAVKGFYPSDIAVEWESNGQP ENNYKTTPPVLDSDGSFFLVSKLTVDKSRWQQGNVFSCSVM HEALHNHYTQKSLSLSPGK |
| 68 | VLCL(DP47) Light chain | EVQLLESGGGLVQPGGSLRLSCAASGFTFSSYAMSWVRQAP GKGLEWVSAISGSGGSTYYADSVKGRFTISRDNSKNTLYLQM NSLRAEDTAVYYCAKGSGFDYWGQGTLVTVSSASVAAPSVF IFPPSDEQLKSGTASVVCLLNNFYPREAKVQWKVDNALQSGN SQESVTEQDSKDSTYSLSSTLTLSKADYEKHKVYACEVTHQG LSSPVTKSFNRGEC |

**Table 5:** amino acid sequences of bispecific 2+1 untargeted DP47 anti-ICOS(JMAb136) human IgG1 P329G LALA (Figure IE).

| SEQ ID NO: | Description | Sequence |
|---|---|---|
| 29 | VLCL(JMAb136) Light chain | DIQMTQSPSSVSASVGDRVTITCRASQGISRLLAWYQQKPGK APKLLIYVASSLQSGVPSRFSGSGSGTDFTLTISSLQPEDFATY YCQQANSFPWTFGQGTKVEIKRTVAAPSVFIFPPSDRKLKSGT ASVVCLLNNFYPREAKVQWKVDNALQSGNSQESVTEQDSKD STYSLSSTLTLSKADYEKHKVYACEVTHQGLSSPVTKSFNRG EC |
| 69 | VHCH1 (JMAb136)-Fc knob chain-VH (DP47) | QVQLVQSGAEVKKPGASVKVSCKASGYTFTGYYMHWVRQA PGQGLEWMGWINPHSGGTNYAQKFQGRVTMTRDTSISTAY MELSRLRSDDTAVYYCARTYYYDSSGYYHDAFDIWGQGTM VTVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPEAAGGPS VFLFPPKPKDTLMISRTPEVTCVVVDVSHEDPEVKFNWYVDG VEVHNAKTKPREEQYNSTYRVVSVLTVLHQDWLNGKEYKC KVSNKALGAPIEKTISKAKGQPREPQVYTLPPCRDELTKNQVS LWCLVKGFYPSDIAVEWESNGQPENNYKTTPPVLDSDGSFFL YSKLTVDKSRWQQGNVFSCSVMHEALHNHYTQKSLSLSPGG GGGSGGGGSGGGGSGGGGSEIVLTQSPGTLSLSPGERATLSC RASQSVSSSYLAWYQQKPGQAPRLLIYGASSRATGIPDRFSGS GSGTDFTLTISRLEPEDFAVYYCQQYGSSPLTFGQGTKVEIK |
| 70 | VHCH1 (JMAb136)-Fc hole chain-VL (DP47) | QVQLVQSGAEVKKPGASVKVSCKASGYTFTGYYMHWVRQA PGQGLEWMGWINPHSGGTNYAQKFQGRVTMTRDTSISTAY MELSRLRSDDTAVYYCARTYYYDSSGYYHDAFDIWGQGTM VTVSSASTKGPSVFPLAPSSKSTSGGTAALGCLVKDYFPEPVT VSWNSGALTSGVHTFPAVLQSSGLYSLSSVVTVPSSSLGTQT YICNVNHKPSNTKVDKKVEPKSCDKTHTCPPCPAPEAAGGPS VFLFPPKPKDTLMISRTPEVTCVVVDVSHEDPEVKFNWYVDG VEVHNAKTKPREEQYNSTYRVVSVLTVLHQDWLNGKEYKC KVSNKALGAPIEKTISKAKGQPREPQVCTLPPSRDELTKNQVS LSCAVKGFYPSDIAVEWESNGQPENNYKTTPPVLDSDGSFFL |
| | | VSKLTVDKSRWQQGNVFSCSVMHEALHNHYTQKSLSLSPGG GGGSGGGGSGGGGSGGGGSEVQLLESGGGLVQPGGSLRLSC AASGFTFSSYAMSWVRQAPGKGLEWVSAISGSGGSTYYADS VKGRFTISRDNSKNTLYLQMNSLRAEDTAVYYCAKGSGFDY WGQGTLVTVSS |

The results of the biochemical analysis of the bispecific molecules with a monovalent or bivalent binding to ICOS (JMAb136) and a monovalent binding to FAP (4B9) or DP47 produced as described in Examples 1 and 2 is summarized in Table 6.

**Table 6:** Biochemical analysis of bispecific FAP-ICOS or DP47-ICOS molecules

| Molecule | Monomer [%] | Yield [mg/L] | CE-SDS (non-reduced) [%] |
|---|---|---|---|
| 1A) FAP-ICOS_1+1 | 91.0 | 13.9 | 100.0 |
| 1B) FAP-ICOS_1+1_HT | 97.3 | 2.6 | 98.3 |

(continued)

| Molecule | Monomer [%] | Yield [mg/L] | CE-SDS (non-reduced) [%] |
|---|---|---|---|
| 1C) FAP-ICOS_2+1 | 96.6 | 6.3 | 94.0 |
| 1D) DP47-ICOS_1+1 | 91.0 | 37.5 | 98.4 |
| 1E) DP47-ICOS_2+1 | 100.0 | 11.7 | 98.5 |

**Example 3**

**Generation of FAP-targeted or untargeted (control) bivalent murine ICOS Ligand constructs**

The following bispecific murine ICOS ligand (ICOSL) constructs containing a monovalent binding for FAP or an untargeted moiety have been prepared as depicted in **Figures 2A and 2B.**

2A) FAP-targeted mICOSL, mIgG1 DAPG, bivalent murine ICOSL (Gly47 - Lys279) monovalent FAP (28H1) c-terminal fused of the heavy chain C region (Figure 2A, SEQ ID NO 18-19)
2B) Untargeted mICOSL, mIgG1 DAPG, bivalent murine ICOSL (Gly47 - Lys279), monovalent untargeted moiety c-terminal fused of the heavy chain C region (Figure 2B, SEQ ID NO 20-21)

In this example the FAP-targeted mICOSL construct HC1 was comprised of murine ICOSL, murine Fc DAPG KK, at which C-terminus a VH of anti-FAP binder (28H1) was fused. HC2 was comprised of murine ICOSL followed by murine Fc DAPG DD, at which C-terminus a VL of anti-FAP binder (28H1) was fused.

The ICOSL amino acid sequence was obtained from Uniprot Q9JHJ8, from which Gly47 till Ser279 was used for cloning. The amino acid sequences for murine ICOS ligand (Gly47 - Ser279) can be found respectively in Table 7.

Combination of the Fc DD with the Fc KK chain allows generation of a heterodimer. The DAPG mutations have been introduced in the constant region of the knob and hole heavy chains to abrogate binding to Fcγ receptor according to the method described in International Patent Appl.Publ. No.WO2012/130831A1.

The corresponding cDNAs were cloned into evitria's vector system using conventional (non-PCR based) cloning techniques. The evitria vector plasmids were gene synthesized. Plasmid DNA was prepared under low-endotoxin conditions based on anion exchange chromatography. DNA concentration was determined by measuring the absorption at a wavelength of 260 nm. Correctness of the sequences was verified with Sanger sequencing (with up to two sequencing reactions per plasmid depending on the size of the cDNA.)

Suspension-adapted CHO K1 cells (originally received from ATCC and adapted to serum-free growth in suspension culture at evitria) were used for production. The seed was grown in eviGrow medium, a chemically defined, animal-component free, serum-free medium. Cells were transfected with eviFect, evitria's custom-made, proprietary transfection reagent, and cells were grown after transfection in eviMake2, an animal-component free, serum-free medium. Supernatant was harvested by centrifugation and subsequent filtration (0.2 μm filter).

Secreted proteins were purified from cell culture supernatants by affinity chromatography using Protein A, followed by size exclusion chromatography. For affinity chromatography, the supernatant was loaded on a Protein A MabSelectSure column (CV = 5 mL, GE Healthcare) equilibrated with 40 mL 20 mM sodium phosphate, 20 mM sodium citrate pH 7.5. Unbound protein was removed by washing with at least 10 column volumes of 20 mM sodium phosphate, 20 mM sodium citrate containing buffer (pH 7.5). The bound protein was eluted using a linear pH-gradient of sodium chloride (from 20 to 100 mM) created over 15 column volumes of 20 mM sodium citrate, 100 mM NaCl, 100 mM Glycine, 0.01% Tween20 pH 3.0. The column was then washed with 10 column volumes of 20 mM sodium citrate, 100 mM NaCl, 100 mM Glycine, 0.01% Tween20 pH 3.0. The pH of collected fractions was adjusted by adding 1/40 (v/v) of 2M Tris, pH8.0. The protein was concentrated and filtered prior to loading on a HiLoad Superdex 50/600 S200 column (GE Healthcare) equilibrated with 20 mM Histidine, 140 mM NaCl, 0.01% Tween20 pH6.0.

The protein concentration of purified bispecific constructs was determined by measuring the OD at 280 nm, using the molar extinction coefficient calculated on the basis of the amino acid sequence. Purity and molecular weight of the bispecific constructs were analyzed by CE-SDS in the presence and absence of a reducing agent (Invitrogen, USA) using a LabChipGXII (Caliper). The aggregate content of bispecific constructs was analyzed using a TSKgel G3000 SW XL analytical size-exclusion column (Tosoh) equilibrated in a 25 mM $K_2HPO_4$, 125 mM NaCl, 200mM L-Arginine Monohydrocloride, 0.02 % (w/v) $NaN_3$, pH 6.7 running buffer at 25 °C.

**Table 7**- amino acid sequences of FAP(28H1)-targeted mICOSL, mIgG1 DAPG.

| SEQ ID NO: | Description | Sequence |
|---|---|---|
| 71 | Murine ICOSL Linker muIgG1 Fc (DAPG KK) 4GS linker FAP(28H1) VH | ETEVGAMVGSNVVLSCIDPHRRHFNLSGLYVYWQIENPEV SVTYYLPYKSPGINVDSSYKNRGHLSLDSMKQGNFSLYLK NVTPQDTQEFTCRVFMNTATELVKILEEVVRLRVAANFSTP VISTSDSSNPGQERTYTCMSKNGYPEPNLYWINTTDNSLIDT ALQNNTVYLNKLGLYDVISTLRLPWTSRGDVLCCVENVAL HQNITSISQAESFTGNNTKNPQETHNNELKGSPGSSSSSGSA DGCKPCICTVPEVSSVFIFPPKPKDVLTITLTPKVTCVVVAIS KDDPEVQFSWFVDDVEVHTAQTKPREEQINSTFRSVSELPI MHQDWLNGKEFKCRVNSAAFGAPIEKTISKTKGRPKAPQV YTIPPPKKQMAKDKVSLTCMITNFFPEDITVEWQWNGQPA ENYKNTQPIMKTDGSYFVYSKLNVQKSNWEAGNTFTCSVL HEGLHNHHTEKSLSHSPGGGGGSGGGGSGGGGSGGGGSEV QLLESGGGLVQPGGSLRLSCAASGFTFSSHAMSWVRQAPG KGLEWVSAIWASGEQYYADSVKGRFTISRDNSKNTLYLQM NSLRAEDTAVYYCAKGWLGNFDYWGQGTLVTVSS |
| 72 | Murine ICOSL linker muIgG1 Fc (DAPG DD) 4GS linker FAP(28H1) VL | ETEVGAMVGSNVVLSCIDPHRRHFNLSGLYVYWQIENPEV SVTYYLPYKSPGINVDSSYKNRGHLSLDSMKQGNFSLYLK NVTPQDTQEFTCRVFMNTATELVKILEEVVRLRVAANFSTP VISTSDSSNPGQERTYTCMSKNGYPEPNLYWINTTDNSLIDT ALQNNTVYLNKLGLYDVISTLRLPWTSRGDVLCCVENVAL HQNITSISQAESFTGNNTKNPQETHNNELKGSPGSSSSSGSA GSPGSSSSSGSADGCKPCICTVPEVSSVFIFPPKPKDVLTITL TPKVTCVVVAISKDDPEVQFSWFVDDVEVHTAQTKPREEQI NSTFRSVSELPIMHQDWLNGKEFKCRVNSAAFGAPIEKTIS KTKGRPKAPQVYTIPPPKEQMAKDKVSLTCMITNFFPEDIT VEWQWNGQPAENYDNTQPIMDTDGSYFVYSDLNVQKSN WEAGNTFTCSVLHEGLHNHHTEKSLSHSPGGGGGSGGGGS |
| | | GGGGSGGGGSEIVLTQSPGTLSLSPGERATLSCRASQSVSRS YLAWYQQKPGQAPRLLIIGASTRATGIPDRFSGSGSGTDFTL TISRLEPEDFAVYYCQQGQVIPPTFGQGTKVEIK |

**Table 8** - amino acid sequences of DP47-untargeted mICOSL, mIgG1 DAPG.

| SEQ ID NO: | Description | Sequence |
|---|---|---|
| 73 | Murine ICOSL Linker muIgG 1 Fc (DAPG KK) G4S linker DP47 VH | ETEVGAMVGSNVVLSCIDPHRRHFNLSGLYVYWQIENPEVS VTYYLPYKSPGINVDSSYKNRGHLSLDSMKQGNFSLYLKN VTPQDTQEFTCRVFMNTATELVKILEEVVRLRVAANFSTPV ISTSDSSNPGQERTYTCMSKNGYPEPNLYWINTTDNSLIDTA LQNNTVYLNKLGLYDVISTLRLPWTSRGDVLCCVENVALH QNITSISQAESFTGNNTKNPQETHNNELKGSPGSSSSSGSAG SPGSSSSSGSADGCKPCICTVPEVSSVFIFPPKPKDVLTITLTP KVTCVVVAISKDDPEVQFSWFVDDVEVHTAQTKPREEQINS TFRSVSELPIMHQDWLNGKEFKCRVNSAAFGAPIEKTISKTK GRPKAPQVYTIPPPKKQMAKDKVSLTCMITNFFPEDITVEW QWNGQPAENYKNTQPIMKTDGSYFVYSKLNVQKSNWEAG NTFTCSVLHEGLHNHHTEKSLSHSPGGGGGSGGGGSGGGG SGGGGSEIVLTQSPGTLSLSPGERATLSCRASQSVSSSYLAW YQQKPGQAPRLLIYGASSRATGIPDRFSGSGSGTDFTLTISRL EPEDFAVYYCQQYGSSPLTFGQGTKVEIK |
| 74 | Murine ICOSL linker muIgG1 Fc (DAPG DD) 4GS linker DP47 VL | ETEVGAMVGSNVVLSCIDPHRRHFNLSGLYVYWQIENPEVS VTYYLPYKSPGINVDSSYKNRGHLSLDSMKQGNFSLYLKN VTPQDTQEFTCRVFMNTATELVKILEEVVRLRVAANFSTPV ISTSDSSNPGQERTYTCMSKNGYPEPNLYWINTTDNSLIDTA LQNNTVYLNKLGLYDVISTLRLPWTSRGDVLCCVENVALH QNITSISQAESFTGNNTKNPQETHNNELKGSPGSSSSSGSAG SPGSSSSSGSADGCKPCICTVPEVSSVFIFPPKPKDVLTITLT PKVTCVVVAISKDDPEVQFSWFVDDVEVHTAQTKPREEQIN STFRSVSELPIMHQDWLNGKEFKCRVNSAAFGAPIEKTISKT KGRPKAPQVYTIPPPKEQMAKDKVSLTCMITNFFPEDITVE WQWNGQPAENYDNTQPIMDTDGSYFVYSDLNVQKSNWE AGNTFTCSVLHEGLHNHHTEKSLSHSPGGGGGSGGGGSGG GGSGGGGSEVQLLESGGGLVQPGGSLRLSCAASGFTFSSYA MSWVRQAPGKGLEWVSAISGSGGSTYYADSVKGRFTISRD NSKNTLYLQMNSLRAEDTAVYYCAKGSGFDYWGQGTLVT VSS |

The results of the biochemical analysis of the bispecific molecules comprising mICOSL as produced as described in this example are summarized in Table 9.

**Table 9:** Biochemical analysis of bispecific FAP-mICOS-L or DP47-mICOS-L molecules

| Molecule | Titer [mg/l] | Recovery [%] | Yield [mg/l] | Analytical SEC (HMW/Monomer/LMW) |
|---|---|---|---|---|
| 2A | 28.01 | 48 | 2.69 | 2.9/97.1/0 |
| 2B | 22.30 | 82.96 | 3.72 | 4/96/0 |

**Example 4**

**Preparation, purification and characterization of T-cell bispecific (TCB) antibodies**

**4.1 Preparation of TCB antibodies with human or humanized binders**

TCB molecules have been prepared according to the methods described in WO 2014/131712 A1 or WO 2016/079076 A1.

The preparation of the anti-CEA/anti-CD3 bispecific antibody (CEA CD3 TCB or CEA TCB) used in the experiments is described in Example 3 of WO 2014/131712 A1. CEA CD3 TCB is a "2+1 IgG CrossFab" antibody and is comprised of two different heavy chains and two different light chains. Point mutations in the CH3 domain ("knobs into holes") were introduced to promote the assembly of the two different heavy chains. Exchange of the VH and VL domains in the CD3 binding Fab were made in order to promote the correct assembly of the two different light chains. 2+1 means that the molecule has two antigen binding domains specific for CEA and one antigen binding domain specific for CD3. CEACAM5 CD3 TCB has the same format, but comprises another CEA binder and comprises point mutations in the CH and CL domains of the CD3 binder in order to support correct pairing of the light chains.

CEA CD3 TCB comprises the amino acid sequences of SEQ ID NO:58, SEQ ID NO:59, SEQ ID NO:60 and SEQ ID NO:61. CEACAM5 CD TCB comprises the amino acid sequences of SEQ ID NO:62, SEQ ID NO:63, SEQ ID NO:64 and SEQ ID NO:65. A schematic scheme of the bispecific antibody in 2+1 format is shown in **Figure 1F.**

**4.2 Preparation of anti-CEA/anti-CD3 T cell bispecific antibody in 2+1 format (bivalent for murine CEA and monovalent for murine CD3)**

The anti-CEA(CH1A1A 98/99 2F1)/anti-CD3(2C11) T cell bispecific 2+1 surrogate molecule was prepared consisting of one CD3-Fab, and two CEA-Fabs and a Fc domain, wherein the two CEA-Fabs are linked via their C-termini to the hinge region of said Fc part and wherein the CD3-Fab is linked with its C-terminus to the N-terminus of one CEA-Fab. The CD3 binding moiety is a crossover Fab molecule wherein either the variable or the constant regions of the Fab light chain and the Fab heavy chain are exchanged.

The Fc domain of the murine surrogate molecule is a mu IgG1 Fc domain, wherein DDKK mutations have been introduced to enhance antibody Fc heterodimer formation as inter alia described by Gunasekaran et al., J. Biol. Chem. 2010,19637-19646. The Fc part of the first heavy chain comprises the mutations Lys392Asp and Lys409Asp (termed Fc-DD) and the Fc part of the second heavy chain comprises the mutations Glu356Lys and Asp399Lys (termed Fc-KK). The numbering is according to Kabat EU index. Furthermore, DAPG mutations were introduced in the constant regions of the heavy chains to abrogate binding to mouse Fc gamma receptors according to the method described e.g. in Baudino et al. J. Immunol. (2008), 181, 6664-6669, or in WO 2016/030350 A1. Briefly, the Asp265Ala and Pro329Gly mutations have been introduced in the constant region of the Fc-DD and Fc-KK heavy chains to abrogate binding to Fc gamma receptors (numbering according to Kabat EU index; i.e. D265A, P329G).

**Table 10** - amino acid sequences of murine anti-CEA/anti-CD3 T cell bispecific antibody.

| SEQ ID NO: | Description | Sequence |
|---|---|---|
| 75 | VHCH1(CH1A1 A 98/99 2F1)-Fc(KK) DAPG chain | QVQLVQSGAEVKKPGASVKVSCKASGYTFTEFGMNWVRQ APGQGLEWMGWINTKTGEATYVEEFKGRVTFTTDTSTSTA YMELRSLRSDDTAVYYCARWDFAYYVEAMDYWGQGTTV TVSSAKTTPPSVYPLAPGSAAQTNSMVTLGCLVKGYFPEPV TVTWNSGSLSSGVHTFPAVLQSDLYTLSSSVTVPSSTWPSQ TVTCNVAHPASSTKVDKKIVPRDCGCKPCICTVPEVSSVFIF PPKPKDVLTITLTPKVTCVVVAISKDDPEVQFSWFVDDVEV HTAQTKPREEQINSTFRSVSELPIMHQDWLNGKEFKCRVNS AAFGAPIEKTISKTKGRPKAPQVYTIPPPKKQMAKDKVSLT CMITNFFPEDITVEWQWNGQPAENYKNTQPIMKTDGSYFV YSKLNVQKSNWEAGNTFTCSVLHEGLHNHHTEKSLSHSPG K |
| 76 | VLCL (CH1A1A 98/99 2F1) Light chain | DIQMTQSPSSLSASVGDRVTITCKASAAVGTYVAWYQQKP GKAPKLLIYSASYRKRGVPSRFSGSGSGTDFTLTISSLQPEDF ATYYCHQYYTYPLFTFGQGTKLEIKRADAAPTVSIFPPSSEQ LTSGGASVVCFLNNFYPKDINVKWKIDGSERQNGVLNSWT |
| | | DQDSKDSTYSMSSTLTLTKDEYERHNSYTCEATHKTSTSPI VKSFNRNEC |

(continued)

| SEQ ID NO: | Description | Sequence |
|---|---|---|
| 77 | VHCL VHCH1 (2C11-CH1A1A 98/99 2F1)-Fc (DD) DAPG chain | EVQLVESGGGLVQPGKSLKLSCEASGFTFSGYGMHWVRQA PGRGLESVAYITSSSINIKYADAVKGRFTVSRDNAKNLLFLQ MNILKSEDTAMYYCARFDWDKNYWGQGTMVTVSSASDA APTVSIFPPSSEQLTSGGASVVCFLNNFYPKDINVKWKIDGS ERQNGVLNSWTDQDSKDSTYSMSSTLTLTKDEYERHNSYT CEATHKTSTSPIVKSFNRNECGGGGSGGGGSQVQLVQSGAE VKKPGASVKVSCKASGYTFTEFGMNWVRQAPGQGLEWM GWINTKTGEATYVEEFKGRVTFTTDTSTSTAYMELRSLRSD DTAVYYCARWDFAYYVEAMDYWGQGTTVTVSSAKTTPPS VYPLAPGSAAQTNSMVTLGCLVKGYFPEPVTVTWNSGSLS SGVHTFPAVLQSDLYTLSSSVTVPSSTWPSQTVTCNVAHPA SSTKVDKKIVPRDCGCKPCICTVPEVSSVFIFPPKPKDVLTIT LTPKVTCVVVAISKDDPEVQFSWFVDDVEVHTAQTKPREE QINSTFRSVSELPIMHQDWLNGKEFKCRVNSAAFGAPIEKTI SKTKGRPKAPQVYTIPPPKEQMAKDKVSLTCMITNFFPEDIT VEWQWNGQPAENYDNTQPIMDTDGSYFVYSDLNVQKSN WEAGNTFTCSVLHEGLHNHHTEKSLSHSPGK |
| 78 | VLCH1 (2C11) Light chain | DIQMTQSPSSLPASLGDRVTINCQASQDISNYLNWYQQKPG KAPKLLIYYTNKLADGVPSRFSGSGSGRDSSFTISSLESEDIG SYYCQQYYNYPWTFGPGTKLEIKSSAKTTPPSVYPLAPGSA AQTNSMVTLGCLVKGYFPEPVTVTWNSGSLSSGVHTFPAV LQSDLYTLSSSVTVPSSTWPSQTVTCNVAHPASSTKVDKKI VPRDC |

## Example 5

### 5.1 PBMC isolation

PBMCs were isolated from fresh blood of healthy donors. Briefly, blood was diluted 2:1 with PBS. About 30 ml of the blood/PBS mixture was layered on 15 ml of Histopaque and centrifuged for 30 min at 450 g without brake. The lymphocytes were collected with a 10 ml pipette into 50 ml tubes containing PBS. The tubes were filled up to 50 ml with PBS and centrifuged 10 min at 350 g. The supernatant was discarded, the pellet re-suspended in 50 ml PBS and centrifuged for 10 min at 300 g. The washing step was repeated once. The cells were re-suspended in RPMI containing 10 % FBS and 1 % GlutaMax and stored at 37°C, 5 % $CO_2$ in the incubator until assay start (not longer than 24h).

### 5.2 Splenocyte isolation

Spleens of C57B1/6 mice or hCEA(HO)Tg mice were transferred into gentleMACS C-tubes (Miltenyi) and MACS buffer (PBS + 0.5 % BSA + 2 mM EDTA) was added to each tube. Spleens were dissociated using the GentleMACS Dissociator, tubes were spun down shortly and cells were passed through a 100 μm nylon cell strainer. Thereafter, tubes were rinsed with 3 ml RPMI1640 medium (SIGMA, Cat.-No. R7388) and centrifuged for 8 min at 350 x g. The supernatant was discarded, the cell suspension passed through a 70 μm nylon cell strainer and washed with medium. After another centrifugation (350 x g, 8 min), supernatants were discarded and 5 ml ACK Lysis Buffer was added. After 5 min incubation at RT cells were washed with RPMI medium.

Afterwards the cells were re-suspended and the pellets pooled in assay medium (RPMI1640, 2 % FBS, 1 % Glutamax), for cell counting (Vi-Cell-Settings leukocytes, 1:10 dilution).

**Example 6**

**ICOS Expression on healthy human T cell subpopulations or tumour-infiltrating Lymphocytes of various tumour indications**

To compare the relative intensities of ICOS expressed on different T cell populations on healthy PBMCs versus TILs of different tumour indications, ICOS expression was evaluated using multi-color flow cytometry. In addition, for some patients PBMCs were isolated from normal tissue adjacent to the tumour tissue and ICOS expression was assessed as well (Figures 5 A to 5D). Tumour samples were derived from the following indications: pancreatic cancer (PancCa), lymph node metastasis (portio), penis adenocarcinoma, renal cell carcinoma (RCC), colorectal cancer (CRC), melanoma, mesothelioma, sarcomatoid soft tissue neoplasia, paracardiac tumour node, colon metastasis of an ovarian cancer, seminoma, head & neck cancer, Thymom, gastrointestinal stroma tumor (GIST), lung metastasis of an adenoid cystic carcinoma, kidney cancer, a rectum metastasis of a liver adenocarcinoma, small intestine cancer, lipomatous soft tissue tumour, and lung cancer (LC).

**6.1 Digestion of Human Tumor or adjacent normal tissue samples**

For the analysis of ICOS expression on T-cells from tumour or normal tissue, samples were digested and single-cell suspensions were analyzed by flow cytometry.

Briefly, a digestion mix was prepared, using 4.4 ml tissue storage solution (#130-100-008, Miltenyi Biotech), 5 ml Accutase (#A6964, Sigma), 333 µl 1 % BSA (#A9576, Sigma) and 260 µl enzyme mix (275 U/ml Collagenase IV, #LS004189, Worthington, 10 U/ml DNAse I Type 4, #10 U/ml DNAse I Type 4, Sigma and 471 U/ml Hyaluronidase #H6254, Sigma).

Tumor, respective adjacent normal tissue pieces were cut in small pieces at room temperature and incubated in the digestion mix for 40 minutes at 37 °C, using a rotating device. Cells were further separated, using a 70 µm cell strainer (#352350, Corning). The cell strainer was washed with 10 ml cold PBS (#352350, GIBCO) and the cell suspension was centrifuged for 10 minutes at 250 x g at 4°C. After an additional washing step with cold PBS, followed by centrifugation as described above, erythrocytes were lysed by incubating the cell pellets for 5 to 15 minutes in 1x PharmLyse buffer (#555899, BD Biosciences) at room temperature. The lysis was stopped by addition of cold PBS and centrifugation for 10 minutes at 250 x g and 4 °C. Cells were washed a second time with cold PBS and number of living cells was determined using trypan blue staining.

**6.2 Digestion of Human Tumor or adjacent normal tissue samples**

Expression level of ICOS on human T-cells from healthy donors, on TILs or on T-cells isolated from normal tissue of cancer patients was determined as follows: 0.05-0.3 million cells were washed once, using PBS and centrifugation for 5 minutes at 350 x g. For the discrimination of live and dead cells, PBMCs were incubated with Zombie UV (#423108, BioLegend) for 20 minutes at room temperature. Cells were washed with PBS and FACS buffer (PBS, containing 2 % FCS, 5 mM EDTA and 0.25 % sodium acide) and stained for CD4 (#317438, BioLegend), CD8 (#301044, BioLegend) and ICOS (#313520, BioLegend) for 20 minutes at room temperature. After two washing steps with FACS buffer, staining was fixed by incubation of cells in 2 % PFA-containing PBS for 15 minutes at room temperature. Cells were washed with FACS buffer once and analyzed by flow cytometry (FACS Fortessa instrument, equipped with Diva Software).

**Example 7**

**CEACAM5-TCB mediated up-regulation of ICOS on peripheral T-cells and TILs of Tumor Patients (FACS)**

Enhanced expression of human ICOS upon TCB-mediated activation of human T-cells in vitro was evaluated by flow cytometry (Figures 3E-3H). Briefly, 0.2 Mio PBMCs or TILs isolated from an ovarian cancer patient with a colon metastasis (6.6.1 and 6.4) were re-suspended in DMEM, including 10 % FCS, 1 % Glutamax, 1 mM Sodium Pyruvate, 1 x NEAA + Pen/Strep and incubated with 0.04 Mio CEA-expressing Lovo tumor cells (#CCL-229, ATCC) and 10 nM CEACAM5-TCB, using flat-bottom-96-well plates.

In another example (Figures 4A and 4B), 0.25 million PBMCs of healthy donors were incubated with 0.025 Mio CEA-positive MKN45 and varying concentrations of CEACAM5-TCB (0.1 pM to 20 nM) for 48 hours in flat-bottom-96-well plates. Up-regulation of ICOS was assessed after 48 h of incubation at 37°C, 5% $CO_2$ after staining of surface markers CD4, CD8 and ICOS, as described below.

PBMCs were harvested from assay plates and transferred to fresh round-bottom 96-well-plates for subsequent staining at 4°C. Cells were washed once with FACS buffer and then stained for 30 min at 4°C, using anti-human CD4 (#300506, BioLegend), anti-human CD8 (#344722, BioLegend), anti-human CD25 (#356120, BioLegend), anti-human CD69(#310930, BioLegend), and anti-human ICOS (#310931 BioLegend) according to the manufacturers' instructions. Cells were washed twice using FACS buffer and fixed for 15 minutes at room temperature in FACS buffer, containing 2 % PFA. After one washing step with FACS buffer, cells were re-suspended in FACS buffer and analyzed using a BD FACS Fortessa machine.

**Example 8**

**Murine CEA-TCB mediated up-regulation of murine ICOS on murine splenocytes (FACS)**

Enhanced expression of murine ICOS upon TCB-mediated activation of murine T-cells ex vivo was evaluated by flow cytometry (Figures 5A-5F). For this, a classical tumor cell lysis experiment was performed as described in Example 14. After 48h, splenocytes were harvested and transferred into a fresh round-bottom 96-well plate for subsequent staining.

Cells were washed once with PBS and stained using the UV Zombie dye (#423108 BioLegend), diluted 1:1000 in PBS for 30 minutes at 4°C. Cells were washed with FACS buffer twice and surface staining was performed for 30 minutes at 4°C, using anti-mouse TCR (#109228 BioLegend), anti-mouse ICOS (#135220 BioLegend), anti-mouse CD4 (#100422 BioLegend), anti-mouse CD8 (#100747 BioLegend) according to the manufacturers' instructions. Cells were washed with FACS buffer twice and intracellular staining was started with addition of Perm/Fix Buffer (#421403 BioLegend) for 30 - 45 minutes at room temperature protected from light. Cells were washed three times with Perm Buffer (#421402 BioLegend) and stained in perm buffer, containing anti-murine FoxP3 (#320014 BioLegend). Cells were washed twice using FACS buffer and fixed for 15 minutes at room temperature in FACS buffer, containing 2 % PFA. After one washing step with FACS buffer, cells were re-suspended in FACS buffer and analyzed using a BD FACS Fortessa machine.

**Example 9**

**Binding of FAP-targeted ICOS and murine ICOS-L, respective untargeted reference molecules to FAP- and ICOS-expressing cells**

The binding of several FAP-ICOS molecules prepared in Examples 1 and 2 was tested using human fibroblast activating protein (huFAP) expressing NIH/3T3-huFAP clone 19. This cell line was generated by the transfection of the mouse embryonic fibroblast NIH/3T3 cell line (ATCC CRL-1658) with the expression vector pETR4921 to express huFAP under 1.5$\mu$g/mL Puromycin selection. The binding to human ICOS was tested with activated human T-Cells.

Furthermore, mICOS-L-targeting bispecific molecules prepared in Example 3 were tested for their binding to murine FAP (mFAP), using 3T3-mFAP cells (parental cell line ATCC #CCL-92, modified to stably overexpress murine FAP, and the binding to murine ICOS-expressing cells was tested using CHO mICOS (parental cell line CHO-k1 ATCC #CCL-61, modified to stably overexpress murine ICOS). Briefly, cells were harvested, counted, checked for viability and re-suspended at 1 million cells per ml in FACS buffer (PBS with 0.1% BSA). 100 $\mu$l of the cell suspension (containing 0.1 million cells) were incubated in round-bottom 96-well plates for 30 min at 4°C with increasing concentrations of the FAP-targeted ICOS or mICOS-L constructs (7 pM - 120 nM for the binding of FAP-ICOS constructs to T-Cells / 3T3-huFAP cells, respective 4 pM - 300 nM for the binding of mICOS-L constructs), cells were washed twice with cold PBS 0.1% BSA, re-incubated for further 30 min at 4°C with the PE-conjugated, donkey anti human H+L PE (Jackson Immuno Research Lab #709-116-149) and washed twice with cold PBS 0.1% BSA. The staining was fixed for 20 min at 4°C in the dark, using 75 $\mu$l of 1% PFA in FACS buffer per well. Fluorescence was analyzed by FACS using a FACS Fortessa (Software FACS Diva). Binding curves were obtained using GraphPadPrism6 and are shown in Figures 6A-6C and 7A-7B, respectively.

The $EC_{50}$ values, as determined by binding of different FAP- or DP47-ICOS molecules to human ICOS- and human FAP-expressing cells are shown in Table 11 and the EC50 values determined for binding of FAP-mICOS-L to murine ICOS-expressing CHO transfectants and murine FAP-expressing 3T3 cells are presented in Table 12 below.

**Table 11:** $EC_{50}$ values of binding of different FAP- or DP47-ICOS molecules to human ICOS- and human FAP-expressing cells

| Molecule | Human ICOS on CD4$^+$ T-Cells $EC_{50}$ (nM) | Human ICOS on CD8+ T-Cells $EC_{50}$ (nM) | Human FAP $EC_{50}$ (nM) |
|---|---|---|---|
| FAP-ICOS_1+1 | 34.86 | 50.72 | 2.98 |

(continued)

| Molecule | Human ICOS on CD4+ T-Cells $EC_{50}$ (nM) | Human ICOS on CD8+ T-Cells $EC_{50}$ (nM) | Human FAP $EC_{50}$ (nM) |
|---|---|---|---|
| FAP-ICOS_1+1_HT | 42.62 | 56.43 | 6.73 |
| FAP-ICOS_2+1 | 10.74 | 14.38 | 5.19 |
| DP47-ICOS_1+1 | 19.85 | 25.7 | no binding |
| DP47-ICOS_2+1 | 9.99 | 12.36 | no binding |

**Table 12:** $EC_{50}$ values, binding of FAP-mICOS-L to murine ICOS-expressing CHO transfectants and murine FAP-expressing 3T3 cells

| Molecule | Target | $EC_{50}$ (nM) |
|---|---|---|
| FAP-mICOS-L | Murine ICOS | 28.05 |
| DP47-mICOS-L | Murine ICOS | 23.57 |
| FAP-mICOS-L | Murine FAP | 71.8 |

**Example 10**

**_In vitro_ Functional Characterization of FAP-targeted ICOS, respective mICOS-L molecules**

Several cell-based in vitro assays were performed to evaluate the activity of FAP-targeted ICOS or mICOS-L molecules versus their corresponding untargeted (DP47) reference molecules.

The assays were designed to show additional agonistic/co-stimulatory activity of the anti-ICOS bispecific molecules in presence of T-cell bispecific-(TCB) mediated activation of T-cells.

1. Jurkat assay (reporter cell line with NFAT-regulated expression of luciferase, induced upon engagement of the CD3/TCR and ICOS), wherein ICOS IgG molecules, plate-bound vs. in solution and in absence versus presence of a coated CD3 IgG stimulus were measured

2. Primary human PBMC co-culture assay, wherein FAP-targeted ICOS molecules, cross-linked by simultaneous binding to human ICOS on T-cells and human FAP, expressed on 3T3-hFAP cells (parental cell line ATCC #CCL-92, modified to stably overexpress human FAP), in the presence of a TCB molecule being crosslinked by simultaneous binding to CD3 on T-cells and human CEA on tumor cells were tested. The FAP-targeted cross-linked ICOS molecules versus untargeted DP47-ICOS molecules were measured in solution, in absence versus presence of a TCB molecule and CEA-positive tumor cells. T-cell activation, T-cell differentiation or T-cell proliferation as determined by flow cytometry were obtained as readouts.

3. Primary murine splenocyte co-culture assay, wherein FAP-targeted mICOS-L molecule, cross-linked by simultaneous binding to murine ICOS on T-cells and murine FAP, expressed on 3T3-mFAP cells (parental cell line ATCC #CCL-92, modified to stably overexpress murine FAP), in presence of a TCB molecule being crosslinked by simultaneous binding to mCD3 on murine T-cells and human CEA on MC38-hCEA tumor cells was measured.

**Example 11**

**Jurkat Reporter Cell Line Assay**

The Dependency on a simultaneous TCR engagement was assessed by using an engineered Jurkat Cell Line expressing Luciferase in response to NFAT nuclear translocation.

GloResponse Jurkat NFAT-RE-luc2P (Promega #CS176501) reporter cell line was preactivated to induce ICOS expression using Cell Culture Flasks coated with 1.5 $\mu$g/ml aCD3 (BioLegend #317304) and 2 $\mu$g/ml aCD28 (BioLegend, #302914) in JurkatNFAT culture Medium (RPMI1640 medium containing 10% FCS, 1% GluMax, 25 mM HEPES, 1x NEAA, 1 % So-Pyruvate; selection: 200 ug/ml Hygromycin B) .

Cells were starved (JurkatNFAT culture Medium without Stimulation) overnight before the assay. Assay Plates (Greiner, 96 well, white wall, clear bottom; # 655098) were coated (4 °C overnight) simultaneously with either 0.5 μg/ml aCD3 (BioLegend #317304) plus FAP-ICOS / DP47-ICOS molecules or FAP-ICOS / DP47-ICOS molecules only at the indicated concentrations (range of 4 pM - 65000 pM in triplicates). The next day the plates were washed once with DPBS (Gibco, #14190136) and 0.1 Mio stimulated and starved GloResponse Jurkat NFAT-RE-luc2P were added. NFAT mediated signaling was assessed after 5 h of incubation at 37°C, 5% $CO_2$ by Luminescence Reading using Promega OneGlo Assay System (Promega, # E6120) according to manufacturer instructions. Plates were read on a Tecan Spark10M Plate Reader (Luminescence Reading, 1000 ms Integration Time, Auto Attenuation Setting).

## Example 12

### Primary human PBMC co-culture Assay

Enhanced T-cell activation, T-Cell proliferation or T-Cell differentiation mediated by combining CEACAM5-TCB and the FAP-ICOS molecules was assessed on CEA-expressing MKN45 (DSMZ #ACC 409) cells and huFAP-expressing Fibroblasts (NIH / 3T3-huFAP clone 19).

Human PBMCs were used as effector cells. T- Cell Activation was detected after 48 h, T-Cell Proliferation and -Differentiation after 96 h of incubation with FAP-ICOS and CEACAM5-TCB. Briefly, adherent target cells and Fibroblasts were harvested with Trypsin/EDTA, washed, and plated at density of 10 000 cells/well using flat-bottom 96-well plates one day before the experiment. Cells were left to adhere overnight. Peripheral blood mononuclear cells (PBMCs) were prepared by Histopaque (Sigma-Aldrich, Cat No. 10771-500ML Histopaque-1077) density centrifugation of enriched lymphocyte preparations of heparinized blood obtained from a Buffy Coat ("Blutspende Zurich"). The blood was diluted 1:2 with sterile DPBS and layered over Histopaque gradient (Sigma, #H8889). After centrifugation (450 x g, 30 minutes, room temperature), the plasma above the PBMC-containing interphase was discarded and PBMCs transferred in a new falcon tube subsequently filled with 50 ml of PBS. The mixture was centrifuged (400 x g, 10 minutes, room temperature), the supernatant discarded and the PBMC pellet washed twice with sterile PBS (centrifugation steps 350 x g, 10 minutes). The resulting PBMC population was counted automatically (Cedex HiRes) and stored in RPMI1640 medium containing 10% FCS and 1% Glutamax (Gibco 35050061) at 37°C, in a humidified incubator until the assay was started. To measure T-Cell Activation (Figures 9A-9H), the FAP-ICOS molecules were added at the indicated concentrations (range of 0.11 pM - 5000 pM in triplicates) and the CEACAM5-TCB was added at a fixed concentration (80pM). As controls, wells containing only the TCB molecule or only the FAP-ICOS molecules were included. For T-Cell Proliferation and Differentiation (Figures 10A and 10B), CEACAM5-TCB was added with increasing concentrations (range of 1.28 pM - 20000 pM in triplicates) and the FAP-ICOS molecules were added at a fixed concentration (1000 pM)

PBMCs were added to target cells and Fibroblasts to obtain a final E:T ratio of 5:1:1. T-Cell Activation was assessed after 48 h of incubation at 37°C, 5% $CO_2$ by flow cytometric analysis, using antibodies recognizing the T cell activation markers CD69 (early activation marker) and CD25 (late activation marker). T Cell Proliferation was assessed after 96 h of incubation at 37°C, 5% $CO_2$ by Flow cytometric analysis, using absolute Counts of CD4+ and CD8+ cells normalized to Counting Beads. T Cell Differentiation was assessed after 96 h of incubation at 37°C, 5% $CO_2$ by Flow cytometric analysis, using antibodies against CD45R0 and CCR7 to discriminate the memory subsets.

Briefly, PBMCs were centrifuged at 400 x g for 4 min and washed twice with PBS containing 0.1% BSA (FACS buffer). Surface staining for CD8 (PerCP/Cy5.5 anti-human CD8a, BioLegend #301032), CD4 (APC/Cy7 anti-human CD4, BioLegend # 300518), CD69 (BV421 anti-human CD69, BioLegend #310930), CD25 (PE anti-human CD25, BioLegend #356104), CD45R0 (APC anti-human CD45R0, BioLegend # 304210) and CCR7 (FITC anti-human CCR7, BioLegend # 353216) was performed according to the suppliers' indications. Absolute T-Cell Counts were obtained using Counting Beads (Precision Count Beads, BioLegend #424902) according to manufacturer's instruction. Cells were thenn washed twice with 150 μl/well PBS containing 0.1% BSA and fixed for 15 - 30 min at 4°C using 75 μl/well FACS buffer, containing 1% PFA. After centrifugation, the samples were re-suspended in 150 μl/well FACS buffer and analyzed using BD FACS Fortessa.

## Example 13

### Primary Murine Splenocyte co-culture Assay

T-cell activation induced by mCEA-TCB upon co-incubation of murine splenocyte effector cells and human CEA-expressing MC38-hCEA target cells (parental cell line as described in Cancer Res 1975, 35:2434-2440, 3T3modified in-house to stably overexpress human CEA) in the presence or absence of mICOS-L molecules was assessed as follows (Figures 11A and 11B):

1.25 million splenocytes isolated from spleens of hCEA(HO) transgenic C57/BL6 mice or C57/BL6 wildtype mice (6.5) were added to 25 000 pre-plated MC38-hCEA tumor target cells per well of a flat-bottom-96-well plate. A fixed concentration of 1.5 nM of mCEA-TCB and 50 nM of a FAP-targeted or an untargeted DP47-mICOS-L molecule was added and the assay plate was incubated for 48h at 37 °C and 5% $CO_2$ in a humidified incubator in T cell media (RPMI1640, containing 10% FCS, 1x GlutaMax (Gibco #35050061), 50 mM x β-MEtoH (Sigma,#M3148-100), 200 U/ml IL2 (Proleukin, Novartis), 1 x antibiotic - antimycotic (Gibco #15240062), 1mM Sodium Pyruvate(Gibco #11360070)).

**Example 14**

***In vivo* Functional Characterization of FAP-targeted ICOS Molecule in combination with CEACAM5-TCB**

**14.1 Pharmacokinetic profile of FAP-ICOS (1+1) after single injection in NSG mice**

A single dose of 2.5 mg/kg of FAP-ICOS (1+1) was injected into NSG mice. All mice were injected i.v. with 200 μl of the appropriate solution. To obtain the proper amount of compounds per 200 μl, the stock solution (Table 12, FAP-ICOS) was diluted with histidine buffer. Three mice per time point were bled at 10min, 1hr, 3hr, 6hr, 24hr, 48hr, 72hr, 96hr, 6 days, 8 days, 10 days and 12 days. The injected compound was analyzed in serum samples by ELISA. Detection of the molecule was carried out by huIgG ELISA (detection via human Fc Antibody part). The plate was washed three times after each step to remove unbound substances. Finally, the peroxidase-bound complex was visualized by adding ABTS substrate solution to form a colored reaction product. The reaction product intensity which was photometrically determined at 405 nm (with reference wavelength at 490 nm) was proportional to the analyte concentration in the serum sample. The result (Figure 12) showed a stable PK-behavior ($T_{1/2}$: 9.52 days) which suggested a once weekly schedule for subsequent efficacy studies.

**Table 13:** Description of tested composition

| Compound | Formulation buffer | Concentration (mg/mL) |
|---|---|---|
| FAP-ICOS (1+1) | 20mM Histidine, 140mM NaCl, pH6.0 | 2.74 (= stock solution) |

**14.2 *In vivo* efficacy of FAP-ICOS (1+1) in combination with CEACAM5-TCB in MKN45 cografted with 3T3-huFAP in fully humanized NOG mice**

The first proof of concept study for the combination of FAP-ICOS and CEACAM5-TCB was aimed to understand the potency in terms of tumor regression and Immuno-PD in fully humanized NSG mice.

Human MKN45 cells (human gastric carcinoma) were originally obtained from ATCC and after expansion deposited in the Glycart internal cell bank. Cells were cultured in DMEM containing 10% FCS at 37 °C in a water-saturated atmosphere at 5 % $CO_2$. In vitro passage 12 was used for subcutaneous injection at a viability of 97%. Human fibroblasts NIH-3T3 were originally obtained from ATCC (ATCC #CCL-92), engineered at Roche Nutley to express human FAP and cultured in DMEM containing 10% Calf serum,1x Sodium Pyruvate and 1.5 μg/ml Puromycin. Clone 39 was used at an in vitro passage number 18 and at a viability of 98.2%.

50 microliters cell suspension (1x106 MKN45 cells + 1x106 3T3-huFAP) mixed with 50 microliters Matrigel were injected subcutaneously in the flank of anaesthetized mice with a 22G to 30G needle.

Female NSG mice, age 4-5 weeks at start of the experiment (Jackson Laboratory) were maintained under specific-pathogen-free condition with daily cycles of 12 h light / 12 h darkness according to committed guidelines (GV-Solas; Felasa; TierschG). The experimental study protocol was reviewed and approved by local government (P 2011/128). After arrival, animals were maintained for one week to get accustomed to the new environment and for observation. Continuous health monitoring was carried out on a regular basis.

Female NSG mice were injected i.p. with 15 mg/kg of Busulfan followed one day later by an i.v. injection of 1x105 human hematopoietic stem cells isolated from cord blood. At week 14-16 after stem cell injection mice were bled sublingual and blood was analyzed by flow cytometry for successful humanization. Efficiently engrafted mice were randomized according to their human T cell frequencies into the different treatment groups. At that time, mice were injected with tumor cells and fibroblasts s.c. as described (Figure 13) and treated once weekly with the compounds or Histidine buffer (Vehicle) when tumor size reached appr. 200 mm³ (day 23). All mice were injected i.v. with 200 μl of the appropriate solution. To obtain the proper amount of compounds per 200 μl, the stock solutions (Table 13) were diluted with Histidine buffer when necessary. For combination therapy (Group C, Figure 13) with FAP-ICOS and CEACAM5-TCB the respective compositions were injected concomant. Tumor growth was measured twice weekly using a caliper (Figure 2) and tumor volume was calculated as followed:

$$T_v: (W^2/2) \times L \qquad (W: Width, L: Length)$$

At termination (day 44), mice were sacrificed, tumors and spleen were removed, weighted and single cell suspensions were prepared through an enzymatic digestion with Collagenase V, Dispase II and DNAse for subsequent FACS-analysis. Single cells where stained for human CD45, CD3, CD8, CD4, CD25 and FoxP3 (intracellular) and analyzed at FACS Fortessa.

Small pieces (30 mg) of tumor tissues were snap frozen and whole protein was isolated. Protein suspensions were analysed for cytokine content by Multiplex analysis.

Figures 14A-14E show the tumor growth kinetics (Mean) in all treatment groups as well as the tumor weights at study termination. CEACAM5-TCB as a single agent induced some tumor growth inhibition. However, the combination with FAP-ICOS showed significant improved tumor growth inhibition that was also reflected by tumor weight at study termination. Interestingly, the Immuno-PD data (Figures 15A-15D) of tumors from animals sacrificed at study termination, revealed a depletion of human Treg cells specifically in the tumor tissue. No depletion was detected in spleen of treated animals (Figures 15C and 15D, flow cytometry). The depletion of Treg shifted the CD8/Treg towards CD8 cells in the combination treatment.

Furthermore, the cytokine analyses showed evaluated levels of CXCL13, TNF-$\alpha$ and CCL3 within the tumor tissue in the combination group (Figures 16A, 16B and 16C).

**Table 14:** Description of tested composition

| Compound | Formulation buffer | Concentration (mg/mL) |
|---|---|---|
| FAP-ICOS (1+1) | 20mM Histidine, 140mM NaCl, pH6.0 | 2.74 (= stock solution) |
| CEACAM 5 TCB | 20mM Histidine, 140mM NaCl, pH6.0 | 4.7 (= stock solution) |

**Example 15**

**Gene Expression Analysis**

To identify ICOS-regulated genes, tumour tissues of the above efficacy study in humanized NSG mice (Exampe 14) were analyzed using the nCounter® Human Immunology Panel (NanoString Technologies, Seattle, USA).

Briefly, fresh frozen humanized mouse tumour tissues (30-70 mg) were ruptured and homogenized in RNA lysis buffer (Qiagen, Hilden, Germany), followed by total RNA extraction using the RNEasy Mini Kit (Qiagen, Hilden, Germany). Gene expression was quantified using the NanoString nCounter platform.

200 ng of total RNA was analyzed with the nCounter® Human Immunology Panel (NanoString Technologies, Seattle, USA) comprising a panel of barcoded probes matching 594 different immunology-related human genes. The codeset was hybridized with the RNA over night at 65°C. RNA transcripts were immobilized and barcoded probes were counted using the NanoString nCounter Digital Analyzer. Normalized raw expression data (nSolver Analysis software) were analyzed when 2 standard deviations above the geometric mean of the codeset-internal negative control probes were reached. Genes were excluded from further analysis if 90% of their expression was below the background threshold. The genes that remained after background-filtering were normalized to the geometric mean of the internal positive controls as well as to 5 housekeeping genes (GAPDH, HPRT1, ALAS1, GUSB and TUBB) and then log2-transformed.

Data was analyzed with Qlucore Omics Explorer (QOE) software (Qlucore AB, Lund, Sweden). A PCA and hierarchical clustering were performed to determine the differences in gene expression between the treatment groups. Genes were considered differentially expressed when displaying a 2-fold difference in transcript level.

Figures 17A and 17B show TNFAIP6 and CXCL13 being the two strongest ICOS-up-regulated genes in combination with CEACAM5-TCB-mediated T-cell activation.

**CONCLUSIONS**

**1. *In-vitro* characterization of FAP-ICOS molecules**

To understand a potential clinical relevance of ICOS as a cancer immunotherapeutic target, the expression of ICOS on TILs of various tumour indications was assessed.

As depicted in Figures 3A-3D, tumor-infiltrating-lymphocytes of both subtypes, CD4[+] and CD8[+] T-cells, show on average higher ICOS expression as T-cells from the same tumour patients isolated from normal tissue, or (peripheral) T-cells from healthy donors. Significant donor variations in expression level, as well as percent of ICOS-expressing T-cells could be observed. Upon ex vivo co-incubation of patient-derived tumour pieces with TILs, Lovo tumor cells and CEACAM5-TCB, the ICOS level on TILs could be increased only marginally, since most of the T-cells were already ICOS-positive at baseline (Figures 3E-3H).

However, co-incubation of Lovo tumor cells with CEACAM5-TCB and freshly isolated PBMCs of healthy donors shows much lower baseline levels of ICOS on CD4$^+$ and CD8$^+$ T-cells and a profound increase of ICOS upon TCB-mediated activation of T-cells.

The up-regulation of ICOS on both, CD4$^+$ and CD8$^+$ T-cells, directly correlates with the TCB concentration used for the T-cell activation and is seen consistently with different TCB molecules (Figures 4A to 4D).

Likewise, the co-incubation of mCEA-TCB or mCEACAM5-TCB with CEA-positive tumor cells and CD3-positive T-cells is able to up-regulate the expression of murine ICOS on CD4+, CD8+ and Treg cells in a concentration-dependent manner (Figures 5A-5F).

This validates ICOS as a therapeutic target being up-regulated at baseline already for a lot of tumour indications, as well as a potential combination partner for TCBs in settings with low ICOS baseline expression on T-cells.

In vitro cell binding assays verify that the ICOS-FAP molecules are able to bind to both human FAP as well as human ICOS on cells in a concentration dependent manner (Figures 6A and 6B: Binding to hICOS on human T-Cells; Figure 6C: Binding to huFAP on 3T3-huFAP cells). As expected, the untargeted ICOS-DP47 control molecules show no binding to huFAP (Figure 8B). The molecules that target FAP with a c-terminal fusion (ICOS-FAP 2+1 / ICOS-FAP 1+1 HT) show a slightly higher EC50 value for binding to human FAP (Table 10). No significant differences between the formats can be found for the binding to human ICOS.

As depicted in Figures 7A and 7B, both molecules, the untargeted DP47-mFAP-L, as well as the FAP-mICOSL bind to murine ICOS in a concentration dependent manner. In addition, FAP-mICOS-L shows concentration-dependent binding to murine FAP, as expected.

Characterization of FAP-ICOS molecules using Jurkat-NFAT Reporter cells or primary cells demonstrated dependency of ICOS signaling on simultaneous CD3/TCR engagement (Figure 8A). All ICOS molecules were crosslinked by direct binding to the plate and therefore, the DP47-ICOS and the FAP-ICOS molecules showed similar activities in the presence of a simultaneous CD3 trigger. However, in the absence of a simultaneous CD3 trigger, no downstream signaling could be observed.

In a second approach, the effect of targeting, respective crosslinking on the induction of ICOS signaling was assessed: cross-linking of the anti-ICOS antibodies via binding of their FAP moiety to human FAP expressing cells led to much more effective signaling downstream of ICOS, as compared to the untargeted DP47-ICOS molecules, which were assessed in solution (Figures 8B-8E and 8F-8I). It should be noted, that in this model system, also the untargeted molecules show a certain level of activity, however to a much lower extent than their respective targeted versions. This clearly indicates the importance of crosslinking to exploit full power of the ICOS signaling.

Given that ICOS is upregulated upon CEACAM5-TCB treatment (Figures 3A-3H and Figures 4A-4D) the potential of the FAP-ICOS molecules to enhance CEACAM5-TCB mediated T-Cell Activation was assessed (Figures 9A-9H). Figures 9A to 9D show exemplary graphs of enhanced CD25 and CD69 expression on CD4$^+$ (9A and 9B) and CD8$^+$ (9C and 9D) T-Cells, revealing a dose dependent increase in CD25 and CD69 expression above CEACAM5-TCB treatment only. Figures 9E-9H depict a summary of up to 5 different healthy human PBMC donors and all five FAP-ICOS or DP47-ICOS molecules. It should be noted that the enhancing effects of FAP-ICOS, as well as the baseline activation levels due to CEACAM5-TCB treatment vary quite strongly between different donors, with some donors not displaying an enhancing effect of FAP-ICOS at all (e.g. Donor 4).

In line with the observations above, the co-incubation of FAP-targeted mICOS-L molecules in combination with a mCEA-TCB molecule leads to enhanced T-cell activation as compared to the TCB effect alone, which could be seen in a dose-dependent up-regulation of CD25 and CD69 on CD4$^+$ and CD8+ T-cells (Figures 11A and 11B).

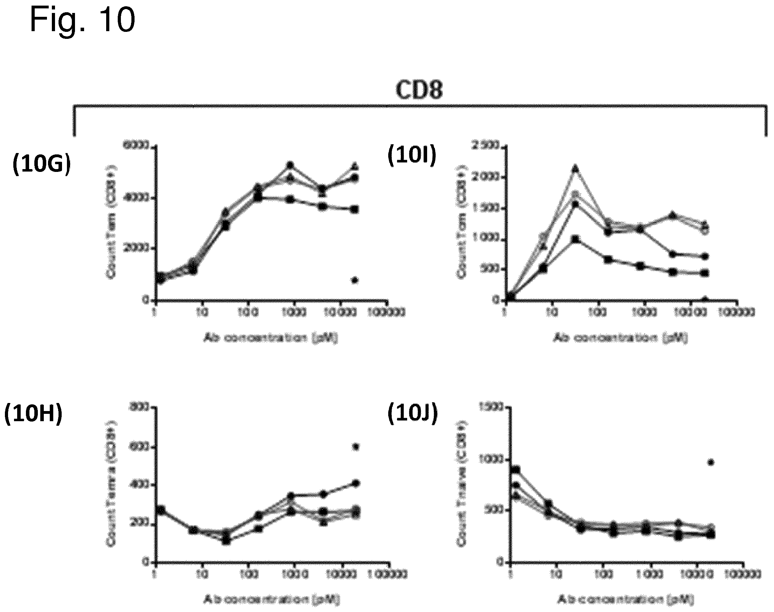

To further characterize the effects of combining CEACAM5-TCB with a targeted ICOS therapy, we characterized the response of primary cells after 96 hours in terms of T-Cell proliferation and T-Cell differentiation (Figure 10). As depicted, the combination of CEACAM5-TCB and FAP-ICOS induced proliferation of both, CD4$^+$ and CD8$^+$ T-cells as compared to CEACAM5-TCB treatment only (Figures 10A and 10B). All three formats perform comparably well. Figures 12C-12F and 12G-12J show that increased numbers of effector memory and central memory T-Cells upon combination treatment with CEACAM5-TCB and FAP-ICOS compared to CEACAM5-TCB monotherapy at increasing TCB concentrations were observed, respective at a fixed concentration of 2.9 pM of the CEACAM5-TCB (Figure 10K), again all three formats performing equally well. This supports the finding, that the boosting effect on early T-Cell Activation (Figure 9) propagates to an increase in total number of T-Cells and an increase in the formation of desired memory T-Cell Subsets, which are supposed to enable long-term efficient anti-tumor responses in patients.

## 2. *In-vivo* characterization of FAP-ICOS molecules

The FAP-ICOS_1+1 molecule from the screening assays above was evaluated in vivo using humanized NSG mice, co-grafted with human CEA-expressing MKN45 tumor cells and 3T3-hFAP expressing fibroblasts. As expected, the monotherapy of CEACAM5-TCB led to a delay in tumor growth. However, the combination of CEACAM5-TCB and FAP-ICOS induced a much stronger anti-tumor response, which was due to a tumor-specific Treg depletion, activation of T effector cells and consequently an improved ratio of CD8 effector to Treg cells.

Moreover, cytokine analysis of remaining tumor samples, revealed significantly up-regulated levels of CXCL13, TNF-alpha and CCL3 in the combination group compared to the monotherapy with CEACAM5-TCB (Figures 16A to 16C).

CCL3 is a cytokine belonging to the CC chemokine family and has been shown to interact with CCL4. It is also known as MIP1alpha and is described to attract macrophages, monocytes and neutrophils. In the tumor microenvironment CCL3 augments the antitumor immune response in a vaccine-context (Allen, 2016).

In line with the cytokine analysis above, CXCL13 and TNFAIP6 were identified as the two strongest ICOS-specific regulated genes (Figures 17A and 17B).

Tumor necrosis factor-inducible gene 6 protein also known as TNF-stimulated gene 6 protein or TSG-6. It is described as a potential biomarker of disease activity in inflammatory bowel disease. It exhibits a hyaluronan-binding domain that is known to be involved in extracellular matrix stability and cell migration.

The chemokine (C-X-C motif) ligand 13 (CXCL13) is also known as B lymphocyte chemoattractant (BLC) or B cell-attracting chemokine 1 (BCA-1). CXCR5(hi)ICOS(hi) CD4 T cells are the most potent inducers of IgG production that also secrete large amounts of the B cell-attracting chemokine CXCL13. Therefore, CXCL13 and its receptor CXCR5 control the organization of B cells within follicles of lymphoid tissues and might play an important role in the formation of tertiary lymphoid structures in several tumor indications.

The B cell chemoattractant CXCL13 has recently been linked with TFH cell infiltration and improved survival in human cancer, due to local memory B cell differentiation induced by CXCL13-producing (CXCR5) follicular helper T cells (Gu-Trantien 2017). This again highlights a potential important role of ICOS in mediating potent anti-tumor responses via different secondary mechanisms.

Taken together, ICOS may serve as a potent anti-tumor molecule as monotherapy in several inflamed tumor indications with significant ICOS baseline expression of tumor-responsive T-cells. Using a FAP-targeted ICOS molecule allows tumor-specific activation of ICOS-expressing activated T effector cells and therefore is supposed to have a preferential safety and potency profile as compared to untargeted ICOS molecules.

T-cell bispecifics are potent immune engangers, which can induce T-cell activation, as well as enhance T-cell infiltration into tumours. Elevated ICOS expression upon TCB therapy could enable the targeting of a much broader patient population as compared to ICOS monotherapy approaches by combining a potent TCB with a tumor-specific ICOS molecule, which is expected to lead to enhanced and prolonged anti-tumor responses compared to a TCB or ICOS monotherapy.

## References

Allen F., Bobanga J., et al., CCL3 in the tumor microenvironment augments the antitumor immune response. J Immunol May 1, 2016, 196 (75.11)

Allison J, Sharma P, Quezada, S.A., Fu T. Combination immunotherapy for the treatment of cancer, WO2011/041613A2 2009

Bacac M, Fauti T, Sam J, et al. A Novel Carcinoembryonic Antigen T-Cell Bispecific Antibody (CEA TCB) for the Treatment of Solid Tumours. Clin Cancer Res. 2016 Jul 1; 22(13):3286-97.

Bacac M, Klein C, Umana P. CEA TCB: A novel head-to-tail 2:1 T cell bispecific antibody for treatment of CEA-positive solid tumours. Oncoimmunology. 2016 Jun 24;5(8).

Carthon B C et al., "Preoperative CTLA-4 blockade: Tolerability and immune monitoring in the setting of a presurgical clinical trial Clin Cancer Res. 2010 16(10); 2861-71.

Dammeijer F., Lau S.P., van Eijck C.H.J., van der Burg S.H., Aerts J.G.J.V. Rationally combining immunotherapies to improve efficacy of immune checkpoint blockade in solid tumours. Cytokine & Growth Factor Reviews 2017 Aug; 36: 5-15.

Davidson E.H., Hood L., Dimitrov K., Direct multiplexed measurement of gene expression with color-coded probe pairs. Nature biotechnology 2008;26:317-325.

Fu T et al., The ICOS/ICOSL pathway is required for optimal antitumour responses mediated by anti-CTLA-4 therapy. Cancer Res. 2011, 71(16); 5445-54.

Geiss G.K. et al., Direct multiplexed measurement of gene expression with color-coded probe pairs. Nat Biotechnol. 2008 Mar;26(3):317-25.

Giacomo A M D et al., "Long-term survival and immunological parameters in metastatic melanoma patients who respond to ipilimumab 10 mg/kg within an expanded access program", Cancer Immunol Immunother. 2013, 62(6); 1021-8.

Gu-Trantien C., Migliori E., et al., CXCL13-producing TFH cells link immune suppression and adaptive memory in human breast cancer. JCI Insight. 2017 Jun 2;2(11).

Hutloff A., Dittrich A. M., Beier K. C., Eljaschewitsch B., Kraft R., Anagnostopoulos I., Kroczek R. A. ICOS is an inducible T-cell co-stimulator structurally and functionally related to CD28. Nature. 1999 Jan 21;397(6716):263-6.

Im S.J., Hashimoto M., et al.. Defining CD8+ T cells that provide the proliferative burst after PD-1 therapy. Nature. 2016 Sep 15; 537(7620): 417-421.

Liakou C I et al., CTLA-4 blockade increases IFN-gamma producing CD4+ICOShi cells to shift the ratio of effector to regulatory T cells in cancer patients. Proc Natl Acad Sci USA 2008,105(39); 14987-92.

Manzoor A. M., Developing Costimulatory Molecules for Immunotherapy of Diseases, Academic Press, 2015; eBook ISBN 9780128026755

Paulos C. M., Carpenito C., Plesa G., Suhoski M. M., Varela-Rohena A., Golovina T. N., Carroll R. G., Riley J. L., June C. H. The inducible costimulator (ICOS) is critical for the development of human T(H)17 cells. Sci Transl Med. 2010 Oct 27;2(55):55ra78.

Sharma P, Allison J 2015. The future of immune checkpoint therapy. Science 2015; 348: 56-61

Simpson T. R., Quezada S. A., Allison J. P. Regulation of CD4 T cell activation and effector function by inducible costimulator (ICOS). Current Opinion in Immunology 2010, 22.

Vonderheide R H et al., Tremelimumab in combination with exemestane in patients with advanced breast cancer and treatment-associated modulation of inducible costimulator expression on patient T cells, Clin Cancer Res. 2010, 16(13); 3485-94.

Wakamatsu E., Mathis D., Benoist C. Convergent and divergent effects of costimulatory molecules in conventional and regulatory CD4+ T cells. Proc Natl Acad Sci USA. 2013 Jan 15; 110(3):1023-8.

Warnatz K., et al., Human ICOS deficiency abrogates the germinal center reaction and provides a monogenic model for common variable immunodeficiency. Blood 2006 107:3045-3052

Yao S., Zhu Y., Zhu G., Augustine M., Zheng L., Goode D. J., Broadwater M., Ruff W., Flies S., Xu H., Flies D., Luo L., Wang S., Chen L. B7-h2 is a costimulatory ligand for CD28 in human. Immunity. 2011 May 27;34(5):729-40.

Young, M. R. I., Th17 Cells in Protection from Tumor or Promotion of Tumor Progression. J Clin Cell Immunol. 2016 Jun; 7(3): 431.

Yuraszeck et al., Translation and Clinical Development of Bispecific T-cell Engaging Antibodies for Cancer Treatment. Clinical Pharmacology & Therapeutics 2017, 101

**Claims**

1. An agonistic ICOS-binding molecule comprising at least one antigen binding domain that binds to a tumor-associated antigen for use in a method for treating or delaying progression of cancer, wherein the agonistic ICOS-binding molecule comprising at least one antigen binding domain that binds to a tumor-associated antigen is used in combination with a T-cell activating anti-CD3 bispecific antibody specific for a tumor-associated antigen.

2. The agonistic ICOS-binding molecule comprising at least one antigen binding domain that binds to a tumor-associated antigen for use in a method of claim 1, wherein the agonistic ICOS-binding molecule comprising at least one antigen binding domain that binds to a tumor-associated antigen and the T-cell activating anti-CD3 bispecific antibody are administered together in a single composition or administered separately in two or more different compositions.

3. The agonistic ICOS-binding molecule comprising at least one antigen binding domain that binds to a tumor-associated antigen for use in a method of claims 1 or 2, wherein the agonistic ICOS-binding molecule comprising at least one antigen binding domain that binds to a tumor-associated antigen comprises at least one ICOS-L comprising the amino acid sequence of SEQ ID NO:1 or SEQ ID NO:2.

4. The agonistic ICOS-binding molecule comprising at least one antigen binding domain that binds to a tumor-associated antigen for use in a method of claims 1 or 2, wherein the agonistic ICOS-binding molecule comprising at least one antigen binding domain that binds to a tumor-associated antigen comprises at least one antigen binding domain that is capable of agonistic binding to human ICOS comprising the amino acid sequence of SEQ ID NO:3.

5. The agonistic ICOS-binding molecule comprising at least one antigen binding domain that binds to a tumor-associated antigen for use in a method of any one of claims 1 to 4, wherein the agonistic ICOS-binding molecule comprising at least one antigen binding domain that binds to a tumor-associated antigen is an agonistic ICOS-binding molecule comprising at least one antigen binding domain that binds to anti-Fibroblast activation protein (FAP).

6. The agonistic ICOS-binding molecule comprising at least one antigen binding domain that binds to a tumor-associated antigen for use in a method of any one of claims 1 to 5, wherein the agonistic ICOS-binding molecule comprises at least one antigen binding domain capable of specific binding to FAP comprising

(a) a heavy chain variable region ($V_H$FAP) comprising (i) CDR-H1 comprising the amino acid sequence of SEQ ID NO:4, (ii) CDR-H2 comprising the amino acid sequence of SEQ ID NO:5, and (iii) CDR-H3 comprising the amino acid sequence of SEQ ID NO:6, and a light chain variable region ($V_L$FAP) comprising (iv) CDR-L1 comprising the amino acid sequence of SEQ ID NO:7, (v) CDR-L2 comprising the amino acid sequence of SEQ ID NO:8, and (vi) CDR-L3 comprising the amino acid sequence of SEQ ID NO:9, or
(b) a heavy chain variable region ($V_H$FAP) comprising (i) CDR-H1 comprising the amino acid sequence of SEQ ID NO:12, (ii) CDR-H2 comprising the amino acid sequence of SEQ ID NO:13, and (iii) CDR-H3 comprising the amino acid sequence of SEQ ID NO:14, and a light chain variable region ($V_L$FAP) comprising (iv) CDR-L1 comprising the amino acid sequence of SEQ ID NO:15, (v) CDR-L2 comprising the amino acid sequence of SEQ ID NO:16, and (vi) CDR-L3 comprising the amino acid sequence of SEQ ID NO:17.

7. The agonistic ICOS-binding molecule comprising at least one antigen binding domain that binds to a tumor-associated antigen for use in a method of any one of claims 1 to 6, wherein the agonistic ICOS-binding molecule comprises at least one antigen binding domain capable of specific binding to FAP comprising a heavy chain variable region ($V_H$FAP) comprising an amino acid sequence of SEQ ID NO:10 and a light chain variable region ($V_L$FAP) comprising an amino acid sequence of SEQ ID NO:11 or wherein the antigen binding domain capable of specific binding to FAP comprises a heavy chain variable region ($V_H$FAP) comprising an amino acid sequence of SEQ ID NO:18 and a light chain variable region ($V_L$FAP) comprising an amino acid sequence of SEQ ID NO:19.

8. The agonistic ICOS-binding molecule comprising at least one antigen binding domain that binds to a tumor-associated antigen for use in a method of any one of claims 1 to 2 or 4 to 7, wherein the agonistic ICOS-binding molecule comprising at least one antigen binding domain that binds to a tumor-associated antigen comprises at least one antigen binding domain capable of specific binding to ICOS comprising a heavy chain variable region ($V_H$ICOS) comprising (i) CDR-H1 comprising the amino acid sequence of SEQ ID NO:20, (ii) CDR-H2 comprising the amino acid sequence of SEQ ID NO:21, and (iii) CDR-H3 comprising the amino acid sequence of SEQ ID NO:22, and a light chain variable region ($V_L$ICOS) comprising (iv) CDR-L1 comprising the amino acid sequence of SEQ ID NO:23, (v) CDR-L2 comprising the amino acid sequence of SEQ ID NO:24, and (vi) CDR-L3 comprising the amino acid sequence of SEQ ID NO:25.

9. The agonistic ICOS-binding molecule comprising at least one antigen binding domain that binds to a tumor-associated antigen for use in a method of any one of claims 1 to 2 or 4 to 8, wherein the agonistic ICOS-binding molecule comprising at least one antigen binding domain that binds to a tumor-associated antigen comprises at least one antigen binding domain comprising a heavy chain variable region ($V_H$ICOS) comprising an amino acid sequence of SEQ ID NO:26 and a light chain variable region ($V_L$ICOS) comprising an amino acid sequence of SEQ ID NO:27.

10. The agonistic ICOS-binding molecule comprising at least one antigen binding domain that binds to a tumor-associated antigen for use in a method of any one of claims 1 to 9, wherein the agonistic ICOS-binding molecule comprising at least one antigen binding domain that binds to a tumor-associated antigen comprises a Fc domain that comprises one or more amino acid substitution that reduces binding to an Fc receptor and/or effector function.

11. The agonistic ICOS-binding molecule comprising at least one antigen binding domain that binds to a tumor-associated antigen for use in a method of any one of claims 1 to 2 or 4 to 10, wherein the agonistic ICOS-binding molecule comprises a first heavy chain comprising an amino acid sequence of SEQ ID NO:28, a first light chain comprising an amino acid sequence of SEQ ID NO:29, a second heavy chain comprising an amino acid sequence of SEQ ID NO:30, and a second light chain comprising an amino acid sequence of SEQ ID NO:31 or wherein the agonistic ICOS-binding molecule comprises a first heavy chain comprising an amino acid sequence of SEQ ID NO:32, a second heavy chain comprising an amino acid sequence of SEQ ID NO:66, and one light chain comprising the amino acid sequence of SEQ ID NO:29.

12. The agonistic ICOS-binding molecule comprising at least one antigen binding domain that binds to a tumor-associated antigen for use in a method of any one of claims 1 to 2 or 4 to 11, wherein the agonistic ICOS-binding molecule comprises monovalent binding to a tumor associated target and bivalent binding to ICOS.

13. The agonistic ICOS-binding molecule comprising at least one antigen binding domain that binds to a tumor-associated antigen for use in a method of any one of claims 1 to 12, wherein the T-cell activating anti-CD3 bispecific antibody is an anti-CEA/anti-CD3 bispecific antibody.

14. The agonistic ICOS-binding molecule comprising at least one antigen binding domain that binds to a tumor-associated antigen for use in a method of any one of claims 1 to 13, wherein the T-cell activating anti-CD3 bispecific antibody comprises a first antigen binding domain comprising a heavy chain variable region ($V_H$CD3) and a light chain variable region ($V_L$CD3), and a second antigen binding domain comprising a heavy chain variable region ($V_H$CEA) and a light chain variable region ($V_L$CEA).

15. The agonistic ICOS-binding molecule comprising at least one antigen binding domain that binds to a tumor-associated antigen for use in a method of any one of claims 1 to 14, wherein the T-cell activating anti-CD3 bispecific antibody comprises a first antigen binding domain comprising a heavy chain variable region ($V_H$CD3) comprising the amino acid sequence of SEQ ID NO:40 and/or a light chain variable region ($V_L$CD3) comprising the amino acid sequence of SEQ ID NO:41.

16. A pharmaceutical product comprising (A) a first composition comprising as active ingredient an agonistic ICOS-binding molecule comprising at least one antigen binding domain that binds to a tumor-associated antigen and a pharmaceutically acceptable excipient; and (B) a second composition comprising a T-cell activating anti-CD3 bispecific antibody specific for a tumor-associated antigen and a pharmaceutically acceptable excipient, for use in the combined, sequential or simultaneous, treatment of a disease, in particular cancer.

17. An agonistic ICOS-binding molecule comprising at least one antigen binding domain that binds to a tumor-associated antigen, wherein the tumor-associated antigen is selected from the group consisting of Fibroblast Activation Protein (FAP), Carcinoembryonic Antigen (CEA), Folate receptor alpha (FolR1), Melanoma-associated Chondroitin Sulfate Proteoglycan (MCSP), Epidermal Growth Factor Receptor (EGFR), human epidermal growth factor receptor 2 (HER2) and p95HER2.

18. The agonistic ICOS-binding molecule comprising at least one antigen binding domain that binds to a tumor-associated antigen of claim 17, wherein the agonistic ICOS-binding molecule comprises at least one antigen binding domain capable of specific binding to FAP comprising

(a) a heavy chain variable region ($V_H$FAP) comprising (i) CDR-H1 comprising the amino acid sequence of SEQ ID NO:4, (ii) CDR-H2 comprising the amino acid sequence of SEQ ID NO:5, and (iii) CDR-H3 comprising the amino acid sequence of SEQ ID NO:6, and a light chain variable region ($V_L$FAP) comprising (iv) CDR-L1 comprising the amino acid sequence of SEQ ID NO:7, (v) CDR-L2 comprising the amino acid sequence of SEQ ID NO:8, and (vi) CDR-L3 comprising the amino acid sequence of SEQ ID NO:9, or
(b) a heavy chain variable region ($V_H$FAP) comprising (i) CDR-H1 comprising the amino acid sequence of SEQ ID NO:12, (ii) CDR-H2 comprising the amino acid sequence of SEQ ID NO:13, and (iii) CDR-H3 comprising the amino acid sequence of SEQ ID NO:14, and a light chain variable region ($V_L$FAP) comprising (iv) CDR-L1 comprising the amino acid sequence of SEQ ID NO:15, (v) CDR-L2 comprising the amino acid sequence of SEQ ID NO:16, and (vi) CDR-L3 comprising the amino acid sequence of SEQ ID NO:17.

19. The agonistic ICOS-binding molecule comprising at least one antigen binding domain that binds to a tumor-associated antigen of claims 17 or 18, wherein the agonistic ICOS-binding molecule comprises at least one antigen binding domain capable of specific binding to ICOS comprising a heavy chain variable region ($V_H$ICOS) comprising (i) CDR-H1 comprising the amino acid sequence of SEQ ID NO:20, (ii) CDR-H2 comprising the amino acid sequence of SEQ ID NO:21, and (iii) CDR-H3 comprising the amino acid sequence of SEQ ID NO:22, and a light chain variable region (V$_L$ICOS) comprising (iv) CDR-L1 comprising the amino acid sequence of SEQ ID NO:23, (v) CDR-L2 comprising the amino acid sequence of SEQ ID NO:24, and (vi) CDR-L3 comprising the amino acid sequence of SEQ ID NO:25.

20. The agonistic ICOS-binding molecule comprising at least one antigen binding domain that binds to a tumor-associated antigen of any one of claims 17 to 19, wherein the agonistic ICOS-binding molecule comprises monovalent binding to a tumor associated target and bivalent binding to ICOS.

Fig. 1

# Fig. 1

**(1E)**

**(1F)**

EP 3 502 140 A1

# Fig. 2

**(2A)**

**(2B)**

murine ICOS Ligand
(Gly47-Lys279)

muFc(DD)
DAPG

muFc(KK)
DAPG

anti FAP

VL          VH

murine ICOS Ligand
(Gly47-Lys279)

muFc(DD)
DAPG

muFc(KK)
DAPG

anti DP47

VL          VH

**(2C)**

VH

Cκ

CH1

VL

Cκ

VH          VH

CH1    CH1

VL

Cκ

CH1

VL

Fc(KK)
DAPG

Fc(DD)
DAPG

Fig. 3

(3A)

(3B)

(3C)

(3D)

Fig. 3

(3E) % ICOS+ CD8+   (3F) % ICOS+ CD4+

(3G) ICOS+ CD8+ MFI   (3H) ICOS+ CD4+ MFI

Fig. 4

(4A)

(4B)

(4C)

(4D)

Fig. 5

(5A) % ICOS+ CD4+ T cells vs concentration [pM]
(5B) % ICOS+ Treg cells vs concentration [pM]
(5C) % ICOS+ CD8+ T cells vs concentration [pM]

# Fig. 5

**(5D)**

**(5E)**

**(5F)**

EP 3 502 140 A1

# Fig. 6

**(6A)**

**(6B)**

Legend: ICOS-FAP 1+1 · ICOS-DP47 1+1 · ICOS-FAP 2+1 · ICOS-DP47 2+1 · ICOS-FAP 1+1 HT

**(6C)**

Legend:
ICOS-FAP 1+1
ICOS-DP47 1+1
ICOS-FAP 2+1
ICOS-DP47 2+1
ICOS-FAP 1+1 HT

EP 3 502 140 A1

# Fig. 7

(7A)

(7B)

FAP-mICOS-L ▪ DP47-mICOSL ♦ 2nd only ✳ cells only

Fig. 8

(8A)

# Fig. 8

**(8B)**

**(8D)**

**(8C)**

**(8E)**

Fig. 8

# Fig. 9

**(9A)**

**(9C)**

**(9B)**

**(9D)**

TCB only    TCB + FAP-ICOS_2+1

EP 3 502 140 A1

Fig. 9

# Fig. 10

(10A)

(10B)

Fig. 10

# Fig. 10

**CD8**

(10G)

(10I)

(10H)

(10J)

Fig. 10

(10K)

(10L)

EP 3 502 140 A1

# Fig. 11

**(11A)**

**(11B)**

☐ mTCB only    ■ mTCB + FAP-mICOS-L    ▦ mTCB + untargeted mICOS-L

# Fig. 12

# Fig. 13

**Humanized mice**

| d0 | d23 | d30 | d37 | Day 44 |
|----|-----|-----|-----|--------|
| Injection of cancer cells | Therapy injection | Therapy injection | Therapy injection | Termination |

*(MKN45: 1x106; sc. +
3T3-huFAP: 1x106; sc. )*

*(ex vivo analysis)*

| Group | No. of animals | Compound | Dose (mg/kg) | Therapy | No. of treatments |
|-------|----------------|----------|--------------|---------|-------------------|
| A | 12 | Vehicle | -- | i.v. | 3 (once weekly) |
| B | 12 | CEACAM5 TCB | 0.5 | i.v. | 3 (once weekly) |
| C | 12 | CEACAM5 TCB + FAP-ICOS_1+1 | 5 | i.v. | 3 (once weekly) |

Fig. 14

**(14A)**

EP 3 502 140 A1

Fig. 14

(14B) Vehicle

(14C) TCB only

(14D) TCB + FAP-ICOS 1+1

(14E)

Fig. 15

Fig. 16

(16A)  CCL3

(16B)  TNF-α

(16C)  CXCL13

# Fig. 17

**(17A)**

TNFAIP6

p=0.1, q=0.99. 2 genes left: TNFAIP6 & CXCL13

**(17B)**

CXCL13

p=0.05, q=0.99. 1 gene left

| Gene Name | Fold Change |
|-----------|-------------|
| CXCL13 | 3.1 +/- 1.28 |
| TNFAIP6 | 2.94 +/- 1.86 |

Europäisches
Patentamt

European
Patent Office

Office européen
des brevets

**EUROPEAN SEARCH REPORT**

Application Number

EP 17 20 9444

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2016/020444 A1 (AFFIMED GMBH [DE]) 11 February 2016 (2016-02-11) * claims 1-4 * | 1-20 | INV.<br>C07K16/30<br>C07K16/28<br>C07K16/40<br><br>ADD.<br>A61K39/00 |
| A | WO 2017/205738 A1 (ABBVIE BIOTHERAPEUTICS INC [US]) 30 November 2017 (2017-11-30) * claims 64-69 * | 1-20 | |
| A | WO 2016/115274 A1 (COMPASS THERAPEUTICS LLC [US]; SABZEVARI HELEN [US]; SCHMIDT MICHAEL M) 21 July 2016 (2016-07-21) * claims 1, 12-15; examples 15-22 * | 1-20 | |
| A | WO 2014/131694 A1 (ROCHE GLYCART AG [CH]) 4 September 2014 (2014-09-04) * the whole document * | 1-20 | |
| A | WO 2015/112534 A2 (MEDIMMUNE LLC [US]) 30 July 2015 (2015-07-30) * 163-164; paragraphs [0163], [0164], [0229] * | 1-20 | |
| A | Anonymous: "Roche - Roche presents the first Phase I efficacy and safety data on CEA-TCB (CEA CD3 TCB), a novel T-cell bispecific antibody targeting solid tumours", Roche press release, 18 May 2017 (2017-05-18), XP055461395, Retrieved from the Internet: URL:https://www.roche.com/investors/updates/inv-update-2017-05-18.htm [retrieved on 2018-03-21] * the whole document * | 1-20 | TECHNICAL FIELDS SEARCHED (IPC)<br><br>C07K<br>A61K |
| A | WO 2014/131712 A1 (ROCHE GLYCART AG [CH]) 4 September 2014 (2014-09-04) * claims 1-4 * | 1-20 | |

-/--

The present search report has been drawn up for all claims

| Place of search | Date of completion of the search | Examiner |
|---|---|---|
| Munich | 22 March 2018 | Lechner, Oskar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

page 1 of 2

Europäisches
Patentamt

European
Patent Office

Office européen
des brevets

## EUROPEAN SEARCH REPORT

Application Number

EP 17 20 9444

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2013/026837 A1 (ROCHE GLYCART AG [CH]; AST OLIVER [CH]; FAUTI TANJA [CH]; JAEGER CHRIS) 28 February 2013 (2013-02-28) * claims 1, 15, 16 * | 1-20 | |
| A | WO 2015/063187 A1 (KIPRIJANOV SERGEJ MICHAILOVIC [NO]) 7 May 2015 (2015-05-07) * paragraphs [0071] - [0072] * | 1-20 | |

TECHNICAL FIELDS
SEARCHED      (IPC)

The present search report has been drawn up for all claims

| Place of search | Date of completion of the search | Examiner |
|---|---|---|
| Munich | 22 March 2018 | Lechner, Oskar |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 2

## ANNEX TO THE EUROPEAN SEARCH REPORT
## ON EUROPEAN PATENT APPLICATION NO.

EP 17 20 9444

This annex lists the patent family members relating to the patent documents cited in the above-mentioned European search report.
The members are as contained in the European Patent Office EDP file on
The European Patent Office is in no way liable for these particulars which are merely given for the purpose of information.

22-03-2018

| Patent document cited in search report | | Publication date | Patent family member(s) | | Publication date |
|---|---|---|---|---|---|
| WO 2016020444 | A1 | 11-02-2016 | AU | 2015299039 A1 | 02-03-2017 |
| | | | CA | 2957462 A1 | 11-02-2016 |
| | | | CN | 107001468 A | 01-08-2017 |
| | | | EP | 2982693 A1 | 10-02-2016 |
| | | | EP | 3177646 A1 | 14-06-2017 |
| | | | JP | 2017529067 A | 05-10-2017 |
| | | | US | 2016039934 A1 | 11-02-2016 |
| | | | WO | 2016020444 A1 | 11-02-2016 |
| WO 2017205738 | A1 | 30-11-2017 | US | 2017342169 A1 | 30-11-2017 |
| | | | WO | 2017205738 A1 | 30-11-2017 |
| WO 2016115274 | A1 | 21-07-2016 | AU | 2016206707 A1 | 10-08-2017 |
| | | | CA | 2973720 A1 | 21-07-2016 |
| | | | CN | 107614522 A | 19-01-2018 |
| | | | EP | 3245227 A1 | 22-11-2017 |
| | | | JP | 2018503399 A | 08-02-2018 |
| | | | WO | 2016115274 A1 | 21-07-2016 |
| WO 2014131694 | A1 | 04-09-2014 | CA | 2896370 A1 | 04-09-2014 |
| | | | CN | 105143270 A | 09-12-2015 |
| | | | EP | 2961773 A1 | 06-01-2016 |
| | | | HK | 1218425 A1 | 17-02-2017 |
| | | | JP | 2016514098 A | 19-05-2016 |
| | | | KR | 20150122761 A | 02-11-2015 |
| | | | RU | 2015140915 A | 03-04-2017 |
| | | | US | 2016145354 A1 | 26-05-2016 |
| | | | WO | 2014131694 A1 | 04-09-2014 |
| WO 2015112534 | A2 | 30-07-2015 | CA | 2936244 A1 | 30-07-2015 |
| | | | EP | 3096782 A2 | 30-11-2016 |
| | | | US | 2017015758 A1 | 19-01-2017 |
| | | | WO | 2015112534 A2 | 30-07-2015 |
| WO 2014131712 | A1 | 04-09-2014 | AR | 094896 A1 | 09-09-2015 |
| | | | AU | 2014222779 A1 | 04-06-2015 |
| | | | CA | 2891493 A1 | 04-09-2014 |
| | | | CL | 2015001988 A1 | 20-11-2015 |
| | | | CN | 104936986 A | 23-09-2015 |
| | | | CR | 20150393 A | 21-08-2015 |
| | | | EA | 201500876 A1 | 29-02-2016 |
| | | | EP | 2961771 A1 | 06-01-2016 |
| | | | HK | 1211300 A1 | 20-05-2016 |
| | | | JP | 6133444 B2 | 24-05-2017 |
| | | | JP | 2016508723 A | 24-03-2016 |
| | | | JP | 2017158565 A | 14-09-2017 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**

EP 17 20 9444

22-03-2018

| Patent document cited in search report | Publication date | Patent family member(s) | Publication date |
|---|---|---|---|
| | | KR 20150109475 A | 01-10-2015 |
| | | PE 14102015 A1 | 18-09-2015 |
| | | PH 12015501340 A1 | 02-09-2015 |
| | | SG 11201504497T A | 29-09-2015 |
| | | TW 201437227 A | 01-10-2014 |
| | | TW 201641513 A | 01-12-2016 |
| | | US 2014242079 A1 | 28-08-2014 |
| | | WO 2014131712 A1 | 04-09-2014 |
| WO 2013026837 A1 | 28-02-2013 | US 2013078250 A1 | 28-03-2013 |
| | | WO 2013026837 A1 | 28-02-2013 |
| WO 2015063187 A1 | 07-05-2015 | GB 2519786 A | 06-05-2015 |
| | | WO 2015063187 A1 | 07-05-2015 |

page 2 of 2

**EP 3 502 140 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011041613 A2, Allison
- WO 2002020565 A
- WO 9316185 A
- US 5571894 A
- US 5587458 A
- US 5869046 A
- EP 404097 A
- WO 199301161 A
- US 6248516 B1
- US 7166697 B1
- US 7250297 B1
- US 20070224633 A
- EP 1641818 A1
- US 20040132028 A1
- US 20080139791 A
- WO 2005056764 A
- US 6818418 B1
- WO 2008098796 A
- US 5821333 A
- US 5731168 A
- US 7695936 B
- US 20030157108 A, Presta, L.
- US 20040093621 A
- WO 2003011878 A, Jean-Mairet
- US 6602684 B, Umana
- US 20050123546 A, Umana
- WO 199730087 A, Patel
- WO 199858964 A, Raju, S.
- WO 199922764 A, Raju, S.
- US 7521541 B
- WO 2012130831 A1
- US 6737056 B
- US 7332581 B
- WO 2012130831 A

- US 20050014934 A
- US 7371826 B
- US 5648260 A
- US 5624821 A
- WO 9429351 A
- US 5500362 A
- US 5821337 A
- WO 2016030350 A1
- WO 2014131694 A1
- WO 2009089004 PCT
- WO 2014131712 A1
- WO 2007071426 A
- WO 2014131712 A
- US 5959177 A
- US 6040498 A
- US 6420548 B
- US 7125978 B
- US 6417429 B
- US 4186567 A
- US 5969108 A, McCafferty
- US 5565332 A, Winter
- US 7527791 B
- US 6982321 B
- US 7087409 B
- WO 2012020006 PCT
- US 20040132066 A
- US 20050260186 A
- US 20060104968 A
- US 6267958 B
- US 6171586 B
- WO 2006044908 A
- US 20080199466 A1
- WO 2016079076 A1

### Non-patent literature cited in the description

- **HUTLOFF et al.** *Nature,* 1999, 397
- **OGASAWARA et al.** *J Immunol.,* 2002, vol. 169
- **PAULOS CM et al.** *Sci Transl Med,* 2010, vol. 2
- **WAKAMATSU et al.** *Proc Natal Acad Sci USA,* 2013, vol. 110
- **SIMPSON et al.** *Current Opinion in Immunology,* 2010, vol. 22
- **YAO et al.** *Immunity,* 2011, vol. 34

- **SIMPSON et al.** *Current Opinion in Immunology,* 2010
- **SHARPE.** *Immunol Rev.,* 2009, vol. 229
- **RITA YOUNG.** *J Clin Cell Immunol.,* 2016, vol. 7
- **WARNATZ et al.** *Blood,* 2006
- **YURASZECK et al.** *Clinical Pharmacology & Therapeutics,* 2017, vol. 101
- **FU T et al.** *Cancer Res,* 2011, vol. 71

239

- **GIACOMO et al.** *Cancer Immunol Immunother.,* 2013, vol. 62
- **CARTHON et al.** *Clin Cancer Res.,* 2010, vol. 16
- **VONDERHEIDE et al.** *Clin Cancer Res.,* 2010, vol. 16
- **LIAKOU et al.** *Proc Natl Acad Sci USA,* 2008, vol. 105
- **HUDSON et al.** *Nat Med,* 2003, vol. 9, 129-134
- **PLÜCKTHUN.** The Pharmacology of Monoclonal Antibodies. Springer-Verlag, 1994, vol. 113, 269-315
- **HOLLINGER et al.** *Proc Natl Acad Sci USA,* 1993, vol. 90, 6444-6448
- **HOUSTON, J.S.** *Methods in Enzymol.,* 1991, vol. 203, 46-96
- *Curr Opin Chem Biol,* 2009, vol. 13, 245-255
- **STUMPP et al.** Darpins: A new generation of protein therapeutics. *Drug Discovery Today,* 2008, vol. 13, 695-701
- *Journal of Immunological Methods,* 2001, vol. 248 (1-2), 31-45
- *Biochim Biophys Acta,* 2000, vol. 1482, 337-350
- *Protein Eng. Des. Sel.,* 2004, vol. 17, 455-462
- *Nature Biotechnology,* 2005, vol. 23 (12), 1556-1561
- *Expert Opinion on Investigational Drugs,* June 2007, vol. 16 (6), 909-917
- *J. Biol. Chem,* 1999, vol. 274, 24066-24073
- *J. Mol. Biol.,* 2003, vol. 332, 489-503
- *PNAS,* 2003, vol. 100 (4), 1700-1705
- *J. Mol. Biol.,* 2007, vol. 369, 1015-1028
- *Protein Eng. Des. Sel.,* 2005, vol. 18, 435-444
- *Expert Opin. Biol. Ther.,* 2005, vol. 5, 783-797
- **LILJEBLAD et al.** *Glyco J,* 2000, vol. 17, 323-329
- **HEELEY.** *Endocr Res,* 2002, vol. 28, 217-229
- **GOLD ; FREEDMAN.** *J Exp Med.,* 1965, vol. 121, 439-462
- **BERINSTEIN N. L.** *J Clin Oncol.,* 2002, vol. 20, 2197-2207
- **NAP et al.** *Tumour Biol.,* 1988, vol. 9 (2-3), 145-53
- **NAP et al.** *Cancer Res.,* 1992, vol. 52 (8), 2329-23339
- **HAMMARSTRÖM S.** *Semin Cancer Biol.,* 1999, vol. 9 (2), 67-81
- **MARSHALL J.** *Semin Oncol.,* 2003, vol. 30 (8), 30-6
- **GOLDENBERG D M.** *The International Journal of Biological Markers,* 1992, vol. 7, 183-188
- **CHAU I. et al.** *J Clin Oncol.,* 2004, vol. 22, 1420-1429
- **FLAMINI et al.** *Clin Cancer Res,* 2006, vol. 12 (23), 6985-6988
- **NECELA et al.** *PloS One,* 2015, vol. 10 (3), e0127133
- **ROSS et al.** *Arch. Biochem. Biophys.,* 1983, vol. 225, 370-38
- **SLAMON, D. J. et al.** *Science,* 1989, vol. 244, 707-712
- **ANIDO et al.** *EMBO J,* 2006, vol. 25, 3234-44
- **PEDERSEN et al.** *Mol Cell Biol,* 2009, vol. 29, 3319-31
- **YAO et al.** *Immunity,* 2011, vol. 34, 729-740
- **KINDT et al.** Kuby Immunology. W.H. Freeman and Co, 2007, 91
- **CHOTHIA ; LESK.** *J. Mol. Biol.,* 1987, vol. 196, 901-917
- **KABAT et al.** Sequences of Proteins of Immunological Interest. Public Health Service, National Institutes of Health, 1991
- **KABAT et al.** Sequences of Proteins of Immunological Interest. U.S. Dept. of Health and Human Services, 1983
- **CHOTHIA et al.** *J. Mol. Biol.,* 1987, vol. 196, 901-917
- **KABAT et al.** Sequence of Proteins of Immunological Interest. U.S. Dept. of Health and Human Services, 1983
- **ALMAGRO ; FRANSSON.** *Front. Biosci.,* 2008, vol. 13, 1619-1633
- **RIDGWAY et al.** *Prot Eng,* 1996, vol. 9, 617-621
- **CARTER.** *J Immunol Meth,* 2001, vol. 248, 7-15
- **CARTER.** *J Immunol Methods,* 2001, vol. 248, 7-15
- **BOWIE, J. U. et al.** *Science,* 1990, vol. 247, 1306-10
- **VAN DE WINKEL, J.G. ; ANDERSON, C.L.** *J. Leukoc. Biol.,* 1991, vol. 49, 511-524
- **RAVETCH, J.V. ; KINET, J.P.** *Annu. Rev. Immunol.,* 1991, vol. 9, 457-492
- **CAPEL, P.J. et al.** *Immunomethods,* 1994, vol. 4, 25-34
- **DE HAAS, M. et al.** *J. Lab. Clin. Med.,* 1995, vol. 126, 330-341
- **GESSNER, J.E. et al.** *Ann. Hematol.,* 1998, vol. 76, 231-248
- **ARMOUR, K.L. et al.** *Eur. J. Immunol.,* 1999, vol. 29, 2613-2624
- **SHIELDS, R.L. et al.** *J. Biol. Chem.,* 2001, vol. 276, 6591-6604
- **CUNNINGHAM ; WELLS.** *Science,* 1989, vol. 244, 1081-1085
- **WRIGHT et al.** *TIBTECH,* 1997, vol. 15, 26-32
- **KAM, N.W. et al.** *Proc. Natl. Acad. Sci. USA,* 2005, vol. 102, 11600-11605
- **GUYER, R.L. et al.** *J. Immunol.,* 1976, vol. 117, 587-593
- **KIM, J.K. et al.** *J. Immunol.,* 1994, vol. 24, 2429-2434

- **DUNCAN, A.R. ; WINTER, G.** *Nature,* 1988, vol. 322, 738-740
- **HELLSTROM et al.** *Proc Natl Acad Sci USA,* 1986, vol. 83, 7059-7063
- **HELLSTROM et al.** *Proc Natl Acad Sci USA,* 1985, vol. 82, 1499-1502
- **BRUGGEMANN et al.** *J Exp Med,* 1987, vol. 166, 1351-1361
- **CLYNES et al.** *Proc Natl Acad Sci USA,* 1998, vol. 95, 652-656
- **MANIATIS et al.** MOLECULAR CLONING: A LABORATORY MANUAL. Cold Spring Harbor Laboratory, 1989
- **AUSUBEL et al.** CURRENT PROTOCOLS IN MOLECULAR BIOLOGY. Greene Publishing Associates and Wiley Interscience, 1989
- **GERNGROSS.** *Nat Biotech,* 2004, vol. 22, 1409-1414
- **LI et al.** *Nat Biotech,* 2006, vol. 24, 210-215
- **GRAHAM et al.** *J Gen Virol,* 1977, vol. 36, 59
- **MATHER.** *Biol Reprod,* 1980, vol. 23, 243-251
- **MATHER et al.** *Annals N.Y. Acad Sci,* 1982, vol. 383, 44-68
- **URLAUB et al.** *Proc Natl Acad Sci USA,* 1980, vol. 77, 4216
- **YAZAKI ; WU.** Methods in Molecular Biology. Humana Press, 2003, vol. 248, 255-268
- **HARLOW ; LANE.** Antibodies, a laboratory manual. Cold Spring Harbor Laboratory, 1988
- **ALMAGRO ; FRANSSON.** *Front Biosci,* 2008, vol. 13, 1619-1633
- **RIECHMANN et al.** *Nature,* 1988, vol. 332, 323-329
- **QUEEN et al.** *Proc Natl Acad Sci USA,* 1989, vol. 86, 10029-10033
- **JONES et al.** *Nature,* 1986, vol. 321, 522-525
- **MORRISON et al.** *Proc Natl Acad Sci,* 1984, vol. 81, 6851-6855
- **MORRISON ; OI.** *Adv Immunol,* 1988, vol. 44, 65-92
- **VERHOEYEN et al.** *Science,* 1988, vol. 239, 1534-1536
- **PADLAN.** *Molec Immun,* 1994, vol. 31 (3), 169-217
- **KASHMIRI et al.** *Methods,* 2005, vol. 36, 25-34
- **PADLAN.** *Mol Immunol,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br J Cancer,* 2000, vol. 83, 252-260
- **VAN DIJK ; VAN DE WINKEL.** *Curr Opin Pharmacol,* 2001, vol. 5, 368-74
- **LONBERG.** *Curr Opin Immunol,* 2008, vol. 20, 450-459
- Monoclonal Antibody Production Techniques and Applications. Marcel Dekker, Inc, 1987, 51-63
- **LONBERG.** *Nat Biotech,* 2005, vol. 23, 1117-1125
- **HOOGENBOOM et al.** Methods in Molecular Biology. Human Press, 2001, vol. 178, 1-37
- **MCCAFFERTY et al.** *Nature,* vol. 348, 552-554
- **CLACKSON et al.** *Nature,* 1991, vol. 352, 624-628
- Epitope Mapping Protocols. **MORRIS.** Methods in Molecular Biology. Humana Press, 1996, vol. 66
- **HARLOW ; LANE.** Antibodies: A Laboratory Manual. Cold Spring Harbor Laboratory, 1988
- Remington's Pharmaceutical Sciences. Mack Printing Company, 1990
- **KABAT, E.A. et al.** Sequences of Proteins of Immunological Interest. Public Health Service, National Institutes of Health, 1991
- **SAMBROOK et al.** Molecular cloning: A laboratory manual. Cold Spring Harbor Laboratory Press, 1989
- **KABAT, E.A. et al.** Sequences of Proteins of Immunological Interest. NIH, 1991
- Current Protocols in Cell Biology. John Wiley & Sons, Inc, 2000
- **GUNASEKARAN et al.** *J. Biol. Chem.,* 2010, 19637-19646
- **BAUDINO et al.** *J. Immunol.,* 2008, vol. 181, 6664-6669
- *Cancer Res,* 1975, vol. 35, 2434-2440
- **ALLEN F. ; BOBANGA J. et al.** CCL3 in the tumor microenvironment augments the antitumor immune response. *J Immunol,* 01 May 2016, vol. 196 (75.11
- **ALLISON J ; SHARMA P ; QUEZADA, S.A. ; FU T.** *Combination immunotherapy for the treatment of cancer*
- **BACAC M ; FAUTI T ; SAM J et al.** A Novel Carcinoembryonic Antigen T-Cell Bispecific Antibody (CEA TCB) for the Treatment of Solid Tumours. *Clin Cancer Res.,* 01 July 2016, vol. 22 (13), 3286-97
- **BACAC M ; KLEIN C ; UMANA P.** CEA TCB: A novel head-to-tail 2:1 T cell bispecific antibody for treatment of CEA-positive solid tumours. *Oncoimmunology,* 24 June 2016, vol. 5 (8
- **CARTHON B C et al.** *Preoperative CTLA-4 blockade: Tolerability and immune monitoring in the setting of a presurgical clinical trial Clin Cancer Res,* 2010, vol. 16 (10), 2861-71
- **DAMMEIJER F. ; LAU S.P. ; VAN EIJCK C.H.J. ; VAN DER BURG S.H. ; AERTS J.G.J.V.** Rationally combining immunotherapies to improve efficacy of immune checkpoint blockade in solid tumours. *Cytokine & Growth Factor Reviews,* August 2017, vol. 36, 5-15

- **DAVIDSON E.H. ; HOOD L. ; DIMITROV K.** Direct multiplexed measurement of gene expression with color-coded probe pairs. *Nature biotechnology,* 2008, vol. 26, 317-325
- **FU T et al.** The ICOS/ICOSL pathway is required for optimal antitumour responses mediated by anti-CTLA-4 therapy. *Cancer Res.,* 2011, vol. 71 (16), 5445-54
- **GEISS G.K. et al.** Direct multiplexed measurement of gene expression with color-coded probe pairs. *Nat Biotechnol.,* March 2008, vol. 26 (3), 317-25
- **GIACOMO A M D et al.** Long-term survival and immunological parameters in metastatic melanoma patients who respond to ipilimumab 10 mg/kg within an expanded access program. *Cancer Immunol Immunother.,* 2013, vol. 62 (6), 1021-8
- **GU-TRANTIEN C. ; MIGLIORI E. et al.** CXCL13-producing TFH cells link immune suppression and adaptive memory in human breast cancer. *JCI Insight.,* 02 June 2017, vol. 2 (11
- **HUTLOFF A. ; DITTRICH A. M. ; BEIER K. C. ; EL-JASCHEWITSCH B. ; KRAFT R. ; ANAGNOSTOPOULOS I. ; KROCZEK R. A.** ICOS is an inducible T-cell co-stimulator structurally and functionally related to CD28. *Nature,* 21 January 1999, vol. 397 (6716), 263-6
- **IM S.J. ; HASHIMOTO M. et al.** Defining CD8+ T cells that provide the proliferative burst after PD-1 therapy. *Nature,* 15 September 2016, vol. 537 (7620), 417-421
- **LIAKOU C I et al.** CTLA-4 blockade increases IFN-gamma producing CD4+ICOShi cells to shift the ratio of effector to regulatory T cells in cancer patients. *Proc Natl Acad Sci USA,* 2008, vol. 105 (39), 14987-92
- **MANZOOR A. M.** Developing Costimulatory Molecules for Immunotherapy of Diseases. Academic Press, 2015
- **PAULOS C. M. ; CARPENITO C. ; PLESA G. ; SUHOSKI M. M. ; VARELA-ROHENA A. ; GOLOVINA T. N. ; CARROLL R. G. ; RILEY J. L. ; JUNE C. H.** The inducible costimulator (ICOS) is critical for the development of human T(H)17 cells. *Sci Transl Med.,* 27 October 2010, vol. 2 (55), 55ra78
- **SHARMA P ; ALLISON J.** The future of immune checkpoint therapy. *Science,* 2015, vol. 348, 56-61
- **SIMPSON T. R. ; QUEZADA S. A. ; ALLISON J. P.** Regulation of CD4 T cell activation and effector function by inducible costimulator (ICOS). *Current Opinion in Immunology,* 2010, vol. 22
- **VONDERHEIDE R H et al.** Tremelimumab in combination with exemestane in patients with advanced breast cancer and treatment-associated modulation of inducible costimulator expression on patient T cells. *Clin Cancer Res.,* 2010, vol. 16 (13), 3485-94
- **WAKAMATSU E. ; MATHIS D. ; BENOIST C.** Convergent and divergent effects of costimulatory molecules in conventional and regulatory CD4+ T cells. *Proc Natl Acad Sci USA,* 15 January 2013, vol. 110 (3), 1023-8
- **WARNATZ K. et al.** Human ICOS deficiency abrogates the germinal center reaction and provides a monogenic model for common variable immunodeficiency. *Blood,* 2006, vol. 107, 3045-3052
- **YAO S. ; ZHU Y. ; ZHU G. ; AUGUSTINE M. ; ZHENG L. ; GOODE D. J. ; BROADWATER M. ; RUFF W. ; FLIES S. ; XU H.** B7-h2 is a costimulatory ligand for CD28 in human. *Immunity,* 27 May 2011, vol. 34 (5), 729-40
- **YOUNG, M. R. I.** Th17 Cells in Protection from Tumor or Promotion of Tumor Progression. *J Clin Cell Immunol.,* June 2016, vol. 7 (3), 431
- **YURASZECK et al.** Translation and Clinical Development of Bispecific T-cell Engaging Antibodies for Cancer Treatment. *Clinical Pharmacology & Therapeutics,* 2017, vol. 101

SEQUENCE LISTING

<110>  F. Hoffmann-La Roche AG

<120>  Combination therapy of tumor targeted ICOS agonists with T-cell
       bispecific molecules

<130>  P34548-EP

<160>  116

<170>  PatentIn version 3.5

<210>  1
<211>  238
<212>  PRT
<213>  Homo sapiens

<400>  1

Asp Thr Gln Glu Lys Glu Val Arg Ala Met Val Gly Ser Asp Val Glu
1               5                   10                  15


Leu Ser Cys Ala Cys Pro Glu Gly Ser Arg Phe Asp Leu Asn Asp Val
            20                  25                  30


Tyr Val Tyr Trp Gln Thr Ser Glu Ser Lys Thr Val Val Thr Tyr His
            35                  40                  45


Ile Pro Gln Asn Ser Ser Leu Glu Asn Val Asp Ser Arg Tyr Arg Asn
        50                  55                  60


Arg Ala Leu Met Ser Pro Ala Gly Met Leu Arg Gly Asp Phe Ser Leu
65                  70                  75                  80


Arg Leu Phe Asn Val Thr Pro Gln Asp Glu Gln Lys Phe His Cys Leu
                85                  90                  95


Val Leu Ser Gln Ser Leu Gly Phe Gln Glu Val Leu Ser Val Glu Val
                100                 105                 110


Thr Leu His Val Ala Ala Asn Phe Ser Val Pro Val Val Ser Ala Pro
            115                 120                 125


His Ser Pro Ser Gln Asp Glu Leu Thr Phe Thr Cys Thr Ser Ile Asn
        130                 135                 140


Gly Tyr Pro Arg Pro Asn Val Tyr Trp Ile Asn Lys Thr Asp Asn Ser
145                 150                 155                 160


Leu Leu Asp Gln Ala Leu Gln Asn Asp Thr Val Phe Leu Asn Met Arg
                165                 170                 175

```
Gly Leu Tyr Asp Val Val Ser Val Leu Arg Ile Ala Arg Thr Pro Ser
            180               185               190

Val Asn Ile Gly Cys Cys Ile Glu Asn Val Leu Leu Gln Gln Asn Leu
            195               200               205

Thr Val Gly Ser Gln Thr Gly Asn Asp Ile Gly Glu Arg Asp Lys Ile
    210               215               220

Thr Glu Asn Pro Val Ser Thr Gly Glu Lys Asn Ala Ala Thr
225               230               235
```

```
<210>  2
<211>  233
<212>  PRT
<213>  Mus musculus

<400>  2
```

```
Glu Thr Glu Val Gly Ala Met Val Gly Ser Asn Val Val Leu Ser Cys
1               5               10              15

Ile Asp Pro His Arg Arg His Phe Asn Leu Ser Gly Leu Tyr Val Tyr
            20               25               30

Trp Gln Ile Glu Asn Pro Glu Val Ser Val Thr Tyr Tyr Leu Pro Tyr
            35               40               45

Lys Ser Pro Gly Ile Asn Val Asp Ser Ser Tyr Lys Asn Arg Gly His
    50               55               60

Leu Ser Leu Asp Ser Met Lys Gln Gly Asn Phe Ser Leu Tyr Leu Lys
65               70               75               80

Asn Val Thr Pro Gln Asp Thr Gln Glu Phe Thr Cys Arg Val Phe Met
            85               90               95

Asn Thr Ala Thr Glu Leu Val Lys Ile Leu Glu Glu Val Val Arg Leu
            100               105               110

Arg Val Ala Ala Asn Phe Ser Thr Pro Val Ile Ser Thr Ser Asp Ser
            115               120               125

Ser Asn Pro Gly Gln Glu Arg Thr Tyr Thr Cys Met Ser Lys Asn Gly
    130               135               140

Tyr Pro Glu Pro Asn Leu Tyr Trp Ile Asn Thr Thr Asp Asn Ser Leu
145               150               155               160
```

```
Ile Asp Thr Ala Leu Gln Asn Asn Thr Val Tyr Leu Asn Lys Leu Gly
            165                 170                 175

Leu Tyr Asp Val Ile Ser Thr Leu Arg Leu Pro Trp Thr Ser Arg Gly
            180                 185                 190

Asp Val Leu Cys Cys Val Glu Asn Val Ala Leu His Gln Asn Ile Thr
            195                 200                 205

Ser Ile Ser Gln Ala Glu Ser Phe Thr Gly Asn Asn Thr Lys Asn Pro
    210                 215                 220

Gln Glu Thr His Asn Asn Glu Leu Lys
225                 230
```

```
<210>   3
<211>   199
<212>   PRT
<213>   Homo sapiens

<400>   3
```

```
Met Lys Ser Gly Leu Trp Tyr Phe Phe Leu Phe Cys Leu Arg Ile Lys
1               5                 10                  15

Val Leu Thr Gly Glu Ile Asn Gly Ser Ala Asn Tyr Glu Met Phe Ile
            20                  25                  30

Phe His Asn Gly Gly Val Gln Ile Leu Cys Lys Tyr Pro Asp Ile Val
            35                  40                  45

Gln Gln Phe Lys Met Gln Leu Leu Lys Gly Gly Gln Ile Leu Cys Asp
    50                  55                  60

Leu Thr Lys Thr Lys Gly Ser Gly Asn Thr Val Ser Ile Lys Ser Leu
65                  70                  75                  80

Lys Phe Cys His Ser Gln Leu Ser Asn Asn Ser Val Ser Phe Phe Leu
                85                  90                  95

Tyr Asn Leu Asp His Ser His Ala Asn Tyr Tyr Phe Cys Asn Leu Ser
            100                 105                 110

Ile Phe Asp Pro Pro Pro Phe Lys Val Thr Leu Thr Gly Gly Tyr Leu
            115                 120                 125

His Ile Tyr Glu Ser Gln Leu Cys Cys Gln Leu Lys Phe Trp Leu Pro
            130                 135                 140
```

Ile Gly Cys Ala Ala Phe Val Val Val Cys Ile Leu Gly Cys Ile Leu
145                 150                 155                 160

Ile Cys Trp Leu Thr Lys Lys Lys Tyr Ser Ser Ser Val His Asp Pro
                165                 170                 175

Asn Gly Glu Tyr Met Phe Met Arg Ala Val Asn Thr Ala Lys Lys Ser
                180                 185                 190

Arg Leu Thr Asp Val Thr Leu
            195

<210>   4
<211>   5
<212>   PRT
<213>   Artificial Sequence

<220>
<223>   FAP(4B9)  CDR-H1

<400>   4

Ser Tyr Ala Met Ser
1               5

<210>   5
<211>   17
<212>   PRT
<213>   Artificial Sequence

<220>
<223>   FAP(4B9)  CDR-H2

<400>   5

Ala Ile Ile Gly Ser Gly Ala Ser Thr Tyr Tyr Ala Asp Ser Val Lys
1               5               10                  15

Gly

<210>   6
<211>   8
<212>   PRT
<213>   Artificial Sequence

<220>
<223>   FAP(4B9)  CDR-H3

<400>   6

Gly Trp Phe Gly Gly Phe Asn Tyr
1               5

```
<210>  7
<211>  12
<212>  PRT
<213>  Artificial Sequence

<220>
<223>  FAP(4B9)  CDR-L1

<400>  7

Arg Ala Ser Gln Ser Val Ser Arg Ser Tyr Leu Ala
1               5                   10


<210>  8
<211>  7
<212>  PRT
<213>  Artificial Sequence

<220>
<223>  FAP(4B9)  CDR-L2

<400>  8

Val Gly Ser Arg Arg Ala Thr
1               5


<210>  9
<211>  9
<212>  PRT
<213>  Artificial Sequence

<220>
<223>  FAP(4B9)  CDR-L3

<400>  9

Gln Gln Gly Ile Met Leu Pro Pro Thr
1               5


<210>  10
<211>  117
<212>  PRT
<213>  Artificial Sequence

<220>
<223>  FAP(4B9)  VH

<400>  10

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Ser Tyr
            20                  25                  30


Ala Met Ser Trp Val Arg Gln Ala Pro Gly Lys Gly Leu Glu Trp Val
            35                  40                  45
```

```
Ser Ala Ile Ile Gly Ser Gly Ala Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Trp Phe Gly Gly Phe Asn Tyr Trp Gly Gln Gly Thr Leu
            100                 105                 110

Val Thr Val Ser Ser
            115


<210>   11
<211>   108
<212>   PRT
<213>   Artificial Sequence

<220>
<223>   FAP(4B9)   VL

<400>   11

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Thr Ser Ser
            20                  25                  30

Tyr Leu Ala Trp Tyr Gln Gln Lys Pro Gly Gln Ala Pro Arg Leu Leu
        35                  40                  45

Ile Asn Val Gly Ser Arg Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Ile Met Leu Pro
                85                  90                  95

Pro Thr Phe Gly Gln Gly Thr Lys Val Glu Ile Lys
            100                 105


<210>   12
<211>   5
<212>   PRT
<213>   Artificial Sequence
```

83

<220>
<223> FAP(28H1) CDR-H1

<400> 12

Ser His Ala Met Ser
1               5


<210> 13
<211> 16
<212> PRT
<213> Artificial Sequence

<220>
<223> FAP(28H1) CDR-H2

<400> 13

Ala Ile Trp Ala Ser Gly Glu Gln Tyr Tyr Ala Asp Ser Val Lys Gly
1               5                   10                  15


<210> 14
<211> 8
<212> PRT
<213> Artificial Sequence

<220>
<223> FAP(28H1) CDR-H3

<400> 14

Gly Trp Leu Gly Asn Phe Asp Tyr
1               5


<210> 15
<211> 12
<212> PRT
<213> Artificial Sequence

<220>
<223> FAP(28H1) CDR-L1

<400> 15

Arg Ala Ser Gln Ser Val Ser Arg Ser Tyr Leu Ala
1               5                   10


<210> 16
<211> 7
<212> PRT
<213> Artificial Sequence

<220>
<223> FAP(28H1) CDR-L2

<400> 16

Gly Ala Ser Thr Arg Ala Thr

1                    5

<210> 17
<211> 9
<212> PRT
<213> Artificial Sequence

<220>
<223> FAP(28H1)  CDR-L3

<400> 17

Gln Gln Gly Gln Val Ile Pro Pro Thr
1                    5

<210> 18
<211> 116
<212> PRT
<213> Artificial Sequence

<220>
<223> FAP(28H1) VH

<400> 18

Glu Val Gln Leu Leu Glu Ser Gly Gly Gly Leu Val Gln Pro Gly Gly
1                    5                    10                   15

Ser Leu Arg Leu Ser Cys Ala Ala Ser Gly Phe Thr Phe Ser Ser His
                20                   25                   30

Ala Met Ser Trp Val Arg Gln Ala Pro Gly Lys Gly Leu Glu Trp Val
                35                   40                   45

Ser Ala Ile Trp Ala Ser Gly Glu Gln Tyr Tyr Ala Asp Ser Val Lys
             50                   55                   60

Gly Arg Phe Thr Ile Ser Arg Asp Asn Ser Lys Asn Thr Leu Tyr Leu
65                   70                   75                   80

Gln Met Asn Ser Leu Arg Ala Glu Asp Thr Ala Val Tyr Tyr Cys Ala
                85                   90                   95

Lys Gly Trp Leu Gly Asn Phe Asp Tyr Trp Gly Gln Gly Thr Leu Val
                100                  105                  110

Thr Val Ser Ser
          115

<210> 19
<211> 108
<212> PRT
<213> Artificial Sequence

<220>
<223> FAP(28H1) VL

<400> 19

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Arg Ser
            20                  25                  30

Tyr Leu Ala Trp Tyr Gln Gln Lys Pro Gly Gln Ala Pro Arg Leu Leu
            35                  40                  45

Ile Ile Gly Ala Ser Thr Arg Ala Thr Gly Ile Pro Asp Arg Phe Ser
        50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Gln Val Ile Pro
                85                  90                  95

Pro Thr Phe Gly Gln Gly Thr Lys Val Glu Ile Lys
            100                 105

<210> 20
<211> 10
<212> PRT
<213> Artificial Sequence

<220>
<223> ICOS  CDR-H1

<400> 20

Gly Tyr Thr Phe Thr Gly Tyr Tyr Met His
1               5                   10

<210> 21
<211> 17
<212> PRT
<213> Artificial Sequence

<220>
<223> ICOS  CDR-H2

<400> 21

Trp Ile Asn Pro His Ser Gly Gly Thr Asn Tyr Ala Gln Lys Phe Gln
1               5                   10                  15

Gly

```
<210>  22
<211>  16
<212>  PRT
<213>  Artificial Sequence

<220>
<223>  ICOS  CDR-H3

<400>  22

Thr Tyr Tyr Tyr Asp Ser Ser Gly Tyr Tyr His Asp Ala Phe Asp Ile
1               5                   10                  15


<210>  23
<211>  11
<212>  PRT
<213>  Artificial Sequence

<220>
<223>  ICOS  CDR-L1

<400>  23

Arg Ala Ser Gln Gly Ile Ser Arg Leu Leu Ala
1               5                   10


<210>  24
<211>  7
<212>  PRT
<213>  Artificial Sequence

<220>
<223>  ICOS  CDR-L2

<400>  24

Val Ala Ser Ser Leu Gln Ser
1               5


<210>  25
<211>  9
<212>  PRT
<213>  Artificial Sequence

<220>
<223>  ICOS  CDR-L3

<400>  25

Gln Gln Ala Asn Ser Phe Pro Trp Thr
1               5


<210>  26
<211>  125
<212>  PRT
<213>  Artificial Sequence
```

<220>
<223>   ICOS VH

<400>   26

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
            35                  40                  45

Gly Trp Ile Asn Pro His Ser Gly Gly Thr Asn Tyr Ala Gln Lys Phe
        50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Thr Tyr Tyr Tyr Asp Ser Ser Gly Tyr Tyr His Asp Ala Phe
            100                 105                 110

Asp Ile Trp Gly Gln Gly Thr Met Val Thr Val Ser Ser
            115                 120                 125

<210>   27
<211>   107
<212>   PRT
<213>   Artificial Sequence

<220>
<223>   ICOS VL

<400>   27

Asp Ile Gln Met Thr Gln Ser Pro Ser Ser Val Ser Ala Ser Val Gly
1               5                   10                  15

Asp Arg Val Thr Ile Thr Cys Arg Ala Ser Gln Gly Ile Ser Arg Leu
            20                  25                  30

Leu Ala Trp Tyr Gln Gln Lys Pro Gly Lys Ala Pro Lys Leu Leu Ile
            35                  40                  45

Tyr Val Ala Ser Ser Leu Gln Ser Gly Val Pro Ser Arg Phe Ser Gly
        50                  55                  60

Ser Gly Ser Gly Thr Asp Phe Thr Leu Thr Ile Ser Ser Leu Gln Pro
65                  70              75                  80

Glu Asp Phe Ala Thr Tyr Tyr Cys Gln Gln Ala Asn Ser Phe Pro Trp
                85                  90                  95

Thr Phe Gly Gln Gly Thr Lys Val Glu Ile Lys
            100                 105


<210> 28
<211> 455
<212> PRT
<213> Artificial Sequence

<220>
<223> VHCH1(JMAb136)- Fc knob chain

<400> 28

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
            35                  40                  45

Gly Trp Ile Asn Pro His Ser Gly Gly Thr Asn Tyr Ala Gln Lys Phe
        50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Thr Tyr Tyr Tyr Asp Ser Ser Gly Tyr Tyr His Asp Ala Phe
            100                 105                 110

Asp Ile Trp Gly Gln Gly Thr Met Val Thr Val Ser Ser Ala Ser Thr
        115                 120                 125

Lys Gly Pro Ser Val Phe Pro Leu Ala Pro Ser Ser Lys Ser Thr Ser
    130                 135                 140

Gly Gly Thr Ala Ala Leu Gly Cys Leu Val Glu Asp Tyr Phe Pro Glu
145                 150                 155                 160

```
Pro Val Thr Val Ser Trp Asn Ser Gly Ala Leu Thr Ser Gly Val His
              165             170             175

Thr Phe Pro Ala Val Leu Gln Ser Ser Gly Leu Tyr Ser Leu Ser Ser
              180             185             190

Val Val Thr Val Pro Ser Ser Ser Leu Gly Thr Gln Thr Tyr Ile Cys
              195             200             205

Asn Val Asn His Lys Pro Ser Asn Thr Lys Val Asp Glu Lys Val Glu
    210             215             220

Pro Lys Ser Cys Asp Lys Thr His Thr Cys Pro Pro Cys Pro Ala Pro
225             230             235             240

Glu Ala Ala Gly Gly Pro Ser Val Phe Leu Phe Pro Pro Lys Pro Lys
              245             250             255

Asp Thr Leu Met Ile Ser Arg Thr Pro Glu Val Thr Cys Val Val Val
              260             265             270

Asp Val Ser His Glu Asp Pro Glu Val Lys Phe Asn Trp Tyr Val Asp
              275             280             285

Gly Val Glu Val His Asn Ala Lys Thr Lys Pro Arg Glu Glu Gln Tyr
    290             295             300

Asn Ser Thr Tyr Arg Val Val Ser Val Leu Thr Val Leu His Gln Asp
305             310             315             320

Trp Leu Asn Gly Lys Glu Tyr Lys Cys Lys Val Ser Asn Lys Ala Leu
              325             330             335

Gly Ala Pro Ile Glu Lys Thr Ile Ser Lys Ala Lys Gly Gln Pro Arg
              340             345             350

Glu Pro Gln Val Tyr Thr Leu Pro Pro Cys Arg Asp Glu Leu Thr Lys
              355             360             365

Asn Gln Val Ser Leu Trp Cys Leu Val Lys Gly Phe Tyr Pro Ser Asp
    370             375             380

Ile Ala Val Glu Trp Glu Ser Asn Gly Gln Pro Glu Asn Asn Tyr Lys
385             390             395             400

Thr Thr Pro Pro Val Leu Asp Ser Asp Gly Ser Phe Phe Leu Tyr Ser
              405             410             415
```

90

```
Lys Leu Thr Val Asp Lys Ser Arg Trp Gln Gln Gly Asn Val Phe Ser
        420             425             430

Cys Ser Val Met His Glu Ala Leu His Asn His Tyr Thr Gln Lys Ser
        435             440             445

Leu Ser Leu Ser Pro Gly Lys
    450             455


<210>  29
<211>  214
<212>  PRT
<213>  Artificial Sequence

<220>
<223>  VLCL(JMAb136) Light chain

<400>  29

Asp Ile Gln Met Thr Gln Ser Pro Ser Ser Val Ser Ala Ser Val Gly
1               5               10              15

Asp Arg Val Thr Ile Thr Cys Arg Ala Ser Gln Gly Ile Ser Arg Leu
        20              25              30

Leu Ala Trp Tyr Gln Gln Lys Pro Gly Lys Ala Pro Lys Leu Leu Ile
        35              40              45

Tyr Val Ala Ser Ser Leu Gln Ser Gly Val Pro Ser Arg Phe Ser Gly
    50              55              60

Ser Gly Ser Gly Thr Asp Phe Thr Leu Thr Ile Ser Ser Leu Gln Pro
65              70              75              80

Glu Asp Phe Ala Thr Tyr Tyr Cys Gln Gln Ala Asn Ser Phe Pro Trp
            85              90              95

Thr Phe Gly Gln Gly Thr Lys Val Glu Ile Lys Arg Thr Val Ala Ala
        100             105             110

Pro Ser Val Phe Ile Phe Pro Pro Ser Asp Arg Lys Leu Lys Ser Gly
        115             120             125

Thr Ala Ser Val Val Cys Leu Leu Asn Asn Phe Tyr Pro Arg Glu Ala
    130             135             140

Lys Val Gln Trp Lys Val Asp Asn Ala Leu Gln Ser Gly Asn Ser Gln
145             150             155             160
```

```
Glu Ser Val Thr Glu Gln Asp Ser Lys Asp Ser Thr Tyr Ser Leu Ser
                165                 170                 175


Ser Thr Leu Thr Leu Ser Lys Ala Asp Tyr Glu Lys His Lys Val Tyr
                180                 185                 190


Ala Cys Glu Val Thr His Gln Gly Leu Ser Ser Pro Val Thr Lys Ser
            195                 200                 205


Phe Asn Arg Gly Glu Cys
        210
```

<210> 30
<211> 440
<212> PRT
<213> Artificial Sequence

<220>
<223> VHCH1 (4B9)- Fc hole chain

<400> 30

```
Glu Ile Val Leu Thr Gln Ser Pro Gly Thr Leu Ser Leu Ser Pro Gly
1                   5                   10                  15


Glu Arg Ala Thr Leu Ser Cys Arg Ala Ser Gln Ser Val Thr Ser Ser
                20                  25                  30


Tyr Leu Ala Trp Tyr Gln Gln Lys Pro Gly Gln Ala Pro Arg Leu Leu
            35                  40                  45


Ile Asn Val Gly Ser Arg Arg Ala Thr Gly Ile Pro Asp Arg Phe Ser
            50                  55                  60


Gly Ser Gly Ser Gly Thr Asp Phe Thr Leu Thr Ile Ser Arg Leu Glu
65                  70                  75                  80


Pro Glu Asp Phe Ala Val Tyr Tyr Cys Gln Gln Gly Ile Met Leu Pro
                85                  90                  95


Pro Thr Phe Gly Gln Gly Thr Lys Val Glu Ile Lys Ser Ser Ala Ser
                100                 105                 110


Thr Lys Gly Pro Ser Val Phe Pro Leu Ala Pro Ser Ser Lys Ser Thr
            115                 120                 125


Ser Gly Gly Thr Ala Ala Leu Gly Cys Leu Val Lys Asp Tyr Phe Pro
            130                 135                 140


Glu Pro Val Thr Val Ser Trp Asn Ser Gly Ala Leu Thr Ser Gly Val
```

145                    150                    155                    160

His Thr Phe Pro Ala Val Leu Gln Ser Ser Gly Leu Tyr Ser Leu Ser
            165                    170                    175

Ser Val Val Thr Val Pro Ser Ser Ser Leu Gly Thr Gln Thr Tyr Ile
            180                    185                    190

Cys Asn Val Asn His Lys Pro Ser Asn Thr Lys Val Asp Lys Lys Val
            195                    200                    205

Glu Pro Lys Ser Cys Asp Lys Thr His Thr Cys Pro Pro Cys Pro Ala
    210                    215                    220

Pro Glu Ala Ala Gly Gly Pro Ser Val Phe Leu Phe Pro Pro Lys Pro
225                    230                    235                    240

Lys Asp Thr Leu Met Ile Ser Arg Thr Pro Glu Val Thr Cys Val Val
            245                    250                    255

Val Asp Val Ser His Glu Asp Pro Glu Val Lys Phe Asn Trp Tyr Val
            260                    265                    270

Asp Gly Val Glu Val His Asn Ala Lys Thr Lys Pro Arg Glu Glu Gln
            275                    280                    285

Tyr Asn Ser Thr Tyr Arg Val Val Ser Val Leu Thr Val Leu His Gln
    290                    295                    300

Asp Trp Leu Asn Gly Lys Glu Tyr Lys Cys Lys Val Ser Asn Lys Ala
305                    310                    315                    320

Leu Gly Ala Pro Ile Glu Lys Thr Ile Ser Lys Ala Lys Gly Gln Pro
            325                    330                    335

Arg Glu Pro Gln Val Cys Thr Leu Pro Pro Ser Arg Asp Glu Leu Thr
            340                    345                    350

Lys Asn Gln Val Ser Leu Ser Cys Ala Val Lys Gly Phe Tyr Pro Ser
            355                    360                    365

Asp Ile Ala Val Glu Trp Glu Ser Asn Gly Gln Pro Glu Asn Asn Tyr
    370                    375                    380

Lys Thr Thr Pro Pro Val Leu Asp Ser Asp Gly Ser Phe Phe Leu Val
385                    390                    395                    400

```
Ser Lys Leu Thr Val Asp Lys Ser Arg Trp Gln Gln Gly Asn Val Phe
            405             410                 415

Ser Cys Ser Val Met His Glu Ala Leu His Asn His Tyr Thr Gln Lys
            420             425                 430

Ser Leu Ser Leu Ser Pro Gly Lys
        435             440
```

<210> 31
<211> 224
<212> PRT
<213> Artificial Sequence

<220>
<223> VLCL(4B9) Light chain

<400> 31

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20              25                  30

Ala Met Ser Trp Val Arg Gln Ala Pro Gly Lys Gly Leu Glu Trp Val
        35              40                  45

Ser Ala Ile Ile Gly Ser Gly Ala Ser Thr Tyr Tyr Ala Asp Ser Val
        50              55                  60

Lys Gly Arg Phe Thr Ile Ser Arg Asp Asn Ser Lys Asn Thr Leu Tyr
65                  70              75                  80

Leu Gln Met Asn Ser Leu Arg Ala Glu Asp Thr Ala Val Tyr Tyr Cys
            85              90                  95

Ala Lys Gly Trp Phe Gly Gly Phe Asn Tyr Trp Gly Gln Gly Thr Leu
            100             105                 110

Val Thr Val Ser Ser Ala Ser Val Ala Ala Pro Ser Val Phe Ile Phe
            115             120                 125

Pro Pro Ser Asp Glu Gln Leu Lys Ser Gly Thr Ala Ser Val Val Cys
        130             135                 140

Leu Leu Asn Asn Phe Tyr Pro Arg Glu Ala Lys Val Gln Trp Lys Val
145                 150             155                 160

Asp Asn Ala Leu Gln Ser Gly Asn Ser Gln Glu Ser Val Thr Glu Gln
```

165                    170                    175

Asp Ser Lys Asp Ser Thr Tyr Ser Leu Ser Ser Thr Leu Thr Leu Ser
      180              185              190

Lys Ala Asp Tyr Glu Lys His Lys Val Tyr Ala Cys Glu Val Thr His
      195              200              205

Gln Gly Leu Ser Ser Pro Val Thr Lys Ser Phe Asn Arg Gly Glu Cys
      210              215              220

<210>  32
<211>  591
<212>  PRT
<213>  Artificial Sequence

<220>
<223>  VHCH1 (JMAb136)-Fc knob chain-VH (4B9)

<400>  32

Gln Val Gln Leu Val Gln Ser Gly Ala Glu Val Lys Lys Pro Gly Ala
1            5              10              15

Ser Val Lys Val Ser Cys Lys Ala Ser Gly Tyr Thr Phe Thr Gly Tyr
      20              25              30

Tyr Met His Trp Val Arg Gln Ala Pro Gly Gln Gly Leu Glu Trp Met
      35              40              45

Gly Trp Ile Asn Pro His Ser Gly Gly Thr Asn Tyr Ala Gln Lys Phe
      50              55              60

Gln Gly Arg Val Thr Met Thr Arg Asp Thr Ser Ile Ser Thr Ala Tyr
65            70            75              80

Met Glu Leu Ser Arg Leu Arg Ser Asp Asp Thr Ala Val Tyr Tyr Cys
           85           90              95

Ala Arg Thr Tyr Tyr Tyr Asp Ser Ser Gly Tyr Tyr His Asp Ala Phe
      100           105          110

Asp Ile Trp Gly Gln Gly Thr Met Val Thr Val Ser Ser Ala Ser Thr
      115           120          125

Lys Gly Pro Ser Val Phe Pro Leu Ala Pro Ser Ser Lys Ser Thr Ser
      130           135          140

Gly Gly Thr Ala Ala Leu Gly Cys Leu Val Lys Asp Tyr Phe Pro Glu
      145           150          155          160

```
Pro Val Thr Val Ser Trp Asn Ser Gly Ala Leu Thr Ser Gly Val His
                165             170             175

Thr Phe Pro Ala Val Leu Gln Ser Ser Gly Leu Tyr Ser Leu Ser Ser
            180             185             190

Val Val Thr Val Pro Ser Ser Ser Leu Gly Thr Gln Thr Tyr Ile Cys
            195             200             205

Asn Val Asn His Lys Pro Ser Asn Thr Lys Val Asp Lys Lys Val Glu
        210             215             220

Pro Lys Ser Cys Asp Lys Thr His Thr Cys Pro Pro Cys Pro Ala Pro
225             230             235             240

Glu Ala Ala Gly Gly Pro Ser Val Phe Leu Phe Pro Pro Lys Pro Lys
            245             250             255

Asp Thr Leu Met Ile Ser Arg Thr Pro Glu Val Thr Cys Val Val Val
            260             265             270

Asp Val Ser His Glu Asp Pro Glu Val Lys Phe Asn Trp Tyr Val Asp
            275             280             285

Gly Val Glu Val His Asn Ala Lys Thr Lys Pro Arg Glu Glu Gln Tyr
    290             295             300

Asn Ser Thr Tyr Arg Val Val Ser Val Leu Thr Val Leu His Gln Asp
305             310             315             320

Trp Leu Asn Gly Lys Glu Tyr Lys Cys Lys Val Ser Asn Lys Ala Leu
            325             330             335

Gly Ala Pro Ile Glu Lys Thr Ile Ser Lys Ala Lys Gly Gln Pro Arg
            340             345             350

Glu Pro Gln Val Tyr Thr Leu Pro Pro Cys Arg Asp Glu Leu Thr Lys
            355             360             365

Asn Gln Val Ser Leu Trp Cys Leu Val Lys Gly Phe Tyr Pro Ser Asp
            370             375             380

Ile Ala Val Glu Trp Glu Ser Asn Gly Gln Pro Glu Asn Asn Tyr Lys
385             390             395             400

Thr Thr Pro Pro Val Leu Asp Ser Asp Gly Ser Phe Phe Leu Tyr Ser
```

```
                    405                      410                      415
```

Lys Leu Thr Val Asp Lys Ser Arg Trp Gln Gln Gly Asn Val Phe Ser
          420                      425                      430

Cys Ser Val Met His Glu Ala Leu His Asn His Tyr Thr Gln Lys Ser
          435                      440                      445

Leu Ser Leu Ser Pro Gly Gly Gly Gly Ser Gly Gly Gly Gly Ser
          450                      455                      460

Gly Gly Gly Gly Ser Gly Gly Gly Gly Ser Glu Val Gln Leu Leu Glu
465                      470                      475                      480

Ser Gly Gly Gly Leu Val Gln Pro Gly Gly Ser Leu Arg Leu Ser Cys
                    485                      490                      495

Ala Ala Ser Gly Phe Thr Phe Ser Ser Tyr Ala Met Ser Trp Val Arg
          500                      505                      510

Gln Ala Pro Gly Lys Gly Leu Glu Trp Val Ser Ala Ile Ile Gly Ser
          515                      520                      525

Gly Ala Ser Thr Tyr Tyr Ala Asp Ser Val Lys Gly Arg Phe Thr Ile
          530                      535                      540

Ser Arg Asp Asn Ser Lys Asn Thr Leu Tyr Leu Gln Met Asn Ser Leu
545                      550                      555                      560

Arg Ala Glu Asp Thr Ala Val Tyr Tyr Cys Ala Lys Gly Trp Phe Gly
                    565                      570                      575

Gly Phe Asn Tyr Trp Gly Gln Gly Thr Leu Val Thr Val Ser Ser
          580                      585                      590

```
<210>  33
<211>  582
<212>  PRT
<213>  Artificial Sequence

<220>
<223>  VHCH1 (JMAb136)-Fc hole chain-VL (4B9)

<400>  33
```

Gln Val Gln Leu Val Gln Ser Gly Ala Glu Val Lys Lys Pro Gly Ala
1                   5                       10                      15

Ser Val Lys Val Ser Cys Lys Ala Ser Gly Tyr Thr Phe Thr Gly Tyr
          20                      25                      30

Tyr Met His Trp Val Arg Gln Ala Pro Gly Gln Gly Leu Glu Trp Met
        35              40              45

Gly Trp Ile Asn Pro His Ser Gly Gly Thr Asn Tyr Ala Gln Lys Phe
        50              55              60

Gln Gly Arg Val Thr Met Thr Arg Asp Thr Ser Ile Ser Thr Ala Tyr
65              70              75              80

Met Glu Leu Ser Arg Leu Arg Ser Asp Asp Thr Ala Val Tyr Tyr Cys
                85              90              95

Ala Arg Thr Tyr Tyr Tyr Asp Ser Ser Gly Tyr Tyr His Asp Ala Phe
        100             105             110

Asp Ile Trp Gly Gln Gly Thr Met Val Thr Val Ser Ser Ala Ser Thr
        115             120             125

Lys Gly Pro Ser Val Phe Pro Leu Ala Pro Ser Ser Lys Ser Thr Ser
    130             135             140

Gly Gly Thr Ala Ala Leu Gly Cys Leu Val Lys Asp Tyr Phe Pro Glu
145             150             155             160

Pro Val Thr Val Ser Trp Asn Ser Gly Ala Leu Thr Ser Gly Val His
            165             170             175

Thr Phe Pro Ala Val Leu Gln Ser Ser Gly Leu Tyr Ser Leu Ser Ser
        180             185             190

Val Val Thr Val Pro Ser Ser Ser Leu Gly Thr Gln Thr Tyr Ile Cys
    195             200             205

Asn Val Asn His Lys Pro Ser Asn Thr Lys Val Asp Lys Lys Val Glu
    210             215             220

Pro Lys Ser Cys Asp Lys Thr His Thr Cys Pro Pro Cys Pro Ala Pro
225             230             235             240

Glu Ala Ala Gly Gly Pro Ser Val Phe Leu Phe Pro Pro Lys Pro Lys
            245             250             255

Asp Thr Leu Met Ile Ser Arg Thr Pro Glu Val Thr Cys Val Val Val
        260             265             270

Asp Val Ser His Glu Asp Pro Glu Val Lys Phe Asn Trp Tyr Val Asp

275            280            285

Gly Val Glu Val His Asn Ala Lys Thr Lys Pro Arg Glu Glu Gln Tyr
    290             295             300

Asn Ser Thr Tyr Arg Val Val Ser Val Leu Thr Val Leu His Gln Asp
305             310             315             320

Trp Leu Asn Gly Lys Glu Tyr Lys Cys Lys Val Ser Asn Lys Ala Leu
          325             330             335

Gly Ala Pro Ile Glu Lys Thr Ile Ser Lys Ala Lys Gly Gln Pro Arg
          340             345             350

Glu Pro Gln Val Cys Thr Leu Pro Pro Ser Arg Asp Glu Leu Thr Lys
          355             360             365

Asn Gln Val Ser Leu Ser Cys Ala Val Lys Gly Phe Tyr Pro Ser Asp
    370             375             380

Ile Ala Val Glu Trp Glu Ser Asn Gly Gln Pro Glu Asn Asn Tyr Lys
385             390             395             400

Thr Thr Pro Pro Val Leu Asp Ser Asp Gly Ser Phe Phe Leu Val Ser
          405             410             415

Lys Leu Thr Val Asp Lys Ser Arg Trp Gln Gln Gly Asn Val Phe Ser
          420             425             430

Cys Ser Val Met His Glu Ala Leu His Asn His Tyr Thr Gln Lys Ser
          435             440             445

Leu Ser Leu Ser Pro Gly Gly Gly Gly Ser Gly Gly Gly Gly Ser
          450             455             460

Gly Gly Gly Gly Ser Gly Gly Gly Gly Ser Glu Ile Val Leu Thr Gln
465             470             475             480

Ser Pro Gly Thr Leu Ser Leu Ser Pro Gly Glu Arg Ala Thr Leu Ser
          485             490             495

Cys Arg Ala Ser Gln Ser Val Thr Ser Ser Tyr Leu Ala Trp Tyr Gln
          500             505             510

Gln Lys Pro Gly Gln Ala Pro Arg Leu Leu Ile Asn Val Gly Ser Arg
          515             520             525

Arg Ala Thr Gly Ile Pro Asp Arg Phe Ser Gly Ser Gly Ser Gly Thr
    530                 535                 540

Asp Phe Thr Leu Thr Ile Ser Arg Leu Glu Pro Glu Asp Phe Ala Val
545                 550                 555                 560

Tyr Tyr Cys Gln Gln Gly Ile Met Leu Pro Pro Thr Phe Gly Gln Gly
                565                 570                 575

Thr Lys Val Glu Ile Lys
            580


<210>   34
<211>   5
<212>   PRT
<213>   Artificial Sequence

<220>
<223>   CD3-HCDR1

<400>   34

Thr Tyr Ala Met Asn
1                   5


<210>   35
<211>   19
<212>   PRT
<213>   Artificial Sequence

<220>
<223>   CD3-HCDR2

<400>   35

Arg Ile Arg Ser Lys Tyr Asn Asn Tyr Ala Thr Tyr Tyr Ala Asp Ser
1               5                   10                  15

Val Lys Gly


<210>   36
<211>   14
<212>   PRT
<213>   Artificial Sequence

<220>
<223>   CD3-HCDR3

<400>   36

His Gly Asn Phe Gly Asn Ser Tyr Val Ser Trp Phe Ala Tyr
1               5                   10


<210>   37


100

<211> 14
<212> PRT
<213> Artificial Sequence

<220>
<223> CD3-LCDR1

<400> 37

Gly Ser Ser Thr Gly Ala Val Thr Thr Ser Asn Tyr Ala Asn
1               5                   10


<210> 38
<211> 7
<212> PRT
<213> Artificial Sequence

<220>
<223> CD3-LCDR2

<400> 38

Gly Thr Asn Lys Arg Ala Pro
1                   5


<210> 39
<211> 9
<212> PRT
<213> Artificial Sequence

<220>
<223> CD3-LCDR3

<400> 39

Ala Leu Trp Tyr Ser Asn Leu Trp Val
1                   5


<210> 40
<211> 125
<212> PRT
<213> Artificial Sequence

<220>
<223> CD3 VH

<400> 40

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Thr Tyr
            20                  25                  30


Ala Met Asn Trp Val Arg Gln Ala Pro Gly Lys Gly Leu Glu Trp Val
            35                  40                  45

```
Ser Arg Ile Arg Ser Lys Tyr Asn Asn Tyr Ala Thr Tyr Tyr Ala Asp
    50                  55                  60

Ser Val Lys Gly Arg Phe Thr Ile Ser Arg Asp Asp Ser Lys Asn Thr
65                  70                  75                  80

Leu Tyr Leu Gln Met Asn Ser Leu Arg Ala Glu Asp Thr Ala Val Tyr
                85                  90                  95

Tyr Cys Val Arg His Gly Asn Phe Gly Asn Ser Tyr Val Ser Trp Phe
            100                 105                 110

Ala Tyr Trp Gly Gln Gly Thr Leu Val Thr Val Ser Ser
        115                 120                 125
```

```
<210>   41
<211>   109
<212>   PRT
<213>   Artificial Sequence

<220>
<223>   CD3 VL

<400>   41
```

```
Gln Ala Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Thr Ser
            20                  25                  30

Asn Tyr Ala Asn Trp Val Gln Glu Lys Pro Gly Gln Ala Phe Arg Gly
        35                  40                  45

Leu Ile Gly Gly Thr Asn Lys Arg Ala Pro Gly Thr Pro Ala Arg Phe
    50                  55                  60

Ser Gly Ser Leu Leu Gly Gly Lys Ala Ala Leu Thr Leu Ser Gly Ala
65                  70                  75                  80

Gln Pro Glu Asp Glu Ala Glu Tyr Tyr Cys Ala Leu Trp Tyr Ser Asn
                85                  90                  95

Leu Trp Val Phe Gly Gly Gly Thr Lys Leu Thr Val Leu
            100                 105
```

```
<210>   42
<211>   5
<212>   PRT
<213>   Artificial Sequence
```

<220>
<223> CEA-HCDR1

<400> 42

Glu Phe Gly Met Asn
1               5


<210> 43
<211> 17
<212> PRT
<213> Artificial Sequence

<220>
<223> CEA-HCDR2

<400> 43

Trp Ile Asn Thr Lys Thr Gly Glu Ala Thr Tyr Val Glu Glu Phe Lys
1               5                   10                  15


Gly


<210> 44
<211> 12
<212> PRT
<213> Artificial Sequence

<220>
<223> CEA-HCDR3

<400> 44

Trp Asp Phe Ala Tyr Tyr Val Glu Ala Met Asp Tyr
1               5                   10


<210> 45
<211> 11
<212> PRT
<213> Artificial Sequence

<220>
<223> CEA-LCDR1

<400> 45

Lys Ala Ser Ala Ala Val Gly Thr Tyr Val Ala
1               5                   10


<210> 46
<211> 7
<212> PRT
<213> Artificial Sequence

<220>
<223> CEA-LCDR2

<400> 46

Ser Ala Ser Tyr Arg Lys Arg
1                   5


<210> 47
<211> 10
<212> PRT
<213> Artificial Sequence

<220>
<223> CEA-LCDR3

<400> 47

His Gln Tyr Tyr Thr Tyr Pro Leu Phe Thr
1                   5                   10


<210> 48
<211> 121
<212> PRT
<213> Artificial Sequence

<220>
<223> CEA VH

<400> 48

Gln Val Gln Leu Val Gln Ser Gly Ala Glu Val Lys Lys Pro Gly Ala
1                   5                   10                  15


Ser Val Lys Val Ser Cys Lys Ala Ser Gly Tyr Thr Phe Thr Glu Phe
            20                  25                  30


Gly Met Asn Trp Val Arg Gln Ala Pro Gly Gln Gly Leu Glu Trp Met
            35                  40                  45


Gly Trp Ile Asn Thr Lys Thr Gly Glu Ala Thr Tyr Val Glu Glu Phe
        50                  55                  60


Lys Gly Arg Val Thr Phe Thr Thr Asp Thr Ser Thr Ser Thr Ala Tyr
65                  70                  75                  80


Met Glu Leu Arg Ser Leu Arg Ser Asp Asp Thr Ala Val Tyr Tyr Cys
                85                  90                  95


Ala Arg Trp Asp Phe Ala Tyr Tyr Val Glu Ala Met Asp Tyr Trp Gly
                100                 105                 110


Gln Gly Thr Thr Val Thr Val Ser Ser
            115                 120


<210> 49

```
<211>   108
<212>   PRT
<213>   Artificial Sequence

<220>
<223>   CEA VL

<400>   49

Asp Ile Gln Met Thr Gln Ser Pro Ser Ser Leu Ser Ala Ser Val Gly
1               5                   10                  15


Asp Arg Val Thr Ile Thr Cys Lys Ala Ser Ala Ala Val Gly Thr Tyr
            20                  25                  30


Val Ala Trp Tyr Gln Gln Lys Pro Gly Lys Ala Pro Lys Leu Leu Ile
        35                  40                  45


Tyr Ser Ala Ser Tyr Arg Lys Arg Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys His Gln Tyr Tyr Thr Tyr Pro Leu
                85                  90                  95


Phe Thr Phe Gly Gln Gly Thr Lys Leu Glu Ile Lys
                100                 105


<210>   50
<211>   5
<212>   PRT
<213>   Artificial Sequence

<220>
<223>   CEA-HCDR1 (CEACAM5)

<400>   50

Asp Thr Tyr Met His
1               5


<210>   51
<211>   17
<212>   PRT
<213>   Artificial Sequence

<220>
<223>   CEA-HCDR2 (CEACAM5)

<400>   51

Arg Ile Asp Pro Ala Asn Gly Asn Ser Lys Tyr Val Pro Lys Phe Gln
1               5                   10                  15
```

105

Gly


<210> 52
<211> 12
<212> PRT
<213> Artificial Sequence

<220>
<223> CEA-HCDR3 (CEACAM5)

<400> 52


Phe Gly Tyr Tyr Val Ser Asp Tyr Ala Met Ala Tyr
1               5                   10


<210> 53
<211> 15
<212> PRT
<213> Artificial Sequence

<220>
<223> CEA-LCDR1 (CEACAM5)

<400> 53

Arg Ala Gly Glu Ser Val Asp Ile Phe Gly Val Gly Phe Leu His
1               5                   10                  15


<210> 54
<211> 7
<212> PRT
<213> Artificial Sequence

<220>
<223> CEA-LCDR2 (CEACAM5)

<400> 54

Arg Ala Ser Asn Arg Ala Thr
1               5


<210> 55
<211> 9
<212> PRT
<213> Artificial Sequence

<220>
<223> CEA-LCDR3 (CEACAM5)

<400> 55

Gln Gln Thr Asn Glu Asp Pro Tyr Thr
1               5


<210> 56

<210> 56
<211> 121
<212> PRT
<213> Artificial Sequence

<220>
<223> CEA VH (CEACAM5)

<400> 56

```
Gln Val Gln Leu Val Gln Ser Gly Ala Glu Val Lys Lys Pro Gly Ser
1               5                   10                  15

Ser Val Lys Val Ser Cys Lys Ala Ser Gly Phe Asn Ile Lys Asp Thr
            20                  25                  30

Tyr Met His Trp Val Arg Gln Ala Pro Gly Gln Gly Leu Glu Trp Met
        35                  40                  45

Gly Arg Ile Asp Pro Ala Asn Gly Asn Ser Lys Tyr Val Pro Lys Phe
    50                  55                  60

Gln Gly Arg Val Thr Ile Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Pro Phe Gly Tyr Tyr Val Ser Asp Tyr Ala Met Ala Tyr Trp Gly
            100                 105                 110

Gln Gly Thr Leu Val Thr Val Ser Ser
        115                 120
```

<210> 57
<211> 111
<212> PRT
<213> Artificial Sequence

<220>
<223> CEA VL (CEACAM5)

<400> 57

```
Glu Ile Val Leu Thr Gln Ser Pro Ala Thr Leu Ser Leu Ser Pro Gly
1               5                   10                  15

Glu Arg Ala Thr Leu Ser Cys Arg Ala Gly Glu Ser Val Asp Ile Phe
            20                  25                  30

Gly Val Gly Phe Leu His Trp Tyr Gln Gln Lys Pro Gly Gln Ala Pro
        35                  40                  45
```

Arg Leu Leu Ile Tyr Arg Ala Ser Asn Arg Ala Thr Gly Ile Pro Ala
50                55                60

Arg Phe Ser Gly Ser Gly Ser Gly Thr Asp Phe Thr Leu Thr Ile Ser
65                70                75                80

Ser Leu Glu Pro Glu Asp Phe Ala Val Tyr Tyr Cys Gln Gln Thr Asn
85                90                95

Glu Asp Pro Tyr Thr Phe Gly Gln Gly Thr Lys Leu Glu Ile Lys
100               105               110

<210> 58
<211> 215
<212> PRT
<213> Artificial Sequence

<220>
<223> Light chain CEA (CEA TCB)

<400> 58

Asp Ile Gln Met Thr Gln Ser Pro Ser Ser Leu Ser Ala Ser Val Gly
1                 5                 10                15

Asp Arg Val Thr Ile Thr Cys Lys Ala Ser Ala Ala Val Gly Thr Tyr
20                25                30

Val Ala Trp Tyr Gln Gln Lys Pro Gly Lys Ala Pro Lys Leu Leu Ile
35                40                45

Tyr Ser Ala Ser Tyr Arg Lys Arg Gly Val Pro Ser Arg Phe Ser Gly
50                55                60

Ser Gly Ser Gly Thr Asp Phe Thr Leu Thr Ile Ser Ser Leu Gln Pro
65                70                75                80

Glu Asp Phe Ala Thr Tyr Tyr Cys His Gln Tyr Tyr Thr Tyr Pro Leu
85                90                95

Phe Thr Phe Gly Gln Gly Thr Lys Leu Glu Ile Lys Arg Thr Val Ala
100               105               110

Ala Pro Ser Val Phe Ile Phe Pro Pro Ser Asp Glu Gln Leu Lys Ser
115               120               125

Gly Thr Ala Ser Val Val Cys Leu Leu Asn Asn Phe Tyr Pro Arg Glu
130               135               140

Ala Lys Val Gln Trp Lys Val Asp Asn Ala Leu Gln Ser Gly Asn Ser

```
            145                  150                  155                  160


            Gln Glu Ser Val Thr Glu Gln Asp Ser Lys Asp Ser Thr Tyr Ser Leu
                         165                  170                  175


            Ser Ser Thr Leu Thr Leu Ser Lys Ala Asp Tyr Glu Lys His Lys Val
                         180                  185                  190


            Tyr Ala Cys Glu Val Thr His Gln Gly Leu Ser Ser Pro Val Thr Lys
                         195                  200                  205


            Ser Phe Asn Arg Gly Glu Cys
                210                  215



            <210>  59
            <211>  214
            <212>  PRT
            <213>  Artificial Sequence

            <220>
            <223>  Light chain CD3 (CEA TCB)

            <400>  59

            Gln Ala Val Val Thr Gln Glu Pro Ser Leu Thr Val Ser Pro Gly Gly
            1               5                   10                  15


            Thr Val Thr Leu Thr Cys Gly Ser Ser Thr Gly Ala Val Thr Thr Ser
                         20                  25                  30


            Asn Tyr Ala Asn Trp Val Gln Glu Lys Pro Gly Gln Ala Phe Arg Gly
                         35                  40                  45


            Leu Ile Gly Gly Thr Asn Lys Arg Ala Pro Gly Thr Pro Ala Arg Phe
                50                  55                  60


            Ser Gly Ser Leu Leu Gly Gly Lys Ala Ala Leu Thr Leu Ser Gly Ala
            65                  70                  75                  80


            Gln Pro Glu Asp Glu Ala Glu Tyr Tyr Cys Ala Leu Trp Tyr Ser Asn
                         85                  90                  95


            Leu Trp Val Phe Gly Gly Gly Thr Lys Leu Thr Val Leu Ser Ser Ala
                         100                 105                 110


            Ser Thr Lys Gly Pro Ser Val Phe Pro Leu Ala Pro Ser Ser Lys Ser
                         115                 120                 125


            Thr Ser Gly Gly Thr Ala Ala Leu Gly Cys Leu Val Lys Asp Tyr Phe
                         130                 135                 140
```

```
Pro Glu Pro Val Thr Val Ser Trp Asn Ser Gly Ala Leu Thr Ser Gly
145                 150             155                 160

Val His Thr Phe Pro Ala Val Leu Gln Ser Ser Gly Leu Tyr Ser Leu
                165             170                 175

Ser Ser Val Val Thr Val Pro Ser Ser Ser Leu Gly Thr Gln Thr Tyr
            180             185                 190

Ile Cys Asn Val Asn His Lys Pro Ser Asn Thr Lys Val Asp Lys Lys
        195             200             205

Val Glu Pro Lys Ser Cys
        210
```

<210> 60
<211> 694
<212> PRT
<213> Artificial Sequence

<220>
<223> CEA CD3 crossfab VHck fc knob P329GLALA (CEA TCB)

<400> 60

```
Gln Val Gln Leu Val Gln Ser Gly Ala Glu Val Lys Lys Pro Gly Ala
1               5               10                  15

Ser Val Lys Val Ser Cys Lys Ala Ser Gly Tyr Thr Phe Thr Glu Phe
            20              25                  30

Gly Met Asn Trp Val Arg Gln Ala Pro Gly Gln Gly Leu Glu Trp Met
        35              40                  45

Gly Trp Ile Asn Thr Lys Thr Gly Glu Ala Thr Tyr Val Glu Glu Phe
    50              55                  60

Lys Gly Arg Val Thr Phe Thr Thr Asp Thr Ser Thr Ser Thr Ala Tyr
65              70              75                  80

Met Glu Leu Arg Ser Leu Arg Ser Asp Asp Thr Ala Val Tyr Tyr Cys
            85              90                  95

Ala Arg Trp Asp Phe Ala Tyr Tyr Val Glu Ala Met Asp Tyr Trp Gly
        100             105                 110

Gln Gly Thr Thr Val Thr Val Ser Ser Ala Ser Thr Lys Gly Pro Ser
        115             120                 125
```

Val Phe Pro Leu Ala Pro Ser Ser Lys Ser Thr Ser Gly Gly Thr Ala
    130             135             140

Ala Leu Gly Cys Leu Val Lys Asp Tyr Phe Pro Glu Pro Val Thr Val
145             150             155             160

Ser Trp Asn Ser Gly Ala Leu Thr Ser Gly Val His Thr Phe Pro Ala
            165             170             175

Val Leu Gln Ser Ser Gly Leu Tyr Ser Leu Ser Ser Val Val Thr Val
        180             185             190

Pro Ser Ser Ser Leu Gly Thr Gln Thr Tyr Ile Cys Asn Val Asn His
    195             200             205

Lys Pro Ser Asn Thr Lys Val Asp Lys Lys Val Glu Pro Lys Ser Cys
    210             215             220

Asp Gly Gly Gly Gly Ser Gly Gly Gly Gly Ser Glu Val Gln Leu Leu
225             230             235             240

Glu Ser Gly Gly Gly Leu Val Gln Pro Gly Gly Ser Leu Arg Leu Ser
            245             250             255

Cys Ala Ala Ser Gly Phe Thr Phe Ser Thr Tyr Ala Met Asn Trp Val
            260             265             270

Arg Gln Ala Pro Gly Lys Gly Leu Glu Trp Val Ser Arg Ile Arg Ser
    275             280             285

Lys Tyr Asn Asn Tyr Ala Thr Tyr Tyr Ala Asp Ser Val Lys Gly Arg
    290             295             300

Phe Thr Ile Ser Arg Asp Asp Ser Lys Asn Thr Leu Tyr Leu Gln Met
305             310             315             320

Asn Ser Leu Arg Ala Glu Asp Thr Ala Val Tyr Tyr Cys Val Arg His
            325             330             335

Gly Asn Phe Gly Asn Ser Tyr Val Ser Trp Phe Ala Tyr Trp Gly Gln
            340             345             350

Gly Thr Leu Val Thr Val Ser Ser Ala Ser Val Ala Ala Pro Ser Val
            355             360             365

Phe Ile Phe Pro Pro Ser Asp Glu Gln Leu Lys Ser Gly Thr Ala Ser
    370             375             380

```
Val Val Cys Leu Leu Asn Asn Phe Tyr Pro Arg Glu Ala Lys Val Gln
385                 390             395                     400

Trp Lys Val Asp Asn Ala Leu Gln Ser Gly Asn Ser Gln Glu Ser Val
                405             410                 415

Thr Glu Gln Asp Ser Lys Asp Ser Thr Tyr Ser Leu Ser Ser Thr Leu
            420             425                 430

Thr Leu Ser Lys Ala Asp Tyr Glu Lys His Lys Val Tyr Ala Cys Glu
            435             440                 445

Val Thr His Gln Gly Leu Ser Ser Pro Val Thr Lys Ser Phe Asn Arg
        450             455                 460

Gly Glu Cys Asp Lys Thr His Thr Cys Pro Pro Cys Pro Ala Pro Glu
465             470                 475                     480

Ala Ala Gly Gly Pro Ser Val Phe Leu Phe Pro Pro Lys Pro Lys Asp
                485                 490                 495

Thr Leu Met Ile Ser Arg Thr Pro Glu Val Thr Cys Val Val Val Asp
            500                 505                 510

Val Ser His Glu Asp Pro Glu Val Lys Phe Asn Trp Tyr Val Asp Gly
        515                 520                 525

Val Glu Val His Asn Ala Lys Thr Lys Pro Arg Glu Glu Gln Tyr Asn
        530                 535                 540

Ser Thr Tyr Arg Val Val Ser Val Leu Thr Val Leu His Gln Asp Trp
545                 550                 555                     560

Leu Asn Gly Lys Glu Tyr Lys Cys Lys Val Ser Asn Lys Ala Leu Gly
                565                 570                 575

Ala Pro Ile Glu Lys Thr Ile Ser Lys Ala Lys Gly Gln Pro Arg Glu
            580                 585                 590

Pro Gln Val Tyr Thr Leu Pro Pro Cys Arg Asp Glu Leu Thr Lys Asn
        595                 600                 605

Gln Val Ser Leu Trp Cys Leu Val Lys Gly Phe Tyr Pro Ser Asp Ile
        610                 615                 620

Ala Val Glu Trp Glu Ser Asn Gly Gln Pro Glu Asn Asn Tyr Lys Thr
```

625            630            635            640

```
Thr Pro Pro Val Leu Asp Ser Asp Gly Ser Phe Phe Leu Tyr Ser Lys
            645                 650                 655

Leu Thr Val Asp Lys Ser Arg Trp Gln Gln Gly Asn Val Phe Ser Cys
            660                 665                 670

Ser Val Met His Glu Ala Leu His Asn His Tyr Thr Gln Lys Ser Leu
            675                 680                 685

Ser Leu Ser Pro Gly Lys
            690


<210>  61
<211>  451
<212>  PRT
<213>  Artificial Sequence

<220>
<223>  CEA VHCH1 Fc hole P329GLALA (CEA TCB)

<400>  61

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Glu Phe
            20                  25                  30

Gly Met Asn Trp Val Arg Gln Ala Pro Gly Gln Gly Leu Glu Trp Met
            35                  40                  45

Gly Trp Ile Asn Thr Lys Thr Gly Glu Ala Thr Tyr Val Glu Glu Phe
            50                  55                  60

Lys Gly Arg Val Thr Phe Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
            85                  90                  95

Ala Arg Trp Asp Phe Ala Tyr Tyr Val Glu Ala Met Asp Tyr Trp Gly
            100                 105                 110

Gln Gly Thr Thr Val Thr Val Ser Ser Ala Ser Thr Lys Gly Pro Ser
            115                 120                 125

Val Phe Pro Leu Ala Pro Ser Ser Lys Ser Thr Ser Gly Gly Thr Ala
            130                 135                 140
```

```
Ala Leu Gly Cys Leu Val Lys Asp Tyr Phe Pro Glu Pro Val Thr Val
145             150             155             160

Ser Trp Asn Ser Gly Ala Leu Thr Ser Gly Val His Thr Phe Pro Ala
                165             170             175

Val Leu Gln Ser Ser Gly Leu Tyr Ser Leu Ser Ser Val Val Thr Val
            180             185             190

Pro Ser Ser Ser Leu Gly Thr Gln Thr Tyr Ile Cys Asn Val Asn His
            195             200             205

Lys Pro Ser Asn Thr Lys Val Asp Lys Lys Val Glu Pro Lys Ser Cys
            210             215             220

Asp Lys Thr His Thr Cys Pro Pro Cys Pro Ala Pro Glu Ala Ala Gly
225             230             235             240

Gly Pro Ser Val Phe Leu Phe Pro Pro Lys Pro Lys Asp Thr Leu Met
                245             250             255

Ile Ser Arg Thr Pro Glu Val Thr Cys Val Val Val Asp Val Ser His
            260             265             270

Glu Asp Pro Glu Val Lys Phe Asn Trp Tyr Val Asp Gly Val Glu Val
            275             280             285

His Asn Ala Lys Thr Lys Pro Arg Glu Glu Gln Tyr Asn Ser Thr Tyr
    290             295             300

Arg Val Val Ser Val Leu Thr Val Leu His Gln Asp Trp Leu Asn Gly
305             310             315             320

Lys Glu Tyr Lys Cys Lys Val Ser Asn Lys Ala Leu Gly Ala Pro Ile
                325             330             335

Glu Lys Thr Ile Ser Lys Ala Lys Gly Gln Pro Arg Glu Pro Gln Val
            340             345             350

Cys Thr Leu Pro Pro Ser Arg Asp Glu Leu Thr Lys Asn Gln Val Ser
            355             360             365

Leu Ser Cys Ala Val Lys Gly Phe Tyr Pro Ser Asp Ile Ala Val Glu
            370             375             380

Trp Glu Ser Asn Gly Gln Pro Glu Asn Asn Tyr Lys Thr Thr Pro Pro
```

385 390 395 400

Val Leu Asp Ser Asp Gly Ser Phe Phe Leu Val Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Lys
450

```
<210>  62
<211>  232
<212>  PRT
<213>  Artificial Sequence

<220>
<223>  CD3 VH-CL (CEACAM5 TCB)

<400>  62
```

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Thr Tyr
20 25 30

Ala Met Asn Trp Val Arg Gln Ala Pro Gly Lys Gly Leu Glu Trp Val
35 40 45

Ser Arg Ile Arg Ser Lys Tyr Asn Asn Tyr Ala Thr Tyr Tyr Ala Asp
50 55 60

Ser Val Lys Gly Arg Phe Thr Ile Ser Arg Asp Asp Ser Lys Asn Thr
65 70 75 80

Leu Tyr Leu Gln Met Asn Ser Leu Arg Ala Glu Asp Thr Ala Val Tyr
85 90 95

Tyr Cys Val Arg His Gly Asn Phe Gly Asn Ser Tyr Val Ser Trp Phe
100 105 110

Ala Tyr Trp Gly Gln Gly Thr Leu Val Thr Val Ser Ser Ala Ser Val
115 120 125

Ala Ala Pro Ser Val Phe Ile Phe Pro Pro Ser Asp Glu Gln Leu Lys
130 135 140

```
Ser Gly Thr Ala Ser Val Val Cys Leu Leu Asn Asn Phe Tyr Pro Arg
145             150                 155                 160


Glu Ala Lys Val Gln Trp Lys Val Asp Asn Ala Leu Gln Ser Gly Asn
                165                 170                 175


Ser Gln Glu Ser Val Thr Glu Gln Asp Ser Lys Asp Ser Thr Tyr Ser
            180                 185                 190


Leu Ser Ser Thr Leu Thr Leu Ser Lys Ala Asp Tyr Glu Lys His Lys
            195                 200                 205


Val Tyr Ala Cys Glu Val Thr His Gln Gly Leu Ser Ser Pro Val Thr
    210                 215                 220


Lys Ser Phe Asn Arg Gly Glu Cys
225                 230



<210>   63
<211>   449
<212>   PRT
<213>   Artificial Sequence

<220>
<223>   CEACAM5 VH-CH1(EE)-Fc (hole, P329G LALA)

<400>   63

Gln Val Gln Leu Val Gln Ser Gly Ala Glu Val Lys Lys Pro Gly Ser
1               5                   10                  15


Ser Val Lys Val Ser Cys Lys Ala Ser Gly Phe Asn Ile Lys Asp Thr
            20                  25                  30


Tyr Met His Trp Val Arg Gln Ala Pro Gly Gln Gly Leu Glu Trp Met
            35                  40                  45


Gly Arg Ile Asp Pro Ala Asn Gly Asn Ser Lys Tyr Val Pro Lys Phe
    50                  55                  60


Gln Gly Arg Val Thr Ile Thr Ala Asp Thr Ser Thr Ser Thr Ala Tyr
65                  70                  75                  80


Met Glu Leu Ser Ser Leu Arg Ser Glu Asp Thr Ala Val Tyr Tyr Cys
                85                  90                  95


Ala Pro Phe Gly Tyr Tyr Val Ser Asp Tyr Ala Met Ala Tyr Trp Gly
                100                 105                 110
```

Gln Gly Thr Leu Val Thr Val Ser Ser Ala Ser Thr Lys Gly Pro Ser
115                120                125

Val Phe Pro Leu Ala Pro Ser Ser Lys Ser Thr Ser Gly Gly Thr Ala
130                135                140

Ala Leu Gly Cys Leu Val Glu Asp Tyr Phe Pro Glu Pro Val Thr Val
145                150                155                160

Ser Trp Asn Ser Gly Ala Leu Thr Ser Gly Val His Thr Phe Pro Ala
165                170                175

Val Leu Gln Ser Ser Gly Leu Tyr Ser Leu Ser Ser Val Val Thr Val
180                185                190

Pro Ser Ser Ser Leu Gly Thr Gln Thr Tyr Ile Cys Asn Val Asn His
195                200                205

Lys Pro Ser Asn Thr Lys Val Asp Glu Lys Val Glu Pro Lys Ser Cys
210                215                220

Asp Lys Thr His Thr Cys Pro Pro Cys Pro Ala Pro Glu Ala Ala Gly
225                230                235                240

Gly Pro Ser Val Phe Leu Phe Pro Pro Lys Pro Lys Asp Thr Leu Met
245                250                255

Ile Ser Arg Thr Pro Glu Val Thr Cys Val Val Val Asp Val Ser His
260                265                270

Glu Asp Pro Glu Val Lys Phe Asn Trp Tyr Val Asp Gly Val Glu Val
275                280                285

His Asn Ala Lys Thr Lys Pro Arg Glu Glu Gln Tyr Asn Ser Thr Tyr
290                295                300

Arg Val Val Ser Val Leu Thr Val Leu His Gln Asp Trp Leu Asn Gly
305                310                315                320

Lys Glu Tyr Lys Cys Lys Val Ser Asn Lys Ala Leu Gly Ala Pro Ile
325                330                335

Glu Lys Thr Ile Ser Lys Ala Lys Gly Gln Pro Arg Glu Pro Gln Val
340                345                350

Cys Thr Leu Pro Pro Ser Arg Asp Glu Leu Thr Lys Asn Gln Val Ser
355                360                365

Leu Ser Cys Ala Val Lys Gly Phe Tyr Pro Ser Asp Ile Ala Val Glu
    370             375             380

Trp Glu Ser Asn Gly Gln Pro Glu Asn Asn Tyr Lys Thr Thr Pro Pro
385             390             395             400

Val Leu Asp Ser Asp Gly Ser Phe Phe Leu Val Ser Lys Leu Thr Val
            405             410             415

Asp Lys Ser Arg Trp Gln Gln Gly Asn Val Phe Ser Cys Ser Val Met
        420             425             430

His Glu Ala Leu His Asn His Tyr Thr Gln Lys Ser Leu Ser Leu Ser
        435             440             445

Pro


<210> 64
<211> 674
<212> PRT
<213> Artificial Sequence

<220>
<223> CEACAM5 VH-CH1(EE)-CD3 VL-CH1-Fc (knob, P329G LALA)

<400> 64

Gln Val Gln Leu Val Gln Ser Gly Ala Glu Val Lys Lys Pro Gly Ser
1               5               10              15

Ser Val Lys Val Ser Cys Lys Ala Ser Gly Phe Asn Ile Lys Asp Thr
            20              25              30

Tyr Met His Trp Val Arg Gln Ala Pro Gly Gln Gly Leu Glu Trp Met
        35              40              45

Gly Arg Ile Asp Pro Ala Asn Gly Asn Ser Lys Tyr Val Pro Lys Phe
        50              55              60

Gln Gly Arg Val Thr Ile Thr Ala Asp Thr Ser Thr Ser Thr Ala Tyr
65              70              75              80

Met Glu Leu Ser Ser Leu Arg Ser Glu Asp Thr Ala Val Tyr Tyr Cys
            85              90              95

Ala Pro Phe Gly Tyr Tyr Val Ser Asp Tyr Ala Met Ala Tyr Trp Gly
            100             105             110

118

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
        130                 135                 140

Ala Leu Gly Cys Leu Val Glu Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
                180                 185                 190

Pro Ser Ser Ser Leu Gly Thr Gln Thr Tyr Ile Cys Asn Val Asn His
        195                 200                 205

Lys Pro Ser Asn Thr Lys Val Asp Glu Lys Val Glu Pro Lys Ser Cys
        210                 215                 220

Asp Gly Gly Gly Gly Ser Gly Gly Gly Gly Ser Gln Ala Val Val Thr
225                 230                 235                 240

Gln Glu Pro Ser Leu Thr Val Ser Pro Gly Gly Thr Val Thr Leu Thr
                245                 250                 255

Cys Gly Ser Ser Thr Gly Ala Val Thr Thr Ser Asn Tyr Ala Asn Trp
                260                 265                 270

Val Gln Glu Lys Pro Gly Gln Ala Phe Arg Gly Leu Ile Gly Gly Thr
        275                 280                 285

Asn Lys Arg Ala Pro Gly Thr Pro Ala Arg Phe Ser Gly Ser Leu Leu
        290                 295                 300

Gly Gly Lys Ala Ala Leu Thr Leu Ser Gly Ala Gln Pro Glu Asp Glu
305                 310                 315                 320

Ala Glu Tyr Tyr Cys Ala Leu Trp Tyr Ser Asn Leu Trp Val Phe Gly
                325                 330                 335

Gly Gly Thr Lys Leu Thr Val Leu Ser Ser Ala Ser Thr Lys Gly Pro
        340                 345                 350

Ser Val Phe Pro Leu Ala Pro Ser Ser Lys Ser Thr Ser Gly Gly Thr
        355                 360                 365

```
Ala Ala Leu Gly Cys Leu Val Lys Asp Tyr Phe Pro Glu Pro Val Thr
    370             375             380

Val Ser Trp Asn Ser Gly Ala Leu Thr Ser Gly Val His Thr Phe Pro
385             390             395                         400

Ala Val Leu Gln Ser Ser Gly Leu Tyr Ser Leu Ser Ser Val Val Thr
            405             410                         415

Val Pro Ser Ser Ser Leu Gly Thr Gln Thr Tyr Ile Cys Asn Val Asn
        420             425             430

His Lys Pro Ser Asn Thr Lys Val Asp Lys Lys Val Glu Pro Lys Ser
        435             440             445

Cys Asp Lys Thr His Thr Cys Pro Pro Cys Pro Ala Pro Glu Ala Ala
    450             455             460

Gly Gly Pro Ser Val Phe Leu Phe Pro Pro Lys Pro Lys Asp Thr Leu
465             470             475                         480

Met Ile Ser Arg Thr Pro Glu Val Thr Cys Val Val Val Asp Val Ser
            485             490                         495

His Glu Asp Pro Glu Val Lys Phe Asn Trp Tyr Val Asp Gly Val Glu
        500             505             510

Val His Asn Ala Lys Thr Lys Pro Arg Glu Glu Gln Tyr Asn Ser Thr
    515             520             525

Tyr Arg Val Val Ser Val Leu Thr Val Leu His Gln Asp Trp Leu Asn
    530             535             540

Gly Lys Glu Tyr Lys Cys Lys Val Ser Asn Lys Ala Leu Gly Ala Pro
545             550             555                         560

Ile Glu Lys Thr Ile Ser Lys Ala Lys Gly Gln Pro Arg Glu Pro Gln
            565             570                         575

Val Tyr Thr Leu Pro Pro Cys Arg Asp Glu Leu Thr Lys Asn Gln Val
        580             585                         590

Ser Leu Trp Cys Leu Val Lys Gly Phe Tyr Pro Ser Asp Ile Ala Val
    595             600             605

Glu Trp Glu Ser Asn Gly Gln Pro Glu Asn Asn Tyr Lys Thr Thr Pro
```

120

```
                    610                      615                      620

        Pro Val Leu Asp Ser Asp Gly Ser Phe Phe Leu Tyr Ser Lys Leu Thr
        625             630             635             640

        Val Asp Lys Ser Arg Trp Gln Gln Gly Asn Val Phe Ser Cys Ser Val
                        645             650             655

        Met His Glu Ala Leu His Asn His Tyr Thr Gln Lys Ser Leu Ser Leu
                660             665             670

        Ser Pro


        <210>  65
        <211>  218
        <212>  PRT
        <213>  Artificial Sequence

        <220>
        <223>  CEACAM5  VL-CL(RK)

        <400>  65

        Glu Ile Val Leu Thr Gln Ser Pro Ala Thr Leu Ser Leu Ser Pro Gly
        1               5               10              15

        Glu Arg Ala Thr Leu Ser Cys Arg Ala Gly Glu Ser Val Asp Ile Phe
                20              25              30

        Gly Val Gly Phe Leu His Trp Tyr Gln Gln Lys Pro Gly Gln Ala Pro
                35              40              45

        Arg Leu Leu Ile Tyr Arg Ala Ser Asn Arg Ala Thr Gly Ile Pro Ala
            50              55              60

        Arg Phe Ser Gly Ser Gly Ser Gly Thr Asp Phe Thr Leu Thr Ile Ser
        65              70              75              80

        Ser Leu Glu Pro Glu Asp Phe Ala Val Tyr Tyr Cys Gln Gln Thr Asn
                        85              90              95

        Glu Asp Pro Tyr Thr Phe Gly Gln Gly Thr Lys Leu Glu Ile Lys Arg
                100             105             110

        Thr Val Ala Ala Pro Ser Val Phe Ile Phe Pro Pro Ser Asp Arg Lys
                115             120             125

        Leu Lys Ser Gly Thr Ala Ser Val Val Cys Leu Leu Asn Asn Phe Tyr
                130             135             140
```

```
Pro Arg Glu Ala Lys Val Gln Trp Lys Val Asp Asn Ala Leu Gln Ser
145             150             155                 160

Gly Asn Ser Gln Glu Ser Val Thr Glu Gln Asp Ser Lys Asp Ser Thr
                165             170                 175

Tyr Ser Leu Ser Ser Thr Leu Thr Leu Ser Lys Ala Asp Tyr Glu Lys
            180             185                 190

His Lys Val Tyr Ala Cys Glu Val Thr His Gln Gly Leu Ser Ser Pro
            195             200             205

Val Thr Lys Ser Phe Asn Arg Gly Glu Cys
    210             215
```

```
<210>  66
<211>  354
<212>  PRT
<213>  Artificial Sequence

<220>
<223>  Fc hole chain-VL (4B9)

<400>  66
```

```
Asp Lys Thr His Thr Cys Pro Pro Cys Pro Ala Pro Glu Ala Ala Gly
1               5               10                  15

Gly Pro Ser Val Phe Leu Phe Pro Pro Lys Pro Lys Asp Thr Leu Met
            20              25              30

Ile Ser Arg Thr Pro Glu Val Thr Cys Val Val Val Asp Val Ser His
            35              40              45

Glu Asp Pro Glu Val Lys Phe Asn Trp Tyr Val Asp Gly Val Glu Val
    50              55              60

His Asn Ala Lys Thr Lys Pro Arg Glu Glu Gln Tyr Asn Ser Thr Tyr
65              70              75                  80

Arg Val Val Ser Val Leu Thr Val Leu His Gln Asp Trp Leu Asn Gly
            85              90                  95

Lys Glu Tyr Lys Cys Lys Val Ser Asn Lys Ala Leu Gly Ala Pro Ile
            100             105             110

Glu Lys Thr Ile Ser Lys Ala Lys Gly Gln Pro Arg Glu Pro Gln Val
            115             120                 125
```

```
Cys Thr Leu Pro Pro Ser Arg Asp Glu Leu Thr Lys Asn Gln Val Ser
    130                 135                 140

Leu Ser Cys Ala Val Lys Gly Phe Tyr Pro Ser Asp Ile Ala Val Glu
    145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Val Ser Lys Leu Thr Val
                180                 185                 190

Asp Lys Ser Arg Trp Gln Gln Gly Asn Val Phe Ser Cys Ser Val Met
            195                 200                 205

His Glu Ala Leu His Asn His Tyr Thr Gln Lys Ser Leu Ser Leu Ser
    210                 215                 220

Pro Gly Gly Gly Gly Gly Ser Gly Gly Gly Gly Ser Gly Gly Gly Gly
225                 230                 235                 240

Ser Gly Gly Gly Gly Ser Glu Ile Val Leu Thr Gln Ser Pro Gly Thr
                245                 250                 255

Leu Ser Leu Ser Pro Gly Glu Arg Ala Thr Leu Ser Cys Arg Ala Ser
            260                 265                 270

Gln Ser Val Thr Ser Ser Tyr Leu Ala Trp Tyr Gln Gln Lys Pro Gly
            275                 280                 285

Gln Ala Pro Arg Leu Leu Ile Asn Val Gly Ser Arg Arg Ala Thr Gly
    290                 295                 300

Ile Pro Asp Arg Phe Ser Gly Ser Gly Ser Gly Thr Asp Phe Thr Leu
305                 310                 315                 320

Thr Ile Ser Arg Leu Glu Pro Glu Asp Phe Ala Val Tyr Tyr Cys Gln
            325                 330                 335

Gln Gly Ile Met Leu Pro Pro Thr Phe Gly Gln Gly Thr Lys Val Glu
            340                 345                 350

Ile Lys


<210>    67
<211>    440
```

<212> PRT
<213> Artificial Sequence

<220>
<223> VHCH1 (DP47)- Fc hole chain

<400> 67

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Leu Thr Phe Gly Gln Gly Thr Lys Val Glu Ile Lys Ser Ser Ala Ser
            100                 105                 110

Thr Lys Gly Pro Ser Val Phe Pro Leu Ala Pro Ser Ser Lys Ser Thr
        115                 120                 125

Ser Gly Gly Thr Ala Ala Leu Gly Cys Leu Val Lys Asp Tyr Phe Pro
    130                 135                 140

Glu Pro Val Thr Val Ser Trp Asn Ser Gly Ala Leu Thr Ser Gly Val
145                 150                 155                 160

His Thr Phe Pro Ala Val Leu Gln Ser Ser Gly Leu Tyr Ser Leu Ser
                165                 170                 175

Ser Val Val Thr Val Pro Ser Ser Ser Leu Gly Thr Gln Thr Tyr Ile
            180                 185                 190

Cys Asn Val Asn His Lys Pro Ser Asn Thr Lys Val Asp Lys Lys Val
            195                 200                 205

Glu Pro Lys Ser Cys Asp Lys Thr His Thr Cys Pro Pro Cys Pro Ala
        210                 215                 220

```
Pro Glu Ala Ala Gly Gly Pro Ser Val Phe Leu Phe Pro Pro Lys Pro
225                 230             235                 240

Lys Asp Thr Leu Met Ile Ser Arg Thr Pro Glu Val Thr Cys Val Val
                245             250                 255

Val Asp Val Ser His Glu Asp Pro Glu Val Lys Phe Asn Trp Tyr Val
                260             265                 270

Asp Gly Val Glu Val His Asn Ala Lys Thr Lys Pro Arg Glu Glu Gln
            275             280             285

Tyr Asn Ser Thr Tyr Arg Val Val Ser Val Leu Thr Val Leu His Gln
    290             295             300

Asp Trp Leu Asn Gly Lys Glu Tyr Lys Cys Lys Val Ser Asn Lys Ala
305             310             315                 320

Leu Gly Ala Pro Ile Glu Lys Thr Ile Ser Lys Ala Lys Gly Gln Pro
                325             330             335

Arg Glu Pro Gln Val Cys Thr Leu Pro Pro Ser Arg Asp Glu Leu Thr
            340             345             350

Lys Asn Gln Val Ser Leu Ser Cys Ala Val Lys Gly Phe Tyr Pro Ser
            355             360             365

Asp Ile Ala Val Glu Trp Glu Ser Asn Gly Gln Pro Glu Asn Asn Tyr
    370             375             380

Lys Thr Thr Pro Pro Val Leu Asp Ser Asp Gly Ser Phe Phe Leu Val
385             390             395                 400

Ser Lys Leu Thr Val Asp Lys Ser Arg Trp Gln Gln Gly Asn Val Phe
                405             410                 415

Ser Cys Ser Val Met His Glu Ala Leu His Asn His Tyr Thr Gln Lys
                420             425             430

Ser Leu Ser Leu Ser Pro Gly Lys
            435             440
```

<210> 68
<211> 222
<212> PRT
<213> Artificial Sequence

<220>

<223> VLCL(DP47) Light chain

<400> 68

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
            85                  90                  95

Ala Lys Gly Ser Gly Phe Asp Tyr Trp Gly Gln Gly Thr Leu Val Thr
            100                 105                 110

Val Ser Ser Ala Ser Val Ala Ala Pro Ser Val Phe Ile Phe Pro Pro
    115                 120                 125

Ser Asp Glu Gln Leu Lys Ser Gly Thr Ala Ser Val Val Cys Leu Leu
    130                 135                 140

Asn Asn Phe Tyr Pro Arg Glu Ala Lys Val Gln Trp Lys Val Asp Asn
145                 150                 155                 160

Ala Leu Gln Ser Gly Asn Ser Gln Glu Ser Val Thr Glu Gln Asp Ser
            165                 170                 175

Lys Asp Ser Thr Tyr Ser Leu Ser Ser Thr Leu Thr Leu Ser Lys Ala
            180                 185                 190

Asp Tyr Glu Lys His Lys Val Tyr Ala Cys Glu Val Thr His Gln Gly
        195                 200                 205

Leu Ser Ser Pro Val Thr Lys Ser Phe Asn Arg Gly Glu Cys
    210                 215                 220

<210> 69
<211> 582
<212> PRT

<213> Artificial Sequence

<220>
<223> VHCH1 (JMAb136)-Fc knob chain-VH (DP47)

<400> 69

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro His Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Thr Tyr Tyr Tyr Asp Ser Ser Gly Tyr Tyr His Asp Ala Phe
            100                 105                 110

Asp Ile Trp Gly Gln Gly Thr Met Val Thr Val Ser Ser Ala Ser Thr
        115                 120                 125

Lys Gly Pro Ser Val Phe Pro Leu Ala Pro Ser Ser Lys Ser Thr Ser
    130                 135                 140

Gly Gly Thr Ala Ala Leu Gly Cys Leu Val Lys Asp Tyr Phe Pro Glu
145                 150                 155                 160

Pro Val Thr Val Ser Trp Asn Ser Gly Ala Leu Thr Ser Gly Val His
                165                 170                 175

Thr Phe Pro Ala Val Leu Gln Ser Ser Gly Leu Tyr Ser Leu Ser Ser
            180                 185                 190

Val Val Thr Val Pro Ser Ser Ser Leu Gly Thr Gln Thr Tyr Ile Cys
            195                 200                 205

Asn Val Asn His Lys Pro Ser Asn Thr Lys Val Asp Lys Lys Val Glu
    210                 215                 220

```
Pro Lys Ser Cys Asp Lys Thr His Thr Cys Pro Pro Cys Pro Ala Pro
225                 230                 235                 240

Glu Ala Ala Gly Gly Pro Ser Val Phe Leu Phe Pro Pro Lys Pro Lys
                245                 250                 255

Asp Thr Leu Met Ile Ser Arg Thr Pro Glu Val Thr Cys Val Val Val
                260                 265                 270

Asp Val Ser His Glu Asp Pro Glu Val Lys Phe Asn Trp Tyr Val Asp
                275                 280                 285

Gly Val Glu Val His Asn Ala Lys Thr Lys Pro Arg Glu Glu Gln Tyr
                290                 295                 300

Asn Ser Thr Tyr Arg Val Val Ser Val Leu Thr Val Leu His Gln Asp
305                 310                 315                 320

Trp Leu Asn Gly Lys Glu Tyr Lys Cys Lys Val Ser Asn Lys Ala Leu
                325                 330                 335

Gly Ala Pro Ile Glu Lys Thr Ile Ser Lys Ala Lys Gly Gln Pro Arg
                340                 345                 350

Glu Pro Gln Val Tyr Thr Leu Pro Pro Cys Arg Asp Glu Leu Thr Lys
                355                 360                 365

Asn Gln Val Ser Leu Trp Cys Leu Val Lys Gly Phe Tyr Pro Ser Asp
                370                 375                 380

Ile Ala Val Glu Trp Glu Ser Asn Gly Gln Pro Glu Asn Asn Tyr Lys
385                 390                 395                 400

Thr Thr Pro Pro Val Leu Asp Ser Asp Gly Ser Phe Phe Leu Tyr Ser
                405                 410                 415

Lys Leu Thr Val Asp Lys Ser Arg Trp Gln Gln Gly Asn Val Phe Ser
                420                 425                 430

Cys Ser Val Met His Glu Ala Leu His Asn His Tyr Thr Gln Lys Ser
                435                 440                 445

Leu Ser Leu Ser Pro Gly Gly Gly Gly Gly Ser Gly Gly Gly Gly Ser
                450                 455                 460

Gly Gly Gly Gly Ser Gly Gly Gly Gly Ser Glu Ile Val Leu Thr Gln
465                 470                 475                 480
```

```
Ser Pro Gly Thr Leu Ser Leu Ser Pro Gly Glu Arg Ala Thr Leu Ser
            485             490                 495

Cys Arg Ala Ser Gln Ser Val Ser Ser Ser Tyr Leu Ala Trp Tyr Gln
            500             505                 510

Gln Lys Pro Gly Gln Ala Pro Arg Leu Leu Ile Tyr Gly Ala Ser Ser
            515             520                 525

Arg Ala Thr Gly Ile Pro Asp Arg Phe Ser Gly Ser Gly Ser Gly Thr
    530             535             540

Asp Phe Thr Leu Thr Ile Ser Arg Leu Glu Pro Glu Asp Phe Ala Val
545             550             555                 560

Tyr Tyr Cys Gln Gln Tyr Gly Ser Ser Pro Leu Thr Phe Gly Gln Gly
            565             570                 575

Thr Lys Val Glu Ile Lys
            580
```

```
<210>  70
<211>  589
<212>  PRT
<213>  Artificial Sequence

<220>
<223>  VHCH1 (JMAb136)-Fc hole chain-VL (DP47)

<400>  70
```

```
Gln Val Gln Leu Val Gln Ser Gly Ala Glu Val Lys Lys Pro Gly Ala
1               5               10                  15

Ser Val Lys Val Ser Cys Lys Ala Ser Gly Tyr Thr Phe Thr Gly Tyr
            20              25                  30

Tyr Met His Trp Val Arg Gln Ala Pro Gly Gln Gly Leu Glu Trp Met
            35              40                  45

Gly Trp Ile Asn Pro His Ser Gly Gly Thr Asn Tyr Ala Gln Lys Phe
    50              55                  60

Gln Gly Arg Val Thr Met Thr Arg Asp Thr Ser Ile Ser Thr Ala Tyr
65              70                  75                  80

Met Glu Leu Ser Arg Leu Arg Ser Asp Asp Thr Ala Val Tyr Tyr Cys
            85              90                  95
```

129

```
Ala Arg Thr Tyr Tyr Tyr Asp Ser Ser Gly Tyr Tyr His Asp Ala Phe
            100             105             110

Asp Ile Trp Gly Gln Gly Thr Met Val Thr Val Ser Ser Ala Ser Thr
            115             120             125

Lys Gly Pro Ser Val Phe Pro Leu Ala Pro Ser Ser Lys Ser Thr Ser
            130             135             140

Gly Gly Thr Ala Ala Leu Gly Cys Leu Val Lys Asp Tyr Phe Pro Glu
145             150             155             160

Pro Val Thr Val Ser Trp Asn Ser Gly Ala Leu Thr Ser Gly Val His
            165             170             175

Thr Phe Pro Ala Val Leu Gln Ser Ser Gly Leu Tyr Ser Leu Ser Ser
            180             185             190

Val Val Thr Val Pro Ser Ser Ser Leu Gly Thr Gln Thr Tyr Ile Cys
            195             200             205

Asn Val Asn His Lys Pro Ser Asn Thr Lys Val Asp Lys Lys Val Glu
            210             215             220

Pro Lys Ser Cys Asp Lys Thr His Thr Cys Pro Pro Cys Pro Ala Pro
225             230             235             240

Glu Ala Ala Gly Gly Pro Ser Val Phe Leu Phe Pro Pro Lys Pro Lys
            245             250             255

Asp Thr Leu Met Ile Ser Arg Thr Pro Glu Val Thr Cys Val Val Val
            260             265             270

Asp Val Ser His Glu Asp Pro Glu Val Lys Phe Asn Trp Tyr Val Asp
            275             280             285

Gly Val Glu Val His Asn Ala Lys Thr Lys Pro Arg Glu Glu Gln Tyr
            290             295             300

Asn Ser Thr Tyr Arg Val Val Ser Val Leu Thr Val Leu His Gln Asp
305             310             315             320

Trp Leu Asn Gly Lys Glu Tyr Lys Cys Lys Val Ser Asn Lys Ala Leu
            325             330             335

Gly Ala Pro Ile Glu Lys Thr Ile Ser Lys Ala Lys Gly Gln Pro Arg
            340             345             350
```

```
Glu Pro Gln Val Cys Thr Leu Pro Pro Ser Arg Asp Glu Leu Thr Lys
        355                 360                 365

Asn Gln Val Ser Leu Ser Cys Ala Val Lys Gly Phe Tyr Pro Ser Asp
        370                 375                 380

Ile Ala Val Glu Trp Glu Ser Asn Gly Gln Pro Glu Asn Asn Tyr Lys
385                 390                 395                 400

Thr Thr Pro Pro Val Leu Asp Ser Asp Gly Ser Phe Phe Leu Val Ser
                405                 410                 415

Lys Leu Thr Val Asp Lys Ser Arg Trp Gln Gln Gly Asn Val Phe Ser
            420                 425                 430

Cys Ser Val Met His Glu Ala Leu His Asn His Tyr Thr Gln Lys Ser
        435                 440                 445

Leu Ser Leu Ser Pro Gly Gly Gly Gly Ser Gly Gly Gly Gly Ser
    450                 455                 460

Gly Gly Gly Gly Ser Gly Gly Gly Gly Ser Glu Val Gln Leu Leu Glu
465                 470                 475                 480

Ser Gly Gly Gly Leu Val Gln Pro Gly Gly Ser Leu Arg Leu Ser Cys
                485                 490                 495

Ala Ala Ser Gly Phe Thr Phe Ser Ser Tyr Ala Met Ser Trp Val Arg
            500                 505                 510

Gln Ala Pro Gly Lys Gly Leu Glu Trp Val Ser Ala Ile Ser Gly Ser
        515                 520                 525

Gly Gly Ser Thr Tyr Tyr Ala Asp Ser Val Lys Gly Arg Phe Thr Ile
    530                 535                 540

Ser Arg Asp Asn Ser Lys Asn Thr Leu Tyr Leu Gln Met Asn Ser Leu
545                 550                 555                 560

Arg Ala Glu Asp Thr Ala Val Tyr Tyr Cys Ala Lys Gly Ser Gly Phe
                565                 570                 575

Asp Tyr Trp Gly Gln Gly Thr Leu Val Thr Val Ser Ser
            580                 585
```

```
<210>   71
<211>   519
```

131

<212> PRT
<213> Artificial Sequence

<220>
<223> Murine ICOSL Linker muIgG1 Fc (DAPG KK) 4GS linker FAP(28H1) VH

<400> 71

```
Gln Asp Thr Gln Glu Phe Thr Cys Arg Val Phe Met Asn Thr Ala Thr
1               5                   10                  15

Glu Leu Val Lys Ile Leu Glu Glu Val Val Arg Leu Arg Val Ala Ala
            20                  25                  30

Asn Phe Ser Thr Pro Val Ile Ser Thr Ser Asp Ser Ser Asn Pro Gly
            35                  40                  45

Gln Glu Arg Thr Tyr Thr Cys Met Ser Lys Asn Gly Tyr Pro Glu Pro
            50                  55                  60

Asn Leu Tyr Trp Ile Asn Thr Thr Asp Asn Ser Leu Ile Asp Thr Ala
65                  70                  75                  80

Leu Gln Asn Asn Thr Val Tyr Leu Asn Lys Leu Gly Leu Tyr Asp Val
                85                  90                  95

Ile Ser Thr Leu Arg Leu Pro Trp Thr Ser Arg Gly Asp Val Leu Cys
            100                 105                 110

Cys Val Glu Asn Val Ala Leu His Gln Asn Ile Thr Ser Ile Ser Gln
            115                 120                 125

Ala Glu Ser Phe Thr Gly Asn Asn Thr Lys Asn Pro Gln Glu Thr His
            130                 135                 140

Asn Asn Glu Leu Lys Gly Ser Pro Gly Ser Ser Ser Ser Ser Gly Ser
145                 150                 155                 160

Ala Asp Gly Cys Lys Pro Cys Ile Cys Thr Val Pro Glu Val Ser Ser
                165                 170                 175

Val Phe Ile Phe Pro Pro Lys Pro Lys Asp Val Leu Thr Ile Thr Leu
                180                 185                 190

Thr Pro Lys Val Thr Cys Val Val Val Ala Ile Ser Lys Asp Asp Pro
            195                 200                 205

Glu Val Gln Phe Ser Trp Phe Val Asp Asp Val Glu Val His Thr Ala
            210                 215                 220
```

```
Gln Thr Lys Pro Arg Glu Glu Gln Ile Asn Ser Thr Phe Arg Ser Val
225                 230                 235                 240

Ser Glu Leu Pro Ile Met His Gln Asp Trp Leu Asn Gly Lys Glu Phe
                245                 250                 255

Lys Cys Arg Val Asn Ser Ala Ala Phe Gly Ala Pro Ile Glu Lys Thr
                260                 265                 270

Ile Ser Lys Thr Lys Gly Arg Pro Lys Ala Pro Gln Val Tyr Thr Ile
                275                 280                 285

Pro Pro Pro Lys Lys Gln Met Ala Lys Asp Lys Val Ser Leu Thr Cys
                290                 295                 300

Met Ile Thr Asn Phe Phe Pro Glu Asp Ile Thr Val Glu Trp Gln Trp
305                 310                 315                 320

Asn Gly Gln Pro Ala Glu Asn Tyr Lys Asn Thr Gln Pro Ile Met Lys
                325                 330                 335

Thr Asp Gly Ser Tyr Phe Val Tyr Ser Lys Leu Asn Val Gln Lys Ser
                340                 345                 350

Asn Trp Glu Ala Gly Asn Thr Phe Thr Cys Ser Val Leu His Glu Gly
                355                 360                 365

Leu His Asn His His Thr Glu Lys Ser Leu Ser His Ser Pro Gly Gly
                370                 375                 380

Gly Gly Gly Ser Gly Gly Gly Ser Gly Gly Gly Gly Ser Gly Gly
385                 390                 395                 400

Gly Gly Ser Glu Val Gln Leu Leu Glu Ser Gly Gly Gly Leu Val Gln
                405                 410                 415

Pro Gly Gly Ser Leu Arg Leu Ser Cys Ala Ala Ser Gly Phe Thr Phe
                420                 425                 430

Ser Ser His Ala Met Ser Trp Val Arg Gln Ala Pro Gly Lys Gly Leu
                435                 440                 445

Glu Trp Val Ser Ala Ile Trp Ala Ser Gly Glu Gln Tyr Tyr Ala Asp
                450                 455                 460

Ser Val Lys Gly Arg Phe Thr Ile Ser Arg Asp Asn Ser Lys Asn Thr
465                 470                 475                 480
```

133

```
Leu Tyr Leu Gln Met Asn Ser Leu Arg Ala Glu Asp Thr Ala Val Tyr
            485                 490                 495

Tyr Cys Ala Lys Gly Trp Leu Gly Asn Phe Asp Tyr Trp Gly Gln Gly
            500                 505                 510

Thr Leu Val Thr Val Ser Ser
            515
```

<210> 72
<211> 607
<212> PRT
<213> Artificial Sequence

<220>
<223> Murine ICOSL linker muIgG1 Fc (DAPG DD) 4GS linker FAP(28H1) VL

<400> 72

```
Glu Thr Glu Val Gly Ala Met Val Gly Ser Asn Val Val Leu Ser Cys
1                   5                   10                  15

Ile Asp Pro His Arg Arg His Phe Asn Leu Ser Gly Leu Tyr Val Tyr
            20                  25                  30

Trp Gln Ile Glu Asn Pro Glu Val Ser Val Thr Tyr Tyr Leu Pro Tyr
            35                  40                  45

Lys Ser Pro Gly Ile Asn Val Asp Ser Ser Tyr Lys Asn Arg Gly His
            50                  55                  60

Leu Ser Leu Asp Ser Met Lys Gln Gly Asn Phe Ser Leu Tyr Leu Lys
65                  70                  75                  80

Asn Val Thr Pro Gln Asp Thr Gln Glu Phe Thr Cys Arg Val Phe Met
            85                  90                  95

Asn Thr Ala Thr Glu Leu Val Lys Ile Leu Glu Glu Val Val Arg Leu
            100                 105                 110

Arg Val Ala Ala Asn Phe Ser Thr Pro Val Ile Ser Thr Ser Asp Ser
            115                 120                 125

Ser Asn Pro Gly Gln Glu Arg Thr Tyr Thr Cys Met Ser Lys Asn Gly
            130                 135                 140

Tyr Pro Glu Pro Asn Leu Tyr Trp Ile Asn Thr Thr Asp Asn Ser Leu
145                 150                 155                 160
```

Ile Asp Thr Ala Leu Gln Asn Asn Thr Val Tyr Leu Asn Lys Leu Gly
                165                 170                 175

Leu Tyr Asp Val Ile Ser Thr Leu Arg Leu Pro Trp Thr Ser Arg Gly
                180                 185                 190

Asp Val Leu Cys Cys Val Glu Asn Val Ala Leu His Gln Asn Ile Thr
                195                 200                 205

Ser Ile Ser Gln Ala Glu Ser Phe Thr Gly Asn Asn Thr Lys Asn Pro
    210                 215                 220

Gln Glu Thr His Asn Asn Glu Leu Lys Gly Ser Pro Gly Ser Ser Ser
225                 230                 235                 240

Ser Ser Gly Ser Ala Gly Ser Pro Gly Ser Ser Ser Ser Gly Ser
                245                 250                 255

Ala Asp Gly Cys Lys Pro Cys Ile Cys Thr Val Pro Glu Val Ser Ser
                260                 265                 270

Val Phe Ile Phe Pro Pro Lys Pro Lys Asp Val Leu Thr Ile Thr Leu
                275                 280                 285

Thr Pro Lys Val Thr Cys Val Val Val Ala Ile Ser Lys Asp Asp Pro
    290                 295                 300

Glu Val Gln Phe Ser Trp Phe Val Asp Asp Val Glu Val His Thr Ala
305                 310                 315                 320

Gln Thr Lys Pro Arg Glu Glu Gln Ile Asn Ser Thr Phe Arg Ser Val
                325                 330                 335

Ser Glu Leu Pro Ile Met His Gln Asp Trp Leu Asn Gly Lys Glu Phe
                340                 345                 350

Lys Cys Arg Val Asn Ser Ala Ala Phe Gly Ala Pro Ile Glu Lys Thr
                355                 360                 365

Ile Ser Lys Thr Lys Gly Arg Pro Lys Ala Pro Gln Val Tyr Thr Ile
    370                 375                 380

Pro Pro Pro Lys Glu Gln Met Ala Lys Asp Lys Val Ser Leu Thr Cys
385                 390                 395                 400

Met Ile Thr Asn Phe Phe Pro Glu Asp Ile Thr Val Glu Trp Gln Trp
                405                 410                 415

135

```
Asn Gly Gln Pro Ala Glu Asn Tyr Asp Asn Thr Gln Pro Ile Met Asp
            420             425             430

Thr Asp Gly Ser Tyr Phe Val Tyr Ser Asp Leu Asn Val Gln Lys Ser
            435             440             445

Asn Trp Glu Ala Gly Asn Thr Phe Thr Cys Ser Val Leu His Glu Gly
        450             455             460

Leu His Asn His His Thr Glu Lys Ser Leu Ser His Ser Pro Gly Gly
465             470             475             480

Gly Gly Gly Ser Gly Gly Gly Ser Gly Gly Gly Ser Gly Gly
            485             490             495

Gly Gly Ser Glu Ile Val Leu Thr Gln Ser Pro Gly Thr Leu Ser Leu
        500             505             510

Ser Pro Gly Glu Arg Ala Thr Leu Ser Cys Arg Ala Ser Gln Ser Val
        515             520             525

Ser Arg Ser Tyr Leu Ala Trp Tyr Gln Gln Lys Pro Gly Gln Ala Pro
    530             535             540

Arg Leu Leu Ile Ile Gly Ala Ser Thr Arg Ala Thr Gly Ile Pro Asp
545             550             555             560

Arg Phe Ser Gly Ser Gly Ser Gly Thr Asp Phe Thr Leu Thr Ile Ser
            565             570             575

Arg Leu Glu Pro Glu Asp Phe Ala Val Tyr Tyr Cys Gln Gln Gly Gln
            580             585             590

Val Ile Pro Pro Thr Phe Gly Gln Gly Thr Lys Val Glu Ile Lys
        595             600             605
```

```
<210>  73
<211>  607
<212>  PRT
<213>  Artificial Sequence

<220>
<223>  Murine ICOSL Linker muIgG1 Fc (DAPG KK) G4S linker DP47 VH

<400>  73
```

```
Glu Thr Glu Val Gly Ala Met Val Gly Ser Asn Val Val Leu Ser Cys
1               5               10              15
```

```
Ile Asp Pro His Arg Arg His Phe Asn Leu Ser Gly Leu Tyr Val Tyr
            20                  25                  30

Trp Gln Ile Glu Asn Pro Glu Val Ser Val Thr Tyr Tyr Leu Pro Tyr
            35                  40                  45

Lys Ser Pro Gly Ile Asn Val Asp Ser Ser Tyr Lys Asn Arg Gly His
            50                  55                  60

Leu Ser Leu Asp Ser Met Lys Gln Gly Asn Phe Ser Leu Tyr Leu Lys
65                  70                  75                  80

Asn Val Thr Pro Gln Asp Thr Gln Glu Phe Thr Cys Arg Val Phe Met
                85                  90                  95

Asn Thr Ala Thr Glu Leu Val Lys Ile Leu Glu Glu Val Val Arg Leu
            100                 105                 110

Arg Val Ala Ala Asn Phe Ser Thr Pro Val Ile Ser Thr Ser Asp Ser
            115                 120                 125

Ser Asn Pro Gly Gln Glu Arg Thr Tyr Thr Cys Met Ser Lys Asn Gly
            130                 135                 140

Tyr Pro Glu Pro Asn Leu Tyr Trp Ile Asn Thr Thr Asp Asn Ser Leu
145                 150                 155                 160

Ile Asp Thr Ala Leu Gln Asn Asn Thr Val Tyr Leu Asn Lys Leu Gly
                165                 170                 175

Leu Tyr Asp Val Ile Ser Thr Leu Arg Leu Pro Trp Thr Ser Arg Gly
                180                 185                 190

Asp Val Leu Cys Cys Val Glu Asn Val Ala Leu His Gln Asn Ile Thr
            195                 200                 205

Ser Ile Ser Gln Ala Glu Ser Phe Thr Gly Asn Asn Thr Lys Asn Pro
            210                 215                 220

Gln Glu Thr His Asn Asn Glu Leu Lys Gly Ser Pro Gly Ser Ser Ser
225                 230                 235                 240

Ser Ser Gly Ser Ala Gly Ser Pro Gly Ser Ser Ser Ser Ser Gly Ser
                245                 250                 255

Ala Asp Gly Cys Lys Pro Cys Ile Cys Thr Val Pro Glu Val Ser Ser
            260                 265                 270
```

Val Phe Ile Phe Pro Pro Lys Pro Lys Asp Val Leu Thr Ile Thr Leu
        275                 280                 285

Thr Pro Lys Val Thr Cys Val Val Val Ala Ile Ser Lys Asp Asp Pro
        290                 295                 300

Glu Val Gln Phe Ser Trp Phe Val Asp Asp Val Glu Val His Thr Ala
305                 310                 315                 320

Gln Thr Lys Pro Arg Glu Glu Gln Ile Asn Ser Thr Phe Arg Ser Val
                325                 330                 335

Ser Glu Leu Pro Ile Met His Gln Asp Trp Leu Asn Gly Lys Glu Phe
                340                 345                 350

Lys Cys Arg Val Asn Ser Ala Ala Phe Gly Ala Pro Ile Glu Lys Thr
        355                 360                 365

Ile Ser Lys Thr Lys Gly Arg Pro Lys Ala Pro Gln Val Tyr Thr Ile
        370                 375                 380

Pro Pro Pro Lys Lys Gln Met Ala Lys Asp Lys Val Ser Leu Thr Cys
385                 390                 395                 400

Met Ile Thr Asn Phe Phe Pro Glu Asp Ile Thr Val Glu Trp Gln Trp
                405                 410                 415

Asn Gly Gln Pro Ala Glu Asn Tyr Lys Asn Thr Gln Pro Ile Met Lys
                420                 425                 430

Thr Asp Gly Ser Tyr Phe Val Tyr Ser Lys Leu Asn Val Gln Lys Ser
        435                 440                 445

Asn Trp Glu Ala Gly Asn Thr Phe Thr Cys Ser Val Leu His Glu Gly
        450                 455                 460

Leu His Asn His His Thr Glu Lys Ser Leu Ser His Ser Pro Gly Gly
465                 470                 475                 480

Gly Gly Gly Ser Gly Gly Gly Gly Ser Gly Gly Gly Ser Gly Gly
                485                 490                 495

Gly Gly Ser Glu Ile Val Leu Thr Gln Ser Pro Gly Thr Leu Ser Leu
        500                 505                 510

Ser Pro Gly Glu Arg Ala Thr Leu Ser Cys Arg Ala Ser Gln Ser Val

                    515                    520                    525

Ser Ser Ser Tyr Leu Ala Trp Tyr Gln Gln Lys Pro Gly Gln Ala Pro
    530                    535                    540

Arg Leu Leu Ile Tyr Gly Ala Ser Ser Arg Ala Thr Gly Ile Pro Asp
545                    550                    555                    560

Arg Phe Ser Gly Ser Gly Ser Gly Thr Asp Phe Thr Leu Thr Ile Ser
                565                    570                    575

Arg Leu Glu Pro Glu Asp Phe Ala Val Tyr Tyr Cys Gln Gln Tyr Gly
            580                    585                    590

Ser Ser Pro Leu Thr Phe Gly Gln Gly Thr Lys Val Glu Ile Lys
        595                    600                    605

<210>  74
<211>  614
<212>  PRT
<213>  Artificial Sequence

<220>
<223>  Murine ICOSL linker muIgG1 Fc (DAPG DD) 4GS linker DP47 VL

<400>  74

Glu Thr Glu Val Gly Ala Met Val Gly Ser Asn Val Val Leu Ser Cys
1                    5                    10                    15

Ile Asp Pro His Arg Arg His Phe Asn Leu Ser Gly Leu Tyr Val Tyr
            20                    25                    30

Trp Gln Ile Glu Asn Pro Glu Val Ser Val Thr Tyr Tyr Leu Pro Tyr
        35                    40                    45

Lys Ser Pro Gly Ile Asn Val Asp Ser Ser Tyr Lys Asn Arg Gly His
    50                    55                    60

Leu Ser Leu Asp Ser Met Lys Gln Gly Asn Phe Ser Leu Tyr Leu Lys
65                    70                    75                    80

Asn Val Thr Pro Gln Asp Thr Gln Glu Phe Thr Cys Arg Val Phe Met
                85                    90                    95

Asn Thr Ala Thr Glu Leu Val Lys Ile Leu Glu Glu Val Val Arg Leu
            100                   105                   110

Arg Val Ala Ala Asn Phe Ser Thr Pro Val Ile Ser Thr Ser Asp Ser
            115                   120                   125

139

Ser Asn Pro Gly Gln Glu Arg Thr Tyr Thr Cys Met Ser Lys Asn Gly
    130                 135                 140

Tyr Pro Glu Pro Asn Leu Tyr Trp Ile Asn Thr Thr Asp Asn Ser Leu
    145                 150                 155                 160

Ile Asp Thr Ala Leu Gln Asn Asn Thr Val Tyr Leu Asn Lys Leu Gly
                    165                 170                 175

Leu Tyr Asp Val Ile Ser Thr Leu Arg Leu Pro Trp Thr Ser Arg Gly
                    180                 185                 190

Asp Val Leu Cys Cys Val Glu Asn Val Ala Leu His Gln Asn Ile Thr
                    195                 200                 205

Ser Ile Ser Gln Ala Glu Ser Phe Thr Gly Asn Asn Thr Lys Asn Pro
    210                 215                 220

Gln Glu Thr His Asn Asn Glu Leu Lys Gly Ser Pro Gly Ser Ser Ser
225                 230                 235                 240

Ser Ser Gly Ser Ala Gly Ser Pro Gly Ser Ser Ser Ser Gly Ser
                    245                 250                 255

Ala Asp Gly Cys Lys Pro Cys Ile Cys Thr Val Pro Glu Val Ser Ser
                    260                 265                 270

Val Phe Ile Phe Pro Pro Lys Pro Lys Asp Val Leu Thr Ile Thr Leu
                    275                 280                 285

Thr Pro Lys Val Thr Cys Val Val Val Ala Ile Ser Lys Asp Asp Pro
    290                 295                 300

Glu Val Gln Phe Ser Trp Phe Val Asp Asp Val Glu Val His Thr Ala
305                 310                 315                 320

Gln Thr Lys Pro Arg Glu Glu Gln Ile Asn Ser Thr Phe Arg Ser Val
                    325                 330                 335

Ser Glu Leu Pro Ile Met His Gln Asp Trp Leu Asn Gly Lys Glu Phe
                    340                 345                 350

Lys Cys Arg Val Asn Ser Ala Ala Phe Gly Ala Pro Ile Glu Lys Thr
                    355                 360                 365

Ile Ser Lys Thr Lys Gly Arg Pro Lys Ala Pro Gln Val Tyr Thr Ile

370 375 380

Pro Pro Pro Lys Glu Gln Met Ala Lys Asp Lys Val Ser Leu Thr Cys
385 390 395 400

Met Ile Thr Asn Phe Phe Pro Glu Asp Ile Thr Val Glu Trp Gln Trp
405 410 415

Asn Gly Gln Pro Ala Glu Asn Tyr Asp Asn Thr Gln Pro Ile Met Asp
420 425 430

Thr Asp Gly Ser Tyr Phe Val Tyr Ser Asp Leu Asn Val Gln Lys Ser
435 440 445

Asn Trp Glu Ala Gly Asn Thr Phe Thr Cys Ser Val Leu His Glu Gly
450 455 460

Leu His Asn His His Thr Glu Lys Ser Leu Ser His Ser Pro Gly Gly
465 470 475 480

Gly Gly Gly Ser Gly Gly Gly Gly Ser Gly Gly Gly Gly Ser Gly Gly
485 490 495

Gly Gly Ser Glu Val Gln Leu Leu Glu Ser Gly Gly Gly Leu Val Gln
500 505 510

Pro Gly Gly Ser Leu Arg Leu Ser Cys Ala Ala Ser Gly Phe Thr Phe
515 520 525

Ser Ser Tyr Ala Met Ser Trp Val Arg Gln Ala Pro Gly Lys Gly Leu
530 535 540

Glu Trp Val Ser Ala Ile Ser Gly Ser Gly Gly Ser Thr Tyr Tyr Ala
545 550 555 560

Asp Ser Val Lys Gly Arg Phe Thr Ile Ser Arg Asp Asn Ser Lys Asn
565 570 575

Thr Leu Tyr Leu Gln Met Asn Ser Leu Arg Ala Glu Asp Thr Ala Val
580 585 590

Tyr Tyr Cys Ala Lys Gly Ser Gly Phe Asp Tyr Trp Gly Gln Gly Thr
595 600 605

Leu Val Thr Val Ser Ser
610

```
<210>   75
<211>   445
<212>   PRT
<213>   Artificial Sequence

<220>
<223>   VHCH1(CH1A1A 98/99 2F1)- Fc(KK) DAPG chain

<400>   75
```

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Glu Phe
            20                  25                  30

Gly Met Asn Trp Val Arg Gln Ala Pro Gly Gln Gly Leu Glu Trp Met
        35                  40                  45

Gly Trp Ile Asn Thr Lys Thr Gly Glu Ala Thr Tyr Val Glu Glu Phe
    50                  55                  60

Lys Gly Arg Val Thr Phe Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
            85                  90                  95

Ala Arg Trp Asp Phe Ala Tyr Tyr Val Glu Ala Met Asp Tyr Trp Gly
        100                 105                 110

Gln Gly Thr Thr Val Thr Val Ser Ser Ala Lys Thr Thr Pro Pro Ser
        115                 120                 125

Val Tyr Pro Leu Ala Pro Gly Ser Ala Ala Gln Thr Asn Ser Met Val
        130                 135                 140

Thr Leu Gly Cys Leu Val Lys Gly Tyr Phe Pro Glu Pro Val Thr Val
145                 150                 155                 160

Thr Trp Asn Ser Gly Ser Leu Ser Ser Gly Val His Thr Phe Pro Ala
            165                 170                 175

Val Leu Gln Ser Asp Leu Tyr Thr Leu Ser Ser Ser Val Thr Val Pro
            180                 185                 190

Ser Ser Thr Trp Pro Ser Gln Thr Val Thr Cys Asn Val Ala His Pro
        195                 200                 205

Ala Ser Ser Thr Lys Val Asp Lys Lys Ile Val Pro Arg Asp Cys Gly

210                          215                          220

Cys Lys Pro Cys Ile Cys Thr Val Pro Glu Val Ser Ser Val Phe Ile
225                 230                 235                 240

Phe Pro Pro Lys Pro Lys Asp Val Leu Thr Ile Thr Leu Thr Pro Lys
                245                 250                 255

Val Thr Cys Val Val Val Ala Ile Ser Lys Asp Asp Pro Glu Val Gln
                260                 265                 270

Phe Ser Trp Phe Val Asp Asp Val Glu Val His Thr Ala Gln Thr Lys
                275                 280                 285

Pro Arg Glu Glu Gln Ile Asn Ser Thr Phe Arg Ser Val Ser Glu Leu
    290                 295                 300

Pro Ile Met His Gln Asp Trp Leu Asn Gly Lys Glu Phe Lys Cys Arg
305                 310                 315                 320

Val Asn Ser Ala Ala Phe Gly Ala Pro Ile Glu Lys Thr Ile Ser Lys
                325                 330                 335

Thr Lys Gly Arg Pro Lys Ala Pro Gln Val Tyr Thr Ile Pro Pro Pro
                340                 345                 350

Lys Lys Gln Met Ala Lys Asp Lys Val Ser Leu Thr Cys Met Ile Thr
                355                 360                 365

Asn Phe Phe Pro Glu Asp Ile Thr Val Glu Trp Gln Trp Asn Gly Gln
    370                 375                 380

Pro Ala Glu Asn Tyr Lys Asn Thr Gln Pro Ile Met Lys Thr Asp Gly
385                 390                 395                 400

Ser Tyr Phe Val Tyr Ser Lys Leu Asn Val Gln Lys Ser Asn Trp Glu
                405                 410                 415

Ala Gly Asn Thr Phe Thr Cys Ser Val Leu His Glu Gly Leu His Asn
                420                 425                 430

His His Thr Glu Lys Ser Leu Ser His Ser Pro Gly Lys
        435                 440                 445

<210>  76
<211>  215
<212>  PRT
<213>  Artificial Sequence

<220>
<223>  VLCL (CH1A1A 98/99 2F1) Light chain

<400>  76

Asp Ile Gln Met Thr Gln Ser Pro Ser Ser Leu Ser Ala Ser Val Gly
1               5                   10                  15

Asp Arg Val Thr Ile Thr Cys Lys Ala Ser Ala Ala Val Gly Thr Tyr
            20                  25                  30

Val Ala Trp Tyr Gln Gln Lys Pro Gly Lys Ala Pro Lys Leu Leu Ile
        35                  40                  45

Tyr Ser Ala Ser Tyr Arg Lys Arg Gly Val Pro Ser Arg Phe Ser Gly
        50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys His Gln Tyr Tyr Thr Tyr Pro Leu
            85                  90                  95

Phe Thr Phe Gly Gln Gly Thr Lys Leu Glu Ile Lys Arg Ala Asp Ala
            100                 105                 110

Ala Pro Thr Val Ser Ile Phe Pro Pro Ser Ser Glu Gln Leu Thr Ser
        115                 120                 125

Gly Gly Ala Ser Val Val Cys Phe Leu Asn Asn Phe Tyr Pro Lys Asp
        130                 135                 140

Ile Asn Val Lys Trp Lys Ile Asp Gly Ser Glu Arg Gln Asn Gly Val
145                 150                 155                 160

Leu Asn Ser Trp Thr Asp Gln Asp Ser Lys Asp Ser Thr Tyr Ser Met
            165                 170                 175

Ser Ser Thr Leu Thr Leu Thr Lys Asp Glu Tyr Glu Arg His Asn Ser
        180                 185                 190

Tyr Thr Cys Glu Ala Thr His Lys Thr Ser Thr Ser Pro Ile Val Lys
        195                 200                 205

Ser Phe Asn Arg Asn Glu Cys
    210                 215

<210>  77

<211> 678
<212> PRT
<213> Artificial Sequence

<220>
<223> VHCL VHCH1 (2C11- CH1A1A 98/99 2F1)-
Fc(DD) DAPG chain

<400> 77

```
Glu Val Gln Leu Val Glu Ser Gly Gly Gly Leu Val Gln Pro Gly Lys
1               5                   10                  15

Ser Leu Lys Leu Ser Cys Glu Ala Ser Gly Phe Thr Phe Ser Gly Tyr
            20                  25                  30

Gly Met His Trp Val Arg Gln Ala Pro Gly Arg Gly Leu Glu Ser Val
        35                  40                  45

Ala Tyr Ile Thr Ser Ser Ser Ile Asn Ile Lys Tyr Ala Asp Ala Val
    50                  55                  60

Lys Gly Arg Phe Thr Val Ser Arg Asp Asn Ala Lys Asn Leu Leu Phe
65                  70                  75                  80

Leu Gln Met Asn Ile Leu Lys Ser Glu Asp Thr Ala Met Tyr Tyr Cys
                85                  90                  95

Ala Arg Phe Asp Trp Asp Lys Asn Tyr Trp Gly Gln Gly Thr Met Val
            100                 105                 110

Thr Val Ser Ser Ala Ser Asp Ala Ala Pro Thr Val Ser Ile Phe Pro
        115                 120                 125

Pro Ser Ser Glu Gln Leu Thr Ser Gly Gly Ala Ser Val Val Cys Phe
        130                 135                 140

Leu Asn Asn Phe Tyr Pro Lys Asp Ile Asn Val Lys Trp Lys Ile Asp
145                 150                 155                 160

Gly Ser Glu Arg Gln Asn Gly Val Leu Asn Ser Trp Thr Asp Gln Asp
                165                 170                 175

Ser Lys Asp Ser Thr Tyr Ser Met Ser Ser Thr Leu Thr Leu Thr Lys
            180                 185                 190

Asp Glu Tyr Glu Arg His Asn Ser Tyr Thr Cys Glu Ala Thr His Lys
            195                 200                 205

Thr Ser Thr Ser Pro Ile Val Lys Ser Phe Asn Arg Asn Glu Cys Gly
```

                    210                        215                        220

Gly Gly Gly Ser Gly Gly Gly Gly Ser Gln Val Gln Leu Val Gln Ser
225             230             235             240

Gly Ala Glu Val Lys Lys Pro Gly Ala Ser Val Lys Val Ser Cys Lys
            245             250             255

Ala Ser Gly Tyr Thr Phe Thr Glu Phe Gly Met Asn Trp Val Arg Gln
            260             265             270

Ala Pro Gly Gln Gly Leu Glu Trp Met Gly Trp Ile Asn Thr Lys Thr
            275             280             285

Gly Glu Ala Thr Tyr Val Glu Glu Phe Lys Gly Arg Val Thr Phe Thr
            290             295             300

Thr Asp Thr Ser Thr Ser Thr Ala Tyr Met Glu Leu Arg Ser Leu Arg
305             310             315             320

Ser Asp Asp Thr Ala Val Tyr Tyr Cys Ala Arg Trp Asp Phe Ala Tyr
            325             330             335

Tyr Val Glu Ala Met Asp Tyr Trp Gly Gln Gly Thr Thr Val Thr Val
            340             345             350

Ser Ser Ala Lys Thr Thr Pro Pro Ser Val Tyr Pro Leu Ala Pro Gly
            355             360             365

Ser Ala Ala Gln Thr Asn Ser Met Val Thr Leu Gly Cys Leu Val Lys
            370             375             380

Gly Tyr Phe Pro Glu Pro Val Thr Val Thr Trp Asn Ser Gly Ser Leu
385             390             395             400

Ser Ser Gly Val His Thr Phe Pro Ala Val Leu Gln Ser Asp Leu Tyr
            405             410             415

Thr Leu Ser Ser Ser Val Thr Val Pro Ser Ser Thr Trp Pro Ser Gln
            420             425             430

Thr Val Thr Cys Asn Val Ala His Pro Ala Ser Ser Thr Lys Val Asp
            435             440             445

Lys Lys Ile Val Pro Arg Asp Cys Gly Cys Lys Pro Cys Ile Cys Thr
450             455             460

Val Pro Glu Val Ser Ser Val Phe Ile Phe Pro Pro Lys Pro Lys Asp
465                 470                 475                 480

Val Leu Thr Ile Thr Leu Thr Pro Lys Val Thr Cys Val Val Val Ala
                485                 490                 495

Ile Ser Lys Asp Asp Pro Glu Val Gln Phe Ser Trp Phe Val Asp Asp
            500                 505                 510

Val Glu Val His Thr Ala Gln Thr Lys Pro Arg Glu Glu Gln Ile Asn
            515                 520                 525

Ser Thr Phe Arg Ser Val Ser Glu Leu Pro Ile Met His Gln Asp Trp
        530                 535                 540

Leu Asn Gly Lys Glu Phe Lys Cys Arg Val Asn Ser Ala Ala Phe Gly
545                 550                 555                 560

Ala Pro Ile Glu Lys Thr Ile Ser Lys Thr Lys Gly Arg Pro Lys Ala
                565                 570                 575

Pro Gln Val Tyr Thr Ile Pro Pro Lys Glu Gln Met Ala Lys Asp
                580                 585                 590

Lys Val Ser Leu Thr Cys Met Ile Thr Asn Phe Phe Pro Glu Asp Ile
                595                 600                 605

Thr Val Glu Trp Gln Trp Asn Gly Gln Pro Ala Glu Asn Tyr Asp Asn
        610                 615                 620

Thr Gln Pro Ile Met Asp Thr Asp Gly Ser Tyr Phe Val Tyr Ser Asp
625                 630                 635                 640

Leu Asn Val Gln Lys Ser Asn Trp Glu Ala Gly Asn Thr Phe Thr Cys
                645                 650                 655

Ser Val Leu His Glu Gly Leu His Asn His His Thr Glu Lys Ser Leu
            660                 665                 670

Ser His Ser Pro Gly Lys
            675

<210> 78
<211> 211
<212> PRT
<213> Artificial Sequence

<220>
<223> VLCH1 (2C11) Light chain

<400> 78

Asp Ile Gln Met Thr Gln Ser Pro Ser Ser Leu Pro Ala Ser Leu Gly
1               5                   10                  15

Asp Arg Val Thr Ile Asn Cys Gln Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Asn Lys Leu Ala Asp Gly Val Pro Ser Arg Phe Ser Gly
    50                  55                  60

Ser Gly Ser Gly Arg Asp Ser Ser Phe Thr Ile Ser Ser Leu Glu Ser
65                  70                  75                  80

Glu Asp Ile Gly Ser Tyr Tyr Cys Gln Gln Tyr Tyr Asn Tyr Pro Trp
            85                  90                  95

Thr Phe Gly Pro Gly Thr Lys Leu Glu Ile Lys Ser Ser Ala Lys Thr
        100                 105                 110

Thr Pro Pro Ser Val Tyr Pro Leu Ala Pro Gly Ser Ala Ala Gln Thr
        115                 120                 125

Asn Ser Met Val Thr Leu Gly Cys Leu Val Lys Gly Tyr Phe Pro Glu
    130                 135                 140

Pro Val Thr Val Thr Trp Asn Ser Gly Ser Leu Ser Ser Gly Val His
145                 150                 155                 160

Thr Phe Pro Ala Val Leu Gln Ser Asp Leu Tyr Thr Leu Ser Ser Ser
            165                 170                 175

Val Thr Val Pro Ser Ser Thr Trp Pro Ser Gln Thr Val Thr Cys Asn
        180                 185                 190

Val Ala His Pro Ala Ser Ser Thr Lys Val Asp Lys Lys Ile Val Pro
    195                 200                 205

Arg Asp Cys
    210

<210> 79
<211> 760
<212> PRT
<213> Homo sapiens

<400> 79

```
Met Lys Thr Trp Val Lys Ile Val Phe Gly Val Ala Thr Ser Ala Val
1               5                   10                  15

Leu Ala Leu Leu Val Met Cys Ile Val Leu Arg Pro Ser Arg Val His
            20                  25                  30

Asn Ser Glu Glu Asn Thr Met Arg Ala Leu Thr Leu Lys Asp Ile Leu
        35                  40                  45

Asn Gly Thr Phe Ser Tyr Lys Thr Phe Phe Pro Asn Trp Ile Ser Gly
        50                  55                  60

Gln Glu Tyr Leu His Gln Ser Ala Asp Asn Asn Ile Val Leu Tyr Asn
65                  70                  75                  80

Ile Glu Thr Gly Gln Ser Tyr Thr Ile Leu Ser Asn Arg Thr Met Lys
                85                  90                  95

Ser Val Asn Ala Ser Asn Tyr Gly Leu Ser Pro Asp Arg Gln Phe Val
            100                 105                 110

Tyr Leu Glu Ser Asp Tyr Ser Lys Leu Trp Arg Tyr Ser Tyr Thr Ala
        115                 120                 125

Thr Tyr Tyr Ile Tyr Asp Leu Ser Asn Gly Glu Phe Val Arg Gly Asn
    130                 135                 140

Glu Leu Pro Arg Pro Ile Gln Tyr Leu Cys Trp Ser Pro Val Gly Ser
145                 150                 155                 160

Lys Leu Ala Tyr Val Tyr Gln Asn Asn Ile Tyr Leu Lys Gln Arg Pro
            165                 170                 175

Gly Asp Pro Pro Phe Gln Ile Thr Phe Asn Gly Arg Glu Asn Lys Ile
        180                 185                 190

Phe Asn Gly Ile Pro Asp Trp Val Tyr Glu Glu Glu Met Leu Ala Thr
        195                 200                 205

Lys Tyr Ala Leu Trp Trp Ser Pro Asn Gly Lys Phe Leu Ala Tyr Ala
    210                 215                 220

Glu Phe Asn Asp Thr Asp Ile Pro Val Ile Ala Tyr Ser Tyr Tyr Gly
225                 230                 235                 240
```

```
Asp Glu Gln Tyr Pro Arg Thr Ile Asn Ile Pro Tyr Pro Lys Ala Gly
            245             250             255

Ala Lys Asn Pro Val Val Arg Ile Phe Ile Ile Asp Thr Thr Tyr Pro
            260             265             270

Ala Tyr Val Gly Pro Gln Glu Val Pro Val Pro Ala Met Ile Ala Ser
            275             280             285

Ser Asp Tyr Tyr Phe Ser Trp Leu Thr Trp Val Thr Asp Glu Arg Val
            290             295             300

Cys Leu Gln Trp Leu Lys Arg Val Gln Asn Val Ser Val Leu Ser Ile
305             310             315             320

Cys Asp Phe Arg Glu Asp Trp Gln Thr Trp Asp Cys Pro Lys Thr Gln
            325             330             335

Glu His Ile Glu Glu Ser Arg Thr Gly Trp Ala Gly Gly Phe Phe Val
            340             345             350

Ser Thr Pro Val Phe Ser Tyr Asp Ala Ile Ser Tyr Tyr Lys Ile Phe
            355             360             365

Ser Asp Lys Asp Gly Tyr Lys His Ile His Tyr Ile Lys Asp Thr Val
            370             375             380

Glu Asn Ala Ile Gln Ile Thr Ser Gly Lys Trp Glu Ala Ile Asn Ile
385             390             395             400

Phe Arg Val Thr Gln Asp Ser Leu Phe Tyr Ser Ser Asn Glu Phe Glu
            405             410             415

Glu Tyr Pro Gly Arg Arg Asn Ile Tyr Arg Ile Ser Ile Gly Ser Tyr
            420             425             430

Pro Pro Ser Lys Lys Cys Val Thr Cys His Leu Arg Lys Glu Arg Cys
            435             440             445

Gln Tyr Tyr Thr Ala Ser Phe Ser Asp Tyr Ala Lys Tyr Tyr Ala Leu
            450             455             460

Val Cys Tyr Gly Pro Gly Ile Pro Ile Ser Thr Leu His Asp Gly Arg
465             470             475             480

Thr Asp Gln Glu Ile Lys Ile Leu Glu Glu Asn Lys Glu Leu Glu Asn
            485             490             495
```

Ala Leu Lys Asn Ile Gln Leu Pro Lys Glu Glu Ile Lys Lys Leu Glu
500 505 510

Val Asp Glu Ile Thr Leu Trp Tyr Lys Met Ile Leu Pro Pro Gln Phe
515 520 525

Asp Arg Ser Lys Lys Tyr Pro Leu Leu Ile Gln Val Tyr Gly Gly Pro
530 535 540

Cys Ser Gln Ser Val Arg Ser Val Phe Ala Val Asn Trp Ile Ser Tyr
545 550 555 560

Leu Ala Ser Lys Glu Gly Met Val Ile Ala Leu Val Asp Gly Arg Gly
565 570 575

Thr Ala Phe Gln Gly Asp Lys Leu Leu Tyr Ala Val Tyr Arg Lys Leu
580 585 590

Gly Val Tyr Glu Val Glu Asp Gln Ile Thr Ala Val Arg Lys Phe Ile
595 600 605

Glu Met Gly Phe Ile Asp Glu Lys Arg Ile Ala Ile Trp Gly Trp Ser
610 615 620

Tyr Gly Gly Tyr Val Ser Ser Leu Ala Leu Ala Ser Gly Thr Gly Leu
625 630 635 640

Phe Lys Cys Gly Ile Ala Val Ala Pro Val Ser Ser Trp Glu Tyr Tyr
645 650 655

Ala Ser Val Tyr Thr Glu Arg Phe Met Gly Leu Pro Thr Lys Asp Asp
660 665 670

Asn Leu Glu His Tyr Lys Asn Ser Thr Val Met Ala Arg Ala Glu Tyr
675 680 685

Phe Arg Asn Val Asp Tyr Leu Leu Ile His Gly Thr Ala Asp Asp Asn
690 695 700

Val His Phe Gln Asn Ser Ala Gln Ile Ala Lys Ala Leu Val Asn Ala
705 710 715 720

Gln Val Asp Phe Gln Ala Met Trp Tyr Ser Asp Gln Asn His Gly Leu
725 730 735

Ser Gly Leu Ser Thr Asn His Leu Tyr Thr His Met Thr His Phe Leu
740 745 750

151

```
Lys Gln Cys Phe Ser Leu Ser Asp
        755                 760


<210>  80
<211>  748
<212>  PRT
<213>  Artificial Sequence

<220>
<223>  hu FAP ectodomain+poly-lys-tag+his6-tag

<400>  80

Arg Pro Ser Arg Val His Asn Ser Glu Glu Asn Thr Met Arg Ala Leu
1               5               10                  15

Thr Leu Lys Asp Ile Leu Asn Gly Thr Phe Ser Tyr Lys Thr Phe Phe
            20                  25                  30

Pro Asn Trp Ile Ser Gly Gln Glu Tyr Leu His Gln Ser Ala Asp Asn
            35                  40                  45

Asn Ile Val Leu Tyr Asn Ile Glu Thr Gly Gln Ser Tyr Thr Ile Leu
        50                  55                  60

Ser Asn Arg Thr Met Lys Ser Val Asn Ala Ser Asn Tyr Gly Leu Ser
65                  70                  75                  80

Pro Asp Arg Gln Phe Val Tyr Leu Glu Ser Asp Tyr Ser Lys Leu Trp
                85                  90                  95

Arg Tyr Ser Tyr Thr Ala Thr Tyr Tyr Ile Tyr Asp Leu Ser Asn Gly
            100                 105                 110

Glu Phe Val Arg Gly Asn Glu Leu Pro Arg Pro Ile Gln Tyr Leu Cys
            115                 120                 125

Trp Ser Pro Val Gly Ser Lys Leu Ala Tyr Val Tyr Gln Asn Asn Ile
        130                 135                 140

Tyr Leu Lys Gln Arg Pro Gly Asp Pro Pro Phe Gln Ile Thr Phe Asn
145                 150                 155                 160

Gly Arg Glu Asn Lys Ile Phe Asn Gly Ile Pro Asp Trp Val Tyr Glu
                165                 170                 175

Glu Glu Met Leu Ala Thr Lys Tyr Ala Leu Trp Trp Ser Pro Asn Gly
            180                 185                 190
```

152

Lys Phe Leu Ala Tyr Ala Glu Phe Asn Asp Thr Asp Ile Pro Val Ile
195                         200                     205

Ala Tyr Ser Tyr Tyr Gly Asp Glu Gln Tyr Pro Arg Thr Ile Asn Ile
210                         215                     220

Pro Tyr Pro Lys Ala Gly Ala Lys Asn Pro Val Val Arg Ile Phe Ile
225                 230                     235                 240

Ile Asp Thr Thr Tyr Pro Ala Tyr Val Gly Pro Gln Glu Val Pro Val
                245                     250                     255

Pro Ala Met Ile Ala Ser Ser Asp Tyr Tyr Phe Ser Trp Leu Thr Trp
            260                     265                     270

Val Thr Asp Glu Arg Val Cys Leu Gln Trp Leu Lys Arg Val Gln Asn
            275                     280                     285

Val Ser Val Leu Ser Ile Cys Asp Phe Arg Glu Asp Trp Gln Thr Trp
290                         295                     300

Asp Cys Pro Lys Thr Gln Glu His Ile Glu Glu Ser Arg Thr Gly Trp
305                 310                     315                 320

Ala Gly Gly Phe Phe Val Ser Thr Pro Val Phe Ser Tyr Asp Ala Ile
                325                     330                     335

Ser Tyr Tyr Lys Ile Phe Ser Asp Lys Asp Gly Tyr Lys His Ile His
            340                     345                     350

Tyr Ile Lys Asp Thr Val Glu Asn Ala Ile Gln Ile Thr Ser Gly Lys
            355                     360                     365

Trp Glu Ala Ile Asn Ile Phe Arg Val Thr Gln Asp Ser Leu Phe Tyr
    370                     375                     380

Ser Ser Asn Glu Phe Glu Glu Tyr Pro Gly Arg Arg Asn Ile Tyr Arg
385                     390                     395                 400

Ile Ser Ile Gly Ser Tyr Pro Pro Ser Lys Lys Cys Val Thr Cys His
                405                     410                     415

Leu Arg Lys Glu Arg Cys Gln Tyr Tyr Thr Ala Ser Phe Ser Asp Tyr
            420                     425                     430

Ala Lys Tyr Tyr Ala Leu Val Cys Tyr Gly Pro Gly Ile Pro Ile Ser
            435                     440                     445

Thr Leu His Asp Gly Arg Thr Asp Gln Glu Ile Lys Ile Leu Glu Glu
450                 455                 460

Asn Lys Glu Leu Glu Asn Ala Leu Lys Asn Ile Gln Leu Pro Lys Glu
465                 470                 475                 480

Glu Ile Lys Lys Leu Glu Val Asp Glu Ile Thr Leu Trp Tyr Lys Met
                485                 490                 495

Ile Leu Pro Pro Gln Phe Asp Arg Ser Lys Lys Tyr Pro Leu Leu Ile
                500                 505                 510

Gln Val Tyr Gly Gly Pro Cys Ser Gln Ser Val Arg Ser Val Phe Ala
                515                 520                 525

Val Asn Trp Ile Ser Tyr Leu Ala Ser Lys Glu Gly Met Val Ile Ala
530                 535                 540

Leu Val Asp Gly Arg Gly Thr Ala Phe Gln Gly Asp Lys Leu Leu Tyr
545                 550                 555                 560

Ala Val Tyr Arg Lys Leu Gly Val Tyr Glu Val Glu Asp Gln Ile Thr
                565                 570                 575

Ala Val Arg Lys Phe Ile Glu Met Gly Phe Ile Asp Glu Lys Arg Ile
                580                 585                 590

Ala Ile Trp Gly Trp Ser Tyr Gly Gly Tyr Val Ser Ser Leu Ala Leu
                595                 600                 605

Ala Ser Gly Thr Gly Leu Phe Lys Cys Gly Ile Ala Val Ala Pro Val
610                 615                 620

Ser Ser Trp Glu Tyr Tyr Ala Ser Val Tyr Thr Glu Arg Phe Met Gly
625                 630                 635                 640

Leu Pro Thr Lys Asp Asp Asn Leu Glu His Tyr Lys Asn Ser Thr Val
                645                 650                 655

Met Ala Arg Ala Glu Tyr Phe Arg Asn Val Asp Tyr Leu Leu Ile His
                660                 665                 670

Gly Thr Ala Asp Asp Asn Val His Phe Gln Asn Ser Ala Gln Ile Ala
                675                 680                 685

Lys Ala Leu Val Asn Ala Gln Val Asp Phe Gln Ala Met Trp Tyr Ser

```
                690                      695                     700

        Asp Gln Asn His Gly Leu Ser Gly Leu Ser Thr Asn His Leu Tyr Thr
        705             710             715                 720

        His Met Thr His Phe Leu Lys Gln Cys Phe Ser Leu Ser Asp Gly Lys
                    725             730                 735

        Lys Lys Lys Lys Lys Gly His His His His His His
                    740             745


        <210>   81
        <211>   761
        <212>   PRT
        <213>   murine

        <400>   81

        Met Lys Thr Trp Leu Lys Thr Val Phe Gly Val Thr Thr Leu Ala Ala
        1               5               10                  15

        Leu Ala Leu Val Val Ile Cys Ile Val Leu Arg Pro Ser Arg Val Tyr
                    20              25                  30

        Lys Pro Glu Gly Asn Thr Lys Arg Ala Leu Thr Leu Lys Asp Ile Leu
                    35              40                  45

        Asn Gly Thr Phe Ser Tyr Lys Thr Tyr Phe Pro Asn Trp Ile Ser Glu
                    50              55                  60

        Gln Glu Tyr Leu His Gln Ser Glu Asp Asp Asn Ile Val Phe Tyr Asn
        65                  70                  75                  80

        Ile Glu Thr Arg Glu Ser Tyr Ile Ile Leu Ser Asn Ser Thr Met Lys
                        85              90                  95

        Ser Val Asn Ala Thr Asp Tyr Gly Leu Ser Pro Asp Arg Gln Phe Val
                    100             105                 110

        Tyr Leu Glu Ser Asp Tyr Ser Lys Leu Trp Arg Tyr Ser Tyr Thr Ala
                    115             120                 125

        Thr Tyr Tyr Ile Tyr Asp Leu Gln Asn Gly Glu Phe Val Arg Gly Tyr
                    130             135                 140

        Glu Leu Pro Arg Pro Ile Gln Tyr Leu Cys Trp Ser Pro Val Gly Ser
        145                 150                 155                 160

        Lys Leu Ala Tyr Val Tyr Gln Asn Asn Ile Tyr Leu Lys Gln Arg Pro
```

|      |     |     | 165 |     |     |     | 170 |     |     |     | 175 |     |     |     |     |
|------|-----|-----|-----|-----|-----|-----|-----|-----|-----|-----|-----|-----|-----|-----|-----|

Gly Asp Pro Pro Phe Gln Ile Thr Tyr Thr Gly Arg Glu Asn Arg Ile
        180             185             190

Phe Asn Gly Ile Pro Asp Trp Val Tyr Glu Glu Glu Met Leu Ala Thr
        195             200             205

Lys Tyr Ala Leu Trp Trp Ser Pro Asp Gly Lys Phe Leu Ala Tyr Val
        210             215             220

Glu Phe Asn Asp Ser Asp Ile Pro Ile Ile Ala Tyr Ser Tyr Tyr Gly
225             230             235             240

Asp Gly Gln Tyr Pro Arg Thr Ile Asn Ile Pro Tyr Pro Lys Ala Gly
            245             250             255

Ala Lys Asn Pro Val Val Arg Val Phe Ile Val Asp Thr Thr Tyr Pro
        260             265             270

His His Val Gly Pro Met Glu Val Pro Val Pro Glu Met Ile Ala Ser
        275             280             285

Ser Asp Tyr Tyr Phe Ser Trp Leu Thr Trp Val Ser Ser Glu Arg Val
290             295             300

Cys Leu Gln Trp Leu Lys Arg Val Gln Asn Val Ser Val Leu Ser Ile
305             310             315             320

Cys Asp Phe Arg Glu Asp Trp His Ala Trp Glu Cys Pro Lys Asn Gln
            325             330             335

Glu His Val Glu Glu Ser Arg Thr Gly Trp Ala Gly Gly Phe Phe Val
        340             345             350

Ser Thr Pro Ala Phe Ser Gln Asp Ala Thr Ser Tyr Tyr Lys Ile Phe
        355             360             365

Ser Asp Lys Asp Gly Tyr Lys His Ile His Tyr Ile Lys Asp Thr Val
370             375             380

Glu Asn Ala Ile Gln Ile Thr Ser Gly Lys Trp Glu Ala Ile Tyr Ile
385             390             395             400

Phe Arg Val Thr Gln Asp Ser Leu Phe Tyr Ser Ser Asn Glu Phe Glu
            405             410             415

```
Gly Tyr Pro Gly Arg Arg Asn Ile Tyr Arg Ile Ser Ile Gly Asn Ser
            420                 425             430

Pro Pro Ser Lys Lys Cys Val Thr Cys His Leu Arg Lys Glu Arg Cys
            435                 440             445

Gln Tyr Tyr Thr Ala Ser Phe Ser Tyr Lys Ala Lys Tyr Tyr Ala Leu
            450                 455             460

Val Cys Tyr Gly Pro Gly Leu Pro Ile Ser Thr Leu His Asp Gly Arg
465                 470                 475                 480

Thr Asp Gln Glu Ile Gln Val Leu Glu Glu Asn Lys Glu Leu Glu Asn
                485                 490                 495

Ser Leu Arg Asn Ile Gln Leu Pro Lys Val Glu Ile Lys Lys Leu Lys
            500                 505             510

Asp Gly Gly Leu Thr Phe Trp Tyr Lys Met Ile Leu Pro Pro Gln Phe
            515                 520             525

Asp Arg Ser Lys Lys Tyr Pro Leu Leu Ile Gln Val Tyr Gly Gly Pro
            530                 535                 540

Cys Ser Gln Ser Val Lys Ser Val Phe Ala Val Asn Trp Ile Thr Tyr
545                 550                 555                 560

Leu Ala Ser Lys Glu Gly Ile Val Ile Ala Leu Val Asp Gly Arg Gly
                565                 570                 575

Thr Ala Phe Gln Gly Asp Lys Phe Leu His Ala Val Tyr Arg Lys Leu
            580                 585                 590

Gly Val Tyr Glu Val Glu Asp Gln Leu Thr Ala Val Arg Lys Phe Ile
            595                 600                 605

Glu Met Gly Phe Ile Asp Glu Glu Arg Ile Ala Ile Trp Gly Trp Ser
            610                 615                 620

Tyr Gly Gly Tyr Val Ser Ser Leu Ala Leu Ala Ser Gly Thr Gly Leu
625                 630                 635                 640

Phe Lys Cys Gly Ile Ala Val Ala Pro Val Ser Ser Trp Glu Tyr Tyr
            645                 650                 655

Ala Ser Ile Tyr Ser Glu Arg Phe Met Gly Leu Pro Thr Lys Asp Asp
            660                 665                 670
```

```
Asn Leu Glu His Tyr Lys Asn Ser Thr Val Met Ala Arg Ala Glu Tyr
        675             680                 685

Phe Arg Asn Val Asp Tyr Leu Leu Ile His Gly Thr Ala Asp Asp Asn
        690             695                 700

Val His Phe Gln Asn Ser Ala Gln Ile Ala Lys Ala Leu Val Asn Ala
705             710             715                 720

Gln Val Asp Phe Gln Ala Met Trp Tyr Ser Asp Gln Asn His Gly Ile
            725             730                 735

Ser Ser Gly Arg Ser Gln Asn His Leu Tyr Thr His Met Thr His Phe
            740             745             750

Leu Lys Gln Cys Phe Ser Leu Ser Asp
            755             760


<210>   82
<211>   749
<212>   PRT
<213>   Artificial Sequence

<220>
<223>   Murine FAP ectodomain+poly-lys-tag+his6-tag

<400>   82

Arg Pro Ser Arg Val Tyr Lys Pro Glu Gly Asn Thr Lys Arg Ala Leu
1               5               10                  15

Thr Leu Lys Asp Ile Leu Asn Gly Thr Phe Ser Tyr Lys Thr Tyr Phe
            20              25                  30

Pro Asn Trp Ile Ser Glu Gln Glu Tyr Leu His Gln Ser Glu Asp Asp
            35              40                  45

Asn Ile Val Phe Tyr Asn Ile Glu Thr Arg Glu Ser Tyr Ile Ile Leu
        50              55                  60

Ser Asn Ser Thr Met Lys Ser Val Asn Ala Thr Asp Tyr Gly Leu Ser
65              70              75                  80

Pro Asp Arg Gln Phe Val Tyr Leu Glu Ser Asp Tyr Ser Lys Leu Trp
            85              90                  95

Arg Tyr Ser Tyr Thr Ala Thr Tyr Tyr Ile Tyr Asp Leu Gln Asn Gly
            100             105                 110
```

Glu Phe Val Arg Gly Tyr Glu Leu Pro Arg Pro Ile Gln Tyr Leu Cys
    115                 120             125

Trp Ser Pro Val Gly Ser Lys Leu Ala Tyr Val Tyr Gln Asn Asn Ile
    130                 135             140

Tyr Leu Lys Gln Arg Pro Gly Asp Pro Pro Phe Gln Ile Thr Tyr Thr
145             150             155             160

Gly Arg Glu Asn Arg Ile Phe Asn Gly Ile Pro Asp Trp Val Tyr Glu
                165             170             175

Glu Glu Met Leu Ala Thr Lys Tyr Ala Leu Trp Trp Ser Pro Asp Gly
        180             185             190

Lys Phe Leu Ala Tyr Val Glu Phe Asn Asp Ser Asp Ile Pro Ile Ile
        195             200             205

Ala Tyr Ser Tyr Tyr Gly Asp Gly Gln Tyr Pro Arg Thr Ile Asn Ile
    210             215             220

Pro Tyr Pro Lys Ala Gly Ala Lys Asn Pro Val Val Arg Val Phe Ile
225             230             235             240

Val Asp Thr Thr Tyr Pro His His Val Gly Pro Met Glu Val Pro Val
            245             250             255

Pro Glu Met Ile Ala Ser Ser Asp Tyr Tyr Phe Ser Trp Leu Thr Trp
            260             265             270

Val Ser Ser Glu Arg Val Cys Leu Gln Trp Leu Lys Arg Val Gln Asn
    275             280             285

Val Ser Val Leu Ser Ile Cys Asp Phe Arg Glu Asp Trp His Ala Trp
    290             295             300

Glu Cys Pro Lys Asn Gln Glu His Val Glu Glu Ser Arg Thr Gly Trp
305             310             315             320

Ala Gly Gly Phe Phe Val Ser Thr Pro Ala Phe Ser Gln Asp Ala Thr
            325             330             335

Ser Tyr Tyr Lys Ile Phe Ser Asp Lys Asp Gly Tyr Lys His Ile His
        340             345             350

Tyr Ile Lys Asp Thr Val Glu Asn Ala Ile Gln Ile Thr Ser Gly Lys
    355             360             365

```
Trp Glu Ala Ile Tyr Ile Phe Arg Val Thr Gln Asp Ser Leu Phe Tyr
    370             375             380

Ser Ser Asn Glu Phe Glu Gly Tyr Pro Gly Arg Arg Asn Ile Tyr Arg
385             390             395             400

Ile Ser Ile Gly Asn Ser Pro Pro Ser Lys Lys Cys Val Thr Cys His
            405             410             415

Leu Arg Lys Glu Arg Cys Gln Tyr Tyr Thr Ala Ser Phe Ser Tyr Lys
            420             425             430

Ala Lys Tyr Tyr Ala Leu Val Cys Tyr Gly Pro Gly Leu Pro Ile Ser
            435             440             445

Thr Leu His Asp Gly Arg Thr Asp Gln Glu Ile Gln Val Leu Glu Glu
    450             455             460

Asn Lys Glu Leu Glu Asn Ser Leu Arg Asn Ile Gln Leu Pro Lys Val
465             470             475             480

Glu Ile Lys Lys Leu Lys Asp Gly Gly Leu Thr Phe Trp Tyr Lys Met
            485             490             495

Ile Leu Pro Pro Gln Phe Asp Arg Ser Lys Lys Tyr Pro Leu Leu Ile
            500             505             510

Gln Val Tyr Gly Gly Pro Cys Ser Gln Ser Val Lys Ser Val Phe Ala
            515             520             525

Val Asn Trp Ile Thr Tyr Leu Ala Ser Lys Glu Gly Ile Val Ile Ala
    530             535             540

Leu Val Asp Gly Arg Gly Thr Ala Phe Gln Gly Asp Lys Phe Leu His
545             550             555             560

Ala Val Tyr Arg Lys Leu Gly Val Tyr Glu Val Glu Asp Gln Leu Thr
            565             570             575

Ala Val Arg Lys Phe Ile Glu Met Gly Phe Ile Asp Glu Glu Arg Ile
            580             585             590

Ala Ile Trp Gly Trp Ser Tyr Gly Gly Tyr Val Ser Ser Leu Ala Leu
            595             600             605

Ala Ser Gly Thr Gly Leu Phe Lys Cys Gly Ile Ala Val Ala Pro Val
610             615             620
```

```
Ser Ser Trp Glu Tyr Tyr Ala Ser Ile Tyr Ser Glu Arg Phe Met Gly
625             630             635             640

Leu Pro Thr Lys Asp Asp Asn Leu Glu His Tyr Lys Asn Ser Thr Val
                645             650             655

Met Ala Arg Ala Glu Tyr Phe Arg Asn Val Asp Tyr Leu Leu Ile His
            660             665             670

Gly Thr Ala Asp Asp Asn Val His Phe Gln Asn Ser Ala Gln Ile Ala
            675             680             685

Lys Ala Leu Val Asn Ala Gln Val Asp Phe Gln Ala Met Trp Tyr Ser
    690             695             700

Asp Gln Asn His Gly Ile Leu Ser Gly Arg Ser Gln Asn His Leu Tyr
705             710             715             720

Thr His Met Thr His Phe Leu Lys Gln Cys Phe Ser Leu Ser Asp Gly
            725             730             735

Lys Lys Lys Lys Lys Lys Gly His His His His His His
        740             745
```

<210> 83
<211> 748
<212> PRT
<213> Artificial Sequence

<220>
<223> Cynomolgus FAP ectodomain+poly-lys-tag+his6-tag

<400> 83

```
Arg Pro Pro Arg Val His Asn Ser Glu Glu Asn Thr Met Arg Ala Leu
1               5               10              15

Thr Leu Lys Asp Ile Leu Asn Gly Thr Phe Ser Tyr Lys Thr Phe Phe
            20              25              30

Pro Asn Trp Ile Ser Gly Gln Glu Tyr Leu His Gln Ser Ala Asp Asn
        35              40              45

Asn Ile Val Leu Tyr Asn Ile Glu Thr Gly Gln Ser Tyr Thr Ile Leu
    50              55              60

Ser Asn Arg Thr Met Lys Ser Val Asn Ala Ser Asn Tyr Gly Leu Ser
65              70              75              80
```

Pro Asp Arg Gln Phe Val Tyr Leu Glu Ser Asp Tyr Ser Lys Leu Trp
85                          90                  95

Arg Tyr Ser Tyr Thr Ala Thr Tyr Tyr Ile Tyr Asp Leu Ser Asn Gly
100                        105                  110

Glu Phe Val Arg Gly Asn Glu Leu Pro Arg Pro Ile Gln Tyr Leu Cys
115                        120                  125

Trp Ser Pro Val Gly Ser Lys Leu Ala Tyr Val Tyr Gln Asn Asn Ile
130                        135                  140

Tyr Leu Lys Gln Arg Pro Gly Asp Pro Pro Phe Gln Ile Thr Phe Asn
145                  150                  155                  160

Gly Arg Glu Asn Lys Ile Phe Asn Gly Ile Pro Asp Trp Val Tyr Glu
165                        170                  175

Glu Glu Met Leu Ala Thr Lys Tyr Ala Leu Trp Trp Ser Pro Asn Gly
180                        185                  190

Lys Phe Leu Ala Tyr Ala Glu Phe Asn Asp Thr Asp Ile Pro Val Ile
195                        200                  205

Ala Tyr Ser Tyr Tyr Gly Asp Glu Gln Tyr Pro Arg Thr Ile Asn Ile
210                  215                  220

Pro Tyr Pro Lys Ala Gly Ala Lys Asn Pro Phe Val Arg Ile Phe Ile
225                  230                  235                  240

Ile Asp Thr Thr Tyr Pro Ala Tyr Val Gly Pro Gln Glu Val Pro Val
245                  250                  255

Pro Ala Met Ile Ala Ser Ser Asp Tyr Tyr Phe Ser Trp Leu Thr Trp
260                  265                  270

Val Thr Asp Glu Arg Val Cys Leu Gln Trp Leu Lys Arg Val Gln Asn
275                  280                  285

Val Ser Val Leu Ser Ile Cys Asp Phe Arg Glu Asp Trp Gln Thr Trp
290                  295                  300

Asp Cys Pro Lys Thr Gln Glu His Ile Glu Glu Ser Arg Thr Gly Trp
305                  310                  315                  320

Ala Gly Gly Phe Phe Val Ser Thr Pro Val Phe Ser Tyr Asp Ala Ile
325                  330                  335

```
Ser Tyr Tyr Lys Ile Phe Ser Asp Lys Asp Gly Tyr Lys His Ile His
        340             345             350

Tyr Ile Lys Asp Thr Val Glu Asn Ala Ile Gln Ile Thr Ser Gly Lys
        355             360             365

Trp Glu Ala Ile Asn Ile Phe Arg Val Thr Gln Asp Ser Leu Phe Tyr
    370             375             380

Ser Ser Asn Glu Phe Glu Asp Tyr Pro Gly Arg Arg Asn Ile Tyr Arg
385             390             395             400

Ile Ser Ile Gly Ser Tyr Pro Pro Ser Lys Lys Cys Val Thr Cys His
            405             410             415

Leu Arg Lys Glu Arg Cys Gln Tyr Tyr Thr Ala Ser Phe Ser Asp Tyr
        420             425             430

Ala Lys Tyr Tyr Ala Leu Val Cys Tyr Gly Pro Gly Ile Pro Ile Ser
        435             440             445

Thr Leu His Asp Gly Arg Thr Asp Gln Glu Ile Lys Ile Leu Glu Glu
    450             455             460

Asn Lys Glu Leu Glu Asn Ala Leu Lys Asn Ile Gln Leu Pro Lys Glu
465             470             475             480

Glu Ile Lys Lys Leu Glu Val Asp Glu Ile Thr Leu Trp Tyr Lys Met
            485             490             495

Ile Leu Pro Pro Gln Phe Asp Arg Ser Lys Lys Tyr Pro Leu Leu Ile
        500             505             510

Gln Val Tyr Gly Gly Pro Cys Ser Gln Ser Val Arg Ser Val Phe Ala
        515             520             525

Val Asn Trp Ile Ser Tyr Leu Ala Ser Lys Glu Gly Met Val Ile Ala
    530             535             540

Leu Val Asp Gly Arg Gly Thr Ala Phe Gln Gly Asp Lys Leu Leu Tyr
545             550             555             560

Ala Val Tyr Arg Lys Leu Gly Val Tyr Glu Val Glu Asp Gln Ile Thr
            565             570             575

Ala Val Arg Lys Phe Ile Glu Met Gly Phe Ile Asp Glu Lys Arg Ile
```

```
              580                    585                         590

Ala Ile Trp Gly Trp Ser Tyr Gly Gly Tyr Val Ser Ser Leu Ala Leu
        595                 600             605

Ala Ser Gly Thr Gly Leu Phe Lys Cys Gly Ile Ala Val Ala Pro Val
    610                 615             620

Ser Ser Trp Glu Tyr Tyr Ala Ser Val Tyr Thr Glu Arg Phe Met Gly
625                 630             635                 640

Leu Pro Thr Lys Asp Asp Asn Leu Glu His Tyr Lys Asn Ser Thr Val
            645             650                 655

Met Ala Arg Ala Glu Tyr Phe Arg Asn Val Asp Tyr Leu Leu Ile His
            660             665                 670

Gly Thr Ala Asp Asp Asn Val His Phe Gln Asn Ser Ala Gln Ile Ala
        675             680                 685

Lys Ala Leu Val Asn Ala Gln Val Asp Phe Gln Ala Met Trp Tyr Ser
    690             695                 700

Asp Gln Asn His Gly Leu Ser Gly Leu Ser Thr Asn His Leu Tyr Thr
705             710                 715                 720

His Met Thr His Phe Leu Lys Gln Cys Phe Ser Leu Ser Asp Gly Lys
            725             730                 735

Lys Lys Lys Lys Lys Gly His His His His His His
            740             745
```

```
<210>    84
<211>    702
<212>    PRT
<213>    Homo sapiens

<400>    84
```

```
Met Glu Ser Pro Ser Ala Pro Pro His Arg Trp Cys Ile Pro Trp Gln
1                 5                 10                  15

Arg Leu Leu Leu Thr Ala Ser Leu Leu Thr Phe Trp Asn Pro Pro Thr
            20              25                  30

Thr Ala Lys Leu Thr Ile Glu Ser Thr Pro Phe Asn Val Ala Glu Gly
        35              40                  45

Lys Glu Val Leu Leu Leu Val His Asn Leu Pro Gln His Leu Phe Gly
```

50                              55                              60

Tyr Ser Trp Tyr Lys Gly Glu Arg Val Asp Gly Asn Arg Gln Ile Ile
65              70              75                      80

Gly Tyr Val Ile Gly Thr Gln Gln Ala Thr Pro Gly Pro Ala Tyr Ser
                85              90                      95

Gly Arg Glu Ile Ile Tyr Pro Asn Ala Ser Leu Leu Ile Gln Asn Ile
            100             105             110

Ile Gln Asn Asp Thr Gly Phe Tyr Thr Leu His Val Ile Lys Ser Asp
        115             120             125

Leu Val Asn Glu Glu Ala Thr Gly Gln Phe Arg Val Tyr Pro Glu Leu
        130             135             140

Pro Lys Pro Ser Ile Ser Ser Asn Asn Ser Lys Pro Val Glu Asp Lys
145             150             155             160

Asp Ala Val Ala Phe Thr Cys Glu Pro Glu Thr Gln Asp Ala Thr Tyr
            165             170             175

Leu Trp Trp Val Asn Asn Gln Ser Leu Pro Val Ser Pro Arg Leu Gln
        180             185             190

Leu Ser Asn Gly Asn Arg Thr Leu Thr Leu Phe Asn Val Thr Arg Asn
        195             200             205

Asp Thr Ala Ser Tyr Lys Cys Glu Thr Gln Asn Pro Val Ser Ala Arg
        210             215             220

Arg Ser Asp Ser Val Ile Leu Asn Val Leu Tyr Gly Pro Asp Ala Pro
225             230             235             240

Thr Ile Ser Pro Leu Asn Thr Ser Tyr Arg Ser Gly Glu Asn Leu Asn
            245             250             255

Leu Ser Cys His Ala Ala Ser Asn Pro Pro Ala Gln Tyr Ser Trp Phe
            260             265             270

Val Asn Gly Thr Phe Gln Gln Ser Thr Gln Glu Leu Phe Ile Pro Asn
            275             280             285

Ile Thr Val Asn Asn Ser Gly Ser Tyr Thr Cys Gln Ala His Asn Ser
            290             295             300

```
Asp Thr Gly Leu Asn Arg Thr Thr Val Thr Thr Ile Thr Val Tyr Ala
305                 310             315                 320


Glu Pro Pro Lys Pro Phe Ile Thr Ser Asn Asn Ser Asn Pro Val Glu
            325             330                 335


Asp Glu Asp Ala Val Ala Leu Thr Cys Glu Pro Glu Ile Gln Asn Thr
            340             345                 350


Thr Tyr Leu Trp Trp Val Asn Asn Gln Ser Leu Pro Val Ser Pro Arg
        355             360             365


Leu Gln Leu Ser Asn Asp Asn Arg Thr Leu Thr Leu Leu Ser Val Thr
        370             375             380


Arg Asn Asp Val Gly Pro Tyr Glu Cys Gly Ile Gln Asn Lys Leu Ser
385             390             395                 400


Val Asp His Ser Asp Pro Val Ile Leu Asn Val Leu Tyr Gly Pro Asp
            405             410                 415


Asp Pro Thr Ile Ser Pro Ser Tyr Thr Tyr Tyr Arg Pro Gly Val Asn
            420             425             430


Leu Ser Leu Ser Cys His Ala Ala Ser Asn Pro Pro Ala Gln Tyr Ser
        435             440             445


Trp Leu Ile Asp Gly Asn Ile Gln Gln His Thr Gln Glu Leu Phe Ile
    450             455             460


Ser Asn Ile Thr Glu Lys Asn Ser Gly Leu Tyr Thr Cys Gln Ala Asn
465             470             475                 480


Asn Ser Ala Ser Gly His Ser Arg Thr Thr Val Lys Thr Ile Thr Val
            485             490                 495


Ser Ala Glu Leu Pro Lys Pro Ser Ile Ser Ser Asn Asn Ser Lys Pro
        500             505             510


Val Glu Asp Lys Asp Ala Val Ala Phe Thr Cys Glu Pro Glu Ala Gln
        515             520             525


Asn Thr Thr Tyr Leu Trp Trp Val Asn Gly Gln Ser Leu Pro Val Ser
        530             535             540


Pro Arg Leu Gln Leu Ser Asn Gly Asn Arg Thr Leu Thr Leu Phe Asn
545             550             555                 560
```

```
Val Thr Arg Asn Asp Ala Arg Ala Tyr Val Cys Gly Ile Gln Asn Ser
            565             570             575

Val Ser Ala Asn Arg Ser Asp Pro Val Thr Leu Asp Val Leu Tyr Gly
            580             585             590

Pro Asp Thr Pro Ile Ile Ser Pro Pro Asp Ser Ser Tyr Leu Ser Gly
            595             600             605

Ala Asn Leu Asn Leu Ser Cys His Ser Ala Ser Asn Pro Ser Pro Gln
    610             615             620

Tyr Ser Trp Arg Ile Asn Gly Ile Pro Gln Gln His Thr Gln Val Leu
625             630             635             640

Phe Ile Ala Lys Ile Thr Pro Asn Asn Asn Gly Thr Tyr Ala Cys Phe
            645             650             655

Val Ser Asn Leu Ala Thr Gly Arg Asn Asn Ser Ile Val Lys Ser Ile
            660             665             670

Thr Val Ser Ala Ser Gly Thr Ser Pro Gly Leu Ser Ala Gly Ala Thr
            675             680             685

Val Gly Ile Met Ile Gly Val Leu Val Gly Val Ala Leu Ile
    690             695             700
```

<210> 85
<211> 257
<212> PRT
<213> Homo sapiens

<400> 85

```
Met Ala Gln Arg Met Thr Thr Gln Leu Leu Leu Leu Leu Val Trp Val
1           5               10              15

Ala Val Val Gly Glu Ala Gln Thr Arg Ile Ala Trp Ala Arg Thr Glu
            20              25              30

Leu Leu Asn Val Cys Met Asn Ala Lys His His Lys Glu Lys Pro Gly
            35              40              45

Pro Glu Asp Lys Leu His Glu Gln Cys Arg Pro Trp Arg Lys Asn Ala
    50              55              60

Cys Cys Ser Thr Asn Thr Ser Gln Glu Ala His Lys Asp Val Ser Tyr
65              70              75              80
```

167

```
Leu Tyr Arg Phe Asn Trp Asn His Cys Gly Glu Met Ala Pro Ala Cys
                85                  90                  95

Lys Arg His Phe Ile Gln Asp Thr Cys Leu Tyr Glu Cys Ser Pro Asn
                100                 105                 110

Leu Gly Pro Trp Ile Gln Gln Val Asp Gln Ser Trp Arg Lys Glu Arg
        115                 120                 125

Val Leu Asn Val Pro Leu Cys Lys Glu Asp Cys Glu Gln Trp Trp Glu
    130                 135                 140

Asp Cys Arg Thr Ser Tyr Thr Cys Lys Ser Asn Trp His Lys Gly Trp
145                 150                 155                 160

Asn Trp Thr Ser Gly Phe Asn Lys Cys Ala Val Gly Ala Ala Cys Gln
                165                 170                 175

Pro Phe His Phe Tyr Phe Pro Thr Pro Thr Val Leu Cys Asn Glu Ile
            180                 185                 190

Trp Thr His Ser Tyr Lys Val Ser Asn Tyr Ser Arg Gly Ser Gly Arg
        195                 200                 205

Cys Ile Gln Met Trp Phe Asp Pro Ala Gln Gly Asn Pro Asn Glu Glu
    210                 215                 220

Val Ala Arg Phe Tyr Ala Ala Ala Met Ser Gly Ala Gly Pro Trp Ala
225                 230                 235                 240

Ala Trp Pro Phe Leu Leu Ser Leu Ala Leu Met Leu Leu Trp Leu Leu
                245                 250                 255

Ser
```

```
<210>  86
<211>  255
<212>  PRT
<213>  Mus musculus

<400>  86

Met Ala His Leu Met Thr Val Gln Leu Leu Leu Val Met Trp Met
1               5                   10                  15

Ala Glu Cys Ala Gln Ser Arg Ala Thr Arg Ala Arg Thr Glu Leu Leu
            20                  25                  30
```

```
Asn Val Cys Met Asp Ala Lys His His Lys Glu Lys Pro Gly Pro Glu
        35                  40                  45

Asp Asn Leu His Asp Gln Cys Ser Pro Trp Lys Thr Asn Ser Cys Cys
        50                  55                  60

Ser Thr Asn Thr Ser Gln Glu Ala His Lys Asp Ile Ser Tyr Leu Tyr
65                  70                  75                      80

Arg Phe Asn Trp Asn His Cys Gly Thr Met Thr Ser Glu Cys Lys Arg
                85                  90                  95

His Phe Ile Gln Asp Thr Cys Leu Tyr Glu Cys Ser Pro Asn Leu Gly
            100                 105                 110

Pro Trp Ile Gln Gln Val Asp Gln Ser Trp Arg Lys Glu Arg Ile Leu
            115                 120                 125

Asp Val Pro Leu Cys Lys Glu Asp Cys Gln Gln Trp Trp Glu Asp Cys
    130                 135                 140

Gln Ser Ser Phe Thr Cys Lys Ser Asn Trp His Lys Gly Trp Asn Trp
145                 150                 155                 160

Ser Ser Gly His Asn Glu Cys Pro Val Gly Ala Ser Cys His Pro Phe
                165                 170                 175

Thr Phe Tyr Phe Pro Thr Ser Ala Ala Leu Cys Glu Glu Ile Trp Ser
            180                 185                 190

His Ser Tyr Lys Leu Ser Asn Tyr Ser Arg Gly Ser Gly Arg Cys Ile
            195                 200                 205

Gln Met Trp Phe Asp Pro Ala Gln Gly Asn Pro Asn Glu Glu Val Ala
    210                 215                 220

Arg Phe Tyr Ala Glu Ala Met Ser Gly Ala Gly Phe His Gly Thr Trp
225                 230                 235                 240

Pro Leu Leu Cys Ser Leu Ser Leu Val Leu Leu Trp Val Ile Ser
                245                 250                 255
```

<210> 87
<211> 257
<212> PRT
<213> Cynomolgus

<400> 87

```
Met Ala Gln Arg Met Thr Thr Gln Leu Leu Leu Leu Val Trp Val
1               5               10              15

Ala Val Val Gly Glu Ala Gln Thr Arg Thr Ala Arg Ala Arg Thr Glu
            20              25              30

Leu Leu Asn Val Cys Met Asn Ala Lys His His Lys Glu Lys Pro Gly
        35              40              45

Pro Glu Asp Lys Leu His Glu Gln Cys Arg Pro Trp Lys Lys Asn Ala
    50              55              60

Cys Cys Ser Thr Asn Thr Ser Gln Glu Ala His Lys Asp Val Ser Tyr
65              70              75              80

Leu Tyr Arg Phe Asn Trp Asn His Cys Gly Glu Met Ala Pro Ala Cys
            85              90              95

Lys Arg His Phe Ile Gln Asp Thr Cys Leu Tyr Glu Cys Ser Pro Asn
        100             105             110

Leu Gly Pro Trp Ile Gln Gln Val Asp Gln Ser Trp Arg Lys Glu Arg
        115             120             125

Val Leu Asn Val Pro Leu Cys Lys Glu Asp Cys Glu Arg Trp Trp Glu
    130             135             140

Asp Cys Arg Thr Ser Tyr Thr Cys Lys Ser Asn Trp His Lys Gly Trp
145             150             155             160

Asn Trp Thr Ser Gly Phe Asn Lys Cys Pro Val Gly Ala Ala Cys Gln
            165             170             175

Pro Phe His Phe Tyr Phe Pro Thr Pro Thr Val Leu Cys Asn Glu Ile
        180             185             190

Trp Thr Tyr Ser Tyr Lys Val Ser Asn Tyr Ser Arg Gly Ser Gly Arg
        195             200             205

Cys Ile Gln Met Trp Phe Asp Pro Ala Gln Gly Asn Pro Asn Glu Glu
    210             215             220

Val Ala Arg Phe Tyr Ala Ala Ala Met Ser Gly Ala Gly Pro Trp Ala
225             230             235             240

Ala Trp Pro Leu Leu Leu Ser Leu Ala Leu Thr Leu Leu Trp Leu Leu
            245             250             255
```

Ser

<210> 88
<211> 2322
<212> PRT
<213> Homo sapiens

<400> 88

Met Gln Ser Gly Pro Arg Pro Pro Leu Pro Ala Pro Gly Leu Ala Leu
1               5                   10                  15

Ala Leu Thr Leu Thr Met Leu Ala Arg Leu Ala Ser Ala Ala Ser Phe
            20                  25                  30

Phe Gly Glu Asn His Leu Glu Val Pro Val Ala Thr Ala Leu Thr Asp
        35                  40                  45

Ile Asp Leu Gln Leu Gln Phe Ser Thr Ser Gln Pro Glu Ala Leu Leu
    50                  55                  60

Leu Leu Ala Ala Gly Pro Ala Asp His Leu Leu Leu Gln Leu Tyr Ser
65                  70                  75                  80

Gly Arg Leu Gln Val Arg Leu Val Leu Gly Gln Glu Glu Leu Arg Leu
                85                  90                  95

Gln Thr Pro Ala Glu Thr Leu Leu Ser Asp Ser Ile Pro His Thr Val
            100                 105                 110

Val Leu Thr Val Val Glu Gly Trp Ala Thr Leu Ser Val Asp Gly Phe
            115                 120                 125

Leu Asn Ala Ser Ser Ala Val Pro Gly Ala Pro Leu Glu Val Pro Tyr
        130                 135                 140

Gly Leu Phe Val Gly Gly Thr Gly Thr Leu Gly Leu Pro Tyr Leu Arg
145                 150                 155                 160

Gly Thr Ser Arg Pro Leu Arg Gly Cys Leu His Ala Ala Thr Leu Asn
                165                 170                 175

Gly Arg Ser Leu Leu Arg Pro Leu Thr Pro Asp Val His Glu Gly Cys
            180                 185                 190

Ala Glu Glu Phe Ser Ala Ser Asp Asp Val Ala Leu Gly Phe Ser Gly
            195                 200                 205

| | | | | | | | | | | | | | | |
|---|---|---|---|---|---|---|---|---|---|---|---|---|---|---|
| Pro | His | Ser | Leu | Ala | Ala | Phe | Pro | Ala | Trp | Gly | Thr | Gln | Asp | Glu | Gly |
| | 210 | | | | 215 | | | | 220 | | | | | | |

| | | | | | | | | | | | | | | |
|---|---|---|---|---|---|---|---|---|---|---|---|---|---|---|
| Thr | Leu | Glu | Phe | Thr | Leu | Thr | Thr | Gln | Ser | Arg | Gln | Ala | Pro | Leu | Ala |
| 225 | | | | 230 | | | | 235 | | | | | 240 | | |

| | | | | | | | | | | | | | | |
|---|---|---|---|---|---|---|---|---|---|---|---|---|---|---|
| Phe | Gln | Ala | Gly | Gly | Arg | Arg | Gly | Asp | Phe | Ile | Tyr | Val | Asp | Ile | Phe |
| | | | 245 | | | | 250 | | | | | 255 | | | |

Glu Gly His Leu Arg Ala Val Val Glu Lys Gly Gln Gly Thr Val Leu
260 265 270

Leu His Asn Ser Val Pro Val Ala Asp Gly Gln Pro His Glu Val Ser
275 280 285

Val His Ile Asn Ala His Arg Leu Glu Ile Ser Val Asp Gln Tyr Pro
290 295 300

Thr His Thr Ser Asn Arg Gly Val Leu Ser Tyr Leu Glu Pro Arg Gly
305 310 315 320

Ser Leu Leu Leu Gly Gly Leu Asp Ala Glu Ala Ser Arg His Leu Gln
325 330 335

Glu His Arg Leu Gly Leu Thr Pro Glu Ala Thr Asn Ala Ser Leu Leu
340 345 350

Gly Cys Met Glu Asp Leu Ser Val Asn Gly Gln Arg Arg Gly Leu Arg
355 360 365

Glu Ala Leu Leu Thr Arg Asn Met Ala Ala Gly Cys Arg Leu Glu Glu
370 375 380

Glu Glu Tyr Glu Asp Asp Ala Tyr Gly His Tyr Glu Ala Phe Ser Thr
385 390 395 400

Leu Ala Pro Glu Ala Trp Pro Ala Met Glu Leu Pro Glu Pro Cys Val
405 410 415

Pro Glu Pro Gly Leu Pro Pro Val Phe Ala Asn Phe Thr Gln Leu Leu
420 425 430

Thr Ile Ser Pro Leu Val Val Ala Glu Gly Gly Thr Ala Trp Leu Glu
435 440 445

Trp Arg His Val Gln Pro Thr Leu Asp Leu Met Glu Ala Glu Leu Arg

                    450                        455                          460


        Lys Ser Gln Val Leu Phe Ser Val Thr Arg Gly Ala Arg His Gly Glu
        465                 470                 475                     480


        Leu Glu Leu Asp Ile Pro Gly Ala Gln Ala Arg Lys Met Phe Thr Leu
                        485                 490                     495


        Leu Asp Val Val Asn Arg Lys Ala Arg Phe Ile His Asp Gly Ser Glu
                    500                 505                 510


        Asp Thr Ser Asp Gln Leu Val Leu Glu Val Ser Val Thr Ala Arg Val
                    515                 520                 525


        Pro Met Pro Ser Cys Leu Arg Arg Gly Gln Thr Tyr Leu Leu Pro Ile
            530                 535                 540


        Gln Val Asn Pro Val Asn Asp Pro Pro His Ile Ile Phe Pro His Gly
        545                 550                 555                     560


        Ser Leu Met Val Ile Leu Glu His Thr Gln Lys Pro Leu Gly Pro Glu
                        565                 570                     575


        Val Phe Gln Ala Tyr Asp Pro Asp Ser Ala Cys Glu Gly Leu Thr Phe
                    580                 585                     590


        Gln Val Leu Gly Thr Ser Ser Gly Leu Pro Val Glu Arg Arg Asp Gln
                    595                 600                 605


        Pro Gly Glu Pro Ala Thr Glu Phe Ser Cys Arg Glu Leu Glu Ala Gly
            610                 615                 620


        Ser Leu Val Tyr Val His Arg Gly Gly Pro Ala Gln Asp Leu Thr Phe
        625                 630                 635                     640


        Arg Val Ser Asp Gly Leu Gln Ala Ser Pro Pro Ala Thr Leu Lys Val
                        645                 650                     655


        Val Ala Ile Arg Pro Ala Ile Gln Ile His Arg Ser Thr Gly Leu Arg
                    660                 665                 670


        Leu Ala Gln Gly Ser Ala Met Pro Ile Leu Pro Ala Asn Leu Ser Val
                    675                 680                 685


        Glu Thr Asn Ala Val Gly Gln Asp Val Ser Val Leu Phe Arg Val Thr
            690                 695                 700

```
Gly Ala Leu Gln Phe Gly Glu Leu Gln Lys Gln Gly Ala Gly Gly Val
705                 710                 715                 720

Glu Gly Ala Glu Trp Trp Ala Thr Gln Ala Phe His Gln Arg Asp Val
                725                 730                 735

Glu Gln Gly Arg Val Arg Tyr Leu Ser Thr Asp Pro Gln His His Ala
                740                 745                 750

Tyr Asp Thr Val Glu Asn Leu Ala Leu Glu Val Gln Val Gly Gln Glu
                755                 760                 765

Ile Leu Ser Asn Leu Ser Phe Pro Val Thr Ile Gln Arg Ala Thr Val
                770                 775                 780

Trp Met Leu Arg Leu Glu Pro Leu His Thr Gln Asn Thr Gln Gln Glu
785                 790                 795                 800

Thr Leu Thr Thr Ala His Leu Glu Ala Thr Leu Glu Glu Ala Gly Pro
                805                 810                 815

Ser Pro Pro Thr Phe His Tyr Glu Val Val Gln Ala Pro Arg Lys Gly
                820                 825                 830

Asn Leu Gln Leu Gln Gly Thr Arg Leu Ser Asp Gly Gln Gly Phe Thr
                835                 840                 845

Gln Asp Asp Ile Gln Ala Gly Arg Val Thr Tyr Gly Ala Thr Ala Arg
    850                 855                 860

Ala Ser Glu Ala Val Glu Asp Thr Phe Arg Phe Arg Val Thr Ala Pro
865                 870                 875                 880

Pro Tyr Phe Ser Pro Leu Tyr Thr Phe Pro Ile His Ile Gly Gly Asp
                885                 890                 895

Pro Asp Ala Pro Val Leu Thr Asn Val Leu Leu Val Val Pro Glu Gly
                900                 905                 910

Gly Glu Gly Val Leu Ser Ala Asp His Leu Phe Val Lys Ser Leu Asn
                915                 920                 925

Ser Ala Ser Tyr Leu Tyr Glu Val Met Glu Arg Pro Arg His Gly Arg
    930                 935                 940

Leu Ala Trp Arg Gly Thr Gln Asp Lys Thr Thr Met Val Thr Ser Phe
945                 950                 955                 960
```

Thr Asn Glu Asp Leu Leu Arg Gly Arg Leu Val Tyr Gln His Asp Asp
                965                    970                    975

Ser Glu Thr Thr Glu Asp Asp Ile Pro Phe Val Ala Thr Arg Gln Gly
            980                    985                    990

Glu Ser Ser Gly Asp Met Ala Trp  Glu Glu Val Arg Gly  Val Phe Arg
            995                    1000                   1005

Val Ala  Ile Gln Pro Val Asn  Asp His Ala Pro Val  Gln Thr Ile
    1010                  1015                1020

Ser Arg  Ile Phe His Val Ala  Arg Gly Gly Arg Arg  Leu Leu Thr
    1025                  1030                1035

Thr Asp  Asp Val Ala Phe Ser  Asp Ala Asp Ser Gly  Phe Ala Asp
    1040                  1045                1050

Ala Gln  Leu Val Leu Thr Arg  Lys Asp Leu Leu Phe  Gly Ser Ile
    1055                  1060                1065

Val Ala  Val Asp Glu Pro Thr  Arg Pro Ile Tyr Arg  Phe Thr Gln
    1070                  1075                1080

Glu Asp  Leu Arg Lys Arg Arg  Val Leu Phe Val His  Ser Gly Ala
    1085                  1090                1095

Asp Arg  Gly Trp Ile Gln Leu  Gln Val Ser Asp Gly  Gln His Gln
    1100                  1105                1110

Ala Thr  Ala Leu Leu Glu Val  Gln Ala Ser Glu Pro  Tyr Leu Arg
    1115                  1120                1125

Val Ala  Asn Gly Ser Ser Leu  Val Val Pro Gln Gly  Gly Gln Gly
    1130                  1135                1140

Thr Ile  Asp Thr Ala Val Leu  His Leu Asp Thr Asn  Leu Asp Ile
    1145                  1150                1155

Arg Ser  Gly Asp Glu Val His  Tyr His Val Thr Ala  Gly Pro Arg
    1160                  1165                1170

Trp Gly  Gln Leu Val Arg Ala  Gly Gln Pro Ala Thr  Ala Phe Ser
    1175                  1180                1185

Gln Gln  Asp Leu Leu Asp Gly  Ala Val Leu Tyr Ser  His Asn Gly
    1190                  1195                1200

Ser Leu Ser Pro Arg Asp Thr Met Ala Phe Ser Val Glu Ala Gly
1205 1210 1215

Pro Val His Thr Asp Ala Thr Leu Gln Val Thr Ile Ala Leu Glu
1220 1225 1230

Gly Pro Leu Ala Pro Leu Lys Leu Val Arg His Lys Lys Ile Tyr
1235 1240 1245

Val Phe Gln Gly Glu Ala Ala Glu Ile Arg Arg Asp Gln Leu Glu
1250 1255 1260

Ala Ala Gln Glu Ala Val Pro Pro Ala Asp Ile Val Phe Ser Val
1265 1270 1275

Lys Ser Pro Pro Ser Ala Gly Tyr Leu Val Met Val Ser Arg Gly
1280 1285 1290

Ala Leu Ala Asp Glu Pro Pro Ser Leu Asp Pro Val Gln Ser Phe
1295 1300 1305

Ser Gln Glu Ala Val Asp Thr Gly Arg Val Leu Tyr Leu His Ser
1310 1315 1320

Arg Pro Glu Ala Trp Ser Asp Ala Phe Ser Leu Asp Val Ala Ser
1325 1330 1335

Gly Leu Gly Ala Pro Leu Glu Gly Val Leu Val Glu Leu Glu Val
1340 1345 1350

Leu Pro Ala Ala Ile Pro Leu Glu Ala Gln Asn Phe Ser Val Pro
1355 1360 1365

Glu Gly Gly Ser Leu Thr Leu Ala Pro Pro Leu Leu Arg Val Ser
1370 1375 1380

Gly Pro Tyr Phe Pro Thr Leu Leu Gly Leu Ser Leu Gln Val Leu
1385 1390 1395

Glu Pro Pro Gln His Gly Ala Leu Gln Lys Glu Asp Gly Pro Gln
1400 1405 1410

Ala Arg Thr Leu Ser Ala Phe Ser Trp Arg Met Val Glu Glu Gln
1415 1420 1425

Leu Ile Arg Tyr Val His Asp Gly Ser Glu Thr Leu Thr Asp Ser

1430                    1435                    1440

Phe Val  Leu Met Ala Asn Ala  Ser Glu Met Asp Arg  Gln Ser His
    1445             1450             1455

Pro Val  Ala Phe Thr Val Thr  Val Leu Pro Val Asn  Asp Gln Pro
    1460             1465             1470

Pro Ile  Leu Thr Thr Asn Thr  Gly Leu Gln Met Trp  Glu Gly Ala
    1475             1480             1485

Thr Ala  Pro Ile Pro Ala Glu  Ala Leu Arg Ser Thr  Asp Gly Asp
    1490             1495             1500

Ser Gly  Ser Glu Asp Leu Val  Tyr Thr Ile Glu Gln  Pro Ser Asn
    1505             1510             1515

Gly Arg  Val Val Leu Arg Gly  Ala Pro Gly Thr Glu  Val Arg Ser
    1520             1525             1530

Phe Thr  Gln Ala Gln Leu Asp  Gly Gly Leu Val Leu  Phe Ser His
    1535             1540             1545

Arg Gly  Thr Leu Asp Gly Gly  Phe Arg Phe Arg Leu  Ser Asp Gly
    1550             1555             1560

Glu His  Thr Ser Pro Gly His  Phe Phe Arg Val Thr  Ala Gln Lys
    1565             1570             1575

Gln Val  Leu Leu Ser Leu Lys  Gly Ser Gln Thr Leu  Thr Val Cys
    1580             1585             1590

Pro Gly  Ser Val Gln Pro Leu  Ser Ser Gln Thr Leu  Arg Ala Ser
    1595             1600             1605

Ser Ser  Ala Gly Thr Asp Pro  Gln Leu Leu Leu Tyr  Arg Val Val
    1610             1615             1620

Arg Gly  Pro Gln Leu Gly Arg  Leu Phe His Ala Gln  Gln Asp Ser
    1625             1630             1635

Thr Gly  Glu Ala Leu Val Asn  Phe Thr Gln Ala Glu  Val Tyr Ala
    1640             1645             1650

Gly Asn  Ile Leu Tyr Glu His  Glu Met Pro Pro Glu  Pro Phe Trp
    1655             1660             1665

177

Glu Ala His Asp Thr Leu Glu Leu Gln Leu Ser Ser Pro Pro Ala
1670 1675 1680

Arg Asp Val Ala Ala Thr Leu Ala Val Ala Val Ser Phe Glu Ala
1685 1690 1695

Ala Cys Pro Gln Arg Pro Ser His Leu Trp Lys Asn Lys Gly Leu
1700 1705 1710

Trp Val Pro Glu Gly Gln Arg Ala Arg Ile Thr Val Ala Ala Leu
1715 1720 1725

Asp Ala Ser Asn Leu Leu Ala Ser Val Pro Ser Pro Gln Arg Ser
1730 1735 1740

Glu His Asp Val Leu Phe Gln Val Thr Gln Phe Pro Ser Arg Gly
1745 1750 1755

Gln Leu Leu Val Ser Glu Glu Pro Leu His Ala Gly Gln Pro His
1760 1765 1770

Phe Leu Gln Ser Gln Leu Ala Ala Gly Gln Leu Val Tyr Ala His
1775 1780 1785

Gly Gly Gly Gly Thr Gln Gln Asp Gly Phe His Phe Arg Ala His
1790 1795 1800

Leu Gln Gly Pro Ala Gly Ala Ser Val Ala Gly Pro Gln Thr Ser
1805 1810 1815

Glu Ala Phe Ala Ile Thr Val Arg Asp Val Asn Glu Arg Pro Pro
1820 1825 1830

Gln Pro Gln Ala Ser Val Pro Leu Arg Leu Thr Arg Gly Ser Arg
1835 1840 1845

Ala Pro Ile Ser Arg Ala Gln Leu Ser Val Val Asp Pro Asp Ser
1850 1855 1860

Ala Pro Gly Glu Ile Glu Tyr Glu Val Gln Arg Ala Pro His Asn
1865 1870 1875

Gly Phe Leu Ser Leu Val Gly Gly Gly Leu Gly Pro Val Thr Arg
1880 1885 1890

Phe Thr Gln Ala Asp Val Asp Ser Gly Arg Leu Ala Phe Val Ala
1895 1900 1905

```
Asn Gly  Ser Ser Val Ala Gly   Ile Phe Gln Leu Ser  Met Ser Asp
    1910                 1915                 1920

Gly Ala  Ser Pro Pro Leu Pro   Met Ser Leu Ala Val  Asp Ile Leu
    1925                 1930                 1935

Pro Ser  Ala Ile Glu Val Gln   Leu Arg Ala Pro Leu  Glu Val Pro
    1940                 1945                 1950

Gln Ala  Leu Gly Arg Ser Ser   Leu Ser Gln Gln Gln  Leu Arg Val
    1955                 1960                 1965

Val Ser  Asp Arg Glu Glu Pro   Glu Ala Ala Tyr Arg  Leu Ile Gln
    1970                 1975                 1980

Gly Pro  Gln Tyr Gly His Leu   Leu Val Gly Gly Arg  Pro Thr Ser
    1985                 1990                 1995

Ala Phe  Ser Gln Phe Gln Ile   Asp Gln Gly Glu Val  Val Phe Ala
    2000                 2005                 2010

Phe Thr  Asn Phe Ser Ser Ser   His Asp His Phe Arg  Val Leu Ala
    2015                 2020                 2025

Leu Ala  Arg Gly Val Asn Ala   Ser Ala Val Val Asn  Val Thr Val
    2030                 2035                 2040

Arg Ala  Leu Leu His Val Trp   Ala Gly Gly Pro Trp  Pro Gln Gly
    2045                 2050                 2055

Ala Thr  Leu Arg Leu Asp Pro   Thr Val Leu Asp Ala  Gly Glu Leu
    2060                 2065                 2070

Ala Asn  Arg Thr Gly Ser Val   Pro Arg Phe Arg Leu  Leu Glu Gly
    2075                 2080                 2085

Pro Arg  His Gly Arg Val Val   Arg Val Pro Arg Ala  Arg Thr Glu
    2090                 2095                 2100

Pro Gly  Gly Ser Gln Leu Val   Glu Gln Phe Thr Gln  Gln Asp Leu
    2105                 2110                 2115

Glu Asp  Gly Arg Leu Gly Leu   Glu Val Gly Arg Pro  Glu Gly Arg
    2120                 2125                 2130

Ala Pro  Gly Pro Ala Gly Asp   Ser Leu Thr Leu Glu  Leu Trp Ala
    2135                 2140                 2145
```

```
Gln Gly  Val Pro Pro Ala Val  Ala Ser Leu Asp Phe  Ala Thr Glu
    2150                 2155             2160

Pro Tyr  Asn Ala Ala Arg Pro  Tyr Ser Val Ala Leu  Leu Ser Val
    2165                 2170             2175

Pro Glu  Ala Ala Arg Thr Glu  Ala Gly Lys Pro Glu  Ser Ser Thr
    2180                 2185             2190

Pro Thr  Gly Glu Pro Gly Pro  Met Ala Ser Ser Pro  Glu Pro Ala
    2195                 2200             2205

Val Ala  Lys Gly Gly Phe Leu  Ser Phe Leu Glu Ala  Asn Met Phe
    2210                 2215             2220

Ser Val  Ile Ile Pro Met Cys  Leu Val Leu Leu Leu  Leu Ala Leu
    2225                 2230             2235

Ile Leu  Pro Leu Leu Phe Tyr  Leu Arg Lys Arg Asn  Lys Thr Gly
    2240                 2245             2250

Lys His  Asp Val Gln Val Leu  Thr Ala Lys Pro Arg  Asn Gly Leu
    2255                 2260             2265

Ala Gly  Asp Thr Glu Thr Phe  Arg Lys Val Glu Pro  Gly Gln Ala
    2270                 2275             2280

Ile Pro  Leu Thr Ala Val Pro  Gly Gln Gly Pro Pro  Pro Gly Gly
    2285                 2290             2295

Gln Pro  Asp Pro Glu Leu Leu  Gln Phe Cys Arg Thr  Pro Asn Pro
    2300                 2305             2310

Ala Leu  Lys Asn Gly Gln Tyr  Trp Val
    2315                 2320
```

```
<210>  89
<211>  1210
<212>  PRT
<213>  Homo sapiens

<400>  89

Met Arg Pro Ser Gly Thr Ala Gly Ala Ala Leu Leu Ala Leu Leu Ala
1                   5                   10                  15

Ala Leu Cys Pro Ala Ser Arg Ala Leu Glu Glu Lys Lys Val Cys Gln
              20                  25                  30
```

Gly Thr Ser Asn Lys Leu Thr Gln Leu Gly Thr Phe Glu Asp His Phe
                35                  40                  45

Leu Ser Leu Gln Arg Met Phe Asn Asn Cys Glu Val Val Leu Gly Asn
            50                  55                  60

Leu Glu Ile Thr Tyr Val Gln Arg Asn Tyr Asp Leu Ser Phe Leu Lys
65                  70                  75                  80

Thr Ile Gln Glu Val Ala Gly Tyr Val Leu Ile Ala Leu Asn Thr Val
                85                  90                  95

Glu Arg Ile Pro Leu Glu Asn Leu Gln Ile Ile Arg Gly Asn Met Tyr
            100                 105                 110

Tyr Glu Asn Ser Tyr Ala Leu Ala Val Leu Ser Asn Tyr Asp Ala Asn
            115                 120                 125

Lys Thr Gly Leu Lys Glu Leu Pro Met Arg Asn Leu Gln Glu Ile Leu
    130                 135                 140

His Gly Ala Val Arg Phe Ser Asn Asn Pro Ala Leu Cys Asn Val Glu
145                 150                 155                 160

Ser Ile Gln Trp Arg Asp Ile Val Ser Ser Asp Phe Leu Ser Asn Met
            165                 170                 175

Ser Met Asp Phe Gln Asn His Leu Gly Ser Cys Gln Lys Cys Asp Pro
            180                 185                 190

Ser Cys Pro Asn Gly Ser Cys Trp Gly Ala Gly Glu Glu Asn Cys Gln
    195                 200                 205

Lys Leu Thr Lys Ile Ile Cys Ala Gln Gln Cys Ser Gly Arg Cys Arg
    210                 215                 220

Gly Lys Ser Pro Ser Asp Cys Cys His Asn Gln Cys Ala Ala Gly Cys
225                 230                 235                 240

Thr Gly Pro Arg Glu Ser Asp Cys Leu Val Cys Arg Lys Phe Arg Asp
            245                 250                 255

Glu Ala Thr Cys Lys Asp Thr Cys Pro Pro Leu Met Leu Tyr Asn Pro
            260                 265                 270

Thr Thr Tyr Gln Met Asp Val Asn Pro Glu Gly Lys Tyr Ser Phe Gly

181

```
                     275                    280                           285


            Ala Thr Cys Val Lys Lys Cys Pro Arg Asn Tyr Val Val Thr Asp His
                290                 295                 300


            Gly Ser Cys Val Arg Ala Cys Gly Ala Asp Ser Tyr Glu Met Glu Glu
            305             310                 315                     320


            Asp Gly Val Arg Lys Cys Lys Lys Cys Glu Gly Pro Cys Arg Lys Val
                            325                 330                 335


            Cys Asn Gly Ile Gly Ile Gly Glu Phe Lys Asp Ser Leu Ser Ile Asn
                        340                 345                 350


            Ala Thr Asn Ile Lys His Phe Lys Asn Cys Thr Ser Ile Ser Gly Asp
                    355                 360                 365


            Leu His Ile Leu Pro Val Ala Phe Arg Gly Asp Ser Phe Thr His Thr
                370                 375                 380


            Pro Pro Leu Asp Pro Gln Glu Leu Asp Ile Leu Lys Thr Val Lys Glu
            385                 390                 395                     400


            Ile Thr Gly Phe Leu Leu Ile Gln Ala Trp Pro Glu Asn Arg Thr Asp
                            405                 410                 415


            Leu His Ala Phe Glu Asn Leu Glu Ile Ile Arg Gly Arg Thr Lys Gln
                        420                 425                 430


            His Gly Gln Phe Ser Leu Ala Val Val Ser Leu Asn Ile Thr Ser Leu
                        435                 440                 445


            Gly Leu Arg Ser Leu Lys Glu Ile Ser Asp Gly Asp Val Ile Ile Ser
                450                 455                 460


            Gly Asn Lys Asn Leu Cys Tyr Ala Asn Thr Ile Asn Trp Lys Lys Leu
            465                 470                 475                     480


            Phe Gly Thr Ser Gly Gln Lys Thr Lys Ile Ile Ser Asn Arg Gly Glu
                            485                 490                 495


            Asn Ser Cys Lys Ala Thr Gly Gln Val Cys His Ala Leu Cys Ser Pro
                        500                 505                 510


            Glu Gly Cys Trp Gly Pro Glu Pro Arg Asp Cys Val Ser Cys Arg Asn
                        515                 520                 525
```

Val Ser Arg Gly Arg Glu Cys Val Asp Lys Cys Asn Leu Leu Glu Gly
530                     535                 540

Glu Pro Arg Glu Phe Val Glu Asn Ser Glu Cys Ile Gln Cys His Pro
545                 550                 555                     560

Glu Cys Leu Pro Gln Ala Met Asn Ile Thr Cys Thr Gly Arg Gly Pro
                565                 570                     575

Asp Asn Cys Ile Gln Cys Ala His Tyr Ile Asp Gly Pro His Cys Val
                580                 585                 590

Lys Thr Cys Pro Ala Gly Val Met Gly Glu Asn Asn Thr Leu Val Trp
                595                 600                 605

Lys Tyr Ala Asp Ala Gly His Val Cys His Leu Cys His Pro Asn Cys
        610                 615                 620

Thr Tyr Gly Cys Thr Gly Pro Gly Leu Glu Gly Cys Pro Thr Asn Gly
625                 630                 635                     640

Pro Lys Ile Pro Ser Ile Ala Thr Gly Met Val Gly Ala Leu Leu Leu
                645                 650                 655

Leu Leu Val Val Ala Leu Gly Ile Gly Leu Phe Met Arg Arg Arg His
                660                 665                 670

Ile Val Arg Lys Arg Thr Leu Arg Arg Leu Leu Gln Glu Arg Glu Leu
        675                 680                 685

Val Glu Pro Leu Thr Pro Ser Gly Glu Ala Pro Asn Gln Ala Leu Leu
    690                 695                 700

Arg Ile Leu Lys Glu Thr Glu Phe Lys Lys Ile Lys Val Leu Gly Ser
705                 710                 715                     720

Gly Ala Phe Gly Thr Val Tyr Lys Gly Leu Trp Ile Pro Glu Gly Glu
                725                 730                 735

Lys Val Lys Ile Pro Val Ala Ile Lys Glu Leu Arg Glu Ala Thr Ser
                740                 745                 750

Pro Lys Ala Asn Lys Glu Ile Leu Asp Glu Ala Tyr Val Met Ala Ser
                755                 760                 765

Val Asp Asn Pro His Val Cys Arg Leu Leu Gly Ile Cys Leu Thr Ser
770                 775                 780

```
Thr Val Gln Leu Ile Thr Gln Leu Met Pro Phe Gly Cys Leu Leu Asp
785                 790             795                 800

Tyr Val Arg Glu His Lys Asp Asn Ile Gly Ser Gln Tyr Leu Leu Asn
                805             810                 815

Trp Cys Val Gln Ile Ala Lys Gly Met Asn Tyr Leu Glu Asp Arg Arg
            820             825             830

Leu Val His Arg Asp Leu Ala Ala Arg Asn Val Leu Val Lys Thr Pro
            835             840             845

Gln His Val Lys Ile Thr Asp Phe Gly Leu Ala Lys Leu Leu Gly Ala
    850             855             860

Glu Glu Lys Glu Tyr His Ala Glu Gly Gly Lys Val Pro Ile Lys Trp
865             870             875                 880

Met Ala Leu Glu Ser Ile Leu His Arg Ile Tyr Thr His Gln Ser Asp
            885             890             895

Val Trp Ser Tyr Gly Val Thr Val Trp Glu Leu Met Thr Phe Gly Ser
            900             905             910

Lys Pro Tyr Asp Gly Ile Pro Ala Ser Glu Ile Ser Ser Ile Leu Glu
            915             920             925

Lys Gly Glu Arg Leu Pro Gln Pro Pro Ile Cys Thr Ile Asp Val Tyr
    930             935             940

Met Ile Met Val Lys Cys Trp Met Ile Asp Ala Asp Ser Arg Pro Lys
945             950             955                 960

Phe Arg Glu Leu Ile Ile Glu Phe Ser Lys Met Ala Arg Asp Pro Gln
            965             970             975

Arg Tyr Leu Val Ile Gln Gly Asp Glu Arg Met His Leu Pro Ser Pro
            980             985             990

Thr Asp Ser Asn Phe Tyr Arg Ala Leu Met Asp Glu Glu Asp Met Asp
    995             1000                1005

Asp Val Val Asp Ala Asp Glu Tyr Leu Ile Pro Gln Gln Gly Phe
    1010            1015               1020

Phe Ser Ser Pro Ser Thr Ser Arg Thr Pro Leu Leu Ser Ser Leu
    1025            1030               1035
```

```
Ser Ala  Thr Ser Asn Asn Ser  Thr Val Ala Cys Ile  Asp Arg Asn
    1040             1045             1050

Gly Leu  Gln Ser Cys Pro Ile  Lys Glu Asp Ser Phe  Leu Gln Arg
    1055             1060             1065

Tyr Ser  Ser Asp Pro Thr Gly  Ala Leu Thr Glu Asp  Ser Ile Asp
    1070             1075             1080

Asp Thr  Phe Leu Pro Val Pro  Glu Tyr Ile Asn Gln  Ser Val Pro
    1085             1090             1095

Lys Arg  Pro Ala Gly Ser Val  Gln Asn Pro Val Tyr  His Asn Gln
    1100             1105             1110

Pro Leu  Asn Pro Ala Pro Ser  Arg Asp Pro His Tyr  Gln Asp Pro
    1115             1120             1125

His Ser  Thr Ala Val Gly Asn  Pro Glu Tyr Leu Asn  Thr Val Gln
    1130             1135             1140

Pro Thr  Cys Val Asn Ser Thr  Phe Asp Ser Pro Ala  His Trp Ala
    1145             1150             1155

Gln Lys  Gly Ser His Gln Ile  Ser Leu Asp Asn Pro  Asp Tyr Gln
    1160             1165             1170

Gln Asp  Phe Phe Pro Lys Glu  Ala Lys Pro Asn Gly  Ile Phe Lys
    1175             1180             1185

Gly Ser  Thr Ala Glu Asn Ala  Glu Tyr Leu Arg Val  Ala Pro Gln
    1190             1195             1200

Ser Ser  Glu Phe Ile Gly Ala
    1205             1210


<210>   90
<211>   1255
<212>   PRT
<213>   Homo sapiens


<400>   90

Met Glu Leu Ala Ala Leu Cys Arg Trp Gly Leu Leu Leu Ala Leu Leu
1               5                 10                      15

Pro Pro Gly Ala Ala Ser Thr Gln Val Cys Thr Gly Thr Asp Met Lys
          20                25                      30
```

Leu Arg Leu Pro Ala Ser Pro Glu Thr His Leu Asp Met Leu Arg His
        35              40                  45

Leu Tyr Gln Gly Cys Gln Val Val Gln Gly Asn Leu Glu Leu Thr Tyr
        50              55                  60

Leu Pro Thr Asn Ala Ser Leu Ser Phe Leu Gln Asp Ile Gln Glu Val
65              70              75                          80

Gln Gly Tyr Val Leu Ile Ala His Asn Gln Val Arg Gln Val Pro Leu
                85              90                  95

Gln Arg Leu Arg Ile Val Arg Gly Thr Gln Leu Phe Glu Asp Asn Tyr
            100             105             110

Ala Leu Ala Val Leu Asp Asn Gly Asp Pro Leu Asn Asn Thr Thr Pro
        115             120             125

Val Thr Gly Ala Ser Pro Gly Gly Leu Arg Glu Leu Gln Leu Arg Ser
        130             135             140

Leu Thr Glu Ile Leu Lys Gly Gly Val Leu Ile Gln Arg Asn Pro Gln
145             150             155             160

Leu Cys Tyr Gln Asp Thr Ile Leu Trp Lys Asp Ile Phe His Lys Asn
            165             170             175

Asn Gln Leu Ala Leu Thr Leu Ile Asp Thr Asn Arg Ser Arg Ala Cys
        180             185             190

His Pro Cys Ser Pro Met Cys Lys Gly Ser Arg Cys Trp Gly Glu Ser
        195             200             205

Ser Glu Asp Cys Gln Ser Leu Thr Arg Thr Val Cys Ala Gly Gly Cys
    210             215             220

Ala Arg Cys Lys Gly Pro Leu Pro Thr Asp Cys Cys His Glu Gln Cys
225             230             235             240

Ala Ala Gly Cys Thr Gly Pro Lys His Ser Asp Cys Leu Ala Cys Leu
            245             250             255

His Phe Asn His Ser Gly Ile Cys Glu Leu His Cys Pro Ala Leu Val
        260             265             270

Thr Tyr Asn Thr Asp Thr Phe Glu Ser Met Pro Asn Pro Glu Gly Arg

```
                    275                          280                          285

Tyr Thr Phe Gly Ala Ser Cys Val Thr Ala Cys Pro Tyr Asn Tyr Leu
    290                 295                 300

Ser Thr Asp Val Gly Ser Cys Thr Leu Val Cys Pro Leu His Asn Gln
305                 310                 315                 320

Glu Val Thr Ala Glu Asp Gly Thr Gln Arg Cys Glu Lys Cys Ser Lys
                325                 330                 335

Pro Cys Ala Arg Val Cys Tyr Gly Leu Gly Met Glu His Leu Arg Glu
                340                 345                 350

Val Arg Ala Val Thr Ser Ala Asn Ile Gln Glu Phe Ala Gly Cys Lys
                355                 360                 365

Lys Ile Phe Gly Ser Leu Ala Phe Leu Pro Glu Ser Phe Asp Gly Asp
    370                 375                 380

Pro Ala Ser Asn Thr Ala Pro Leu Gln Pro Glu Gln Leu Gln Val Phe
385                 390                 395                 400

Glu Thr Leu Glu Glu Ile Thr Gly Tyr Leu Tyr Ile Ser Ala Trp Pro
                405                 410                 415

Asp Ser Leu Pro Asp Leu Ser Val Phe Gln Asn Leu Gln Val Ile Arg
                420                 425                 430

Gly Arg Ile Leu His Asn Gly Ala Tyr Ser Leu Thr Leu Gln Gly Leu
                435                 440                 445

Gly Ile Ser Trp Leu Gly Leu Arg Ser Leu Arg Glu Leu Gly Ser Gly
    450                 455                 460

Leu Ala Leu Ile His His Asn Thr His Leu Cys Phe Val His Thr Val
465                 470                 475                 480

Pro Trp Asp Gln Leu Phe Arg Asn Pro His Gln Ala Leu Leu His Thr
                485                 490                 495

Ala Asn Arg Pro Glu Asp Glu Cys Val Gly Glu Gly Leu Ala Cys His
                500                 505                 510

Gln Leu Cys Ala Arg Gly His Cys Trp Gly Pro Gly Pro Thr Gln Cys
    515                 520                 525
```

Val Asn Cys Ser Gln Phe Leu Arg Gly Gln Glu Cys Val Glu Glu Cys
    530                 535                 540

Arg Val Leu Gln Gly Leu Pro Arg Glu Tyr Val Asn Ala Arg His Cys
545                 550                 555                 560

Leu Pro Cys His Pro Glu Cys Gln Pro Gln Asn Gly Ser Val Thr Cys
                565                 570                 575

Phe Gly Pro Glu Ala Asp Gln Cys Val Ala Cys Ala His Tyr Lys Asp
                580                 585                 590

Pro Pro Phe Cys Val Ala Arg Cys Pro Ser Gly Val Lys Pro Asp Leu
                595                 600                 605

Ser Tyr Met Pro Ile Trp Lys Phe Pro Asp Glu Glu Gly Ala Cys Gln
    610                 615                 620

Pro Cys Pro Ile Asn Cys Thr His Ser Cys Val Asp Leu Asp Asp Lys
625                 630                 635                 640

Gly Cys Pro Ala Glu Gln Arg Ala Ser Pro Leu Thr Ser Ile Ile Ser
                645                 650                 655

Ala Val Val Gly Ile Leu Leu Val Val Val Leu Gly Val Val Phe Gly
                660                 665                 670

Ile Leu Ile Lys Arg Arg Gln Gln Lys Ile Arg Lys Tyr Thr Met Arg
    675                 680                 685

Arg Leu Leu Gln Glu Thr Glu Leu Val Glu Pro Leu Thr Pro Ser Gly
    690                 695                 700

Ala Met Pro Asn Gln Ala Gln Met Arg Ile Leu Lys Glu Thr Glu Leu
705                 710                 715                 720

Arg Lys Val Lys Val Leu Gly Ser Gly Ala Phe Gly Thr Val Tyr Lys
                725                 730                 735

Gly Ile Trp Ile Pro Asp Gly Glu Asn Val Lys Ile Pro Val Ala Ile
            740                 745                 750

Lys Val Leu Arg Glu Asn Thr Ser Pro Lys Ala Asn Lys Glu Ile Leu
    755                 760                 765

Asp Glu Ala Tyr Val Met Ala Gly Val Gly Ser Pro Tyr Val Ser Arg
    770                 775                 780

```
Leu Leu Gly Ile Cys Leu Thr Ser Thr Val Gln Leu Val Thr Gln Leu
785             790             795             800

Met Pro Tyr Gly Cys Leu Leu Asp His Val Arg Glu Asn Arg Gly Arg
                805             810             815

Leu Gly Ser Gln Asp Leu Leu Asn Trp Cys Met Gln Ile Ala Lys Gly
            820             825             830

Met Ser Tyr Leu Glu Asp Val Arg Leu Val His Arg Asp Leu Ala Ala
        835             840             845

Arg Asn Val Leu Val Lys Ser Pro Asn His Val Lys Ile Thr Asp Phe
    850             855             860

Gly Leu Ala Arg Leu Leu Asp Ile Asp Glu Thr Glu Tyr His Ala Asp
865             870             875             880

Gly Gly Lys Val Pro Ile Lys Trp Met Ala Leu Glu Ser Ile Leu Arg
            885             890             895

Arg Arg Phe Thr His Gln Ser Asp Val Trp Ser Tyr Gly Val Thr Val
        900             905             910

Trp Glu Leu Met Thr Phe Gly Ala Lys Pro Tyr Asp Gly Ile Pro Ala
        915             920             925

Arg Glu Ile Pro Asp Leu Leu Glu Lys Gly Glu Arg Leu Pro Gln Pro
    930             935             940

Pro Ile Cys Thr Ile Asp Val Tyr Met Ile Met Val Lys Cys Trp Met
945             950             955             960

Ile Asp Ser Glu Cys Arg Pro Arg Phe Arg Glu Leu Val Ser Glu Phe
            965             970             975

Ser Arg Met Ala Arg Asp Pro Gln Arg Phe Val Val Ile Gln Asn Glu
        980             985             990

Asp Leu Gly Pro Ala Ser Pro Leu Asp Ser Thr Phe Tyr Arg Ser Leu
    995             1000            1005

Leu Glu Asp Asp Asp Met Gly Asp Leu Val Asp Ala Glu Glu Tyr
    1010            1015            1020

Leu Val Pro Gln Gln Gly Phe Phe Cys Pro Asp Pro Ala Pro Gly
    1025            1030            1035
```

Ala Gly Gly Met Val His His Arg His Arg Ser Ser Ser Thr Arg
    1040            1045            1050

Ser Gly Gly Gly Asp Leu Thr Leu Gly Leu Glu Pro Ser Glu Glu
    1055            1060            1065

Glu Ala Pro Arg Ser Pro Leu Ala Pro Ser Glu Gly Ala Gly Ser
    1070            1075            1080

Asp Val Phe Asp Gly Asp Leu Gly Met Gly Ala Ala Lys Gly Leu
    1085            1090            1095

Gln Ser Leu Pro Thr His Asp Pro Ser Pro Leu Gln Arg Tyr Ser
    1100            1105            1110

Glu Asp Pro Thr Val Pro Leu Pro Ser Glu Thr Asp Gly Tyr Val
    1115            1120            1125

Ala Pro Leu Thr Cys Ser Pro Gln Pro Glu Tyr Val Asn Gln Pro
    1130            1135            1140

Asp Val Arg Pro Gln Pro Pro Ser Pro Arg Glu Gly Pro Leu Pro
    1145            1150            1155

Ala Ala Arg Pro Ala Gly Ala Thr Leu Glu Arg Pro Lys Thr Leu
    1160            1165            1170

Ser Pro Gly Lys Asn Gly Val Val Lys Asp Val Phe Ala Phe Gly
    1175            1180            1185

Gly Ala Val Glu Asn Pro Glu Tyr Leu Thr Pro Gln Gly Gly Ala
    1190            1195            1200

Ala Pro Gln Pro His Pro Pro Pro Ala Phe Ser Pro Ala Phe Asp
    1205            1210            1215

Asn Leu Tyr Tyr Trp Asp Gln Asp Pro Pro Glu Arg Gly Ala Pro
    1220            1225            1230

Pro Ser Thr Phe Lys Gly Thr Pro Thr Ala Glu Asn Pro Glu Tyr
    1235            1240            1245

Leu Gly Leu Asp Val Pro Val
    1250            1255

<210>  91

190

<211> 645
<212> PRT
<213> Homo sapiens

<400> 91

Met Pro Ile Trp Lys Phe Pro Asp Glu Glu Gly Ala Cys Gln Pro Cys
1               5                   10                  15

Pro Ile Asn Cys Thr His Ser Cys Val Asp Leu Asp Asp Lys Gly Cys
            20                  25                  30

Pro Ala Glu Gln Arg Ala Ser Pro Leu Thr Ser Ile Ile Ser Ala Val
        35                  40                  45

Val Gly Ile Leu Leu Val Val Val Leu Gly Val Val Phe Gly Ile Leu
    50                  55                  60

Ile Lys Arg Arg Gln Gln Lys Ile Arg Lys Tyr Thr Met Arg Arg Leu
65              70                  75                  80

Leu Gln Glu Thr Glu Leu Val Glu Pro Leu Thr Pro Ser Gly Ala Met
            85                  90                  95

Pro Asn Gln Ala Gln Met Arg Ile Leu Lys Glu Thr Glu Leu Arg Lys
            100                 105                 110

Val Lys Val Leu Gly Ser Gly Ala Phe Gly Thr Val Tyr Lys Gly Ile
        115                 120                 125

Trp Ile Pro Asp Gly Glu Asn Val Lys Ile Pro Val Ala Ile Lys Val
    130                 135                 140

Leu Arg Glu Asn Thr Ser Pro Lys Ala Asn Lys Glu Ile Leu Asp Glu
145             150                 155                 160

Ala Tyr Val Met Ala Gly Val Gly Ser Pro Tyr Val Ser Arg Leu Leu
            165                 170                 175

Gly Ile Cys Leu Thr Ser Thr Val Gln Leu Val Thr Gln Leu Met Pro
        180                 185                 190

Tyr Gly Cys Leu Leu Asp His Val Arg Glu Asn Arg Gly Arg Leu Gly
        195                 200                 205

Ser Gln Asp Leu Leu Asn Trp Cys Met Gln Ile Ala Lys Gly Met Ser
    210                 215                 220

Tyr Leu Glu Asp Val Arg Leu Val His Arg Asp Leu Ala Ala Arg Asn

|  | 225 | | | | 230 | | | | 235 | | | | 240 |
|---|---|---|---|---|---|---|---|---|---|---|---|---|---|

Val Leu Val Lys Ser Pro Asn His Val Lys Ile Thr Asp Phe Gly Leu
                    245             250             255

Ala Arg Leu Leu Asp Ile Asp Glu Thr Glu Tyr His Ala Asp Gly Gly
                    260             265             270

Lys Val Pro Ile Lys Trp Met Ala Leu Glu Ser Ile Leu Arg Arg Arg
                    275             280             285

Phe Thr His Gln Ser Asp Val Trp Ser Tyr Gly Val Thr Val Trp Glu
        290             295             300

Leu Met Thr Phe Gly Ala Lys Pro Tyr Asp Gly Ile Pro Ala Arg Glu
305             310             315             320

Ile Pro Asp Leu Leu Glu Lys Gly Glu Arg Leu Pro Gln Pro Pro Ile
                    325             330             335

Cys Thr Ile Asp Val Tyr Met Ile Met Val Lys Cys Trp Met Ile Asp
            340             345             350

Ser Glu Cys Arg Pro Arg Phe Arg Glu Leu Val Ser Glu Phe Ser Arg
        355             360             365

Met Ala Arg Asp Pro Gln Arg Phe Val Val Ile Gln Asn Glu Asp Leu
370             375             380

Gly Pro Ala Ser Pro Leu Asp Ser Thr Phe Tyr Arg Ser Leu Leu Glu
385             390             395             400

Asp Asp Asp Met Gly Asp Leu Val Asp Ala Glu Glu Tyr Leu Val Pro
                    405             410             415

Gln Gln Gly Phe Phe Cys Pro Asp Pro Ala Pro Gly Ala Gly Gly Met
            420             425             430

Val His His Arg His Arg Ser Ser Ser Thr Arg Ser Gly Gly Gly Asp
        435             440             445

Leu Thr Leu Gly Leu Glu Pro Ser Glu Glu Glu Ala Pro Arg Ser Pro
    450             455             460

Leu Ala Pro Ser Glu Gly Ala Gly Ser Asp Val Phe Asp Gly Asp Leu
465             470             475             480

Gly Met Gly Ala Ala Lys Gly Leu Gln Ser Leu Pro Thr His Asp Pro
          485                 490               495

Ser Pro Leu Gln Arg Tyr Ser Glu Asp Pro Thr Val Pro Leu Pro Ser
          500                 505               510

Glu Thr Asp Gly Tyr Val Ala Pro Leu Thr Cys Ser Pro Gln Pro Glu
          515                 520               525

Tyr Val Asn Gln Pro Asp Val Arg Pro Gln Pro Pro Ser Pro Arg Glu
          530                 535               540

Gly Pro Leu Pro Ala Ala Arg Pro Ala Gly Ala Thr Leu Glu Arg Pro
545                 550               555               560

Lys Thr Leu Ser Pro Gly Lys Asn Gly Val Val Lys Asp Val Phe Ala
          565                 570               575

Phe Gly Gly Ala Val Glu Asn Pro Glu Tyr Leu Thr Pro Gln Gly Gly
          580                 585               590

Ala Ala Pro Gln Pro His Pro Pro Pro Ala Phe Ser Pro Ala Phe Asp
          595                 600               605

Asn Leu Tyr Tyr Trp Asp Gln Asp Pro Pro Glu Arg Gly Ala Pro Pro
          610                 615               620

Ser Thr Phe Lys Gly Thr Pro Thr Ala Glu Asn Pro Glu Tyr Leu Gly
625                 630               635               640

Leu Asp Val Pro Val
          645

```
<210>  92
<211>  5
<212>  PRT
<213>  Artificial Sequence

<220>
<223>  Peptide linker G4S

<400>  92

Gly Gly Gly Gly Ser
1               5


<210>  93
<211>  10
<212>  PRT
<213>  Artificial Sequence
```

```
<220>
<223>  Peptide linker (G4S)2

<400>  93

Gly Gly Gly Gly Ser Gly Gly Gly Gly Ser
1               5                   10


<210>  94
<211>  10
<212>  PRT
<213>  Artificial Sequence

<220>
<223>  Peptide linker (SG4)2

<400>  94

Ser Gly Gly Gly Gly Ser Gly Gly Gly Gly
1               5                   10


<210>  95
<211>  14
<212>  PRT
<213>  Artificial Sequence

<220>
<223>  Peptide linker G4(SG4)2

<400>  95

Gly Gly Gly Gly Ser Gly Gly Gly Gly Ser Gly Gly Gly Gly
1               5                   10


<210>  96
<211>  10
<212>  PRT
<213>  Artificial Sequence

<220>
<223>  Peptide linker

<400>  96

Gly Ser Pro Gly Ser Ser Ser Ser Gly Ser
1               5                   10


<210>  97
<211>  15
<212>  PRT
<213>  Artificial Sequence

<220>
<223>  Peptide linker (G4S)3

<400>  97

Gly Gly Gly Gly Ser Gly Gly Gly Gly Ser Gly Gly Gly Gly Ser
1               5                   10                      15
```

```
<210>  98
<211>  20
<212>  PRT
<213>  Artificial Sequence

<220>
<223>  Peptide linker (G4S)4

<400>  98

Gly Gly Gly Gly Ser Gly Gly Gly Gly Ser Gly Gly Gly Gly Ser Gly
1               5                   10                  15


Gly Gly Gly Ser
            20


<210>  99
<211>  8
<212>  PRT
<213>  Artificial Sequence

<220>
<223>  Peptide linker

<400>  99

Gly Ser Gly Ser Gly Ser Gly Ser
1               5


<210>  100
<211>  8
<212>  PRT
<213>  Artificial Sequence

<220>
<223>  Peptide linker

<400>  100

Gly Ser Gly Ser Gly Asn Gly Ser
1               5


<210>  101
<211>  8
<212>  PRT
<213>  Artificial Sequence

<220>
<223>  Peptide linker

<400>  101

Gly Gly Ser Gly Ser Gly Ser Gly
1               5


<210>  102
```

<211>   6
<212>   PRT
<213>   Artificial Sequence

<220>
<223>   Peptide linker

<400>   102

Gly Gly Ser Gly Ser Gly
1               5


<210>   103
<211>   4
<212>   PRT
<213>   Artificial Sequence

<220>
<223>   Peptide linker

<400>   103

Gly Gly Ser Gly
1


<210>   104
<211>   8
<212>   PRT
<213>   Artificial Sequence

<220>
<223>   Peptide linker

<400>   104

Gly Gly Ser Gly Asn Gly Ser Gly
1               5


<210>   105
<211>   8
<212>   PRT
<213>   Artificial Sequence

<220>
<223>   Peptide linker

<400>   105

Gly Gly Asn Gly Ser Gly Ser Gly
1               5


<210>   106
<211>   6
<212>   PRT
<213>   Artificial Sequence

<220>
<223>   Peptide linker

```
<400>  106

Gly Gly Asn Gly Ser Gly
1               5


<210>  107
<211>  290
<212>  PRT
<213>  Homo sapiens

<400>  107

Met Arg Ile Phe Ala Val Phe Ile Phe Met Thr Tyr Trp His Leu Leu
1               5                   10                  15


Asn Ala Phe Thr Val Thr Val Pro Lys Asp Leu Tyr Val Val Glu Tyr
            20                  25                  30


Gly Ser Asn Met Thr Ile Glu Cys Lys Phe Pro Val Glu Lys Gln Leu
        35                  40                  45


Asp Leu Ala Ala Leu Ile Val Tyr Trp Glu Met Glu Asp Lys Asn Ile
        50                  55                  60


Ile Gln Phe Val His Gly Glu Glu Asp Leu Lys Val Gln His Ser Ser
65                  70                  75                  80


Tyr Arg Gln Arg Ala Arg Leu Leu Lys Asp Gln Leu Ser Leu Gly Asn
                85                  90                  95


Ala Ala Leu Gln Ile Thr Asp Val Lys Leu Gln Asp Ala Gly Val Tyr
            100                 105                 110


Arg Cys Met Ile Ser Tyr Gly Gly Ala Asp Tyr Lys Arg Ile Thr Val
            115                 120                 125


Lys Val Asn Ala Pro Tyr Asn Lys Ile Asn Gln Arg Ile Leu Val Val
            130                 135                 140


Asp Pro Val Thr Ser Glu His Glu Leu Thr Cys Gln Ala Glu Gly Tyr
145                 150                 155                 160


Pro Lys Ala Glu Val Ile Trp Thr Ser Ser Asp His Gln Val Leu Ser
                165                 170                 175


Gly Lys Thr Thr Thr Thr Asn Ser Lys Arg Glu Glu Lys Leu Phe Asn
                180                 185                 190


Val Thr Ser Thr Leu Arg Ile Asn Thr Thr Thr Asn Glu Ile Phe Tyr
            195                 200                 205
```

```
Cys Thr Phe Arg Arg Leu Asp Pro Glu Glu Asn His Thr Ala Glu Leu
    210                 215             220

Val Ile Pro Glu Leu Pro Leu Ala His Pro Pro Asn Glu Arg Thr His
    225             230             235                 240

Leu Val Ile Leu Gly Ala Ile Leu Leu Cys Leu Gly Val Ala Leu Thr
                245             250                 255

Phe Ile Phe Arg Leu Arg Lys Gly Arg Met Met Asp Val Lys Lys Cys
            260             265             270

Gly Ile Gln Asp Thr Asn Ser Lys Lys Gln Ser Asp Thr His Leu Glu
            275             280             285

Glu Thr
    290


<210>  108
<211>  288
<212>  PRT
<213>  Homo sapiens

<400>  108

Met Gln Ile Pro Gln Ala Pro Trp Pro Val Val Trp Ala Val Leu Gln
1               5               10                  15

Leu Gly Trp Arg Pro Gly Trp Phe Leu Asp Ser Pro Asp Arg Pro Trp
            20              25              30

Asn Pro Pro Thr Phe Ser Pro Ala Leu Leu Val Val Thr Glu Gly Asp
        35              40              45

Asn Ala Thr Phe Thr Cys Ser Phe Ser Asn Thr Ser Glu Ser Phe Val
    50              55              60

Leu Asn Trp Tyr Arg Met Ser Pro Ser Asn Gln Thr Asp Lys Leu Ala
65              70              75                  80

Ala Phe Pro Glu Asp Arg Ser Gln Pro Gly Gln Asp Cys Arg Phe Arg
            85              90              95

Val Thr Gln Leu Pro Asn Gly Arg Asp Phe His Met Ser Val Val Arg
            100             105             110

Ala Arg Arg Asn Asp Ser Gly Thr Tyr Leu Cys Gly Ala Ile Ser Leu
        115             120             125
```

```
Ala Pro Lys Ala Gln Ile Lys Glu Ser Leu Arg Ala Glu Leu Arg Val
    130             135             140

Thr Glu Arg Arg Ala Glu Val Pro Thr Ala His Pro Ser Pro Ser Pro
145             150             155             160

Arg Pro Ala Gly Gln Phe Gln Thr Leu Val Val Gly Val Val Gly Gly
            165             170             175

Leu Leu Gly Ser Leu Val Leu Leu Val Trp Val Leu Ala Val Ile Cys
            180             185             190

Ser Arg Ala Ala Arg Gly Thr Ile Gly Ala Arg Arg Thr Gly Gln Pro
        195             200             205

Leu Lys Glu Asp Pro Ser Ala Val Pro Val Phe Ser Val Asp Tyr Gly
    210             215             220

Glu Leu Asp Phe Gln Trp Arg Glu Lys Thr Pro Glu Pro Pro Val Pro
225             230             235             240

Cys Val Pro Glu Gln Thr Glu Tyr Ala Thr Ile Val Phe Pro Ser Gly
            245             250             255

Met Gly Thr Ser Ser Pro Ala Arg Arg Gly Ser Ala Asp Gly Pro Arg
            260             265             270

Ser Ala Gln Pro Leu Arg Pro Glu Asp Gly His Cys Ser Trp Pro Leu
        275             280             285
```

```
<210>  109
<211>  118
<212>  PRT
<213>  Artificial Sequence

<220>
<223>  VH (PD-L1)

<400>  109
```

```
Glu Val Gln Leu Val Glu Ser Gly Gly Gly Leu Val Gln Pro Gly Gly
1               5               10              15

Ser Leu Arg Leu Ser Cys Ala Ala Ser Gly Phe Thr Phe Ser Asp Ser
            20              25              30

Trp Ile His Trp Val Arg Gln Ala Pro Gly Lys Gly Leu Glu Trp Val
            35              40              45
```

```
Ala Trp Ile Ser Pro Tyr Gly Gly Ser Thr Tyr Tyr Ala Asp Ser Val
    50                  55              60

Lys Gly Arg Phe Thr Ile Ser Ala Asp Thr Ser Lys Asn Thr Ala Tyr
65                  70              75                  80

Leu Gln Met Asn Ser Leu Arg Ala Glu Asp Thr Ala Val Tyr Tyr Cys
                85                  90                  95

Ala Arg Arg His Trp Pro Gly Gly Phe Asp Tyr Trp Gly Gln Gly Thr
            100                 105                 110

Leu Val Thr Val Ser Ser
            115
```

```
<210>  110
<211>  107
<212>  PRT
<213>  Artificial Sequence

<220>
<223>  VL (PD-L1)

<400>  110
```

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Ser Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Leu Tyr His Pro Ala
                85                  90                  95

Thr Phe Gly Gln Gly Thr Lys Val Glu Ile Lys
            100                 105
```

```
<210>  111
<211>  121
<212>  PRT
<213>  Artificial Sequence
```

200

<220>
<223>  VH (PD-L1) 2

<400>   111

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Arg Tyr
            20                  25                  30

Trp Met Ser Trp Val Arg Gln Ala Pro Gly Lys Gly Leu Glu Trp Val
        35                  40                  45

Ala Asn Ile Lys Gln Asp Gly Ser Glu Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Gly Trp Phe Gly Glu Leu Ala Phe Asp Tyr Trp Gly
            100                 105                 110

Gln Gly Thr Leu Val Thr Val Ser Ser
        115                 120

<210>   112
<211>   108
<212>   PRT
<213>   Artificial Sequence

<220>
<223>  VL (PD-L1) 2

<400>   112

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Arg Val Ser Ser Ser
            20                  25                  30

Tyr Leu Ala Trp Tyr Gln Gln Lys Pro Gly Gln Ala Pro Arg Leu Leu
        35                  40                  45

Ile Tyr Asp Ala Ser Ser Arg Ala Thr Gly Ile Pro Asp Arg Phe Ser
    50                  55                  60

```
Gly Ser Gly Ser Gly Thr Asp Phe Thr Leu Thr Ile Ser Arg Leu Glu
65                  70                  75                  80


Pro Glu Asp Phe Ala Val Tyr Tyr Cys Gln Gln Tyr Gly Ser Leu Pro
                85                  90                  95


Trp Thr Phe Gly Gln Gly Thr Lys Val Glu Ile Lys
            100                 105


<210>   113
<211>   120
<212>   PRT
<213>   Artificial Sequence

<220>
<223>   VH (PD-1)

<400>   113

Gln Val Gln Leu Val Gln Ser Gly Val Glu Val Lys Lys Pro Gly Ala
1                   5                   10                  15


Ser Val Lys Val Ser Cys Lys Ala Ser Gly Tyr Thr Phe Thr Asn Tyr
                20                  25                  30


Tyr Met Tyr Trp Val Arg Gln Ala Pro Gly Gln Gly Leu Glu Trp Met
            35                  40                  45


Gly Gly Ile Asn Pro Ser Asn Gly Gly Thr Asn Phe Asn Glu Lys Phe
        50                  55                  60


Lys Asn Arg Val Thr Leu Thr Thr Asp Ser Ser Thr Thr Thr Ala Tyr
65                  70                  75                  80


Met Glu Leu Lys Ser Leu Gln Phe Asp Asp Thr Ala Val Tyr Tyr Cys
                85                  90                  95


Ala Arg Arg Asp Tyr Arg Phe Asp Met Gly Phe Asp Tyr Trp Gly Gln
            100                 105                 110


Gly Thr Thr Val Thr Val Ser Ser
        115                 120


<210>   114
<211>   111
<212>   PRT
<213>   Artificial Sequence

<220>
<223>   VL (PD-1)
```

<400> 114

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Lys Gly Val Ser Thr Ser
            20                  25                  30

Gly Tyr Ser Tyr Leu His Trp Tyr Gln Gln Lys Pro Gly Gln Ala Pro
        35                  40                  45

Arg Leu Leu Ile Tyr Leu Ala Ser Tyr Leu Glu Ser Gly Val Pro Ala
    50                  55                  60

Arg Phe Ser Gly Ser Gly Ser Gly Thr Asp Phe Thr Leu Thr Ile Ser
65              70                  75                  80

Ser Leu Glu Pro Glu Asp Phe Ala Val Tyr Tyr Cys Gln His Ser Arg
                85                  90                  95

Asp Leu Pro Leu Thr Phe Gly Gly Gly Thr Lys Val Glu Ile Lys
            100                 105                 110

<210> 115
<211> 113
<212> PRT
<213> Artificial Sequence

<220>
<223> VH (PD-1) 2

<400> 115

Gln Val Gln Leu Val Glu Ser Gly Gly Gly Val Val Gln Pro Gly Arg
1               5                   10                  15

Ser Leu Arg Leu Asp Cys Lys Ala Ser Gly Ile Thr Phe Ser Asn Ser
            20                  25                  30

Gly Met His Trp Val Arg Gln Ala Pro Gly Lys Gly Leu Glu Trp Val
        35                  40                  45

Ala Val Ile Trp Tyr Asp Gly Ser Lys Arg Tyr Tyr Ala Asp Ser Val
        50                  55                  60

Lys Gly Arg Phe Thr Ile Ser Arg Asp Asn Ser Lys Asn Thr Leu Phe
65              70                  75                  80

Leu Gln Met Asn Ser Leu Arg Ala Glu Asp Thr Ala Val Tyr Tyr Cys
                85                  90                  95

```
Ala Thr Asn Asp Asp Tyr Trp Gly Gln Gly Thr Leu Val Thr Val Ser
            100                 105                 110

Ser
```

```
<210>  116
<211>  107
<212>  PRT
<213>  Artificial Sequence

<220>
<223>  VL (PD-1) 2

<400>  116
```

```
Glu Ile Val Leu Thr Gln Ser Pro Ala Thr Leu Ser Leu Ser Pro Gly
1               5               10              15

Glu Arg Ala Thr Leu Ser Cys Arg Ala Ser Gln Ser Val Ser Ser Tyr
            20              25              30

Leu Ala Trp Tyr Gln Gln Lys Pro Gly Gln Ala Pro Arg Leu Leu Ile
            35              40              45

Tyr Asp Ala Ser Asn Arg Ala Thr Gly Ile Pro Ala Arg Phe Ser Gly
            50              55              60

Ser Gly Ser Gly Thr Asp Phe Thr Leu Thr Ile Ser Ser Leu Glu Pro
65                  70              75                  80

Glu Asp Phe Ala Val Tyr Tyr Cys Gln Gln Ser Ser Asn Trp Pro Arg
                85              90                  95

Thr Phe Gly Gln Gly Thr Lys Val Glu Ile Lys
            100             105
```